# EUROPEAN PATENT APPLICATION

(11) **EP 1 686 135 A1**
(43) Date of publication of application: **02.08.2006**
(21) Application number: 05021917.9
(22) Date of filing: 23.02.2001
(51) Int. Cl.: C07K 14/72, C12N 15/12, C12N 15/861, C12N 15/863, C12N 15/864, C12N 15/867, C12N 15/869, C12N 5/10, C07K 16/28, G01N 33/53, C12Q 1/68

(54) **G protein-coupled receptors**

(30) Priority: 23.02.2000 US 184305 P; 23.02.2000 US 184304 P; 23.02.2000 US 184303 P; 23.02.2000 US 184397 P; 23.02.2000 US 184247 P; 02.03.2000 US 186457 P; 03.03.2000 US 186810 P; 09.03.2000 US 188064 P; 13.03.2000 US 188880 P; 03.04.2000 US 194344 P; 23.06.2000 US 213861 P; 11.07.2000 US 217370 P; 11.07.2000 US 217369 P; 14.07.2000 US 218337 P; 20.07.2000 US 219492 P
(62) Divisional of application: 01912924.6
(71) Applicant: Pharmacia & Upjohn Company LLC, Kalamazoo, MI 49001 (US)
(72) Inventor: Vogeli, Gabriel, Encinitas, CA 92024 (US); Wood, Linda S., Michigan 49024 (US); Parodi, Luis A., 11543 Stockholm (SE); Lind, Peter, 75129 Uppsala (SE)
(74) Representative: Perry, Robert Edward

(57) **Abstract**

The present invention provides a gene encoding a G protein-coupled receptor termed nGPCR-x; constructs and recombinant host cells incorporating the genes; the nGPCR-x polypeptides encoded by the gene; antibodies to the nGPCR-x polypeptides; and methods of making and using all of the foregoing.

SEQUENCE LISTING

<110> Pharmacia & Upjohn Company
      Vogeli, Gabriel
      Parodi, Luis A.
      Hiebsch, Ronald R.
      Lind, Peter
      Kaytes, Paul S.
      Ruff, Valerie
      Huff, Rita M.
      Wood, Linda S.

<120>  Novel G Protein-Coupled Receptors Cross-Reference To Related Applications

<130>  00325.PCT1

<150>  60/184,305
<151>  2000-02-23

<150>  60/184,304
<151>  2000-02-23

<150>  60/184,303
<151>  2000-02-23

<150>  60/184,397
<151>  2000-02-23

<150>  60/184,247
<151>  2000-02-23

<150>  60/188,880
<151>  2000-03-13

<150>  60/217,369
<151>  2000-07-11

<150>  60/217,370
<151>  2000-07-11

<150>  60/218,492
<151>  2000-07-20

<150>  60/186,810
<151>  2000-03-03

<150>  60/188,064
<151>  2000-03-09

<150>  60/186,457
<151>  2000-03-02

<150>  60/213,861
<151>  2000-06-23

<150>  60/194,344
<151>  2000-04-03

<150>  60/218,337

<151>  2000-07-14

<160>  184

<170>  PatentIn version 3.0

<210>  1
<211>  545
<212>  DNA
<213>  Homo sapiens

<400>  1
cctatgctac aatcggcaga tgggaactag gagccatgat ctctcagatt gcaggtctca      60

ttggaaccac atttattgga ttttcctttt tagtagtact aacatcatac tactcttttg     120

taagccatct gagaaaaata agaacctgta cgtccattat ggagaaagat ttgacttaca     180

gttctgtgaa aagacatctt ttggtcatcc agattctact aatagtttgc ttccttcctt     240

atagtatttt taaacccatt ttttatgttc tacaccaaag agataactgt cagcaattga     300

attatttaat agaaacaaaa aacattctca cctgtcttgc ttcggccaga agtagcacag     360

accccattat atttctttta ttagacaaaa cattcaagaa gacactatat aatctcttta     420

caaagtctaa ttcagcacat atgcaatcat atggttgact tttgaatgga aaaccccaca     480

atattaagaa aagcattcat gtgactttat tagggacact aaactacatc attaacatgt     540

cacag                                                                 545

<210>  2
<211>  1611
<212>  DNA
<213>  Homo sapiens

<400>  2
cagtgagccg agatggtgcc attgcactct agcctggggc aacagagccg actccatctc      60

caaaaaaaaa aggccattct gaggatcaag gcaccactag caacagggag ccccatgggt     120

ctcagaccct ctccccacat ctcctggtcc ctgcccccac ctggcgtaca gggaccagcc     180

ccacggaagg ctcttgaggc caggtaacca tggggagggg aggaatgggg acaccttcct     240

cctgagtgtc ttagggaaga gaagcttagg tcaggtggct gagggtggaa atgagagagg     300

ggtctcctcc tggagggtct caccattccc ttggtcaccc acccaactct catctcccct     360

gatgtgggga ggagcagggg gcatggattc ctgagcccca gactcaactg ttgtggttta     420

caggggcatc aggagagaga gcgagcagaa cacactcctg cagcatcccc tggccccccg     480

ccccatgatg gagcccagag aagctggaca gcacgtgggg gccgccaacg gcgcccagga     540

ggatgtggcc ttcaacctca tcatcctgtc cctcaccgag gggctcggcc tcggtgggct     600

gctggggaat ggggcagtcc tctggctgct cagctccaat gtctacagaa accccttcgc 660
catctacctc ctggacgtgg cctgcgcgga tctcatcttc cttggctgcc acatggtggc 720
catcgtcccc gacttgctgc aaggccggct ggacttcccg ggcttcgtgc agaccagcct 780
ggcaacgctg cgcttcttct gctacatcgt gggcctgagt ctcctggcgg ccgtcagcgt 840
ggagcagtgc ctggccgccc tcttcccagc ctggtactcg tgccgccgcc cacgccacct 900
gaccacctgt gtgtgcgccc tcacctgggc cctctgcctg ctgctgcacc tgctgctcag 960
cggcgcctgc acccagttct tcggggagcc cagccgccac ttgtgccgga cgctgtggct 1020
ggtggcagcg gtgctgctgg ctctgctgtg ttgcaccatg tgtggggcca gccttatgct 1080
gctgctgcgg gtggagcgag gcccccagcg gcccccaccc cggggcttcc ctgggctcat 1140
cctcctcacc gtcctcctct tcctcttctg cggcctgccc ttcggcatct actggctgtc 1200
ccggaacctg ctctggtaca tcccccacta cttctaccac ttcagcttcc tcatggccgc 1260
cgtgcactgc gcggccaagc ccgtcgtcta cttctgcctg ggcagtgccc agggccgcag 1320
gctgcccctc cggctggtcc tccagcgagc gctgggagac gaggctgagc tgggggccgt 1380
cagggagacc tcccgccggg gcctggtgga catagcagcc tgagccctgg ggcccccgac 1440
cccagctgca gcccccgtga ggcaagaggg tgacgtgggg aaggtggtgg ggtcagaggc 1500
tggggccagc cggacctgga ggaggccttg gtgggtgacc cggtcatgtg ctgtcaaagt 1560
tgtgaccctt ggtctggagc atgaggctcc cctgggaggc agctggaaag g 1611

<210> 3
<211> 930
<212> DNA
<213> Homo sapiens

<400> 3
aaaattgctc ttcctcctga gcttgtacac aatgattgag ttcaagatga agaagatgga 60
gcagggcacc aggtagacgg tgaagcagtg gatccagatg aggacgtgat gcacagaggt 120
gctgatgtag tcttcagtcc agatgttggg ccaccagtaa taggggatgc tggtcaggaa 180
gcaggtgatg taaacactta caatgacttt ccgggtgcgg gctgggtatg agaccgtgtg 240
gtacttgagc gggtggcaga cagcgatata cctgtcaatg gttaacggta cagtaatcca 300
tatggaggtg tggatggatg agaattccag cacttctatg atcttgtcgg ggacctgagg 360
catctgcatg ttcaagatga aatcttccaa caggaagtcc acaaacacta tgaaaaagag 420
gaccaagatg tcggcagcag cgagtgccaa gagatagttg taggaggact tctgtcttct 480
tgccaccagc tgggagagga tgatcactgt caagatattt gctgtggaga gaagaaaaac 540

tggtttagct ctgaagcaaa gatgacttcg ttggctccta tgggggccct aggcatatgt 600

ttattttgca ctcccatgga agtgaaaatg attgaatcaa tgcttttgag ggacaacctc 660

agcattacaa atagcacctc atacaattag tggatactat tttaaagtta tgcttatatt 720

ctaacacaac catgagaggt ggtgcctcca ttctcctcat cttagaagtg aaactggggc 780

tctgagagcc tcacacagcc atgagaggtg gtgccgccat tctcctcaca caaccatgag 840

tggtgttgcc gccattctcc tcacacaaca atgagaggtg gtgccaccat tcttctcaca 900

caaccgtgag aggcgatgct gccattatcc 930

<210> 4
<211> 533
<212> DNA
<213> Homo sapiens

<400> 4
ggcccgctcc cacgctgtgt agtgtacttt cattttcaat aaatcacttc attccttcct 60

tgctttgttt gtgcgttttg tccaattctt tgttcaagac gccaagaacc tggacagcct 120

ccaccattaa gaatacaaga gcagtttctg tcacatgtac atatgggggt gggtggatct 180

cgctcagcct ttccaggaca cagcggtttg atggaatgct tttctgaaac tcgtggcaga 240

atgagtacgg aagagggctg atagccgaat tcagccagtg gagccagaag cgtgtctcat 300

agagaaagtg ctggacatgt gacccgtggc aggcagctcc gatgatcacg agcagtgtag 360

ggagcccagc acaggacaaa tgcacacacc atgacgtcaa gcgacgtgga cgctcatttg 420

tcccgggaga cgccgtgctg ggaggctcgg gctgtctgct gccattgcag gcgttctcgc 480

tgccctgtag cgggtcttct gcgtaggatt atcaagtcca cctgcagagg tga 533

<210> 5
<211> 589
<212> DNA
<213> Homo sapiens

<400> 5
   tacttttacc caaaactatt atgttcatta ttagagtttc ctagaaaaat acctaaggag 60
   ttaatggttt catatctttt gctctattat gaagaagaaa aatgttttac aaatattttc 120
   attattggag cattttttgt tgttagtgaa attatcaaaa ctaggattga tttctattct 180
   gtttactttt gttataatct ttatcctttt ctcttaattt ctgtattttg gatgcctaac 240
   ttagaataca ttaccaaagt taccttttca tttagtctct caatacaaga tgatttaaaa 300
   catttatggt tacctttttt aatttttttg ctatgcaaat ttataaaagg gcaaagtctt 360
   tgtgctctaa taatacctgc tttctcatgt tttacatgtt ctacgattta ttttgttttt 420
   ataatgtaat tttcgtttac ctaattgtgc acatagtgaa taatagatta taatgaagaa 480
   aacttggatt aaaatctatt gttaaaaagg tttttcaggc aataataaat cattggattt 540
   ttctgatgta ttttaaaaag atatgtttat ttttgagcaa ctcgtgtgc 589

<210> 6
<211> 864
<212> DNA
<213> Homo sapiens

<400> 6
ggctacttgt tatggaaaag ttatgattgg tgtagaactg tacaggttgc ttctggcact 60

tgaagaaagc cttgagcgtg gcggctgcca tggtgcggaa ccgcttgctg atgaagcagt 120

agaggaagaa gttgatggct gtgttcagaa gggctagcat gttggcaatg tcggacatga 180

tgtgtaccag ccagcggttc tggatgggcg ccccatagag gtggtaaaga atcatgatga 240

tgcgggggc ccaaagtgtg gcaaagatgg aggtaatggt gaacaagatg gcggtggtct 300

tccccgtgga gtagccacgg agacgaaaat tgctcttcct cctgagcttg tacacaatga 360

ttgagttcaa gatgaagaac atggagcagg gcaccaagta gacggtgaag cagtggatcc 420

agatgaggac gtgatgcaca gaggtgctga tgtaatcttc agtccagatg ttgggccacc 480

agtaataggg gatgctggtc aggaagcagg tgatgtaaac acttacaatg actttccggg 540

tgcgggctgg gtatgagacc gtgtggtact tgagcgggtg gcagacagcg atatacctgt 600

caatggttaa cggtacagta atccatatgg aggtgtggat ggatgagaat tccagcactt 660

ctatgatctt gtcggggacc tgaggcatct gcatgttcaa gatgaaatct tccaacagga 720

agtccacaaa cactatgaaa aagaggacca agatgtcggc agcagcgagt gccaagagat 780

agttgtagga ggacttctgt cttcttgcca ccagctggga gaggatgatc actgtcaaga 840

tatttgctgt ggagagaaga aaac 864

<210> 7
<211> 823
<212> DNA
<213> Homo sapiens

<400> 7
   gacacctgcc aacatgttca ttatcaacct cgcggtcagc gacttcctca tgtccttcac 60
   ccaggcccct gtcttcttca ccagtagcct ctataagcag tggctctttg gggagacagg 120
   tagatgctgg ggctcccttt tgctggaggg aggaggaggg ttttgacctg gggatgccct 180
   caatggaggg tggcccaaag gaggtgattt gctgcttctg ggcagagagt gggtagctgc 240
   cctcagtcct gtgagtaagc aagaagggaa gatgcagtgt tggtcctaag gcctctgcca 300
   gccttggcca gatgtggcag gtggaggggg tggagtgcgc tcagtcctgc tcttcctgtg 360
   aggtgaaggc cagagcagag tctaccctgt ccccagaccc tcctccccag gactcagagc 420
   aggggctgtg cccacaggct gcgagttcta tgccttctgt ggagctctct ttggcatttc 480
   ctccatgatc accctgacgg ccatcgccct ggaccgctac ctggtaatca cacgcccgct 540
   ggccaccttt ggtgtggcgt ccaagaggcg tgcggcattt gtcctgctgg gcgtttggct 600
   ctatgccctg gcctggagtc tgccaccctt cttcggctgg agtaagtggg ctgctggaac 660
   tggaaggggg gcagatgggc tgggaggggc acattcaagg gtaagtaggt gacttgggtc 720
   agccagctgg cgggagcagg gtgcccagga gctacctgag cctcaggtga gatggacatt 780
   cagggggaca tgactggcag caagggaaac tgacactgcc cca 823

<210> 8
<211> 619
<212> DNA
<213> Homo sapiens

<400> 8
ttctcccctt gacgggtgac taactctgcc tgcgtgtttc ttttgtcacc agcataggca 60

ctgagtgcgg tctgtgcacc cctttgccac ccaccggtgc cggcactgag cctgcaacct 120

gtctcacgcc ctctggctgt tgccatgacg tccacctgca ccaacagcac gcgcgagagt 180

aacagcagcc acacgtgcat gcccctctcc aaaatgccca tcagcctggc ccacggcatc 240

atccgctcaa ccgtgctggt tatcttcctc gccgcctctt tcgtcggcaa catagtgctg 300

gcgctagtgt tgcagcgcaa gccgcagctg ctgcaggtga ccaaccgttt tatctttaac 360

ctcctcgtca ccgacctgct gcagatttcg ctcgtggccc cctgggtggt ggccacctct 420

gtgcctctct tctggcccct caacagccac ttctgcacgg ccctggttag cctcacccac 480

ctgttcgcct tcgccagcgt caacaccatt gtcgtggtgt cagtggatcg ctacttgtcc 540

atcatccacc ctctcttcta cccgtccaag atgacccagc gccgcgggta cctgctcctc 600

tatggcacct ggattgtgg 619

<210> 9
<211> 550
<212> DNA
<213> Homo sapiens

<400> 9
aggacaacga tggtcactga tttggtgacc ttcgacagtc tccgggcgct ggctccggtc 60

gggcgtcctc cggctaccgc ggcccctcct ttggtccccg ccgcgcggcg gtcggcgatg 120

aagcgcacca gcagcaggta gcacaagata atgatgccca gcggcagcac gaagcccagc 180

agcaccttct gcgagtggta gaggcccagc cagaactgcc tgtcgcggcc cagcaacttg 240

tccgggaaac gcaccaggca cagctcctcg cccatcacct tgaccgtggt ggagaaaatg 300

gcactgggca gcgaggccag cgcggccaaa gcccagatcc acacacacag cgccttggcc 360

gagaagcagc agctgtcccc caggctccgg ccgcagcagt cgccccggcc gtgtcctcgg 420

gtcccggtgg ctcttcagag ccgaggccac cgaatggtag cgcgtcacac tcatggcagt 480

gaggaagaac acgctggcgt acatgttcat gggacgtcac catggacacg atcttacaca 540

tggccttgcc 550

<210> 10
<211> 1104
<212> DNA
<213> Homo sapiens

<400> 10
ctggaaaggg tgctggtggc gcgccgcgac gccgcggcgc gactgcctgc ctggtactca 60

acctcttctg cgcggacctg ctcttcatca gcgctatccc tctggtgctg gccgtgcgct 120

ggactgaggc ctggctgctg ggccccgttg cctgccacct gctcttctac gtgatgaccc 180

tgagcggcag cgtcaccatc ctcacgctgg ccgcggtcag cctggagcgc atggtgtgca 240

tcgtgcacct gcagcgcggc gtgcggggtc ctgggcggcg ggcgcgggca gtgctgctgg 300

cgctcatctg gggctattcg gcggtcgccg ctctgcctct ctgcgtcttc ttccgagtcg 360

tcccgcaacg gctccccggc gccgaccagg tgagcgcccc tctgtgtgtg ccgggcaggt 420

gtcctgcgca ggctgggaag cggggccccg acggaagctg ggatgaggat gatcaagaac 480

aacaatagcc atttattgca cttaatcgtt gtgccaaatc ttgtgcccat ggctgtgaag 540

tttaatctct taaatctcac tacaacgctg tgcacacgcc ctcctaaatg atgtaagtgg 600

agtcccccaa attcttgcaa aatgcaatga ctgttgcgag gttaattaac gagtagttta 660

ggagcgagac ggaactttgg gggtgcaggg tggccaaaca ctttgtattg aatcatgatt 720

cctcgccagg tgctacaata ctgttattat cacacccatt tcacagatga gaaccagagg 780

cacaccgaag tgtataataa cttgcccaga gtattttatc cgtaattcga ggaggagatg 840

ggctccttcc agaagtttac ccgtaattca aggaggagtt gggctcctgt ccagggttgg 900

gttatggtcc tgctttgaaa gcgcgcggac aggcatgtga gacccgggga ccccagatgc 960

aatgctgtct ttaggggact tgtgacagaa ttcccttccg ggtcttcag tttttcagc 1020

tgcaaaacgg aaggattaca ctagaccttc gaggtgtcct gggcgcctga aatgtgcaga 1080

ttacagaggc tggaccgacg agct 1104

<210> 11
<211> 591
<212> DNA
<213> Homo sapiens

<400> 11
ttaatccctg gaagtccacg aacaatgaat ccatttcatg catcttgttg gaacacctct 60

gccgaacttt taaacaaatc ctggaataaa gagtttgctt atcaaactgc cagtgtggta 120

gatacagtca tcctcccttc catgattggg attatctgtt caacagggct ggttggcaac 180

atcctcattg tattcactat aataaggtaa ggaatggctc cttttttttt tttttccttc 240

catactttag gaaactacag tcaaagctcc ctaaatgagt cctttcccct gtagcatttt 300

gcttaatgaa atgcaatttt ggaaatattt gcttaagata attaatgaag attctacaga 360

tattttcgtc atgcattagg taacatctca gttgcaaatc tcaacatgct aagacctagg 420

ccaatgctta ctgctgggtc agtgagtttt tagggaaatg actctcactc tcagtcttag 480

ctgcatatta gaatcatctg gggagcttta aaaactcctg atatgcagtt tcaccccaga 540

cccattaact cagaatctct aagggtaggg cccgggtaag atttaaaact g 591

<210> 12
<211> 378
<212> DNA
<213> Homo sapiens

<400> 12
gggtgacagg aactctggaa ggtgatagtt ttccaggtga agaagggaca gtaggtttcc 60

ctgctttact gctatctccc tatggccttc ggtcaggcct gaactgtgat gagagtctct 120

gcccctgctc tttgggattc ctcaaatatc ctcccttcat tgttccctga attatctaga 180

tcaatacaaa ccaatccctc ctctgcttaa aacttttaat tgcttacagg ggaaagtgca 240

gactccttag ctggcgcaca agccttttgc acctggcaca catagagctt tctagggcta 300

tctcctgcca ttctctagcc tgtctccttc tgctctgggt ctccagaact gcccatgaat 360

tccctcacat gcctttct 378

<210> 13
<211> 503
<212> DNA
<213> Homo sapiens

<400> 13
cctatgcttt cctatagctc atggaccaag ccatttctcc aacatcataa tttttcctta 60

tcatcccttt ccttgcatta ttctccagat gaaaaaagtt caaatgcctc ctccctattt 120

tcagctgaga aggtgagatg cccgtgcctg gatttccagg ctcctgcctg cttggcagac 180

cactgtgacc gtgcacgact aagtccactc ttcccagcca gcatcccctg ctctccccag 240

gccaacttct ttgtgttcta ttcattccta tctccttgcc ggaactactc agacccctct 300

gtctttctgc ctcttgccca atcttccagg ctctagtttg ctggctttct gcctccaaaa 360

atgattttaa gcatttgtga gtctttctct ccacagaact ccagactctt tgaaaatgcc 420

gtagttcgta aattacttcc aatatttaag aagtgcttat catgcttccc tgcgggttca 480

tgctctttgg tattgatttt cct 503

<210> 14
<211> 671
<212> DNA
<213> Homo sapiens

<400> 14
caaatgaggt ggatgcactt agaaggaagg gtagaacaaa tattcgttga tgatatacaa 60

tgtgtcagat acagagatac acagactagg gctgtgggaa tctcagattt taatttgtca 120

acagtgtgtt ttgatttttt tgttttgtat atttgcctcc ccaattaatt tcacaggctt 180

aatcatcttt acaagacatt attttaaaga gaaagcgaac ttactgaagt tttatgcttc 240

cctgattgtg atgagctggt tgattctagc tctagtttca atgttctgga aaatactgaa 300

gtacttccac ctggtggcac ttagtgaaca ttgcagaacc gagtaatagg ttattccgtt 360

gggtttctcg aacaaatctg agttatagct aggaactctc aactaacaat ttatgagaac 420

cttctgctac acatgtgaat ttacatattt attcctttgc agttgaagga tgggatgtat 480

gcaaaggaga atgaactcta ctacagtaat ggaaagaagt gatagaagat gaaactccaa 540

aatgcctatc tgctacttga gtggaactca ctagaatata tcaaatgcag agagaaacat 600

gtgactaggc tctagtcaaa caattgtcat caaatacttg ctgaatatat aacacatttt 660

agggagctgt g 671

<210> 15
<211> 383
<212> DNA
<213> Homo sapiens

<400> 15
aaaaaaatac attcctgtat ggaagactaa atattaaaga aaaaaacagt tatccatatg 60

ttgtttcata gctttaagta tcctagtcta aaatccaaag ggactttttt ggggactcaa 120

aaaaaaaat tctaaagttg atccagaaga gtaaagagac taatatagtc aaaataatat 180

ttgaaaaggt taaataatta tgggaaaaca tgcttagcaa aaatataaac aaacaaaact 240

tcaaacagac aaaaacccta tgaaaaacca aaaacctatt taaaaactaa attctaaatg 300

agaactaaca tacaaatcaa gaatcagcat agatagctaa gaagcaaatt atagcatata 360

aactaattca atttattata aag 383

<210> 16
<211> 742
<212> DNA
<213> Homo sapiens

<400> 16
ctaggctgct cctgtgtggt tataatgaat ctcatcagct acaatcattt cccaaaaagg 60

acagaaagtg aatctgtctt ataaagatgg ttttcaaagc caactacaga tatcatagaa 120

aaaaaaaaaa aaacagtggc agataagacc tttcatcttt ttcttatccc atgggctttt 180

ctgcctccag atttccttgc acatcaaggg ggttctgtgc aaatcagtgg gcctggccct 240

ctgcccaccc tgggctaagc tgtggttgcc atggctacga agacaagaat gacccttggt 300

tttatagagc cctgggtccc ttgctcacag gccttttcag ttgatatttc tttcatcttc 360

ttcagaaccc catgtggcag gtgcaacaag gagttcacca atgctccggc caaagtgagt 420

aatgagggac acatgcacga ccaggcagag cagccctgaa gaagtggttt gtgagggtgt 480

ggaggaaggg cttgcttcca cactctgcac ttctgggtcc taaggcacta atcacacctg 540

gctcaggcat cattctcagc ctcatgctac ttttgtccag ggacagaaca agaggtgcca 600

gcactcaagg gaatcctgca ccacaacagg caggggacca tcatgactgc tgaaataggc 660

agcaggagtc cgaactatgg cataaaaatg tcacagcaac accacagggg ggaacccctg 720

gggggaagta ggggcatggt ga 742

<210> 17
<211> 228
<212> PRT
<213> Homo sapiens

<400> 17

Ala Thr Thr Ala Thr Cys Thr Cys Cys Ala Thr Thr Ala Thr Gly Cys
1               5                   10                  15

Thr Cys Thr Ala Thr Cys Ala Gly Thr Thr Thr Cys Thr Thr Thr Ala
            20                  25                  30

Thr Ala Gly Ala Ala Thr Ala Thr Gly Ala Thr Thr Ala Cys Thr Ala
        35                  40                  45

Cys Thr Thr Ala Gly Thr Gly Ala Thr Ala Ala Ala Gly Cys Thr Thr
    50                  55                  60

Cys Cys Thr Thr Thr Gly Cys Thr Ala Ala Gly Ala Thr Thr Thr Cys
65                  70                  75                  80

Ala Gly Cys Cys Thr Ala Cys Gly Ala Ala Cys Cys Ala Thr Gly Ala
                85                  90                  95

Thr Cys Cys Ala Ala Ala Cys Cys Cys Thr Ala Cys Thr Thr Cys Thr
            100                 105                 110

Ala Ala Ala Ala Cys Ala Thr Ala Thr Ala Ala Ala Cys Ala Thr Gly
        115                 120                 125

Cys Thr Thr Thr Ala Cys Ala Ala Gly Thr Ala Thr Cys Cys Thr Ala
    130                 135                 140

Thr Ala Thr Ala Thr Gly Gly Ala Ala Ala Ala Gly Thr Cys Cys Thr
145                 150                 155                 160

Thr Gly Gly Ala Ala Thr Thr Ala Thr Thr Thr Gly Gly Gly Thr Ala
                165                 170                 175

Ala Thr Thr Ala Ala Cys Cys Thr Gly Gly Thr Ala Thr Thr Ala Cys
            180                 185                 190

Ala Thr Gly Thr Gly Thr Thr Ala Thr Thr Thr Cys Cys Thr Gly Ala
        195                 200                 205

Thr Cys Thr Ala Cys Thr Ala Thr Thr Gly Cys Ala Ala Ala Thr Thr
    210                 215                 220

Gly Ala Cys Ala
225

<210>  18
<211>  751
<212>  DNA
<213>  Homo sapiens

<400>  18
   tcttgattta cacaaaaaac cataactaac taaaatagaa aacttttaat tatctgtatt      60
   gttgtataaa atgttttata tattaggaat ctaaaaattt gttttttgct tttatcctct      120
   gtcataggaa gaagacatca tgtctctgtt tactgtaacc attaataaag gctaataaca      180
   gacagtacat gatgatactt ttaactaggg caaacaaaag taatatttta acaatgaggt      240
   ttggtctttg ctatctatac ctcatgtcta attttcccta caatgtaaat gtcattcctc      300
   ctctctaccc atttgtaagg gtctcagttt tctgctcttg catgacttat tttaaagggt      360
   cacaataagg ccaggtaatt catattttaa aaattccatt tagaataatt acatctaaaa      420
   attcacaaga aagacaattt caatataaaa taataaatta ctaatattgg aatttcaagc      480
   attagtcatg gcaaaaaaga gataatttgt agcagaatat tttaatggca actttcttat 540
   tctatcactt attgtgttct atttgttatg accaaagaaa ttactctata tccactacaa 600
   ttcataaaac aggcatggaa gaagtctttt ttttcttggt gctcatgtct aagaagatga 660
   acctcagaag tatgtcattt ttcaatacta tgttctgaac agacagcaca cattattttt 720
   gaatggacac caaatctcaa acatatatag a 751

<210> 19
<211> 657
<212> DNA
<213> Homo sapiens

<400> 19
gtcctaataa tcttcaatga attttaacta attttcaggg ataacaaagc acttcagatt 60

gaagtcaatt catgtatact atttactcag gaacttttaa tttttcctat cacatacatg 120

ctgttgctgg gtttcttatt tgttaaaaga tatttcattc cctactgtgt ttaccctttg 180

ttagcaaagt tggtttagag tgacatagcc tgatgaaacc cataaaacag ccataaattg 240

ctcttatatg ggataaaaca atatttgaac actatatttc ttaaaaatat aatcttatat 300

tgggtggtta gaagtgatct tcacatcgtg tgtgtgtgtg tgtgtgtgtg tgtaatatat 360

aatatgaaag acttttaaaa gtaactttaa aaatacatat ttttatatac atattttcat 420

acatatttac atacattttc tgttttcaat tcatctaggt ttacattaga ctatgtcctt 480

agtttgagtg ttaaaactat aaaaagagaa taaagttaca gcagaattaa ttgccaagga 540

tatgacagtt cgagcactac agtaaaaaat gagacacagg tttataaaaa catttaaatt 600

ctgaattttt gctttcttag gtttctctgt cagtaataga taattgttag tatgaaa 657

<210> 20
<211> 509
<212> DNA
<213> Homo sapiens

<400> 20
tttctaaatc tttgttacat tcttctttac tctgaggggt tttagtcatc tgctagtaaa 60

ctagttagtc ctttccgtca gctgggtaaa aagctataaa tggttttcaa ctctatgcag 120

ctcccttggg ttgctcattt gatctgagcc aggtttgaag ttgggccatt gagaatgatc 180

tgtcctcatg atgtaggggg tgactccagc caattcttaa tattgctgga attaattaac 240

tttgattcct ctaactgaaa gagggcattt tactctaccg tgtattatta aataccttat 300

aacttgattt ttttgatttt gttttcttgc tcagtgagta cttaaaatat ccctgccgat 360

atagtaattt gttccctgtt taatatggct ttttcctttg tttactctgg actcaagtct 420

ttgtagcttg tttttctaag tcagctcaaa agttgctggt cattggattt ggcagccttt 480

tcaagcttta cccaccagaa tcctacagg 509

<210> 21
<211> 582
<212> DNA
<213> Homo sapiens

<400> 21
cccggcccag tctccacatc ttgtaagtgg ggagataaaa cctatctaag ggggatatta 60

tgaggagcag gagagaaggc atgtcattct cctaattaat tggcattcac taaagagtta 120

tgtgattatt aaatacataa taaaatataa aaatataagg tgctctagtt atataatcat 180

gaggtctgag gatggtgatg actgcacttc agtggactac cttatgaggg actgactgtc 240

aagaagctct gtgcatgtgt gcttgtgtgt gtgcatctgt gtattatata tatacatata 300

cggtatataa ataggcactt ttattatata agaaaatgat gtcacagtaa aaatgcataa 360

atattattga gcatgtttgt ataaacgagt gaacaaagag acatttggga tgcaagacca 420

gtggtataat ctgcccaaac acaagccccg ttttactgtt gctaatgcac aaagagaggg 480

ctggcagaca tggcctgcat gctctgtaca cctgcccatc tgcaccacac tcccccatgg 540

tcttgctgtt gttgctgtgt tgggctcatc ccgcctctca ca 582

<210> 22
<211> 307
<212> DNA
<213> Homo sapiens

<400> 22
aggcagagat tatgtgagac tttgcatgat aggggtctgt gcattcttgc gcatttctta 60

gctctgtgtg agcaatattt aagtagtcat tacataaaat ctattgaacc tatacaaaga 120

agtgaacaca ctgtgatgag ttggctccta ccaagtaagg cctgggggtt ggtcctttat 180

ggttccccag cacacatctt aaaatagttc ctttgtggat aaactctctt tgaattattc 240

tattttgagt gtgccatctg attcttgtta ggactctgac tttaaaaagg tagagcattg 300

ctctcat 307

<210> 23
<211> 633
<212> DNA
<213> Homo sapiens

<400> 23
caaatggatt aatttacata tggatgaaca tgatctgttg ctctccaggt cccaaaggat 60

acataagaag aaaaatttag tgatgttact ggatgatgtc ttttaagaca acacaataca 120

atatctgagt atgtaccctt acgacataga gaagggattt tcaaaatatt ttaacttaaa 180

tagattcact aaaagaaatc accttccaac cactgttcct tgtctctggt caattagggt 240

cataatattg ttttcattgt attacaaaag gtaagaatgt acactgtata aatgaataaa 300

taatatagat tactagataa gcagataaat aaatacaaaa gcacaaaaat acaaaagcaa 360

atgaccactc aactcacttc cacccacttc aaggaaaaca cagttccttt atcatagtta 420

ctattagaag tctttcctgt cttcattttc ctacaagcat gcagaaatat atatgtggac 480

acatttgtgc cagaattcct ttttttctga cactcacttt tttcctccta ctccacaata 540

tgtcaggaca attttctaca agatatagca aatggagtaa catagaatag agcaaaacat 600

gaaaacctca aactcattag tggatgatgt ttt 633

<210> 24
<211> 674
<212> DNA
<213> Homo sapiens

<400> 24
gggaaggctc ttctaagaaa ccacgcccac acacaaatta gtaaattcga caaaacagga 60

aacaacaaaa aattttgtgt taaaagtaac actaaatcaa aatgaaaaga taaaaggcaa 120

acacaaaatt gacaaggatg cttgcaactt gtgtaactga taaagggcta ttttcctgaa 180

tatatttctc tattctataa ttactttctt taatatattt taaaaacttc tgtaagtaag 240

tatgaaatag acaaatgata agaacagtta tcagaaaatg aaatacaaat gattcaaaca 300

tgaaaagatg ctcgagctca gtctataata atagaccctt aaaagtataa tgaaatatct 360

ttttttaaac ccctatctga ttagcaaaga ccagcaagtt ggaaaaacag agggcttttt 420

caaagatcaa gcttgtgagc caaactaaag gattctgtgc tttcaagaat tacactgttt 480

agagtttgga ctttttgaaa aaaatgtaca tgtctatgaa ataaattggc cttttaaaaa 540

agagtttgca gcaatgctga aagtagtggc caaaagcatg atcaagagat aggatattca 600

tctaatctca aaccatctcc ctacaagcta tttatcaatt acaaagtgaa aaatactaac 660

tgcacagtgg tgaa 674

<210> 25
<211> 600
<212> DNA
<213> Homo sapiens

<400> 25
   acaaaagtca ctcctctgct caaaatcttt gcacggtttc ctgctgtact tggaatacaa 60
   ccccaagtcc tttcctaggg cactagcctt gtattggcct tgatcactgc tcctgcccac 120
   ctcataccac ttgtcatttg gccattatag tccaacaaca tccactcatc cgctttctcg 180
   caggccctgg aatattctaa gcactttccc atctcagggt gttgtgaatg ctgtttcctc 240
   tacctgctct catcctccca ctctttatca actaagttct cacatacaca tgtttgatct 300
   caagctgtgt gtgtttactt gtttgttatt tgaagcacta tgctgagcca tagtggaatc 360
   tgaggtaaaa ggaaaaataa gtaatattga ctctgagttt atgtaatatt ttgatttttt 420
   cccatgaagg aattttgcat taatttttat tttaaaaata ttgtattaaa atttatttta 480
   tcttgattac tgagtatttt ggtgtcccct taaaattctc accaaaggac aagtgcctcg 540
   ctcacctcac cctttttcca gtactggtca ttggtctgtc ccaccaaact gtaagtaaac 600

<210> 26
<211> 709
<212> DNA
<213> Homo sapiens

<400> 26
ccgagggctt tccctgtgag aatcagtttc aaatatgagg ttgattttat tttccccggt 60

tgcaattaac tgaaaaaaat atttcaaagc tgtataatta aaggccggca ttttattagg 120

gtttaaaata gtcgctatga aatttgctcc tgccattggt aattacaacg tacatcttaa 180

tatccccctg cttgccagcc ccgagataac agcgctgtgt gtgctgcatt tggctttgca 240

gagaaggtaa aatccttatc ttatctccat ctgcacacaa caaccagcct ctcttccaat 300

tcaaccctaa tcttcttagt aaataatact gatatttttc accataacaa aatggagctg 360

gtgccagatt ctcactgata acacctctcg tttacacacc attctctgtc aaaatcggcc 420

cgatttttct taatctcttt aacttatcaa aaaaaatact ttttacattt ggccatagta 480

agttcttttc taccaaataa taatattata aataaatccc tttgcctaca gctgactcat 540

tgaaaagata ctcagcctcc tcattttctt catgaatgga aattgttctg ctcttcaatt 600

ttcctcccaa gtttgggggt ccacaccaag ggaattgttc ccaggcaggc cctgagccat 660

ctttgtatct tgcagggggt catcttggtc caggatgtgc atgcttctc 709

<210> 27
<211> 397
<212> DNA
<213> Homo sapiens

<400> 27
   agtataaaat ctgggagaaa aatccatcaa attataaaat agaactaata atgctttcat 60
   gggtaagcaa tgaaacaaac caaaggtaaa ccagcgtttt aaagattgat atttctttaa 120
   gaagagaatg gaaaataaat taaaatatac attttatatt gattatcatg aagtttttct 180
   agtttcttac ctgaagtttc ctggaggaac tttaacagca ctcatttgac attaggaaga 240
   agccgaatgt aaataaatcc taggtggata agcataatat aatgagtaat atgatcttaa 300
   atggaaaaaa gattatgtaa agttgtttat actcttaaca ttttccaccc actacactgt 360
   gaagcagatt aaccttttgt caaaggtgta accttct 397

<210> 28
<211> 537
<212> DNA
<213> Homo sapiens

<400> 28
ctttgaagga tgaagagaag ttcaccaagt gaacgtggag gaaaagcagt ccaggcagaa 60

ggaacagctg ctgaaaaggt caggtgccca aaccagaaag gcatgtgagg gaattcatgg 120

gcagttctgg agaaccagag cagaaggaga caggctagag aatggcagga gatagcccta 180

gaaagctcta tgtgtgccag gtgcaaaagg cttgtgcgcc agctaaggag tctgcacttt 240

cccctgtaag caattaaaag ttttaagcag aggagggatt ggtttgtatt gatctagata 300

attcagggaa caatgaaggg aggatatttg aggaatccca aagagcaggg gcagagactc 360

tcatcacagt tcaggcctga ccgaaggcca tagggagata gcagtaaagc agggaaacct 420

actgtccctt cttcacctgg aaaactatca ccttccagag ttcctgtcac ccccagtgaa 480

ggcaaccact gtctcttctg ttacctaaca cgtggtacaa acagctacca tagcatg 537

<210> 29
<211> 578
<212> DNA
<213> Homo sapiens

<400> 29
tataaggtag ttgtgtagct ttaagactaa gacctctaca cctggaaatt ggtatacttt 60

ttcaataaag catcagatag taaagttttg tgggccatat gtctctgctg caactactca 120

gctctgccat tgtgcaaaag cagccacaga taatatgtaa atgaatgaac ttggctgttt 180

tcacaaaaac aggcggctgt taggatttgg cttgctgact cttaacttgg attctcactt 240

ctgaaagcat ggtctaggag accagcagca tcagcattac ctgtgaacct gttagagata 300

cagaatgttg agccccttct catactgatt gctctagagt ctaaaccatg tttctcaaac 360

ttcagcatac ataggactca cctggggatc ttgtgaaagt acagactctg agatagcagg 420

gctgaacacc atactagcct tggggaggca atgttatgac ctggtcagtg ttccttgggg 480

agtccgcaat ctattaggag agactgctat ataaacaaat aatttcaata cagactagta 540

aatgctataa cggaggtatc ttcagcagag gagcacca 578

<210> 30
<211> 589
<212> DNA
<213> Homo sapiens

<400> 30
attttcatgt tgatggcaat catccaagag agagcaggcc atgtcacgca ataatagaac 60

aaggagaaat gcacactttt ctttttccat gtctctgctt gcattacatt tgctaatacc 120

actttagcca gataaattaa cacagcgcag cacatagtca aaaaaataga agaccagaag 180

ttacagaaca actgacatgg tttagaaaag caattaagac catcagtgaa atcagtctac 240

cacaaaaggt aattcatttg ttcacaacgc tgttgaaaag acttatcttc caagacagga 300

aatggttctc cactgaaggg tgaagacatt tcaattttca gtcatttggg gaagagttgg 360

atctccaacg agtaactttc atgcaaggac aagaatttag tagtgaaata gaggttattc 420

gtttttttac cataaaataa ttaataatct tggaggcagt ttcctcatag cagttattat 480

ggcagttgtg ttcatttaca ggaaaactga gaaactctaa gatgtttttg ggaaaaaaaa 540

gtattttgaa agcttgcgag tgttaacttc cacaatagat atactcttc 589

<210> 31
<211> 1750
<212> DNA
<213> Homo sapiens

<400> 31
   cgcacagcgc gcaggtcctc accagagctc tggtggccac ctctgtcccg ccatgctgct 60
   caccgacagt ggccagggcc cacagcacca agaggcttgg gccacaaagt aaagggtcgc 120
   ggagcctcgc cggccgccat gtggagctgc agctggttca acggcacagg gctggtggag 180
   gagctgcctg cctgccagga cctgcagctg gggctgtcac tgttgtcgct gctgggcctg 240
   gtggtgggcg tgccagtggg cctgtgctac aacgccctgc tggtgctggc caacctacac 300
   agcaaggcca gcatgaccat gccggacgtg tactttgtca acatggcagt ggcaggcctg 360
   gtgctcagcg ccctggcccc tgtgcacctg ctcggccccc cgagctcccg gtgggcgctg 420
   tggagtgtgg gcggcgaagt ccacgtggca ctgcagatcc ccttcaatgt gtcctcactg 480
   gtggccatgt actccaccgc cctgctgagc ctcgaccact acatcgagcg tgcactgccg 540
   cggacctaca tggccagcgt gtacaacacg cggcacgtgt gcggcttcgt gtggggtggc 600
   gcgctgctga ccagcttctc ctcgctgctc ttctacatct gcagccatgt gtccacccgc 660
   gcgctagagt gcgccaagat gcagaacgca gaagctgccg acgccacgct ggtgttcatc 720
   ggctacgtgg tgccagcact ggccaccctc tacgcgctgg tgctactctc ccgcgtccgc 780
   agggaggaca cgcccctgga ccgggacacg ggccggctgg agccctcggc acacaggctg 840
   ctggtggcca ccgtgtgcac gcagtttggg ctctggacgc cacactatct gatcctgctg 900
   gggcacacgg gcatcatctc gcgagggaag cccgtggacg cacactacct ggggctactg 960
   cactttgtga aggatttctc caaactcctg gccttctcca gcagctttgt gacaccactt 1020
   ctctaccgct acatgaacca gagcttcccc agcaagctcc aacggctgat gaaaaagctg 1080
   ccctgcgggg accggcactg ctccccggac cacatggggg tgcagcaggt gctggcgtag 1140
   gcggcccagc cctcctgggg agacgtgact ctggtggacg cagagcactt agttaccctg 1200
   gacgctcccc acatccttcc agaaggagac gagctgctgg aagagaagca ggaggggtgt 1260
   ttttcttgaa gtttcctttt tcccacaaat gccactcttg ggccaaggct gtggtccccg 1320
   tggctggcat ctggcttgag tctccccgag gcctgtgcgt ctcccaaaca cgcagctcaa 1380
   ggtccacatc cgcaaaagcc tcctcgcctt cagcctcctc agcattcagt ttgtcaatga 1440
   agtgatgaaa gcttagagcc agtatttata ctttgtggtt aaaatacttg attccccctt 1500
   gtttgtttta caaaaacaga tgtttcctag aaaaatgaca aatagtaaaa tgaacaaaac 1560
   cctacgaaag aatggcaaca gccagggtgg ccgggccctg ccagtgggcg gcgtgtgcta 1620
   gcaaggcctg ccgggtgtgc cgcagtcacc acaggttctg gagaacattt cacagaagtg 1680
   cctgagacgc ggagacatgg ctggtgttaa atggagctat tcaatagcag tgacgcgctc 1740
   tcctcagcca 1750

<210> 32
<211> 551
<212> DNA
<213> Homo sapiens

<400> 32
ccacaagaca gtgccattta gtggcagccc taggataaag atgatactgt aggccaggga 60

gaggtagact tgcttgtact tctctgagaa ctggcagaga ccttgttcct gtgatgtatt 120

catgtccacc ttctccatgt cccgggaggc tccctccagg agcagagctc cacgacggct 180

cccgcttctg cttcccctgg aaggaagcaa aatggacagc ataacatcat gacctcagca 240

tggcatcagc ctctgcccca acccggcatt caactttgtc cccccaagtc tagtcagagg 300

ccttgtttaa aagagaccaa ccccaggaaa atagagtatt caacaagggc caaataatct 360

tccaaagctg ggcgtctaga cttaccaggt ccaaagggtt ggccttacat ggacttgtat 420

gctgtctact ctaggctggg ttgtgataga aatggtggtg ggatgacact gtcatttaaa 480

cagggtactc agaacctcca gctctgtgtc tcctcctgct agaatccatt gcatagagtc 540

agaaaatctc c 551

<210> 33
<211> 426
<212> DNA
<213> Homo sapiens

<400> 33
catcctaagc tcgggcacac aaagtcccag aggcacacag attcagtggc ttggggatag 60

catattccaa ctcttgggcc cctaactcaa aatccccaac aaccctcagt cacctgtggc 120

tccagtcctg ttttggtttt ccacagatca caaaggcatc aaggaagagg ctcacggtaa 180

gcctggccta ctcggagagc caccagatcc gcgtgtccca gcaggacttc cggctcttcc 240

gcaccctctt ccttctaatg gtctccttct tcatcatgtg gagccccatc atcattacca 300

tcctcctaat cctgatccag aacttcaagc aagacctggt catctggccg tccctcttct 360

tctgggtggt gggcttcaca tttgctaatt cagccctaaa ccccatcctc tacaacatga 420

cactgt 426

<210> 34
<211> 537
<212> DNA
<213> Homo sapiens

<400> 34
gctgggaagt gctcctggtg cagccggcgg tagatggcgg agggcacggt gagcagcaag 60

gccagtgtcc aggctgcccc acaggccacc tgcaccccgc acgcccgctg aaccgtatac 120

caccaggcag gcccgagagc caggaagcag aggtcggcac tgagagctgc caggagcagg 180

acgctggcat acatggtcag caggatgatg gagggcagcg cccgacagcc cactgcacca 240

tacggtcagt ggcctccacg ggcaatgggc actgccagga tgggcagaga caaacagcac 300

agcaaatccg ccacggccag gtggagcaac caggtggcac ccaccctccg gcgggccacc 360

ttcccagcca cccaggccac catggcattg cccggcaccc ccaccaggaa gatggcggca 420

tacagtggga gcggggccac gcgcagcggg tcgatggcca ggcaggcgcc atccaggcag 480

tccacagggc ggtccgagag gtcgctgtaa tccccatact cgtagctgac agaatcg 537

<210> 35

<211>  536
<212>  DNA
<213>  Homo sapiens

<400>  35
ggctccactg gcctttcggc aaggggctct gcaggctgac ggcgtttgtg ctctacaccg      60

acacctacgg ggggtctacc tcatggcctg tgtgagcgtg gaccattacc cagctgtggt     120

ctgtgcccac tggggcccgt gcctccgcac ggctggccgc gccaggctgg tctgcgtggc     180

catctggacc ttggtgctgc tgcagacgat gcccttgctc ttgatgccca tgaccaagcc     240

gctggtgggc aagctggcct gcatggagta cagcagcatg gagtcagtcc tcggggctgc     300

ccctcatggt cctggtggcc tttgccattg gcttctgtgg gccagtgggg atcatcctgt     360

cctgctatat gaagatcacc tggaagctgt gcagcacagc tgggagaacc cagtgaccag     420

cgggaaagga caccaccggc ggggcagccc gggaggaccc agtgaccagc aggaaaggac     480

gccaccggcg gggcagccca ggaggaccca gtgaccagcg ggaaaggaca ccaccg         536

<210>  36
<211>  1449
<212>  DNA
<213>  Homo sapiens

<400>  36
gaggcaggct gcagggaagt aaggaggagg catggcacct tctcatcggg catcacaggt      60

ggggttttgc ccccacccctg aacgccctct gtggcgcctt ccacccacct gtaggcccag     120

aaggatgtcg gtctgctacc gtcccccagg gaacgagaca ctgctgagct ggaagacttc     180

gcgggccaca ggcacagcct tcctgctgct ggcggcgctg ctggggctgc ctggcaacgg     240

cttcgtggtg tggagcttgg cgggctggcg gcctgcacgg gggcgaccgc tggcggccac     300

gcttgtgctg cacctggcgc tggccgacgg cgcggtgctg ctgctcacgc cgctctttgt     360

ggccttcctg acccggcagg cctggccgct gggccaggcg ggctgcaagg cggtgtacta     420

cgtgtgcgcg ctcagcatgt acgccagcgt gctgctcacc ggcctgctca gcctgcagcg     480

ctgcctcgca gtcacccgcc ccttcctggc gcctcggctg cgcagcccgg ccctggcccg     540

ccgcctgctg ctggcggtct ggctggccgc cctgttgctc gccgtcccgg ccgccgtcta     600

ccgccacctg tggagggacc gcgtatgcca gctgtgccac ccgtcgccgg tccacgccgc     660

cgcccacctg agcctggaga ctctgaccgc tttcgtgctt cctttcgggc tgatgctcgg     720

ctgctacagc gtgacgctgg cacggctgcg gggcgcccgc tggggctccg ggcggcacgg     780

ggcgcgggtg ggccggctgg tgagcgccat cgtgcttgcc ttcggcttgc tctgggcccc     840

ctaccacgca gtcaaccttc tgcaggcggt cgcagcgctg gctccaccgg aaggggcctt     900

ggcgaagctg ggcggagccg gccaggcggc gcgagcggga actacggcct tggccttctt 960

cagttctagc gtcaacccgg tgctctacgt cttcaccgct ggagatctgc tgccccgggc 1020

aggtccccgt ttcctcacgc ggctcttcga aggctctggg gaggcccgag ggggcggccg 1080

ctctagggaa gggaccatgg agctccgaac tacccctcag ctgaaagtgg tggggcaggg 1140

ccgcggcaat ggagacccgg ggggtgggat ggagaaggac ggtccggaat ggacctttga 1200

cagcagaccc tacaacctgc tgcccttccc tgtccctttc caccccccac ccaccctcca 1260

gaggtcagtg ttctgggaca tttggggacc cttctttgac tagagtttgg atctggctgg 1320

gtaggattac tatacacttg gggcaggccc aggctcctcc aaactgaggg attatgaggg 1380

tggtgatggt ccctgttaag gactattgtg tgcttgcaag ttggcatgta cccatgtgcc 1440

agcattgct 1449

<210> 37
<211> 635
<212> DNA
<213> Homo sapiens

<400> 37
ccagccgtcc aggcgacgcg ggccagcacc aggaaccagg tgacgctggc cacgtggtag 60

cgcgggcaga ccagggggca cgcgctgttg cgcagcaggc cgcaggcgtt ggcgggcagc 120

ggcacggccg gcagggtcgg ggtccacacc agggcgcaga ggacggccga ggcgtgtctg 180

ggccggcagc cctggtagca ggcggggaag aggtcggaga ggcagcgctc cacgctgaag 240

gccgccagca gccagagccc caccgcgaac cacaggaagg tgagcacgaa gtagagtgtg 300

tcctgggcgc ccagggcagc ctgagccacg gagaagccca cacggcagga gaggaacagg 360

aagtcggcgg cggccaggtg cagcaggtag atggagaagg ggcccttctt gatgcggaag 420

ccgaggttcc agagcaccag cccgttacct accggtcccc cgaggcccac gatcagcgtc 480

aggtagaaga ccacactgtc gaaggttctc cagaggccga acagcccaaa catcctggcc 540

ggctcagagg gtgctggcga gggacctgca agatggagac agacatggtc ctcaggagtc 600

ttttgtgccc ccgctggggg cacagggagt ggcat 635

<210> 38
<211> 564
<212> DNA
<213> Homo sapiens

<400> 38
atgtctacag aaaccccttc gccatctacc tcctggtacg tggcctgcag caggatctca 60

tcttccttgg ctgccacatg gtggccatcg tccccgactt gctgcaaggc cggctggact     120

tcccgggctt cgtgcagacc agcctggcaa cgctgcgctt cttctgctac atcgtgggcc     180

tgagtctcct ggcggccgtc agcgtggagc agtgcctggc cgccctcttc ccagcctggt     240

actcgtgccg ccgcccacgc cacctgacca cctgtgtgtg cgccctcacc tgggcccttt     300

gcctgctgct gcacctgctg ctcagcagcg cctgcaccca gttcttcggg gagcccagcc     360

gccacttgtg ccggacgctg tggctggtgg cagcggtgct gctggctctg ctgtgttgca     420

ccatgtgtgg ggccagcctt atgctgctgc tgcgggtgga gcgaggcccc cagcggcccc     480

caccccgggg cttccctggg ctcatccttc ctcaccgtcc tcctcttcct cttctgcggc     540

ctgcccttcg gcatctactg gctg                                            564

<210>  39
<211>  187
<212>  PRT
<213>  Homo sapiens

<400>  39

Val Tyr Arg Asn Pro Phe Ala Ile Tyr Leu Leu Val Arg Gly Leu Gln
1               5                   10                  15

Gln Asp Leu Ile Phe Leu Gly Cys His Met Val Ala Ile Val Pro Asp
            20                  25                  30

Leu Leu Gln Gly Arg Leu Asp Phe Pro Gly Phe Val Gln Thr Ser Leu
        35                  40                  45

Ala Thr Leu Arg Phe Phe Cys Tyr Ile Val Gly Leu Ser Leu Leu Ala
    50                  55                  60

Ala Val Ser Val Glu Gln Cys Leu Ala Ala Leu Phe Pro Ala Trp Tyr
65                  70                  75                  80

Ser Cys Arg Arg Pro Arg His Leu Thr Thr Cys Val Cys Ala Leu Thr
                85                  90                  95

Trp Ala Leu Cys Leu Leu Leu His Leu Leu Leu Ser Ser Ala Cys Thr
            100                 105                 110

Gln Phe Phe Gly Glu Pro Ser Arg His Leu Cys Arg Thr Leu Trp Leu
        115                 120                 125

Val Ala Ala Val Leu Leu Ala Leu Leu Cys Cys Thr Met Cys Gly Ala
    130                 135                 140

Ser Leu Met Leu Leu Leu Arg Val Glu Arg Gly Pro Gln Arg Pro Pro
145                 150                 155                 160

Pro Arg Gly Phe Pro Gly Leu Ile Leu Pro His Arg Pro Pro Leu Pro
                165                 170                 175

Leu Leu Arg Pro Ala Leu Arg His Leu Leu Ala
            180                 185

<210>  40
<211>  544
<212>  PRT
<213>  Homo sapiens

<400>  40

Cys Ala Ala Thr Thr Thr Thr Cys Thr Ala Thr Thr Gly Cys Cys Thr
1               5                   10                  15

Cys Thr Cys Thr Gly Gly Cys Cys Thr Gly Thr Gly Cys Thr Gly Ala
            20                  25                  30

Cys Thr Thr Cys Thr Thr Gly Gly Thr Ala Gly Gly Thr Gly Thr Gly
        35                  40                  45

Ala Cys Thr Gly Thr Gly Ala Thr Gly Cys Thr Thr Thr Cys Ala
    50                  55                  60

Gly Cys Ala Thr Gly Gly Thr Cys Ala Gly Gly Ala Cys Gly Gly Thr
65                  70                  75                  80

Gly Gly Ala Gly Ala Gly Cys Thr Gly Cys Thr Gly Gly Thr Ala Thr
                85                  90                  95

Thr Thr Thr Gly Gly Ala Gly Cys Cys Ala Ala Ala Thr Thr Thr Thr
            100                 105                 110

Gly Thr Ala Cys Thr Cys Thr Thr Cys Ala Cys Ala Gly Thr Thr Gly
        115                 120                 125

Cys Thr Gly Thr Gly Ala Thr Gly Thr Gly Gly Cys Ala Thr Thr Thr
    130                 135                 140

Thr Gly Thr Thr Ala Cys Thr Cys Thr Thr Cys Thr Gly Thr Cys Cys
145                 150                 155                 160

Thr Cys Cys Ala Cys Thr Thr Gly Thr Gly Cys Thr Thr Cys Ala Thr
                165                 170                 175

Cys Thr Gly Cys Ala Thr Cys Gly Ala Cys Ala Gly Gly Thr Ala Cys
            180                 185                 190

Ala Thr Thr Gly Thr Gly Gly Thr Thr Ala Cys Thr Gly Ala Thr Cys
        195                 200                 205

Cys Cys Cys Thr Gly Gly Thr Cys Thr Ala Thr Gly Cys Thr Ala Cys
    210                 215                 220

Cys Ala Ala Gly Thr Thr Cys Ala Cys Cys Gly Thr Gly Thr Cys Thr
225                 230                 235                 240

Gly Thr Gly Thr Cys Gly Gly Gly Ala Ala Thr Thr Thr Gly Cys Ala
                245                 250                 255

Thr Cys Ala Gly Cys Gly Thr Gly Thr Cys Cys Thr Gly Gly Ala Thr

260                 265                 270

Thr Cys Thr Gly Cys Cys Thr Cys Thr Cys Ala Cys Gly Thr Ala Cys
        275                 280                 285

Ala Gly Cys Gly Gly Thr Gly Cys Thr Gly Thr Gly Thr Thr Cys Thr
    290                 295                 300

Ala Cys Ala Cys Ala Gly Gly Thr Gly Thr Cys Ala Ala Thr Gly Ala
305                 310                 315                 320

Thr Gly Ala Thr Gly Gly Gly Cys Thr Gly Gly Ala Gly Gly Ala Ala
                325                 330                 335

Thr Thr Ala Gly Thr Ala Ala Gly Thr Gly Cys Thr Cys Thr Cys Ala
            340                 345                 350

Ala Cys Thr Gly Cys Gly Thr Ala Gly Gly Thr Gly Gly Cys Thr Gly
        355                 360                 365

Thr Cys Ala Ala Ala Thr Thr Ala Thr Thr Gly Thr Ala Ala Gly Thr
    370                 375                 380

Cys Ala Ala Gly Gly Cys Thr Gly Gly Gly Thr Gly Thr Thr Gly Ala
385                 390                 395                 400

Thr Ala Gly Ala Thr Thr Thr Thr Cys Thr Gly Thr Thr Ala Thr Thr
                405                 410                 415

Cys Thr Thr Cys Ala Thr Ala Cys Cys Thr Ala Cys Cys Cys Thr Thr
            420                 425                 430

Gly Thr Thr Ala Thr Gly Ala Thr Ala Ala Thr Thr Cys Thr Thr Thr
        435                 440                 445

Ala Cys Ala Gly Thr Ala Ala Gly Ala Thr Thr Thr Thr Thr Cys Thr
    450                 455                 460

Thr Ala Thr Ala Gly Cys Thr Ala Ala Ala Cys Ala Ala Cys Ala Ala
465                 470                 475                 480

Gly Cys Thr Ala Thr Ala Ala Ala Ala Ala Thr Thr Gly Ala Ala Ala
                485                 490                 495

Cys Thr Ala Cys Thr Ala Gly Thr Ala Gly Cys Ala Ala Ala Gly Thr
            500                 505                 510

Ala Gly Ala Ala Thr Cys Ala Thr Cys Cys Thr Cys Ala Gly Ala Gly
        515                 520                 525

Ala Gly Thr Thr Ala Thr Ala Ala Ala Ala Thr Cys Ala Gly Ala Gly
    530                 535                 540

<210>  41
<211>  595
<212>  DNA
<213>  Homo sapiens

<400>  41

attattcatt cctttagaca ttaaacattc attgagcacc tgctgtatgc aaagcactgg 60

gcacccacac taaggatgaa aaccaaggat aagtaagaca gcatgtagat tctagctgcc 120

tggttgagag gttacagtca gaaagtcctt atagccattc agtgacatac agatagggat 180

aggagaggaa atggggtgag cacagggaag ggataagtat agggtcaggg ctaccaccct 240

tcttttcccc atgaccccat gggggcaaat atgtcctgtc tcttcctggg tgggtgatgt 300

tcactgcccc tttctccatt ccaattggaa cttctagatt gagcccgaag ctagacttgc 360

agatcacaat tttaagaaag ttggatgttc tgcaacaggt tacacaggag ctatcattga 420

gtcatctttt cgttcaccca atcatttatt cattcattca ttcaacaagt atttcccaag 480

caccaattcc atcctcaaca aaagactcct aaatcacagg ccagatgaaa gacgtcacta 540

ttcacttgga aaaattcagc tgattatgat cctggtcgga gtgtactcag cgtcc 595

<210> 42
<211> 651
<212> DNA
<213> Homo sapiens

<400> 42
atggaattag aaaaatgaga aagtaatcaa agattgtaaa aaatactgag atagaattaa 60

aaggaataaa aaatttgtaa atcaaatatg taattctttt taagttgaca cattgtaatt 120

ttatatattt gtggagtatg atttggtaca ttaatttgta tatgtgttgt atgctgatca 180

aatcagggta tgtactaggc catttttgca ttggtataaa gaaatacctg aagctgggta 240

atttaaagaa aagaagttta tttgactcat ggttctatag gctgtgaaca acatctgctt 300

ggcttctggt gggagcctcg gaaagctttc aatagtggca gaaggggaag gggaagctgg 360

agtatcacat ggtgtgaaag ggagcaagag agagagagag agacaggagg tcctagactt 420

ttaaacaacc agatcttgtg tgaactattt catgtgacta agaactcatt catcaccaag 480

cggaaggtgc caagtcattc atgagggatc caccctcata atacactacc ttccaccaag 540

tctcacttac aacattggga atgacatttc agcataagat ttggagagga caaatgtcca 600

aactatatca gggtagttag ctcattcatc acctcatgtc tttatcattt c 651

<210> 43
<211> 668
<212> DNA
<213> Homo sapiens

<400> 43
atgtaaaggg ccttttggaa gaaaactgta aactccactg tggggccatt aaagaagtct 60

tgaatacata gattaaatcc cttggtttga gataataaaa ctctcaatat ttaccatgct 120

tttgtcattt aaatacaaat tgtggacatt tttaccaggg caacagatga gttacaacca 180

tcctttttatc agttgcacag aatcttattg aatgggtata tcctacttat gtcagttctt 240

agtagtgaat attaggttgt ttctaatttt tccatattag aaacaatgtt aaaagaactt 300

gctcttattt gtgtttctta tgtctacact agtattcaat acctttctag gagtggaaat 360

tcctggctca aacaaactgc cttccaagaa gatttactaa tatacattcc tactaatgct 420

gttttagaat gcctttttct ccatattcct atgaacactg agtgttctct ttttaacact 480

ttttttcctc aatctactag actccttcta aaatatatac ttttcaaaac gcctgcttac 540

ccttcaatat attgtctttt aaaagattat ttgtaggtac tgtttatgtg ttaggttggt 600

ggaaaattaa ttgtgatttt gcaaaaactg caattacttt tgcaccaacc taatattata 660

catgtaga 668

<210> 44
<211> 604
<212> DNA
<213> Homo sapiens

<400> 44
tctcttttgg ttcccttttt tcctcattac ctacctcttc tcctttgctg gtccttcaca 60

tcttcctggc ctcttctttg tctgcattta cttgtgtggt cttccgtctt taacacatct 120

acccatcagc tactcccaaa tgtatatatc tattcccgga cctttcctct gaactccaga 180

tttgtatttc caactaccta ctcaatagta ccttcttgga tatttattaa aatttgaatc 240

acgacatgcc taaaattgaa cttcctatct ctgaagcaaa gccctatcct tctattgttt 300

ttcccatccc aataaattgc cactcattct tccagatgct tgggcaacat ttttgcagtc 360

atctttgatt tccttctttc tttgatatcc cacatacatt caccagcata tgctgccctg 420

tctacatttt aaacataacc agcatccaac atttctacaa ttgctactag ccttgtatag 480

ccctatcctc tctcctctgg attactgtat tagcctctga actgggatcc cgcttcaacc 540

ctttccaacc tcctcctccc aaagccaact ctgtgaacag cagctaaagt tgttctttca 600

aaaa 604

<210> 45
<211> 578
<212> DNA
<213> Homo sapiens

<400> 45
caaccctggt aaagcatcgc accttgggtt atgtacctca gggttatttg acgcactggg 60

ctaaaatgtt gaaggacatc ctgtttccag gtggggactg gaacagagcc tggactgttt 120

tagccaatgg ctccttacct caggatgttg cattcccagc acattctggt tggtgttgag 180

aactacaaac aagaaagtgg gaagaactgt tctgcaccat ttatgtaaac ttctaggaaa 240

gcaaactaat gtattgtgac agaaggaaga tgagtgattg cttaaacaca agggaggaat 300

gctacacagg gaaggtctgg agagttggat tacaaagaga gacaaggaca cttttgggag 360

agatggatat gtttattatc ttgcttatgg tgatagtttc ataggtccat aaatcccaaa 420

agcatctcat tgtacactaa ctatgtatga tttatttata ttaattatac ctcaataaag 480

ttgtttttta aaaaagttac cacttaatcc tgtaaataga ccaggaaggc aattaattaa 540

tattttctag tttacttttg agaaactaaa gcttagta 578

<210> 46
<211> 575
<212> PRT
<213> Homo sapiens

<400> 46

Ala Gly Thr Thr Thr Ala Thr Gly Gly Ala Cys Cys Ala Gly Cys Cys
1               5                   10                  15

Thr Thr Cys Cys Cys Thr Gly Thr Gly Ala Ala Ala Thr Thr Thr Gly
            20                  25                  30

Ala Cys Thr Thr Thr Thr Cys Cys Cys Thr Cys Thr Thr Thr Gly Cys
        35                  40                  45

Thr Gly Ala Ala Thr Thr Gly Gly Thr Cys Ala Gly Gly Thr Thr Ala
    50                  55                  60

Ala Cys Ala Ala Thr Gly Gly Thr Thr Ala Cys Cys Cys Cys Thr Gly
65                  70                  75                  80

Gly Ala Thr Thr Ala Cys Ala Gly Gly Ala Ala Gly Gly Gly Cys Ala
                85                  90                  95

Thr Gly Thr Gly Cys Thr Ala Ala Ala Ala Gly Cys Cys Thr Cys Thr
            100                 105                 110

Thr Thr Gly Gly Ala Gly Ala Cys Cys Cys Ala Cys Ala Thr Gly Gly
        115                 120                 125

Cys Cys Cys Thr Cys Ala Gly Ala Thr Gly Ala Gly Cys Ala Ala Thr
    130                 135                 140

Thr Gly Thr Thr Cys Ala Gly Ala Thr Thr Cys Cys Thr Thr Thr Thr
145                 150                 155                 160

Cys Thr Thr Thr Thr Thr Cys Thr Thr Thr Thr Cys Cys Ala Thr Gly
                165                 170                 175

Gly Gly Ala Ala Thr Ala Ala Gly Cys Thr Thr Thr Cys Cys Thr Cys
            180                 185                 190

Thr Cys Thr Cys Cys Ala Ala Ala Gly Thr Ala Cys Ala Thr Gly Thr
        195                 200                 205

Thr Thr Thr Ala Gly Gly Cys Thr Thr Thr Thr Thr Thr Ala Thr Thr
    210                 215                 220

Thr Thr Cys Thr Thr Gly Cys Thr Ala Cys Thr Cys Cys Cys Ala Ala
225                 230                 235                 240

Gly Gly Ala Cys Cys Thr Gly Gly Thr Gly Ala Thr Ala Thr Thr Thr
                245                 250                 255

Thr Thr Cys Thr Thr Thr Ala Cys Cys Ala Thr Gly Cys Ala Thr Thr
            260                 265                 270

Ala Ala Ala Cys Ala Gly Ala Ala Thr Cys Thr Gly Thr Gly Ala Gly
        275                 280                 285

Thr Cys Thr Thr Thr Thr Cys Thr Gly Gly Ala Ala Ala Ala Ala Ala
    290                 295                 300

Ala Ala Ala Ala Gly Gly Cys Ala Gly Gly Ala Gly Gly Gly Ala Ala
305                 310                 315                 320

Cys Ala Thr Ala Cys Thr Ala Gly Thr Thr Ala Ala Ala Ala Ala Gly
                325                 330                 335

Thr Thr Thr Cys Thr Gly Gly Gly Thr Ala Cys Ala Cys Thr Ala Cys
            340                 345                 350

Cys Ala Ala Gly Ala Thr Gly Thr Ala Cys Cys Thr Ala Thr Thr Thr
        355                 360                 365

Ala Thr Thr Gly Ala Thr Ala Thr Ala Cys Ala Ala Ala Thr Gly Gly
    370                 375                 380

Cys Ala Thr Ala Ala Gly Thr Thr Ala Thr Thr Gly Ala Ala Thr Gly
385                 390                 395                 400

Cys Thr Thr Gly Cys Thr Ala Thr Ala Gly Gly Cys Ala Thr Thr Cys
                405                 410                 415

Thr Cys Thr Ala Ala Gly Ala Ala Cys Thr Thr Thr Gly Thr Ala Ala
            420                 425                 430

Gly Ala Ala Thr Thr Gly Ala Cys Thr Thr Ala Cys Ala Thr Gly Ala
        435                 440                 445

Gly Cys Thr Ala Cys Thr Thr Cys Ala Thr Ala Gly Cys Ala Gly Thr
    450                 455                 460

Thr Cys Gly Ala Thr Gly Ala Thr Ala Thr Ala Cys Ala Thr Gly Thr
465                 470                 475                 480

Thr Gly Thr Thr Ala Thr Thr Ala Thr Cys Ala Cys Cys Ala Cys Thr
                485                 490                 495

Thr Thr Ala Cys Ala Gly Ala Thr Ala Ala Gly Gly Ala Ala Ala Thr
            500                 505                 510

Ala Gly Ala Gly Ala Cys Ala Gly Ala Cys Ala Thr Ala Cys Thr Gly
        515                 520                 525

Ala Ala Thr Gly Ala Cys Ala Thr Gly Cys Thr Cys Ala Ala Cys Gly
    530                 535                 540

Cys Cys Ala Cys Thr Cys Cys Ala Cys Thr Ala Gly Cys Ala Ala Gly
545                 550                 555                 560

Thr Gly Gly Cys Ala Gly Ala Ala Cys Cys Cys Ala Ala Gly Cys
                565                 570                 575

<210>  47
<211>  549
<212>  DNA
<213>  Homo sapiens

<400>  47
cgtggcaggt tgcaccaact tttaaaccca caagcaatta tgagaggttc atttcccttc      60

actcacaaca aaactgcatc ttatcacgtt ttaagaaatc tttgccattt tgctgggatt     120

aacttgtctc tcattattct atatttgcat ttctaagcta ttaagcttga ataatttcca     180

tatgggccat ttgtatttgc aaaacaaaca ctaccccttc aatgattttg cctggcttct     240

gccttcacta gtatttattg cctctttaaa acactaagtt aatagtttta tttgttcctt     300

tgtcagcctc ctctgaaaat gatgacactt ttcaaacagt accacatctt tctactcatt     360

tcagttcaat gctcatatag ggcattgtgc atactgactg tccaagctgt gcactaagca     420

ggtgaatctt ccctgccctt ggatcgagta ataacagaac ttaaagaagg ctcctgggta     480

ggaaaatcat gggtactggt aacagagaga tatggattca aatcctggct tctctactta     540

ctcactcaa                                                             549

<210>  48
<211>  726
<212>  DNA
<213>  Homo sapiens

<400>  48
acaccaggaa cacacttgga tgcccatcgg ccccaggttt cttctgctgg tcctgtctgg      60

ccatgtgttt gggtttgaaa gtgtccagat tgcctggttc tcctggatct tcacgcaaaa     120

gaaacgagca catgatggtg acctggaact ctcctcgctg gcgtcactgc atttttgcaa     180

agcctgtaac tgtcctgtct gcattctggg ctccaaggct cagtcctctg atctttcctg     240

acctgtcctt cccctaggct gccttcctct ttttccttat tacagtaaag ttctgtatgt     300

actgcagcat attccattta ttgggaattg aatatatttc ttctatgcca ggatttaaaa 360

ttaggattgt taatattgtg gtttgtgctc tggttacaga atttctaagg tttgggtgtt 420

ctatcccagc accatatttt ctcaaagcct tgctctctgc agtgggtgat tttgcacaat 480

gcaaacttct caggtacttt cttctgagct ccaggtcccc atatccaaca tctactcaac 540

atcttattct gagatgttca cctcaaacat gtgagaatca acatgtcaac atgtcaatac 600

ctcttgcagg atttcctaac tccacagatg gtatccgtcc aattgtgcaa gccaagagca 660

aggcgccagc tgggacgttt cccataccca atctatcatc atgtcccatt aaattttacc 720

agtagt 726

<210> 49
<211> 633
<212> DNA
<213> Homo sapiens

<400> 49
cacttgagag gtgatctcat ctataaagag agtatattta aggagctaat ttacacaggt 60

agatgacaaa accttacacc aactcttcaa gcacaggaag caccttacac gaaacacttt 120

ggtgaatgct catttctact caattttaaa ctaaattcaa ctgaaaatac ctcattttaa 180

agaggatatt cagttgactc actggggaaa aacaattgga taaactggtt gtttgccctt 240

accgagataa aatcactgat aagttgaatg cattttgtaa atgtgtcaag acagatggct 300

ttaatagtca cacatattaa gtatgggata tgtggcgagg aatagaggtt tttatataca 360

gaagtaagaa ctgtagaggg gggaaaccat gaaagataga aaaagatgag aagaaagagg 420

acagagatga tacactcaag aaaagctgcc tatttcagag tagaatatat tgtaagcccc 480

agtatagttg aatgcttaca agaggagaag cttttagcaa ctcctttgtt tcttaatgta 540

tatattatat ctgaacagaa tgcttctttc ctgtgagcta gacagtagga agtaatctat 600

cttcaacctt gtttggtgtg taaataaaac ttt 633

<210> 50
<211> 618
<212> DNA
<213> Homo sapiens

<400> 50
gtgatacggt tcggctgtgt ccccatccaa atctaatctt gaattacagc tcccataatt 60

tccatgtgtt gtggaagggg cctggtggga gataaccgaa tcatgggggc ggctcccccc 120

atactgttct cttggtagtg aataagtctc ataagatctg atcgttttat aaggggaaac 180

cccttttgct tggttcttat tctctctttg ctggctgcca tgtaagatgc ttctttgctc 240

ttcctttgtg ttctgccatg actgtggggc ctctccagcc atgtggaaat gtgaggaaat 300

taaacctctt tcctttataa attgcccagt cttgggtatg tctttatcag cagtgtgaaa 360

tggactaata caccagaagt tcaagactag cttgggcaac acagtaagac cctgtctgta 420

taaagaaaga ataagaaaaa gaaaaatatc agtggcataa tattagacag aatactataa 480

aactgattaa ggcacattaa tatagccaga ataattattc tgattatatc attttgaaag 540

accagataat caatataact gatttttatc ccgagtagct cagacttgca agcatgcact 600

accatacttg gataattt 618

<210> 51
<211> 1612
<212> DNA
<213> Homo sapiens

<400> 51
cagtgagccg agatggtgcc attgcactct agcctggggc aacagagcca gactccatct 60

ccaaaaaaaa aaggccattc tgaggatcaa ggcaccacta gcaacaggga gccccatggg 120

tctcagaccc tctccccaca tctcctggtc cctgccccca cctggcgtac agggaccagc 180

cccacggaag gctcttgagg ccaggtaacc atggggaggg gaggaatggg gacaccttcc 240

tcctgagtgt cttagggaag agaagcttag gtcaggtggc tgagggtgga aatgagagag 300

gggtctcctc ctggagggtc tcaccattcc cttggtcacc cacccaactc tcatctcccc 360

tgatgtgggg aggagcaggg ggcatggatt cctgagcccc agactcaact gttgtggttt 420

acaggggcat caggagagag agcgagcaga acacactcct gcagcatccc ctggcccccc 480

gccccatgat ggagcccaga gaagctggac agcacgtggg ggccgccaac ggcgcccagg 540

aggatgtggc cttcaacctc atcatcctgt ccctcaccga ggggctcggc ctcggtgggc 600

tgctggggaa tggggcagtc ctctggctgc tcagctccaa tgtctacaga aacccctttcg 660

ccatctacct cctggacgtg gcctgcgcgg atctcatctt ccttggctgc cacatggtgg 720

ccatcgtccc cgacttgctg caaggccggc tggacttccc gggcttcgtg cagaccagcc 780

tggcaacgct gcgcttcttc tgctacatcg tgggcctgag tctcctggcg gccgtcagcg 840

tggagcagtg cctggccgcc ctcttcccag cctggtactc gtgccgccgc ccacgccacc 900

tgaccacctg tgtgtgcgcc ctcacctggg ccctctgcct gctgctgcac ctgctgctca 960

gcggcgcctg cacccagttc ttcggggagc ccagccgcca cttgtgccgg acgctgtggc 1020

tggtggcagc ggtgctgctg gctctgctgt gttgcaccat gtgtggggcc agccttatgc 1080

tgctgctgcg ggtggagcga ggcccccagc ggcccccacc ccggggcttc cctgggctca 1140

tcctcctcac cgtcctcctc ttcctcttct gcggcctgcc cttcggcatc tactggctgt 1200

cccggaacct gctctggtac atcccccact acttctacca cttcagcttc ctcatggccg 1260

ccgtgcactg cgcggccaag cccgtcgtct acttctgcct gggcagtgcc cagggccgca 1320

ggctgcccct ccggctggtc ctccagcgag cgctgggaga cgaggctgag ctgggggccg 1380

tcagggagac ctcccgccgg ggcctggtgg acatagcagc ctgagccctg gggcccccga 1440

ccccagctgc agcccccgtg aggcaagagg gtgacgtggg gaaggtggtg gggtcagagg 1500

ctggggccag ccggacctgg aggaggcctt ggtgggtgac ccggtcatgt gctgtcaaag 1560

ttgtgaccct tggtctggag catgaggctc ccctgggagg cagctggaaa gg 1612

<210> 52
<211> 1527
<212> DNA
<213> Homo sapiens

<400> 52
atgacgtcca cctgcaccaa cagcacgcgc gagagtaaca gcagccacac gtgcatgccc 60

ctctccaaaa tgcccatcag cctggcccac ggcatcatcc gctcaaccgt gctggttatc 120

ttcctcgccg cctctttcgt cggcaacata gtgctggcgc tagtgttgca gcgcaagccg 180

cagctgctgc aggtgaccaa ccgttttatc tttaacctcc tcgtcaccga cctgctgcag 240

atttcgctcg tggccccctg ggtggtggcc acctctgtgc ctctcttctg gcccctcaac 300

agccacttct gcacggccct ggttagcctc acccacctgt tcgccttcgc cagcgtcaac 360

accattgtcg tggtgtcagt ggatcgctac ttgtccatca tccaccctct ctcctacccg 420

tccaagatga cccagcgccg cggttacctg ctcctctatg gcacctggat tgtggccatc 480

ctgcagagca ctcctccact ctacggctgg ggccaggctg cctttgatga gcgcaatgct 540

ctctgctcca tgatctgggg ggccagcccc agctacacta ttctcagcgt ggtgtccttc 600

atcgtcattc cactgattgt catgattgcc tgctactccg tggtgttctg tgcagcccgg 660

aggcagcatg ctctgctgta caatgtcaag agacacagct tggaagtgcg agtcaaggac 720

tgtgtggaga atgaggatga agagggagca gagaagaagg aggagttcca ggatgagagt 780

gagtttcgcc gccagcatga aggtgaggtc aaggccaagg agggcagaat ggaagccaag 840

gacggcagcc tgaaggccaa ggaaggaagc acggggacca gtgagagtag tgtagaggcc 900

aggggcagcg aggaggtcag agagagcagc acggtggcca gcgacggcag catggagggt 960

aaggaaggca gcaccaaagt tgaggagaac agcatgaagg cagacaaggg tcgcacagag 1020

gtcaaccagt gcagcattga cttgggtgaa gatgacatgg agtttggtga agacgacatc 1080

aatttcagtg aggatgacgt cgaggcagtg aacatcccgg agagcctccc acccagtcgt 1140

cgtaacagca acagcaaccc tcctctgccc aggtgctacc agtgcaaagc tgctaaagtg 1200

atcttcatca tcattttctc ctatgtgcta tccctggggc cctactgctt tttagcagtc 1260

ctggccgtgt gggtggatgt cgaaacccag gtaccccagt gggtgatcac cataatcatc 1320

tggcttttct tcctgcagtg ctgcatccac ccctatgtct atggctacat gcacaagacc 1380

attaagaagg aaatccagga catgctgaag aagttcttct gcaaggaaaa gcccccgaaa 1440

gaagatagcc acccagacct gcccggaaca gagggtggga ctgaaggcaa gattgtccct 1500

tcctacgatt ctgctacttt tccttga 1527

<210> 53
<211> 939
<212> DNA
<213> Homo sapiens

<400> 53
atgatggagc ccagagaagc tggacagcac gtgggggccg ccaacagcgc ccaggaggat 60

gtggccttca acctcatcat cctgtccctc accgaggggc tcggcctcgg tgggctgctg 120

gggaatgggg cagtcctctg gctgctcagc tccaatgtct acagaaaccc cttcgccatc 180

tacctcctgg acgtggcctg cgcggatctc atcttccttg gctgccacat ggtggccatc 240

gtccccgact tgctgcaagg ccggctggac ttcccgggct tcgtgcagac cagcctggca 300

acgctgcgct tcttctgcta catcgtgggc ctgagtctcc tggcggccgt cagcgtggag 360

cagtgcctgg ccgccctctt cccagcctgg tactcgtgcc gccgcccacg ccacctgacc 420

acctgtgtgt gcgccctcac ctgggccctc tgcctgctgc tgcacctgct gctcagcggc 480

gcctgcaccc agttcttcgg ggagcccagc cgccacttgt gccggacgct gtggctggtg 540

gcagcggtgc tgctggctct gctgtgttgc accatgtgtg gggccagcct tatgctgctg 600

ctgcgggtgg agcgaggccc ccagcggccc ccaccccggg gcttccctgg gctcatcctc 660

ctcaccgtcc tcctcttcct cttctgcggc ctgcccttcg gcatctactg gctgtcccgg 720

aacctgctct ggtacatccc ccactacttc taccacttca gcttcctcat ggccgccgtg 780

cactgcgcgg ccaagcccgt cgtctacttc tgcctgggca gtgcccaggg ccgcaggctg 840

cccctccggc tggtcctcca gcgagcgctg ggagacgagg ctgagctggg ggccgtcagg 900

gagacctccc gccggggcct ggtggacata gcagcctga 939

<210> 54
<211> 1778

<212>  DNA
<213>  Homo sapiens

<400>  54
ggagcctcgc cggccgccat gtggagctgc agctggttca acggcacagg gctggtggag      60

gagctgcctg cctgccagga cctgcagctg gggctgtcac tgttgtcgct gctgggcctg     120

gtggtgggcg tgccagtggg cctgtgctac aacgccctgc tggtgctggc caacctacac     180

agcaaggcca gcatgaccat gccggacgtg tactttgtca acatggcagt ggcaggcctg     240

gtgctcagcg ccctggcccc tgtgcacctg ctcggccccc cgagctcccg gtgggcgctg     300

tggagtgtgg gcggcgaagt ccacgtggca ctgcagatcc ccttcaatgt gtcctcactg     360

gtggccatgt actccaccgc cctgctgagc ctcgaccact acatcgagcg tgcactgccg     420

cggacctaca tggccagcgt gtacaacacg cggcacgtgt gcggcttcgt gtggggtggc     480

gcgctgctga ccagcttctc ctcgctgctc ttctacatct gcagccatgt gtccacccgc     540

gcgctagagt gcgccaagat gcagaacgca gaagctgccg acgccacgct ggtgttcatc     600

ggctacgtgg tgccagcact ggccaccctc tacgcgctgg tgctactctc ccgcgtccgc     660

agggaggaca cgcccctgga ccgggacacg ggccggctgg agccctcggc acacaggctg     720

ctggtggcca ccgtgtgcac gcagtttggg ctctggacgc cacactatct gatcctgctg     780

gggcacacgg tcatcatctc gcgagggaag cccgtggacg cacactacct ggggctactg     840

cactttgtga aggatttctc caaactcctg gccttctcca gcagctttgt gacaccactt     900

ctctaccgct acatgaacca gagcttcccc agcaagctcc aacggctgat gaaaaagctg     960

ccctgcgggg accggcactg ctccccggac cacatggggg tgcagcaggt gctggcgtag    1020

gcggcccagc cctcctgggg agacgtgact ctggtggacg cagagcactt agttaccctg    1080

gacgctcccc acatccttcc agaaggagac gagctgctgg aagagaagca ggaggggtgt    1140

ttttcttgaa gtttcctttt tcccacaaat gccactcttg ggccaaggct gtggtccccg    1200

tggctggcat ctggcttgag tctccccgag gcctgtgcgt ctcccaaaca cgcagctcaa    1260

ggtccacatc cgcaaaagcc tcctcgcctt cagcctcctc agcattcagt ttgtcaatga    1320

agtgatgaaa gcttagagcc agtatttata ctttgtggtt aaaatacttg attccccctt    1380

gtttgtttta caaaaacaga tgtttcctag aaaaatgaca aatagtaaaa tgaacaaaac    1440

cctacgaaag aatggcaaca gccagggtgg ccgggccctg ccagtgggcg gcgtgtgcta    1500

gcaaggcctg ccgggtgtgc cgcagtcacc acagggttct gagaacattt cacagaagtg    1560

cctgagacgc ggagacatgg ctggtgttaa atggagctat tcaatagcag tgacgcgctc    1620

tcctcagcca ccaaatgtcc ctgacaccct ccccagcccc cacagataac atcagctgag    1680

gtttttttca gtatgaacct gtcctaaatc aattcctcaa agtgtgcaca aaactaaaga 1740

atataaataa acaaaagaaa ggcaaaaaaa aaaaaaaa 1778

<210> 55
<211> 1014
<212> DNA
<213> Homo sapiens

<400> 55
atggggaacg attctgtcag ctacgagtat ggggattaca gcgacctctc ggaccgccct 60

gtggactgcc tggatggcgc ctgcctggcc atcgacccgc tgcgcgtggc cccgctccca 120

ctgtatgccg ccatcttcct ggtgggggtg ccgggcaatg ccatggtggc ctgggtggct 180

gggaaggtgg cccgccggag ggtgggtgcc acctggttgc tccacctggc cgtggcggat 240

ttgctgtgct gtttgtctct gcccatcctg gcagtgccca ttgcccgtgg aggccactgg 300

ccgtatggtg cagtgggctg tcgggcgctg ccctccatca tcctgctgac catgtatgcc 360

agcgtcctgc tcctggcagc tctcagtgcc gacctctgct tcctggctct cgggcctgcc 420

tggtggtcta cggttcagcg ggcgtgcggg gtgcaggtgg cctgtggggc agcctggaca 480

ctggccttgc tgctcaccgt gccctccgcc atctaccgcc ggctgcacca ggagcacttc 540

ccagcccggc tgcagtgtgt ggtggactac ggcggctcct ccagcaccga gaatgcggtg 600

actgccatcc ggtttctttt tggcttcctg gggcccctgg tggccgtggc cagctgccac 660

agtgccctcc tgtgctgggc agcccgacgc tgccggccgc tgggcacagc cattgtggtg 720

gggttttttg tctgctgggc accctaccac ctgctggggc tggtgctcac tgtggcggcc 780

ccgaactccg cactcctggc cagggccctg cgggctgaac ccctcatcgt gggccttgcc 840

ctcgctcaca gctgcctcaa tcccatgctc ttcctgtatt ttgggagggc tcaactccgc 900

cggtcactgc cagctgcctg tcactgggcc ctgagggagt cccagggcca ggacgaaagt 960

gtggacagca agaaatccac cagccatgac ctggtctcgg agatggaggt gtag 1014

<210> 56
<211> 1170
<212> DNA
<213> Homo sapiens

<400> 56
atggcacctt ctcatcgggc atcacaggtg gggttttgcc ccacccctga acgccctctg 60

tggcgccttc cacccacctg taggcccaga aggatgtcgg tctgctaccg tcccccaggg 120

aacgagacac tgctgagctg gaagacttcg cgggccacag gcacagcctt cctgctgctg 180

gcggcgctgc tggggctgcc tggcaacggc ttcgtggtgt ggagcttggc gggctggcgg 240

cctgcacggg ggcgaccgct ggcggccacg cttgtgctgc acctggcgct ggccgacggc 300

gcggtgctgc tgctcacgcc gctctttgtg gccttcctga cccggcaggc ctggccgctg 360

ggccaggcgg gctgcaaggc ggtgtactac gtgtgcgcgc tcagcatgta cgccagcgtg 420

ctgctcaccg gcctgctcag cctgcagcgc tgcctcgcag tcacccgccc cttcctggcg 480

cctcggctgc gcagcccggc cctggcccgc cgcctgctgc tggcggtctg gctggccgcc 540

ctgttgctcg ccgtcccggc cgccgtctac cgccacctgt ggagggaccg cgtatgccag 600

ctgtgccacc cgtcgccggt ccacgccgcc gcccacctga gcctggagac tctgaccgct 660

ttcgtgcttc ctttcgggct gatgctcggc tgctacagcg tgacgctggc acggctgcgg 720

ggcgcccgct ggggctccgg gcggcacggg gcgcgggtgg gccggctggt gagcgccatc 780

gtgcttgcct tcggcttgct ctgggccccc taccacgcag tcaaccttct gcaggcggtc 840

gcagcgctgg ctccaccgga aggggccttg gcgaagctgg gcggagccgg ccaggcggcg 900

cgagcgggaa ctacggcctt ggccttcttc agttctagcg tcaacccggt gctctacgtc 960

ttcaccgctg gagatctgct gccccgggca ggtccccgtt tcctcacgcg gctcttcgaa 1020

ggctctgggg aggcccgagg gggcggccgc tctagggaag ggaccatgga gctccgaact 1080

acccctcagc tgaaagtggt ggggcagggc cgcggcaatg gagacccggg gggtgggatg 1140

gagaaggacg gtccggaatg ggacctttga 1170

<210> 57
<211> 1965
<212> DNA
<213> Homo sapiens

<400> 57
   ccgccgcgcg gggaggacgc gagcacccag ctttaatccc tggaaagtcc acgaacaatg 60
   aatccatttc atgcatcttg ttggaacacc tctgccgaac ttttaaacaa atcctggaat 120
   aaagagtttg cttatcaaac tgccagtgtg gtagatacag tcatcctccc ttccatgatt 180
   gggattatct gttcaacagg gctggttggc aacatcctca ttgtattcac tataataaga 240
   tccaggaaaa aaacagtccc tgacatctat atctgcaacc tggctgtggc tgatttggtc 300
   cacatagttg gaatgccttt tcttattcac caatggcccg gagggggaga gtgggtgttt 360
   gggggcctct tctgcaccat catcacatcc ctggatactt gtaaccaatt tgcctgtagt 420
   gccatcatga ctgtaatgag tgtggacagg tactttgccc tcgtccaacc atttcgactg 480
   acacgttgga gaacaaggta caagaccatc cggatcaatt tgggcctttg ggcagcttcc 540
   tttatcctgg cattgcctgt ctgggtctac tcgaaggtca tcaaatttaa agacggtgtt 600
   gagagttgtg cttttgattt gacatcccct gacgatgtac tctggtatac actttatttg 660
   acgataacaa cttttttttt ccctctaccc ttgattttgg tgtgctatat tttaatttta 720
   tgctatactt gggagatgta tcaacagaat aaggatgcca gatgctgcaa tcccagtgta 780
   ccaaaacaga gagtgatgaa gttgacaaag atggtgctgg tgctggtggt agtctttatc 840
   ctgagtgctg ccccttatca tgtgatacaa ctggtgaact tacagatgga acagcccaca 900
   ctggccttct atgtgggtta ttacctctcc atctgtctca gctatgccag cagcagcatt 960
   aacccttttc tctacatcct gctgagtgga aatttccaga aacgtctgcc tcaaatccaa 1020
   agaagagcga ctgagaagga aatcaacaat atgggaaaca ctctgaaatc acacttttag 1080
   gaaagtacat ggatcaccat gagtctagac atgattgtct atcttactgg tattattaga 1140
   aagggcaggt gtaccgatat gtttatgccc attcttcttg tgtacttgtg actcttagca 1200
   gcatggaaga gaagtgtaac catgcaaata caatgagctt aatatgctaa ctttagcaag 1260
   atgtaaaatg ttgatctata ttgtgggtag ggaatgggat agtctgagat acccaggctt 1320
   catgatggtg tatattattt cagcatatta taaactagtc actaatgaaa atggccatcc 1380
   atgaccattg actcaaaact caccaaggaa cctgaccttg ccctccacac tgtggcctca 1440
   ctgtaacagt ttcctcaagg ttcctaggag ggtatcacct tagagtgaag tctaaaattt 1500
   ggctattttt tatctataaa aaatgtcagt tttatatggt ccaatactaa taccctcaac 1560
   aactaagccc caccttttag aataagttac catttattgc acacatgcaa tgtgtaagat 1620
   tacatgtaac aaacctgtga aataagtatt attacctttg tttgctaagg ctcagaaagg 1680
   agaaatgata ggcctaatgc tgcaacagct atctaagagc tgagctaaca ttcagctctg 1740
   cctgtttctt ttctactgcc gaccttgaca acctttactt atcatactgg agaacccagt 1800
   aacttggagt ttcttttgct ttctcctgta gccctacaag aggagaacta aagtctgata 1860
   gaaatgagtt gatgttttaa gcatcatttt ggattatctt gttctcacac ctgctaactg 1920
   tagaaactgg catctggact ttaataataa tactttactt ctgga 1965

<210> 58
<211> 1031
<212> DNA
<213> Homo sapiens

<400> 58
ccatgaccag caatttttcc caacctgttg tgcagctttg ctatgaggat gtgaatggat 60

cttgtattga aactccctat tctcctgggt cccgggtaat tctgtacacg gcgtttagct 120

ttgggtcttt gctggctgta tttggaaatc tcttagtaat gacttctgtt cttcatttta 180

agcagctgca ctctccaacc aattttctca ttgcctctct ggcctgtgct gacttcttgg 240

taggtgtgac tgtgatgctt ttcagcatgg tcaggacggt ggagagctgc tggtattttg 300

gagccaaatt ttgtactctt cacagttgct gtgatgtggc attttgttac tcttctgtcc 360

tccacttgtg cttcatctgc atcgacaggt acattgtggt tactgatccc ctggtctatg 420

ctaccaagtt caccgtgtct gtgtcgggaa tttgcatcag cgtgtcctgg attctgcctc 480

tcacgtacag cggtgctgtg ttctacacag gtgtcaatga tgatgggctg gaggaattag 540

taagtgctct caactgcgta ggtggctgtc aaattattgt aagtcaaggc tgggtgttga 600

tagattttct gttattcttc atacctaccc ttgttatgat aattctttac agtaagattt 660

ttcttatagc taaacaacaa gctataaaaa ttgaaactac tagtagcaaa gtagaatcat 720

cctcagagag ttataaaatc agagtggcca agagagagag gaaagcagct aaaaccctgg 780

gggtcacggt actagcattt gttatttcat ggttaccgta tacagttgat atattaattg 840

atgcctttat gggcttcctg acccctgcct atatctatga aatttgctgt tggagtgctt 900

attataactc agccatgaat cctttgattt atgctctatt ttatccttgg tttaggaaag 960

ccataaaact tattttaagt ggagatgttt taaaggctag ttcatcaacc attagtttat 1020

ttttagaata a 1031

<210> 59
<211> 972
<212> DNA
<213> Homo sapiens

<400> 59
atgccactcc ctgtgccccc agcgggggca caaaagactc ctgaggacca tgtctgtctc 60

catcttgcag gtccctcgcc agcaccctct gagccggcca ggatgtttgg gctgttcggc 120

ctctggagaa ccttcgacag tgtggtcttc tacctgacgc tgatcgtggg cctcggggga 180

ccggtaggta acgggctggt gctctggaac ctcggcttcc gcatcaagaa gggccccttc 240

tccatctacc tgctgcacct ggccgccgcc gacttcctgt tcctctcctg ccgtgtgggc 300

ttctccgtgg ctcaggctgc cctgggcgcc caggacacac tctacttcgt gctcaccttc 360

ctgtggttcg cggtggggct ctggctgctg gcggccttca gcgtggagcg ctgcctctcc 420

gacctcttcc ccgcctgcta ccagggctgc cggcccagac acgcctcggc cgtcctctgc 480

gccctggtgt ggacccccac cctgccggcc gtgccgctgc ccgccaacgc ctgcggcctg 540

ctgcgcaaca gcgcgtgccc cctggtctgc ccgcgctacc acgtggccag cgtcacctgg 600

ttcctggtgc tggcccgcgt cgcctggacg gctggcgtgg tcctctttgt ctgggtgacc 660

tgctgctcca ctcgcccgcg gcccaggctc tacggcatcg tcctgggcgc gctgctcctg 720

ctcttcttct gtggcctgcc ctcggtcttc tactggagcc tgcagcccct gctgaacttc 780

ctgctgcccg tgttttcccc gctggccacg ctgctggcct gcgtcaacag cagctccaag 840

cccctcatct actcggggtt gggccgacag cccgggaagc gggagccgct gaggtcggta 900

ctgcggaggg ccctggggga gggcgccgag ctgggtgcca ggggacagtc cctgcccatg 960

ggtctcctat aa 972

<210> 60
<211> 1011
<212> DNA
<213> Homo sapiens

<400> 60
atgaacaaca atacaacatg tattcaacca tctatgatct cttccatggc tttaccaatc 60

atttacatcc tcctttgtat tgttggtgtt tttggaaaca ctctctctca atggatattt 120

ttaacaaaaa taggtaaaaa aacatcaacg cacatctacc tgtcacacct tgtgactgca 180

aacttacttg tgtgcagtgc catgcctttc atgagtatct atttcctgaa aggtttccaa 240

tgggaatatc aatctgctca atgcagagtg gtcaattttc tgggaactct atccatgcat 300

gcaagtatgt ttgtcagtct cttaatttta agttggattg ccataagccg ctatgctacc 360

ttaatgcaaa aggattcctc gcaagagact acttcatgct atgagaaaat attttatggc 420

catttactga aaaaatttcg ccagcccaac tttgctagaa aactatgcat ttacatatgg 480

ggagttgtac tgggcataat cattccagtt accgtatact actcagtcat agaggctaca 540

gaaggagaag agagcctatg ctacaatcgg cagatggaac taggagccat gatctctcag 600

attgcaggtc tcattggaac cacatttatt ggattttcct ttttagtagt actaacatca 660

tactactctt ttgtaagcca tctgagaaaa ataagaacct gtacgtccat tatggagaaa 720

gatttgactt acagttctgt gaaaagacat cttttggtca tccagattct actaatagtt 780

tgcttccttc cttatagtat ttttaaaccc atttttatg ttctacacca aagagataac 840

tgtcagcaat tgaattattt aatagaaaca aaaaacattc tcacctgtct tgcttcggcc 900

agaagtagca cagaccccat tatatttctt ttattagaca aaacattcaa gaagacacta 960

tataatctct ttacaaagtc taattcagca catatgcaat catatggttg a 1011

<210> 61
<211> 180
<212> PRT

<213> Homo sapiens

<400> 61

Tyr Ala Thr Ile Gly Arg Trp Glu Leu Gly Ala Met Ile Ser Gln Ile
1               5                   10                  15

Ala Gly Leu Ile Gly Thr Thr Phe Ile Gly Phe Ser Phe Leu Val Val
            20                  25                  30

Leu Thr Ser Tyr Tyr Ser Phe Val Ser His Leu Arg Lys Ile Arg Thr
        35                  40                  45

Cys Thr Ser Ile Met Glu Lys Asp Leu Thr Tyr Ser Ser Val Lys Arg
    50                  55                  60

His Leu Leu Val Ile Gln Ile Leu Leu Ile Val Cys Phe Leu Pro Tyr
65                  70                  75                  80

Ser Ile Phe Lys Pro Ile Phe Tyr Val Leu His Gln Arg Asp Asn Cys
                85                  90                  95

Gln Gln Leu Asn Tyr Leu Ile Glu Thr Lys Asn Ile Leu Thr Cys Leu
            100                 105                 110

Ala Ser Ala Arg Ser Ser Thr Asp Pro Ile Ile Phe Leu Leu Leu Asp
        115                 120                 125

Lys Thr Phe Lys Lys Thr Leu Tyr Asn Leu Phe Thr Lys Ser Asn Ser
    130                 135                 140

Ala His Met Gln Ser Tyr Gly Leu Leu Asn Gly Lys Pro His Asn Ile
145                 150                 155                 160

Lys Lys Ser Ile His Val Thr Leu Leu Gly Thr Leu Asn Tyr Ile Ile
                165                 170                 175

Asn Met Ser Gln
            180

<210> 62
<211> 530
<212> PRT
<213> Homo sapiens

<400> 62

<210>  63
<211>  313
<212>  PRT
<213>  Homo sapiens

<400>  63

Gly Trp Gln His Arg Leu Ser Arg Leu Cys Glu Lys Asn Gly Gly Thr
1               5                   10                  15

Thr Ser His Cys Cys Val Arg Arg Met Ala Ala Thr Pro Leu Met Val
            20                  25                  30

Val Gly Glu Trp Arg His His Leu Ser Trp Leu Cys Glu Ala Leu Arg
        35                  40                  45

Ala Pro Val Ser Leu Leu Arg Gly Glu Trp Arg His His Leu Ser Trp
    50                  55                  60

Leu Cys Asn Ile Ser Ile Thr Leu Lys Tyr Pro Leu Ile Val Gly Ala
65                  70                  75                  80

Ile Cys Asn Ala Glu Val Val Pro Gln Lys His Phe Asn His Phe His
                85                  90                  95

Phe His Gly Ser Ala Lys Thr Tyr Ala Gly Pro His Arg Ser Gln Arg
            100                 105                 110

Ser His Leu Cys Phe Arg Ala Lys Pro Val Phe Leu Leu Ser Thr Ala
        115                 120                 125

Asn Ile Leu Thr Val Ile Ile Leu Ser Gln Leu Val Ala Arg Arg Gln
    130                 135                 140

Lys Ser Ser Tyr Asn Tyr Leu Leu Ala Leu Ala Ala Ala Asp Ile Leu
145                 150                 155                 160

Val Leu Phe Phe Ile Val Phe Val Asp Phe Leu Leu Glu Asp Phe Ile
                165                 170                 175

Leu Asn Met Gln Met Pro Gln Val Pro Asp Lys Ile Ile Glu Val Leu
            180                 185                 190

Glu Phe Ser Ser Ile His Thr Ser Ile Trp Ile Thr Val Pro Leu Thr
        195                 200                 205

Ile Asp Arg Tyr Ile Ala Val Cys His Pro Leu Lys Tyr His Thr Val
    210                 215                 220

Ser Tyr Pro Ala Arg Thr Arg Lys Val Ile Val Ser Val Tyr Ile Thr
225                 230                 235                 240

Cys Phe Leu Thr Ser Ile Pro Tyr Tyr Trp Trp Pro Asn Ile Trp Thr
                245                 250                 255

Glu Asp Tyr Ile Ser Thr Ser Val His His Val Leu Ile Trp Ile His
            260                 265                 270

Cys Phe Thr Val Tyr Leu Val Pro Cys Ser Ile Phe Phe Ile Leu Asn
        275                 280                 285

Ser Ile Ile Val Tyr Lys Leu Arg Arg Lys Ser Asn Phe Asp Asn Gly
    290                 295                 300

Ser Ile Ala Ser His Gly Cys Val Arg
305                 310

<210> 64
<211> 173
<212> PRT
<213> Homo sapiens

<400> 64

<210>  65
<211>  187
<212>  PRT
<213>  Homo sapiens

<400>  65

<210> 66
<211> 287
<212> PRT
<213> Homo sapiens

<400> 66

Phe Leu Leu Ser Thr Ala Asn Ile Leu Thr Val Ile Ile Leu Ser Gln
1               5                   10                  15

Leu Val Ala Arg Arg Gln Lys Ser Ser Tyr Asn Tyr Leu Leu Ala Leu
            20                  25                  30

Ala Ala Ala Asp Ile Leu Val Leu Phe Phe Ile Val Phe Val Asp Phe
        35                  40                  45

Leu Leu Glu Asp Phe Ile Leu Asn Met Gln Met Pro Gln Val Pro Asp
    50                  55                  60

Lys Ile Ile Glu Val Leu Glu Phe Ser Ser Ile His Thr Ser Ile Trp
65                  70                  75                  80

Ile Thr Val Pro Leu Thr Ile Asp Arg Tyr Ile Ala Val Cys His Pro
                85                  90                  95

Leu Lys Tyr His Thr Val Ser Tyr Pro Ala Arg Thr Arg Lys Val Ile
            100                 105                 110

Val Ser Val Tyr Ile Thr Cys Phe Leu Thr Ser Ile Pro Tyr Tyr Trp
        115                 120                 125

Trp Pro Asn Ile Trp Thr Glu Asp Tyr Ile Ser Thr Ser Val His His
    130                 135                 140

Val Leu Ile Trp Ile His Cys Phe Thr Val Tyr Leu Val Pro Cys Ser
145                 150                 155                 160

Met Phe Phe Ile Leu Asn Ser Ile Ile Val Tyr Lys Leu Arg Arg Lys
                165                 170                 175

Ser Asn Phe Arg Leu Arg Gly Tyr Ser Thr Gly Lys Thr Thr Ala Ile
            180                 185                 190

Leu Phe Thr Ile Thr Ser Ile Phe Ala Thr Leu Trp Ala Pro Arg Ile
        195                 200                 205

Ile Met Ile Leu Tyr His Leu Tyr Gly Ala Pro Ile Gln Asn Arg Trp
    210                 215                 220

Leu Val His Ile Met Ser Asp Ile Ala Asn Met Leu Ala Leu Leu Asn
225                 230                 235                 240

Thr Ala Ile Asn Phe Phe Leu Tyr Cys Phe Ile Ser Lys Arg Phe Arg
                245                 250                 255

Thr Met Ala Ala Ala Thr Leu Lys Ala Phe Phe Lys Cys Gln Lys Gln
            260                 265                 270

Pro Val Gln Phe Tyr Thr Asn His Asn Phe Ser Ile Thr Ser Ser
        275                 280                 285

<210> 67
<211> 271
<212> PRT
<213> Homo sapiens

<400> 67

<210>  68
<211>  204
<212>  PRT
<213>  Homo sapiens

<400>  68

<210>  69
<211>  177
<212>  PRT
<213>  Homo sapiens

<400>  69

Gln Gly His Val Asp Arg Val His Gly Asp Val Pro Thr Cys Thr Pro

1               5                   10                  15

Ala Cys Ser Ser Ser Leu Pro Val Arg Ala Thr Ile Arg Trp Pro Arg
            20                  25                  30

Leu Arg Ala Thr Gly Thr Arg Gly His Gly Arg Gly Asp Cys Cys Gly
        35                  40                  45

Arg Ser Leu Gly Asp Ser Cys Cys Phe Ser Ala Lys Ala Leu Cys Val
    50                  55                  60

Trp Ile Trp Ala Leu Ala Ala Leu Ala Ser Leu Pro Ser Ala Ile Phe
65                  70                  75                  80

Ser Thr Thr Val Lys Val Met Gly Glu Glu Leu Cys Leu Val Arg Phe
                85                  90                  95

Pro Asp Lys Leu Leu Gly Arg Asp Arg Gln Phe Trp Leu Gly Leu Tyr
            100                 105                 110

His Ser Gln Lys Val Leu Leu Gly Phe Val Leu Pro Leu Gly Ile Ile
        115                 120                 125

Ile Leu Cys Tyr Leu Leu Leu Val Arg Phe Ile Ala Asp Arg Arg Ala
    130                 135                 140

Ala Gly Thr Lys Gly Gly Ala Ala Val Ala Gly Gly Arg Pro Thr Gly
145                 150                 155                 160

Ala Ser Ala Arg Arg Leu Ser Lys Val Thr Lys Ser Val Thr Ile Val
                165                 170                 175

Val


<210>  70
<211>  356
<212>  PRT
<213>  Homo sapiens

<400>  70

<210>  71
<211>  187
<212>  PRT
<213>  Homo sapiens

<400>  71

<210>  72
<211>  123
<212>  PRT
<213>  Homo sapiens

<400>  72

Val Thr Gly Thr Leu Glu Gly Asp Ser Phe Pro Gly Glu Glu Gly Thr
1               5                   10                  15

Val Gly Phe Pro Ala Leu Leu Leu Ser Pro Tyr Gly Leu Arg Ser Gly
            20                  25                  30

Leu Asn Cys Asp Glu Ser Leu Cys Pro Cys Ser Leu Gly Phe Leu Lys
        35                  40                  45

Tyr Pro Pro Phe Ile Val Pro Ile Ile Ile Asn Thr Asn Gln Ser Leu
    50                  55                  60

Leu Cys Leu Lys Leu Leu Ile Ala Tyr Arg Gly Lys Cys Arg Leu Leu
65                  70                  75                  80

Ser Trp Arg Thr Ser Leu Leu His Leu Ala His Ile Glu Leu Ser Arg

85                  90                  95

Ala Ile Ser Cys His Ser Leu Ala Cys Leu Leu Leu Leu Trp Val Ser
            100                 105                 110

Arg Thr Ala His Glu Phe Pro His Met Pro Phe
        115                 120

<210>  73
<211>  157
<212>  PRT
<213>  Homo sapiens

<400>  73

<210>  74
<211>  211
<212>  PRT
<213>  Homo sapiens

<400>  74

<210>  75
<211>  120
<212>  PRT
<213>  Homo sapiens

<400>  75

<210> 76
<211> 239
<212> PRT
<213> Homo sapiens

<400> 76

<210> 77
<211> 228
<212> DNA
<213> Homo sapiens

<400> 77
attatctcca ttatgctcta tcagtttctt tatagaatat gattactact tagtgataaa 60

gcttcctttg ctaagatttc agcctacgaa ccatgatcca aaccctactt ctaaaacata 120

taaacatgct ttacaagtat cctatatatg gaaaagtcct tggaattatt tgggtaatta 180

acctggtatt acatgtgtta tttcctgatc tactattgca aattgaca 228


<210> 78
<211> 239
<212> PRT
<213> Homo sapiens

<400> 78

<210>  79
<211>  203
<212>  PRT
<213>  Homo sapiens

<400>  79

<210>  80
<211>  156
<212>  PRT
<213>  Homo sapiens

<400> 80

Ser Lys Ser Leu Leu His Ser Ser Leu Leu Gly Val Leu Val Ile Cys
1               5                   10                  15

Thr Ser Ser Phe Pro Ser Ala Gly Lys Ala Ile Asn Gly Phe Gln Leu
            20                  25                  30

Tyr Ala Ala Pro Leu Gly Cys Ser Phe Asp Leu Ser Gln Val Ser Trp
        35                  40                  45

Ala Ile Glu Asn Asp Leu Ser Ser Cys Arg Gly Leu Gln Pro Ile Leu
    50                  55                  60

Asn Ile Ala Gly Ile Asn Leu Phe Leu Leu Lys Glu Gly Ile Leu Leu
65                  70                  75                  80

Tyr Arg Val Leu Leu Asn Thr Leu Leu Asp Phe Phe Asp Phe Val Phe
                85                  90                  95

Leu Leu Ser Glu Tyr Leu Lys Tyr Pro Cys Arg Tyr Ser Asn Leu Phe
            100                 105                 110

Pro Val Tyr Gly Phe Phe Leu Cys Leu Leu Trp Thr Gln Val Phe Val
        115                 120                 125

Ala Cys Phe Ser Lys Ser Ala Gln Lys Leu Leu Val Ile Gly Phe Gly
    130                 135                 140

Ser Leu Phe Lys Leu Tyr Pro Pro Glu Ser Tyr Arg
145                 150                 155

<210>  81
<211>  208
<212>  PRT
<213>  Homo sapiens

<400>  81

<210>  82
<211>  97
<212>  PRT
<213>  Homo sapiens

<400>  82

<210>  83
<211>  201
<212>  PRT
<213>  Homo sapiens

<400>  83

<210>  84
<211>  212
<212>  PRT
<213>  Homo sapiens

<400>  84

<210> 85
<211> 192
<212> PRT
<213> Homo sapiens

<400> 85

<210>  86
<211>  227
<212>  PRT
<213>  Homo sapiens

<400>  86

<210> 87
<211> 125
<212> PRT
<213> Homo sapiens

<400> 87

<210> 88
<211> 174
<212> PRT
<213> Homo sapiens

<400> 88

<210>  89
<211>  182
<212>  PRT
<213>  Homo sapiens

<400>  89

<210>  90
<211>  188
<212>  PRT

<213>  Homo sapiens

<400>  90

<210>  91
<211>  333
<212>  PRT
<213>  Homo sapiens

<400>  91

Met Trp Ser Cys Ser Trp Phe Asn Gly Thr Gly Leu Val Glu Glu Leu
1               5                   10                  15

Pro Ala Cys Gln Asp Leu Gln Leu Gly Leu Ser Leu Leu Ser Leu Leu
            20                  25                  30

Gly Leu Val Val Gly Val Pro Val Gly Leu Cys Tyr Asn Ala Leu Leu
        35                  40                  45

Val Leu Ala Asn Leu His Ser Lys Ala Ser Met Thr Met Pro Asp Val
    50                  55                  60

Tyr Phe Val Asn Met Ala Val Ala Gly Leu Val Leu Ser Ala Leu Ala
65                  70                  75                  80

Pro Val His Leu Leu Gly Pro Pro Ser Ser Arg Trp Ala Leu Trp Ser
                85                  90                  95

Val Gly Gly Glu Val His Val Ala Leu Gln Ile Pro Phe Asn Val Ser
            100                 105                 110

Ser Leu Val Ala Met Tyr Ser Thr Ala Leu Leu Ser Leu Asp His Tyr
        115                 120                 125

Ile Glu Arg Ala Leu Pro Arg Thr Tyr Met Ala Ser Val Tyr Asn Thr
    130                 135                 140

Arg His Val Cys Gly Phe Val Trp Gly Gly Ala Leu Leu Thr Ser Phe
145                 150                 155                 160

Ser Ser Leu Leu Phe Tyr Ile Cys Ser His Val Ser Thr Arg Ala Leu
                165                 170                 175

Glu Cys Ala Lys Met Gln Asn Ala Glu Ala Ala Asp Ala Thr Leu Val
            180                 185                 190

Phe Ile Gly Tyr Val Val Pro Ala Leu Ala Thr Leu Tyr Ala Leu Val
        195                 200                 205

Leu Leu Ser Arg Val Arg Arg Glu Asp Thr Pro Leu Asp Arg Asp Thr
    210                 215                 220

Gly Arg Leu Glu Pro Ser Ala His Arg Leu Leu Val Ala Thr Val Cys
225                 230                 235                 240

Thr Gln Phe Gly Leu Trp Thr Pro His Tyr Leu Ile Leu Leu Gly His
                245                 250                 255

Thr Gly Ile Ile Ser Arg Gly Lys Pro Val Asp Ala His Tyr Leu Gly
            260                 265                 270

Leu Leu His Phe Val Lys Asp Phe Ser Lys Leu Leu Ala Phe Ser Ser
        275                 280                 285

Ser Phe Val Thr Pro Leu Leu Tyr Arg Tyr Met Asn Gln Ser Phe Pro
    290                 295                 300

Ser Lys Leu Gln Arg Leu Met Lys Lys Leu Pro Cys Gly Asp Arg His
305                 310                 315                 320

Cys Ser Pro Asp His Met Gly Val Gln Gln Val Leu Ala
                325                 330

<210>  92
<211>  97
<212>  PRT
<213>  Homo sapiens

<400>  92

Thr Arg Leu Trp Gly Thr Lys Leu Asn Ala Gly Leu Gly Gln Arg Leu

1               5                   10                  15

Met Pro Cys Gly His Asp Val Met Leu Ser Ile Leu Leu Pro Ser Arg
            20                  25                  30

Gly Ser Arg Ser Gly Ser Arg Arg Gly Ala Leu Leu Leu Glu Gly Ala
        35                  40                  45

Ser Arg Asp Met Glu Lys Val Asp Met Asn Thr Ser Gln Glu Gln Gly
    50                  55                  60

Leu Cys Gln Phe Ser Glu Lys Tyr Lys Gln Val Tyr Leu Ser Leu Ala
65                  70                  75                  80

Tyr Ser Ile Ile Phe Ile Leu Gly Leu Pro Leu Asn Gly Thr Val Leu
                85                  90                  95

Trp


<210>  93
<211>  140
<212>  PRT
<213>  Homo sapiens

<400>  93

Ser Ala Arg Ala His Lys Val Pro Glu Ala His Arg Phe Ser Gly Leu
1               5                   10                  15

Gly Ile Ala Tyr Ser Asn Ser Trp Ala Pro Asn Ser Lys Ser Pro Thr
            20                  25                  30

Thr Leu Ser His Leu Trp Leu Gln Ser Cys Phe Gly Phe Pro Gln Ile
        35                  40                  45

Thr Lys Ala Ser Arg Lys Arg Leu Thr Val Ser Leu Ala Tyr Ser Glu
    50                  55                  60

Ser His Gln Ile Arg Val Ser Gln Gln Asp Phe Arg Leu Phe Arg Thr
65                  70                  75                  80

Leu Phe Leu Leu Met Val Ser Phe Phe Ile Met Trp Ser Pro Ile Ile
                85                  90                  95

Ile Thr Ile Leu Leu Ile Leu Ile Gln Asn Phe Lys Gln Asp Leu Val
            100                 105                 110

Ile Trp Pro Ser Leu Phe Phe Trp Val Val Gly Phe Thr Phe Ala Asn
        115                 120                 125

Ser Ala Leu Asn Pro Ile Leu Tyr Asn Met Thr Leu
    130                 135                 140

<210>  94
<211>  177
<212>  PRT
<213>  Homo sapiens

<400> 94

Asp Ser Val Ser Tyr Glu Tyr Gly Asp Tyr Ser Asp Leu Ser Asp Arg
1               5                   10                  15

Pro Val Asp Cys Leu Asp Gly Ala Cys Leu Ala Ile Asp Pro Leu Arg
            20                  25                  30

Val Ala Pro Leu Pro Leu Tyr Ala Ala Ile Phe Leu Val Gly Val Pro
        35                  40                  45

Gly Asn Ala Met Val Ala Trp Val Ala Gly Lys Val Ala Arg Arg Arg
    50                  55                  60

Val Gly Ala Thr Trp Leu Leu His Leu Ala Val Ala Asp Leu Leu Cys
65                  70                  75                  80

Cys Leu Ser Leu Pro Ile Leu Ala Val Pro Ile Ala Arg Gly Gly His
                85                  90                  95

Pro Tyr Gly Ala Val Gly Cys Arg Ala Leu Pro Ser Ile Ile Leu Leu
            100                 105                 110

Thr Met Tyr Ala Ser Val Leu Leu Leu Ala Ala Leu Ser Ala Asp Leu
        115                 120                 125

Cys Phe Leu Ala Leu Gly Pro Ala Trp Trp Tyr Thr Val Gln Arg Ala
    130                 135                 140

Cys Gly Val Gln Val Ala Cys Gly Ala Ala Trp Thr Leu Ala Leu Leu
145                 150                 155                 160

Leu Thr Val Pro Ser Ala Ile Tyr Arg Arg Leu His Gln Glu His Phe
                165                 170                 175

Pro


<210>  95
<211>  177
<212>  PRT
<213>  Homo sapiens

<400>  95

Ala Pro Leu Ala Phe Arg Gln Gly Ala Leu Gln Ala Asp Gly Val Cys
1               5                   10                  15

Ala Leu His Arg His Leu Arg Gly Val Tyr Leu Met Ala Cys Val Ser
            20                  25                  30

Val Asp His Tyr Pro Ala Val Val Cys Ala His Trp Gly Pro Cys Leu
        35                  40                  45

Arg Thr Ala Gly Arg Ala Arg Leu Val Cys Val Ala Ile Trp Thr Leu
    50                  55                  60

Val Leu Leu Gln Thr Met Pro Leu Leu Leu Met Pro Met Thr Lys Pro
65                  70                  75                  80

Leu Val Gly Lys Leu Ala Cys Met Glu Tyr Ser Ser Met Glu Ser Val
                85                  90                  95

Leu Gly Ala Ala Pro His Gly Pro Gly Gly Leu Cys His Trp Leu Leu
            100                 105                 110

Trp Ala Ser Gly Asp His Pro Val Leu Leu Tyr Glu Asp His Leu Glu
        115                 120                 125

Ala Val Gln His Ser Trp Glu Asn Pro Val Thr Ser Gly Lys Gly His
    130                 135                 140

His Arg Arg Gly Ser Pro Gly Gly Pro Ser Asp Gln Gln Glu Arg Thr
145                 150                 155                 160

Pro Pro Ala Gly Gln Pro Arg Arg Thr Gln Pro Ala Gly Lys Asp Thr
                165                 170                 175

Thr

<210>  96
<211>  358
<212>  PRT
<213>  Homo sapiens

<400>  96

Met Ser Val Cys Tyr Arg Pro Pro Gly Asn Glu Thr Leu Leu Ser Trp
1               5                   10                  15

Lys Thr Ser Arg Ala Thr Gly Thr Ala Phe Leu Leu Leu Ala Ala Leu
            20                  25                  30

Leu Gly Leu Pro Gly Asn Gly Phe Val Val Trp Ser Leu Ala Gly Trp
        35                  40                  45

Arg Pro Ala Arg Gly Arg Pro Leu Ala Ala Thr Leu Val Leu His Leu
    50                  55                  60

Ala Leu Ala Asp Gly Ala Val Leu Leu Leu Thr Pro Leu Phe Val Ala
65                  70                  75                  80

Phe Leu Thr Arg Gln Ala Trp Pro Leu Gly Gln Ala Gly Cys Lys Ala
                85                  90                  95

Val Tyr Tyr Val Cys Ala Leu Ser Met Tyr Ala Ser Val Leu Leu Thr
            100                 105                 110

Gly Leu Leu Ser Leu Gln Arg Cys Leu Ala Val Thr Arg Pro Phe Leu
        115                 120                 125

Ala Pro Arg Leu Arg Ser Pro Ala Leu Ala Arg Arg Leu Leu Leu Ala
    130                 135                 140

Val Trp Leu Ala Ala Leu Leu Leu Ala Val Pro Ala Ala Val Tyr Arg
145                 150                 155                 160

His Leu Trp Arg Asp Arg Val Cys Gln Leu Cys His Pro Ser Pro Val
                165                 170                 175

His Ala Ala Ala His Leu Ser Leu Glu Thr Leu Thr Ala Phe Val Leu
            180                 185                 190

Pro Phe Gly Leu Met Leu Gly Cys Tyr Ser Val Thr Leu Ala Arg Leu
        195                 200                 205

Arg Gly Ala Arg Trp Gly Ser Gly Arg His Gly Ala Arg Val Gly Arg
    210                 215                 220

Leu Val Ser Ala Ile Val Leu Ala Phe Gly Leu Leu Trp Ala Pro Tyr
225                 230                 235                 240

His Ala Val Asn Leu Leu Gln Ala Val Ala Ala Leu Ala Pro Pro Glu
                245                 250                 255

Gly Ala Leu Ala Lys Leu Gly Gly Ala Gly Gln Ala Ala Arg Ala Gly
            260                 265                 270

Thr Thr Ala Leu Ala Phe Phe Ser Ser Ser Val Asn Pro Val Leu Tyr
        275                 280                 285

Val Phe Thr Ala Gly Asp Leu Leu Pro Arg Ala Gly Pro Arg Phe Leu
    290                 295                 300

Thr Arg Leu Phe Glu Gly Ser Gly Glu Ala Arg Gly Gly Gly Arg Ser
305                 310                 315                 320

Arg Glu Gly Thr Met Glu Leu Arg Thr Thr Pro Gln Leu Lys Val Val
                325                 330                 335

Gly Gln Gly Arg Gly Asn Gly Asp Pro Gly Gly Gly Met Glu Lys Asp
            340                 345                 350

Gly Pro Glu Trp Asp Leu
        355

<210>  97
<211>  211
<212>  PRT
<213>  Homo sapiens

<400>  97

Met Pro Leu Pro Val Pro Pro Ala Gly Ala Gln Lys Thr Pro Glu Asp
1               5                   10                  15

His Val Cys Leu His Leu Ala Gly Pro Ser Pro Ala Pro Ser Glu Pro
            20                  25                  30

Ala Arg Met Phe Gly Leu Phe Gly Leu Trp Arg Thr Phe Asp Ser Val
        35                  40                  45

Val Phe Tyr Leu Thr Leu Ile Val Gly Leu Gly Gly Pro Val Gly Asn
    50                  55                  60

Gly Leu Val Leu Trp Asn Leu Gly Phe Arg Ile Lys Lys Gly Pro Phe

65                  70                  75                  80

Ser Ile Tyr Leu Leu His Leu Ala Ala Ala Asp Phe Leu Phe Leu Ser
                85                  90                  95

Cys Arg Val Gly Phe Ser Val Ala Gln Ala Ala Leu Gly Ala Gln Asp
            100                 105                 110

Thr Leu Tyr Phe Val Leu Thr Phe Leu Trp Phe Ala Val Gly Leu Trp
        115                 120                 125

Leu Leu Ala Ala Phe Ser Val Glu Arg Cys Leu Ser Asp Leu Phe Pro
    130                 135                 140

Ala Cys Tyr Gln Gly Cys Arg Pro Arg His Ala Ser Ala Val Leu Cys
145                 150                 155                 160

Ala Leu Val Trp Thr Pro Thr Leu Pro Ala Val Pro Leu Pro Ala Asn
                165                 170                 175

Ala Cys Gly Leu Leu Arg Asn Ser Ala Cys Pro Leu Val Cys Pro Arg
            180                 185                 190

Tyr His Val Ala Ser Val Thr Trp Phe Leu Val Leu Ala Arg Val Ala
        195                 200                 205

Trp Thr Ala
    210

<210>  98
<211>  187
<212>  PRT
<213>  Homo sapiens

<400>  98

Val Tyr Arg Asn Pro Phe Ala Ile Tyr Leu Leu Val Arg Gly Leu Gln
1               5                   10                  15

Gln Asp Leu Ile Phe Leu Gly Cys His Met Val Ala Ile Val Pro Asp
            20                  25                  30

Leu Leu Gln Gly Arg Leu Asp Phe Pro Gly Phe Val Gln Thr Ser Leu
        35                  40                  45

Ala Thr Leu Arg Phe Phe Cys Tyr Ile Val Gly Leu Ser Leu Leu Ala
    50                  55                  60

Ala Val Ser Val Glu Gln Cys Leu Ala Ala Leu Phe Pro Ala Trp Tyr
65                  70                  75                  80

Ser Cys Arg Arg Pro Arg His Leu Thr Thr Cys Val Cys Ala Leu Thr
                85                  90                  95

Trp Ala Leu Cys Leu Leu Leu His Leu Leu Leu Ser Ser Ala Cys Thr
            100                 105                 110

Gln Phe Phe Gly Glu Pro Ser Arg His Leu Cys Arg Thr Leu Trp Leu
        115                 120                 125

Val Ala Ala Val Leu Leu Ala Leu Leu Cys Cys Thr Met Cys Gly Ala
    130                 135                 140

Ser Leu Met Leu Leu Leu Arg Val Glu Arg Gly Pro Gln Arg Pro Pro
145                 150                 155                 160

Pro Arg Gly Phe Pro Gly Leu Ile Leu Pro His Arg Pro Pro Leu Pro
                165                 170                 175

Leu Leu Arg Pro Ala Leu Arg His Leu Leu Ala
            180                 185

<210>  99
<211>  216
<212>  PRT
<213>  Homo sapiens

<400>  99

<210>  100
<211>  181
<212>  PRT
<213>  Homo sapiens

<400>  100

Gln Phe Ser Ile Ala Ser Leu Ala Cys Ala Asp Phe Leu Val Gly Val
1               5                   10                  15

Thr Val Met Leu Phe Ser Met Val Arg Thr Val Glu Ser Cys Trp Tyr
            20                  25                  30

Phe Gly Ala Lys Phe Cys Thr Leu His Ser Cys Cys Asp Val Ala Phe
        35                  40                  45

Cys Tyr Ser Ser Val Leu His Leu Cys Phe Ile Cys Ile Asp Arg Tyr
    50                  55                  60

Ile Val Val Thr Asp Pro Leu Val Tyr Ala Thr Lys Phe Thr Val Ser
65                  70                  75                  80

Val Ser Gly Ile Cys Ile Ser Val Ser Trp Ile Leu Pro Leu Thr Tyr
                85                  90                  95

Ser Gly Ala Val Phe Tyr Thr Gly Val Asn Asp Asp Gly Leu Glu Glu
            100                 105                 110

Leu Val Ser Ala Leu Asn Cys Val Gly Gly Cys Gln Ile Ile Val Ser
        115                 120                 125

Gln Gly Trp Val Leu Ile Asp Phe Leu Leu Phe Phe Ile Pro Thr Leu
    130                 135                 140

Val Met Ile Ile Leu Tyr Ser Lys Ile Phe Leu Ile Ala Lys Gln Gln
145                 150                 155                 160

Ala Ile Lys Ile Glu Thr Thr Ser Ser Lys Val Glu Ser Ser Ser Glu
                165                 170                 175

Ser Tyr Lys Ile Arg
            180

<210>  101
<211>  188
<212>  PRT
<213>  Homo sapiens

<400>  101

<210> 102
<211> 204
<212> PRT
<213> Homo sapiens

<400> 102

<210>  103
<211>  215
<212>  PRT
<213>  Homo sapiens

<400>  103

<210>  104
<211>  194
<212>  PRT
<213>  Homo sapiens

<400>  104

<210>  105
<211>  186
<212>  PRT
<213>  Homo sapiens

<400>  105

<210>  106
<211>  184
<212>  PRT
<213>  Homo sapiens

<400>  106

<210>  107
<211>  170
<212>  PRT
<213>  Homo sapiens

<400>  107

<210>  108
<211>  239
<212>  PRT
<213>  Homo sapiens

<400>  108

Thr Arg Asn Thr Leu Gly Cys Pro Ser Ala Pro Gly Phe Phe Cys Trp
1               5                   10                  15

Ser Cys Leu Ala Met Cys Leu Gly Leu Lys Val Ser Arg Leu Pro Gly
            20                  25                  30

Ser Pro Gly Ser Ser Arg Lys Arg Asn Glu His Met Met Val Thr Trp
        35                  40                  45

Asn Ser Pro Arg Trp Arg His Cys Ile Phe Ala Lys Pro Val Thr Val
    50                  55                  60

Leu Ser Ala Phe Trp Ala Pro Arg Leu Ser Pro Leu Ile Phe Pro Asp
65                  70                  75                  80

Leu Ser Phe Pro Ala Ala Phe Leu Phe Phe Leu Ile Thr Val Lys Phe
                85                  90                  95

Cys Met Tyr Cys Ser Ile Phe His Leu Leu Gly Ile Glu Tyr Ile Ser
            100                 105                 110

Ser Met Pro Gly Phe Lys Ile Arg Ile Val Asn Ile Val Val Cys Ala
        115                 120                 125

Leu Val Thr Glu Phe Leu Arg Phe Gly Cys Ser Ile Pro Ala Pro Tyr
    130                 135                 140

Phe Leu Lys Ala Leu Leu Ser Ala Val Gly Asp Phe Ala Gln Cys Lys
145                 150                 155                 160

Leu Leu Arg Tyr Phe Leu Leu Ser Ser Arg Ser Pro Tyr Pro Thr Ser
                165                 170                 175

Thr Gln His Leu Ile Leu Arg Cys Ser Pro Gln Thr Cys Glu Asn Gln
            180                 185                 190

His Val Asn Met Ser Ile Pro Leu Ala Gly Phe Pro Asn Ser Thr Asp
        195                 200                 205

Gly Ile Arg Pro Ile Val Gln Ala Lys Ser Lys Ala Pro Ala Gly Thr
    210                 215                 220

Phe Pro Ile Pro Asn Leu Ser Ser Cys Pro Ile Lys Phe Tyr Gln
225                 230                 235

<210>  109
<211>  202
<212>  PRT
<213>  Homo sapiens

<400> 109

<210>  110
<211>  192
<212>  PRT
<213>  Homo sapiens

<400>  110

<210>  111
<211>  311
<212>  PRT
<213>  Homo sapiens

<400>  111

Met Met Glu Pro Arg Glu Ala Gly Gln His Val Gly Ala Ala Asn Gly
1               5                   10                  15

Ala Gln Glu Asp Val Ala Phe Asn Leu Ile Ile Leu Ser Leu Thr Glu
            20                  25                  30

Gly Leu Gly Leu Gly Gly Leu Leu Gly Asn Gly Ala Val Leu Trp Leu
        35                  40                  45

Leu Ser Ser Asn Val Tyr Arg Asn Pro Phe Ala Ile Tyr Leu Leu Asp
    50                  55                  60

Val Ala Cys Ala Asp Leu Ile Phe Leu Gly Cys His Met Val Ala Ile
65                  70                  75                  80

Val Pro Asp Leu Leu Gln Gly Arg Leu Asp Phe Pro Gly Phe Val Gln
                85                  90                  95

Thr Ser Leu Ala Thr Leu Arg Phe Cys Tyr Ile Val Gly Leu Ser Leu
            100                 105                 110

Leu Ala Ala Val Ser Val Glu Gln Cys Leu Ala Ala Leu Phe Pro Ala
        115                 120                 125

Trp Tyr Ser Cys Arg Arg Pro Arg His Leu Thr Thr Cys Val Cys Ala
    130                 135                 140

Leu Thr Trp Ala Leu Cys Leu Leu Leu His Leu Leu Leu Ser Gly Ala
145                 150                 155                 160

Cys Thr Gln Phe Phe Gly Glu Pro Ser Arg His Leu Cys Arg Thr Leu
                165                 170                 175

Trp Leu Val Ala Ala Val Leu Leu Ala Leu Leu Cys Cys Thr Met Cys
            180                 185                 190

Gly Ala Ser Leu Met Leu Leu Leu Arg Val Glu Arg Gly Pro Gln Arg
        195                 200                 205

Pro Pro Pro Arg Gly Phe Pro Gly Leu Ile Leu Leu Thr Val Leu Leu
    210                 215                 220

Phe Leu Phe Cys Gly Leu Pro Phe Gly Ile Tyr Trp Leu Ser Arg Asn
225                 230                 235                 240

Leu Leu Trp Tyr Ile Pro His Tyr Phe Tyr His Phe Ser Phe Leu Met
                245                 250                 255

Ala Ala Val His Cys Ala Ala Lys Pro Val Val Tyr Phe Cys Leu Gly
            260                 265                 270

Ser Ala Gln Gly Arg Arg Leu Pro Leu Arg Leu Val Leu Gln Arg Ala
        275                 280                 285

Leu Gly Asp Glu Ala Glu Leu Gly Ala Val Arg Glu Thr Ser Arg Arg
    290                 295                 300

Gly Leu Val Asp Ile Ala Ala
305                 310

<210>  112
<211>  508
<212>  PRT
<213>  Homo sapiens

<400>  112

Met Thr Ser Thr Cys Thr Asn Ser Thr Arg Glu Ser Asn Ser Ser His
1               5                   10                  15

Thr Cys Met Pro Leu Ser Lys Met Pro Ile Ser Leu Ala His Gly Ile
            20                  25                  30

Ile Arg Ser Thr Val Leu Val Ile Phe Leu Ala Ala Ser Phe Val Gly
        35                  40                  45

Asn Ile Val Leu Ala Leu Val Leu Gln Arg Lys Pro Gln Leu Leu Gln
    50                  55                  60

Val Thr Asn Arg Phe Ile Phe Asn Leu Leu Val Thr Asp Leu Leu Gln
65                  70                  75                  80

Ile Ser Leu Val Ala Pro Trp Val Val Ala Thr Ser Val Pro Leu Phe
                85                  90                  95

Trp Pro Leu Asn Ser His Phe Cys Thr Ala Leu Val Ser Leu Thr His

            100                 105                 110

Leu Phe Ala Phe Ala Ser Val Asn Thr Ile Val Val Val Ser Val Asp
        115                 120                 125

Arg Tyr Leu Ser Ile Ile His Pro Leu Ser Tyr Pro Ser Lys Met Thr
    130                 135                 140

Gln Arg Arg Gly Tyr Leu Leu Leu Tyr Gly Thr Trp Ile Val Ala Ile
145                 150                 155                 160

Leu Gln Ser Thr Pro Pro Leu Tyr Gly Trp Gly Gln Ala Ala Phe Asp
                165                 170                 175

Glu Arg Asn Ala Leu Cys Ser Met Ile Trp Gly Ala Ser Pro Ser Tyr
            180                 185                 190

Thr Ile Leu Ser Val Val Ser Phe Ile Val Ile Pro Leu Ile Val Met
        195                 200                 205

Ile Ala Cys Tyr Ser Val Val Phe Cys Ala Ala Arg Arg Gln His Ala
    210                 215                 220

Leu Leu Tyr Asn Val Lys Arg His Ser Leu Glu Val Arg Val Lys Asp
225                 230                 235                 240

Cys Val Glu Asn Glu Asp Glu Glu Gly Ala Glu Lys Lys Glu Glu Phe
                245                 250                 255

Gln Asp Glu Ser Glu Phe Arg Arg Gln His Glu Gly Glu Val Lys Ala
            260                 265                 270

Lys Glu Gly Arg Met Glu Ala Lys Asp Gly Ser Leu Lys Ala Lys Glu
        275                 280                 285

Gly Ser Thr Gly Thr Ser Glu Ser Ser Val Glu Ala Arg Gly Ser Glu
    290                 295                 300

Glu Val Arg Glu Ser Ser Thr Val Ala Ser Asp Gly Ser Met Glu Gly
305                 310                 315                 320

Lys Glu Gly Ser Thr Lys Val Glu Glu Asn Ser Met Lys Ala Asp Lys
                325                 330                 335

Gly Arg Thr Glu Val Asn Gln Cys Ser Ile Asp Leu Gly Glu Asp Asp
            340                 345                 350

Met Glu Phe Gly Glu Asp Asp Ile Asn Phe Ser Glu Asp Asp Val Glu
        355                 360                 365

Ala Val Asn Ile Pro Glu Ser Leu Pro Pro Ser Arg Arg Asn Ser Asn
    370                 375                 380

Ser Asn Pro Pro Leu Pro Arg Cys Tyr Gln Cys Lys Ala Ala Lys Val
385                 390                 395                 400

Ile Phe Ile Ile Ile Phe Ser Tyr Val Leu Ser Leu Gly Pro Tyr Cys
                405                 410                 415

Phe Leu Ala Val Leu Ala Val Trp Val Asp Val Glu Thr Gln Val Pro
            420                 425                 430

Gln Trp Val Ile Thr Ile Ile Ile Trp Leu Phe Phe Leu Gln Cys Cys
        435                 440                 445

Ile His Pro Tyr Val Tyr Gly Tyr Met His Lys Thr Ile Lys Lys Glu
    450                 455                 460

Ile Gln Asp Met Leu Lys Lys Phe Phe Cys Lys Glu Lys Pro Pro Lys
465                 470                 475                 480

Glu Asp Ser His Pro Asp Leu Pro Gly Thr Glu Gly Gly Thr Glu Gly
                485                 490                 495

Lys Ile Val Pro Ser Tyr Asp Ser Ala Thr Phe Pro
            500                 505

<210>  113
<211>  311
<212>  PRT
<213>  Homo sapiens

<400>  113

Met Met Glu Pro Arg Glu Ala Gly Gln His Val Gly Ala Ala Asn Ser
1               5                   10                  15

Ala Gln Glu Asp Val Ala Phe Asn Leu Ile Ile Leu Ser Leu Thr Glu
            20                  25                  30

Gly Leu Gly Leu Gly Gly Leu Leu Gly Asn Gly Ala Val Leu Trp Leu
        35                  40                  45

Leu Ser Ser Asn Val Tyr Arg Asn Pro Phe Ala Ile Tyr Leu Leu Asp
    50                  55                  60

Val Ala Cys Ala Asp Leu Ile Phe Leu Gly Cys His Met Val Ala Ile
65                  70                  75                  80

Val Pro Asp Leu Leu Gln Gly Arg Leu Asp Phe Pro Gly Phe Val Gln
                85                  90                  95

Thr Ser Leu Ala Thr Leu Arg Phe Cys Tyr Ile Val Gly Leu Ser Leu
            100                 105                 110

Leu Ala Ala Val Ser Val Glu Gln Cys Leu Ala Ala Leu Phe Pro Ala
        115                 120                 125

Trp Tyr Ser Cys Arg Arg Pro Arg His Leu Thr Thr Cys Val Cys Ala
    130                 135                 140

Leu Thr Trp Ala Leu Cys Leu Leu Leu His Leu Leu Leu Ser Gly Ala
145                 150                 155                 160

Cys Thr Gln Phe Phe Gly Glu Pro Ser Arg His Leu Cys Arg Thr Leu
                165                 170                 175

Trp Leu Val Ala Ala Val Leu Leu Ala Leu Leu Cys Cys Thr Met Cys

180                 185                 190

Gly Ala Ser Leu Met Leu Leu Leu Arg Val Glu Arg Gly Pro Gln Arg
        195                 200                 205

Pro Pro Pro Arg Gly Phe Pro Gly Leu Ile Leu Leu Thr Val Leu Leu
    210                 215                 220

Phe Leu Phe Cys Gly Leu Pro Phe Gly Ile Tyr Trp Leu Ser Arg Asn
225                 230                 235                 240

Leu Leu Trp Tyr Ile Pro His Tyr Phe Tyr His Phe Ser Phe Leu Met
                245                 250                 255

Ala Ala Val His Cys Ala Ala Lys Pro Val Val Tyr Phe Cys Leu Gly
            260                 265                 270

Ser Ala Gln Gly Arg Arg Leu Pro Leu Arg Leu Val Leu Gln Arg Ala
        275                 280                 285

Leu Gly Asp Glu Ala Glu Leu Gly Ala Val Arg Glu Thr Ser Arg Arg
    290                 295                 300

Gly Leu Val Asp Ile Ala Ala
305                 310

<210>  114
<211>  333
<212>  PRT
<213>  Homo sapiens

<400>  114

Met Trp Ser Cys Ser Trp Phe Asn Gly Thr Gly Leu Val Glu Glu Leu
1               5                   10                  15

Pro Ala Cys Gln Asp Leu Gln Leu Gly Leu Ser Leu Leu Ser Leu Leu
            20                  25                  30

Gly Leu Val Val Gly Val Pro Val Gly Leu Cys Tyr Asn Ala Leu Leu
        35                  40                  45

Val Leu Ala Asn Leu His Ser Lys Ala Ser Met Thr Met Pro Asp Val
    50                  55                  60

Tyr Phe Val Asn Met Ala Val Ala Gly Leu Val Leu Ser Ala Leu Ala
65                  70                  75                  80

Pro Val His Leu Leu Gly Pro Pro Ser Ser Arg Trp Ala Leu Trp Ser
                85                  90                  95

Val Gly Gly Glu Val His Val Ala Leu Gln Ile Pro Phe Asn Val Ser
            100                 105                 110

Ser Leu Val Ala Met Tyr Ser Thr Ala Leu Leu Ser Leu Asp His Tyr
        115                 120                 125

Ile Glu Arg Ala Leu Pro Arg Thr Tyr Met Ala Ser Val Tyr Asn Thr
    130                 135                 140

Arg His Val Cys Gly Phe Val Trp Gly Gly Ala Leu Leu Thr Ser Phe
145                 150                 155                 160

Ser Ser Leu Leu Phe Tyr Ile Cys Ser His Val Ser Thr Arg Ala Leu
                165                 170                 175

Glu Cys Ala Lys Met Gln Asn Ala Glu Ala Ala Asp Ala Thr Leu Val
            180                 185                 190

Phe Ile Gly Tyr Val Val Pro Ala Leu Ala Thr Leu Tyr Ala Leu Val
        195                 200                 205

Leu Leu Ser Arg Val Arg Arg Glu Asp Thr Pro Leu Asp Arg Asp Thr
    210                 215                 220

Gly Arg Leu Glu Pro Ser Ala His Arg Leu Leu Val Ala Thr Val Cys
225                 230                 235                 240

Thr Gln Phe Gly Leu Trp Thr Pro His Tyr Leu Ile Leu Leu Gly His
                245                 250                 255

Thr Val Ile Ile Ser Arg Gly Lys Pro Val Asp Ala His Tyr Leu Gly
            260                 265                 270

Leu Leu His Phe Val Lys Asp Phe Ser Lys Leu Leu Ala Phe Ser Ser
        275                 280                 285

Ser Phe Val Thr Pro Leu Leu Tyr Arg Tyr Met Asn Gln Ser Phe Pro
    290                 295                 300

Ser Lys Leu Gln Arg Leu Met Lys Lys Leu Pro Cys Gly Asp Arg His
305                 310                 315                 320

Cys Ser Pro Asp His Met Gly Val Gln Gln Val Leu Ala
                325                 330

<210>  115
<211>  337
<212>  PRT
<213>  Homo sapiens

<400>  115

<210>  116
<211>  389
<212>  PRT
<213>  Homo sapiens

<400>  116

Met Ala Pro Ser His Arg Ala Ser Gln Val Gly Phe Cys Pro Thr Pro

1               5                   10                  15

Glu Arg Pro Leu Trp Arg Leu Pro Pro Thr Cys Arg Pro Arg Arg Met
            20                  25                  30

Ser Val Cys Tyr Arg Pro Pro Gly Asn Glu Thr Leu Leu Ser Trp Lys
        35                  40                  45

Thr Ser Arg Ala Thr Gly Thr Ala Phe Leu Leu Leu Ala Ala Leu Leu
    50                  55                  60

Gly Leu Pro Gly Asn Gly Phe Val Val Trp Ser Leu Ala Gly Trp Arg
65                  70                  75                  80

Pro Ala Arg Gly Arg Pro Leu Ala Ala Thr Leu Val Leu His Leu Ala
                85                  90                  95

Leu Ala Asp Gly Ala Val Leu Leu Leu Thr Pro Leu Phe Val Ala Phe
            100                 105                 110

Leu Thr Arg Gln Ala Trp Pro Leu Gly Gln Ala Gly Cys Lys Ala Val
        115                 120                 125

Tyr Tyr Val Cys Ala Leu Ser Met Tyr Ala Ser Val Leu Leu Thr Gly
    130                 135                 140

Leu Leu Ser Leu Gln Arg Cys Leu Ala Val Thr Arg Pro Phe Leu Ala
145                 150                 155                 160

Pro Arg Leu Arg Ser Pro Ala Leu Ala Arg Arg Leu Leu Leu Ala Val
                165                 170                 175

Trp Leu Ala Ala Leu Leu Leu Ala Val Pro Ala Ala Val Tyr Arg His
            180                 185                 190

Leu Trp Arg Asp Arg Val Cys Gln Leu Cys His Pro Ser Pro Val His
        195                 200                 205

Ala Ala Ala His Leu Ser Leu Glu Thr Leu Thr Ala Phe Val Leu Pro
    210                 215                 220

Phe Gly Leu Met Leu Gly Cys Tyr Ser Val Thr Leu Ala Arg Leu Arg
225                 230                 235                 240

Gly Ala Arg Trp Gly Ser Gly Arg His Gly Ala Arg Val Gly Arg Leu
                245                 250                 255

Val Ser Ala Ile Val Leu Ala Phe Gly Leu Leu Trp Ala Pro Tyr His
            260                 265                 270

Ala Val Asn Leu Leu Gln Ala Val Ala Ala Leu Ala Pro Pro Glu Gly
        275                 280                 285

Ala Leu Ala Lys Leu Gly Gly Ala Gly Gln Ala Ala Arg Ala Gly Thr
    290                 295                 300

Thr Ala Leu Ala Phe Phe Ser Ser Ser Val Asn Pro Val Leu Tyr Val
305                 310                 315                 320

Phe Thr Ala Gly Asp Leu Leu Pro Arg Ala Gly Pro Arg Phe Leu Thr
                325                 330                 335

Arg Leu Phe Glu Gly Ser Gly Glu Ala Arg Gly Gly Gly Arg Ser Arg
            340                 345                 350

Glu Gly Thr Met Glu Leu Arg Thr Thr Pro Gln Leu Lys Val Val Gly
        355                 360                 365

Gln Gly Arg Gly Asn Gly Asp Pro Gly Gly Gly Met Glu Lys Asp Gly
    370                 375                 380

Pro Glu Trp Asp Leu
385

<210> 117
<211> 340
<212> PRT
<213> Homo sapiens

<400> 117

Met Asn Pro Phe His Ala Ser Cys Trp Asn Thr Ser Ala Glu Leu Leu
1               5                   10                  15

Asn Lys Ser Trp Asn Lys Glu Phe Ala Tyr Gln Thr Ala Ser Val Val
            20                  25                  30

Asp Thr Val Ile Leu Pro Ser Met Ile Gly Ile Ile Cys Ser Thr Gly
        35                  40                  45

Leu Val Gly Asn Ile Leu Ile Val Phe Thr Ile Ile Arg Ser Arg Lys
    50                  55                  60

Lys Thr Val Pro Asp Ile Tyr Ile Cys Asn Leu Ala Val Ala Asp Leu
65                  70                  75                  80

Val His Ile Val Gly Met Pro Phe Leu Ile His Gln Trp Ala Arg Gly
                85                  90                  95

Gly Glu Trp Val Phe Gly Gly Pro Leu Cys Thr Ile Ile Thr Ser Leu
            100                 105                 110

Asp Thr Cys Asn Gln Phe Ala Cys Ser Ala Ile Met Thr Val Met Ser
        115                 120                 125

Val Asp Arg Tyr Phe Ala Leu Val Gln Pro Phe Arg Leu Thr Arg Trp
    130                 135                 140

Arg Thr Arg Tyr Lys Thr Ile Arg Ile Asn Leu Gly Leu Trp Ala Ala
145                 150                 155                 160

Ser Phe Ile Leu Ala Leu Pro Val Trp Val Tyr Ser Lys Val Ile Lys
                165                 170                 175

Phe Lys Asp Gly Val Glu Ser Cys Ala Phe Asp Leu Thr Ser Pro Asp
            180                 185                 190

Asp Val Leu Trp Tyr Thr Leu Tyr Leu Thr Ile Thr Thr Phe Phe Phe

195                 200                 205

Pro Leu Pro Leu Ile Leu Val Cys Tyr Ile Leu Ile Leu Cys Tyr Thr
    210                 215                 220

Trp Glu Met Tyr Gln Gln Asn Lys Asp Ala Arg Cys Cys Asn Pro Ser
225                 230                 235                 240

Val Pro Lys Gln Arg Val Met Lys Leu Thr Lys Met Val Leu Val Leu
                245                 250                 255

Val Val Val Phe Ile Leu Ser Ala Ala Pro Tyr His Val Ile Gln Leu
            260                 265                 270

Val Asn Leu Gln Met Glu Gln Pro Thr Leu Ala Phe Tyr Val Gly Tyr
        275                 280                 285

Tyr Leu Ser Ile Cys Leu Ser Tyr Ala Ser Ser Ser Ile Asn Pro Phe
    290                 295                 300

Leu Tyr Ile Leu Leu Ser Gly Asn Phe Gln Lys Arg Leu Pro Gln Ile
305                 310                 315                 320

Gln Arg Arg Ala Thr Glu Lys Glu Ile Asn Asn Met Gly Asn Thr Leu
                325                 330                 335

Lys Ser His Phe
            340

<210>  118
<211>  342
<212>  PRT
<213>  Homo sapiens

<400>  118

Met Thr Ser Asn Phe Ser Gln Pro Val Val Gln Leu Cys Tyr Glu Asp
1               5                   10                  15

Val Asn Gly Ser Cys Ile Glu Thr Pro Tyr Ser Pro Gly Ser Arg Val
            20                  25                  30

Ile Leu Tyr Thr Ala Phe Ser Phe Gly Ser Leu Leu Ala Val Phe Gly
        35                  40                  45

Asn Leu Leu Val Met Thr Ser Val Leu His Phe Lys Gln Leu His Ser
    50                  55                  60

Pro Thr Asn Phe Leu Ile Ala Ser Leu Ala Cys Ala Asp Phe Leu Val
65                  70                  75                  80

Gly Val Thr Val Met Leu Phe Ser Met Val Arg Thr Val Glu Ser Cys
                85                  90                  95

Trp Tyr Phe Gly Ala Lys Phe Cys Thr Leu His Ser Cys Cys Asp Val
            100                 105                 110

Ala Phe Cys Tyr Ser Ser Val Leu His Leu Cys Phe Ile Cys Ile Asp
        115                 120                 125

Arg Tyr Ile Val Val Thr Asp Pro Leu Val Tyr Ala Thr Lys Phe Thr
    130                 135                 140

Val Ser Val Ser Gly Ile Cys Ile Ser Val Ser Trp Ile Leu Pro Leu
145                 150                 155                 160

Thr Tyr Ser Gly Ala Val Phe Tyr Thr Gly Val Asn Asp Asp Gly Leu
                165                 170                 175

Glu Glu Leu Val Ser Ala Leu Asn Cys Val Gly Gly Cys Gln Ile Ile
            180                 185                 190

Val Ser Gln Gly Trp Val Leu Ile Asp Phe Leu Leu Phe Phe Ile Pro
        195                 200                 205

Thr Leu Val Met Ile Ile Leu Tyr Ser Lys Ile Phe Leu Ile Ala Lys
    210                 215                 220

Gln Gln Ala Ile Lys Ile Glu Thr Thr Ser Ser Lys Val Glu Ser Ser
225                 230                 235                 240

Ser Glu Ser Tyr Lys Ile Arg Val Ala Lys Arg Glu Arg Lys Ala Ala
                245                 250                 255

Lys Thr Leu Gly Val Thr Val Leu Ala Phe Val Ile Ser Trp Leu Pro
            260                 265                 270

Tyr Thr Val Asp Ile Leu Ile Asp Ala Phe Met Gly Phe Leu Thr Pro
        275                 280                 285

Ala Tyr Ile Tyr Glu Ile Cys Cys Trp Ser Ala Tyr Tyr Asn Ser Ala
    290                 295                 300

Met Asn Pro Leu Ile Tyr Ala Leu Phe Tyr Pro Trp Phe Arg Lys Ala
305                 310                 315                 320

Ile Lys Leu Ile Leu Ser Gly Asp Val Leu Lys Ala Ser Ser Ser Thr
                325                 330                 335

Ile Ser Leu Phe Leu Glu
            340

<210>  119
<211>  323
<212>  PRT
<213>  Homo sapiens

<400>  119

Met Pro Leu Pro Val Pro Pro Ala Gly Ala Gln Lys Thr Pro Glu Asp
1               5                   10                  15

His Val Cys Leu His Leu Ala Gly Pro Ser Pro Ala Pro Ser Glu Pro
            20                  25                  30

Ala Arg Met Phe Gly Leu Phe Gly Leu Trp Arg Thr Phe Asp Ser Val
        35                  40                  45

Val Phe Tyr Leu Thr Leu Ile Val Gly Leu Gly Gly Pro Val Gly Asn
    50                  55                  60

Gly Leu Val Leu Trp Asn Leu Gly Phe Arg Ile Lys Lys Gly Pro Phe
65                  70                  75                  80

Ser Ile Tyr Leu Leu His Leu Ala Ala Ala Asp Phe Leu Phe Leu Ser
                85                  90                  95

Cys Arg Val Gly Phe Ser Val Ala Gln Ala Ala Leu Gly Ala Gln Asp
            100                 105                 110

Thr Leu Tyr Phe Val Leu Thr Phe Leu Trp Phe Ala Val Gly Leu Trp
        115                 120                 125

Leu Leu Ala Ala Phe Ser Val Glu Arg Cys Leu Ser Asp Leu Phe Pro
    130                 135                 140

Ala Cys Tyr Gln Gly Cys Arg Pro Arg His Ala Ser Ala Val Leu Cys
145                 150                 155                 160

Ala Leu Val Trp Thr Pro Thr Leu Pro Ala Val Pro Leu Pro Ala Asn
                165                 170                 175

Ala Cys Gly Leu Leu Arg Asn Ser Ala Cys Pro Leu Val Cys Pro Arg
            180                 185                 190

Tyr His Val Ala Ser Val Thr Trp Phe Leu Val Leu Ala Arg Val Ala
        195                 200                 205

Trp Thr Ala Gly Val Val Leu Phe Val Trp Val Thr Cys Cys Ser Thr
    210                 215                 220

Arg Pro Arg Pro Arg Leu Tyr Gly Ile Val Leu Gly Ala Leu Leu Leu
225                 230                 235                 240

Leu Phe Phe Cys Gly Leu Pro Ser Val Phe Tyr Trp Ser Leu Gln Pro
                245                 250                 255

Leu Leu Asn Phe Leu Leu Pro Val Phe Ser Pro Leu Ala Thr Leu Leu
            260                 265                 270

Ala Cys Val Asn Ser Ser Ser Lys Pro Leu Ile Tyr Ser Gly Leu Gly
        275                 280                 285

Arg Gln Pro Gly Lys Arg Glu Pro Leu Arg Ser Val Leu Arg Arg Ala
    290                 295                 300

Leu Gly Glu Gly Ala Glu Leu Gly Ala Arg Gly Gln Ser Leu Pro Met
305                 310                 315                 320

Gly Leu Leu


<210>  120
<211>  336
<212>  PRT
<213>  Homo sapiens

<400> 120

Met Asn Asn Asn Thr Thr Cys Ile Gln Pro Ser Met Ile Ser Ser Met
1               5                   10                  15

Ala Leu Pro Ile Ile Tyr Ile Leu Leu Cys Ile Val Gly Val Phe Gly
            20                  25                  30

Asn Thr Leu Ser Gln Trp Ile Phe Leu Thr Lys Ile Gly Lys Lys Thr
        35                  40                  45

Ser Thr His Ile Tyr Leu Ser His Leu Val Thr Ala Asn Leu Leu Val
    50                  55                  60

Cys Ser Ala Met Pro Phe Met Ser Ile Tyr Phe Leu Lys Gly Phe Gln
65                  70                  75                  80

Trp Glu Tyr Gln Ser Ala Gln Cys Arg Val Val Asn Phe Leu Gly Thr
                85                  90                  95

Leu Ser Met His Ala Ser Met Phe Val Ser Leu Leu Ile Leu Ser Trp
            100                 105                 110

Ile Ala Ile Ser Arg Tyr Ala Thr Leu Met Gln Lys Asp Ser Ser Gln
        115                 120                 125

Glu Thr Thr Ser Cys Tyr Glu Lys Ile Phe Tyr Gly His Leu Leu Lys
    130                 135                 140

Lys Phe Arg Gln Pro Asn Phe Ala Arg Lys Leu Cys Ile Tyr Ile Trp
145                 150                 155                 160

Gly Val Val Leu Gly Ile Ile Ile Pro Val Thr Val Tyr Tyr Ser Val
                165                 170                 175

Ile Glu Ala Thr Glu Gly Glu Glu Ser Leu Cys Tyr Asn Arg Gln Met
            180                 185                 190

Glu Leu Gly Ala Met Ile Ser Gln Ile Ala Gly Leu Ile Gly Thr Thr
        195                 200                 205

Phe Ile Gly Phe Ser Phe Leu Val Val Leu Thr Ser Tyr Tyr Ser Phe
    210                 215                 220

Val Ser His Leu Arg Lys Ile Arg Thr Cys Thr Ser Ile Met Glu Lys
225                 230                 235                 240

Asp Leu Thr Tyr Ser Ser Val Lys Arg His Leu Leu Val Ile Gln Ile
                245                 250                 255

Leu Leu Ile Val Cys Phe Leu Pro Tyr Ser Ile Phe Lys Pro Ile Phe
            260                 265                 270

Tyr Val Leu His Gln Arg Asp Asn Cys Gln Gln Leu Asn Tyr Leu Ile
        275                 280                 285

Glu Thr Lys Asn Ile Leu Thr Cys Leu Ala Ser Ala Arg Ser Ser Thr
    290                 295                 300

Asp Pro Ile Ile Phe Leu Leu Leu Asp Lys Thr Phe Lys Lys Thr Leu
305                 310                 315                 320

Tyr Asn Leu Phe Thr Lys Ser Asn Ser Ala His Met Gln Ser Tyr Gly
                325                 330                 335

<210>  121
<211>  28
<212>  DNA
<213>  Artificial

<220>
<223>  Primer

<400>  121
ttcaaagctt atgacgtcca cctgcacc                                          28

<210>  122
<211>  35
<212>  DNA
<213>  Artificial

<220>
<223>  Primer

<400>  122
ttcactcgag tcaaggaaaa gtagcagaat cgtag                                  35

<210>  123
<211>  35
<212>  DNA
<213>  Artificial

<220>
<223>  Primer

<400>  123
gatcgaattc atgatggagc ccagagaagc tggac                                  35

<210>  124
<211>  28
<212>  DNA
<213>  Artificial

<220>
<223>  Primer

<400>  124
cgagtcaggc tgctatgtcc accaggcc                                          28

<210>  125
<211>  37
<212>  DNA
<213>  Artificial

<220>
<223>  Primer

<400>  125
gcataagctt ccatgtggag ctgcagctgg ttcaacg                                37

<210>  126
<211>  33
<212>  DNA
<213>  Artificial

<220>
<223>  Primer

<400>  126
gcatctcgag cctacgccag cacctgctgc acc                                    33

<210>  127
<211>  33
<212>  DNA
<213>  Artificial

<220>
<223>  Primer

<400>  127
gcataagctt ccatggggaa cgattctgtc agc                                    33

<210>  128
<211>  33
<212>  DNA
<213>  Artificial

<220>
<223>  Primer

<400>  128
gcatctcgag cctacacctc catctccgag acc                                    33

<210>  129
<211>  31
<212>  DNA
<213>  Artificial

<220>
<223>  Primer

<400>  129
gatcaagctt gcatggcacc ttctcatcgg g                                      31

<210>  130
<211>  31
<212>  DNA
<213>  Artificial

<220>
<223>  Primer

<400>  130
gatcctcgag tcaaaggtcc cattccggac c                                    31


<210>  131
<211>  13
<212>  DNA
<213>  Artificial

<220>
<223>  Primer

<400>  131
tccctgtgcc ccc                                                        13


<210>  132
<211>  23
<212>  DNA
<213>  Artificial

<220>
<223>  Primer

<400>  132
ttataggaga cccatgggca ggg                                             23


<210>  133
<211>  40
<212>  DNA
<213>  Artificial

<220>
<223>  Primer

<400>  133
ggtgctctgg aacctcggct tccgcatcaa gaagggcccc                           40


<210>  134
<211>  40
<212>  DNA
<213>  Artificial

<220>
<223>  Primer

<400>  134
ggggcccttc ttgatgcgga agccgaggtt ccagagcacc                           40


<210>  135
<211>  38
<212>  DNA

<213>  Artificial

<220>
<223>  Primer

<400>  135
ttcaaagctt atgaacaaca atacaacatg tattcaac                                38

<210>  136
<211>  23
<212>  DNA
<213>  Artificial

<220>
<223>  Primer

<400>  136
tcaaacatat gattgcatat gtg                                                23

<210>  137
<211>  39
<212>  DNA
<213>  Artificial

<220>
<223>  Primer

<400>  137
gcacatatgc aatcatatgg ttgactcgag tgaaaaggg                               39

<210>  138
<211>  39
<212>  DNA
<213>  Artificial

<220>
<223>  Primer

<400>  138
cccttttcac tcgagtcaac catatgattg catatgtgc                               39

<210>  139
<211>  30
<212>  DNA
<213>  Artificial

<220>
<223>  Primer

<400>  139
ctccatctgt ctcagctatg ccagcagcag                                         30

<210>  140
<211>  13

<212>  DNA
<213>  Artificial

<220>
<223>  Primer

<400>  140
tctcagctat gcc                                                          13


<210>  141
<211>  19
<212>  DNA
<213>  Artificial

<220>
<223>  Primer

<400>  141
tccttctcag tcgctcttc                                                    19


<210>  142
<211>  19
<212>  DNA
<213>  Artificial

<220>
<223>  Primer

<400>  142
cctccacttg tgcttcatc                                                    19


<210>  143
<211>  21
<212>  DNA
<213>  Artificial

<220>
<223>  Primer

<400>  143
aaaatctatc aacacccagc c                                                 21


<210>  144
<211>  32
<212>  DNA
<213>  Artificial

<220>
<223>  Primer

<400>  144
gatcaagctt accatgacca gcaatttttc cc                                     32


<210>  145

<211>  34
<212>  DNA
<213>  Artificial

<220>
<223>  Primer

<400>  145
gatcctcgag cttattctaa aaataaacta atgg                                34


<210>  146
<211>  25
<212>  DNA
<213>  Artificial

<220>
<223>  Primer

<400>  146
catgatctgg ggggccagcc ccagc                                          25


<210>  147
<211>  25
<212>  DNA
<213>  Artificial

<220>
<223>  Primer

<400>  147
gctggggctg gccccccaga tcatg                                          25


<210>  148
<211>  34
<212>  DNA
<213>  Artificial

<220>
<223>  Primer

<400>  148
gacatcaatt tcagtgagga tgacgtcgag gcag                                34


<210>  149
<211>  33
<212>  DNA
<213>  Artificial

<220>
<223>  Primer

<400>  149
tgcctcgacg tcatcctcac tgaaattgat gtc                                 33

<210> 150
<211> 34
<212> DNA
<213> Artificial

<220>
<223> Primer

<400> 150
cgaactccgc actcctggcc agggccctgc gggc 34


<210> 151
<211> 33
<212> DNA
<213> Artificial

<220>
<223> Primer

<400> 151
gcccgcaggg ccctggccag gagtgcggag ttc 33


<210> 152
<211> 48
<212> DNA
<213> Artificial

<220>
<223> Primer

<400> 152
gcgtaatacg actcactata gggagacctg ccagtgtggt agatacag 48


<210> 153
<211> 19
<212> DNA
<213> Artificial

<220>
<223> Primer

<400> 153
ggatgtgatg atggtgcag 19


<210> 154
<211> 47
<212> DNA
<213> Artificial

<220>
<223> Primer

<400> 154
gcgtaatacg actcactata gggagaccgg atgtgatgat ggtgcag 47

<210>  155
<211>  20
<212>  DNA
<213>  Artificial

<220>
<223>  Primer

<400>  155
tgccagtgtg gtagatacag                                                    20


<210>  156
<211>  48
<212>  DNA
<213>  Artificial

<220>
<223>  Primer

<400>  156
gcgtaatacg actcactata gggagaccac cagcaatttt tcccaacc                     48


<210>  157
<211>  19
<212>  DNA
<213>  Artificial

<220>
<223>  Primer

<400>  157
aataccagca gctctccac                                                     19


<210>  158
<211>  47
<212>  DNA
<213>  Artificial

<220>
<223>  Primer

<400>  158
gcgtaatacg actcactata gggagaccaa taccagcagc tctccac                      47


<210>  159
<211>  20
<212>  DNA
<213>  Artificial

<220>
<223>  Primer

<400>  159
accagcaatt tttcccaacc                                                    20

<210>  160
<211>  19
<212>  DNA
<213>  Artificial

<220>
<223>  Primer

<400>  160
gtgactaact ctgcctgcg                                                    19


<210>  161
<211>  19
<212>  DNA
<213>  Artificial

<220>
<223>  Primer

<400>  161
ttgcgctgca acactagcg                                                    19


<210>  162
<211>  20
<212>  DNA
<213>  Artificial

<220>
<223>  Primer

<400>  162
acagccccaa agccaaacac                                                   20


<210>  163
<211>  22
<212>  DNA
<213>  Artificial

<220>
<223>  Primer

<400>  163
ccgcaggagc aatgaaaatc ag                                                22


<210>  164
<211>  20
<212>  DNA
<213>  Artificial

<220>
<223>  Primer

<400>  164

tctccaaact cctggccttc 20

<210> 165
<211> 20
<212> DNA
<213> Artificial

<220>
<223> Primer

<400> 165
gcagggcagc tttttcatcc 20

<210> 166
<211> 20
<212> DNA
<213> Artificial

<220>
<223> Primer

<400> 166
acgcccgctg aaccgtatac 20

<210> 167
<211> 19
<212> DNA
<213> Artificial

<220>
<223> Primer

<400> 167
gggtgccacc tggttgctc 19

<210> 168
<211> 18
<212> DNA
<213> Artificial

<220>
<223> Primer

<400> 168
atggcacctt ctcatcgg 18

<210> 169
<211> 18
<212> DNA
<213> Artificial

<220>
<223> Primer

<400> 169
acgtagtaca ccgccttg 18

<210> 170
<211> 19
<212> DNA
<213> Artificial

<220>
<223> Primer

<400> 170
agcaggtaga tggagaagg 19

<210> 171
<211> 19
<212> DNA
<213> Artificial

<220>
<223> Primer

<400> 171
gactcctgag gaccatgtc 19

<210> 172
<211> 21
<212> DNA
<213> Artificial

<220>
<223> Primer

<400> 172
cattggaacc acatttattg g 21

<210> 173
<211> 20
<212> DNA
<213> Artificial

<220>
<223> Primer

<400> 173
aagcaagaca ggtgagaatg 20

<210> 174
<211> 20
<212> DNA
<213> Artificial

<220>
<223> Primer

<400>  174
ccatctgtct cagctatgcc                                                    20


<210>  175
<211>  19
<212>  DNA
<213>  Artificial

<220>
<223>  Primer

<400>  175
tccttctcag tcgctcttc                                                     19


<210>  176
<211>  21
<212>  DNA
<213>  Artificial

<220>
<223>  Primer

<400>  176
caaacaacaa acagcagaac c                                                  21


<210>  177
<211>  20
<212>  DNA
<213>  Artificial

<220>
<223>  Primer

<400>  177
tcacagtcac acctaccaag                                                    20


<210>  178
<211>  20
<212>  DNA
<213>  Artificial

<220>
<223>  Primer

<400>  178
ccatctgtct cagctatgcc                                                    20


<210>  179
<211>  19
<212>  DNA
<213>  Artificial

<220>

<223>  Primer

<400>  179
tccttctcag tcgctcttc                                                    19


<210>  180
<211>  21
<212>  DNA
<213>  Artificial

<220>
<223>  Primer

<400>  180
gtctatgcta ccaagttcac c                                                 21


<210>  181
<211>  19
<212>  DNA
<213>  Artificial

<220>
<223>  Primer

<400>  181
attcctccag cccatcatc                                                    19


<210>  182
<211>  9
<212>  PRT
<213>  Artificial

<220>
<223>  Primer

<400>  182

Ala Pro Arg Thr Pro Gly Gly Arg Arg
1               5

<210>  183
<211>  19
<212>  PRT
<213>  Homo sapiens

<400>  183

Asp Phe Asp Met Leu Arg Cys Met Leu Gly Arg Val Tyr Arg Pro Cys
1               5                   10                  15

Trp Gln Val


<210>  184
<211>  17
<212>  PRT

<213>  Salmon

<400>  184

Asp Thr Met Arg Cys Met Val Gly Arg Val Tyr Arg Pro Cys Trp Glu
1               5                   10                  15

Val

## Description

### CROSS-REFERENCE TO RELATED APPLICATIONS

The present application claims priority to Application Serial Nunbers 60/184,305 filed February 23, 2000, 60/184,304 filed February 23,2000, 60/184,303 filed February 23, 2000, 60/184,397 filed February 23, 2000, 60/184,247 filed February 23, 2000, 60/188,880 filed March 13, 2000, 60/217,369 filed July 11, 2000, 60/217,370 filed July 11, 2000, 60/219,492 filed July 20, 2000, 60/186,810 filed March 3, 2000, 60/188,064 filed March 9, 2000, 60/186,457 filed March 2,2000, 60/213,861 filed June 23, 2000, 60/194,344 filed April 3, 2000, and 60/218,337 filed July 14, 2000, each of which is incorporated herein by reference in its entirety.

### FIELD OF THE INVENTION

The present invention relates generally to the fields of genetics and cellular and molecular biology. More particularly, the invention relates to novel G protein coupled receptors, to polynucleotides that encode such novel receptors, to reagents such as antibodies, probes, primers and kits comprising such antibodies, probes, primers related to the same, and to methods which use the novel G protein coupled receptors, polynucleotides or reagents.

### BACKGROUND OF THE INVENTION

The G protein-coupled receptors (GPCRs) form a vast superfamily of cell surface receptors which are characterized by an amino-terminal extracellular domain, a carboxyl-terminal intracellular domain, and a serpentine structure that passes through the cell membrane seven times. Hence, such receptors are sometimes also referred to as seven transmembrane (7TM) receptors. These seven transmembrane domains define three extracellular loops and three intracellular loops, in addition to the amino- and carboxy- terminal domains. The extracellular portions of the receptor have a role in recognizing and binding one or more extracellular binding partners (*e.g.,* ligands), whereas the intracellular portions have a role in recognizing and communicating with downstream molecules in the signal transduction cascade.

The G protein-coupled receptors bind a variety of ligands including calcium ions, hormones, chemokines, neuropeptides, neurotransmitters, nucleotides, lipids, odorants, and even photons, and are important in the normal (and sometimes the aberrant) function of many cell types. (See generally Strosberg, *Eur. J. Biochem.* 196:1-10 (1991) and Bohm *et al., Biochem J.* 322:1-18 (1997)). When a specific ligand binds to its corresponding receptor, the ligand typically stimulates the receptor to activate a specific heterotrimeric guanine-nucleotide-binding regulatory protein (G-protein) that is coupled to the intracellular portion of the receptor. The G protein in turn transmits a signal to an effector molecule within the cell, by either stimulating or inhibiting the activity of that effector molecule. These effector molecules include adenylate cyclase, phospholipases and ion channels. Adenylate cyclase and phospholipases are enzymes that are involved in the production of the second messenger molecules cAMP, inositol triphosphate and diacyglycerol. It is through this sequence of events that an extracellular ligand stimuli exerts intracellular changes through a G protein-coupled receptor. Each such receptor has its own characteristic primary structure, expression pattern, ligand-binding profile, and intracellular effector system.

Because of the vital role of G protein-coupled receptors in the communication between cells and their environment, such receptors are attractive targets for therapeutic intervention, for example by activating or antagonizing such receptors. For receptors having a known ligand, the identification of agonists or antagonists may be sought specifically to enhance or inhibit the action of the ligand. Some G protein-coupled receptors have roles in disease pathogenesis (*e.g*., certain chemokine receptors that act as HIV co-receptors may have a role in AIDS pathogenesis), and are attractive targets for therapeutic intervention even in the absence of knowledge of the natural ligand of the receptor. Other receptors are attractive targets for therapeutic intervention by virtue of their expression pattern in tissues or cell types that are themselves attractive targets for therapeutic intervention. Examples of this latter category of receptors include receptors expressed in immune cells, which can be targeted to either inhibit autoimmune responses or to enhance immune responses to fight pathogens or cancer; and receptors expressed in the brain or other neural organs and tissues, which are likely targets in the treatment of mental disorder, depression, schizophrenia, bipolar disease, or other neurological disorders. This latter category of receptor is also useful as a marker for identifying and/or purifying (*e.g*., via fluorescence-activated cell sorting) cellular subtypes that express the receptor. Unfortunately, only a limited number of G protein receptors from the central nervous system (CNS) are known. Thus, a need exists for G protein-coupled receptors that have been identified and show promise as targets for therapeutic intervention in a variety of animals, including humans.

### SUMMARY OF THE INVENTION

The present invention relates to an isolated nucleic acid molecule that comprises a nucleotide sequence that encodes a polypeptide comprising an amino acid sequence homologous to sequences selected from the group consisting of SEQ ID NO:61 to SEQ ID NO:120, or a fragment thereof. In some embodiments, the nucleic acid molecule encodes at least a portion of SEQ ID NO:111 to SEQ ID NO:120. In some embodiments, the nucleic acid molecule comprises a sequence that encodes a polypeptide comprising a sequence selected from the group consisting of SEQ ID NO:61 to SEQ ID NO:120, or a fragment thereof. In some embodiments, the nucleic acid molecule comprises a sequence homologous to a sequence selected from the group consisting of SEQ ID NO:1 to SEQ ID NO:60, or a fragment thereof In some embodiments, the nucleic acid molecule comprises a sequence selected from the group consisting of SEQ ID NO:1 to SEQ ID NO:60, or fragments thereof.

According to some embodiments, the present invention provides vectors which comprise the nucleic acid molecule of the invention. In some embodiments, the vector is an expression vector.

According to some embodiments, the present invention provides host cells which comprise the vectors of the invention. In some embodiments, the host cells comprise expression vectors.

The present invention provides an isolated nucleic acid molecule comprising a nucleotide sequence complementary to at least a portion of a sequence selected from the group consisting of SEQ ID NO:1 to SEQ ID NO:60, said portion comprising at least 10 nucleotides.

The present invention provides a method of producing a polypeptide comprising a sequence selected from the group consisting of SEQ ID NO:61 to SEQ ID NO:120, or a homolog or fragment thereof. The method comprising the steps of introducing a recombinant expression vector that includes a nucleotide sequence that encodes the polypeptide into a compatible host cell, growing the host cell under conditions for expression of the polypeptide and recovering the polypeptide.

The present invention provides an isolated antibody which binds to an epitope on a polypeptide comprising a sequence selected from the group consisting of SEQ ID NO:61 to SEQ ID NO:120, or a homolog or fragment thereof.

The present invention provides an method of inducing an immune response in a mammal against a polypeptide comprising a sequence selected from the group consisting of SEQ ID NO:61 to SEQ ID NO:120, or a homolog or fragment thereof. The method comprises administering to a mammal an amount of the polypeptide sufficient to induce said immune response.

The present invention provides a method for identifying a compound which binds nGPCR-x. The method comprises the steps of contacting nGPCR-x with a compound and determining whether the compound binds nGPCR-x.

The present invention provides a method for identifying a compound which binds a nucleic acid molecule encoding nGPCR-x. The method comprises the steps of contacting said nucleic acid molecule encoding nGPCR-x with a compound and determining whether said compound binds said nucleic acid molecule.

The present invention provides a method for identifying a compound which modulates the activity of nGPCR-x. The method comprises the steps of contacting nGPCR-x with a compound and determining whether nGPCR-x activity has been modulated.

The present invention provides a method of identifying an animal homolog of nGPCR-x. The method comprises the steps screening a nucleic acid database of the animal with a sequence selected from the group consisting of SEQ ID NO:1 to SEQ ID NO:60, or a portion thereof and determining whether a portion of said library or database is homologous to said sequence selected from the group consisting of SEQ ID NO:1 to SEQ ID NO:60, or portion thereof.

The present invention provides a method of identifying an animal homolog of nGPCR-x. The methods comprises the steps screening a nucleic acid library of the animal with a nucleic acid molecule having a sequence selected from the group consisting of SEQ ID NO:1 to SEQ ID NO:60, or a portion thereof and determining whether a portion of said library or database is homologous to said sequence selected from the group consisting of SEQ ID NO:1 to SEQ ID NO:60, or a portion thereof.

Another aspect of the present invention relates to methods of screening a human subject to diagnose a disorder affecting the brain or genetic predisposition therefor. The methods comprise the steps of assaying nucleic acid of a human subject to determine a presence or an absence of a mutation altering an amino acid sequence, expression, or biological activity of at least one nGPCR that is expressed in the brain. The nGPCR comprise an amino acid sequence selected from the group consisting of SEQ ID Numbers 61, 62, 68, 91, 94, 96, 97, 99, 100, and 111-120, and allelic variants thereof. A diagnosis of the disorder or predisposition is made from the presence or absence of the mutation. The presence of a mutation altering the amino acid sequence, expression, or biological activity of the nGPCR in the nucleic acid correlates with an increased risk of developing the disorder.

The present invention further relates to methods of screening for a hereditary mental disorder genotype related to nGPCR-42, 46, 48, 49, 51, 52, 61, 63, or 70 in a human patient. The methods comprise the steps of providing a biological sample comprising nucleic acid from the patient, in which the nucleic acid includes sequences corresponding to alleles of nGPCR-42, 46, 48, 49, 51, 52, 61, 63, or 70. The presence of one or more mutations in the nGPCR-42, 46, 48, 49, 51, 52, 61, 63, or 70 allele is detected indicative of a hereditary mental disorder genotype.

The present invention provides kits for screening a human subject to diagnose mental disorder or a genetic predisposition therefor. The kits include an oligonucleotide useful as a probe for identifying polymorphisms in a human nGPCR-42, 46, 48, 49, 51, 52, 61, 63, or 70 gene. The oligonucleotide comprises 6-50 nucleotides in a sequence that is identical or complementary to a sequence of a wild type human nGPCR-42, 46, 48, 49, 51, 52, 61, 63, or 70 gene sequence or nGPCR-42, 46, 48, 49, 51, 52, 61, 63, or 70 coding sequence, except for one sequence difference selected from the group consisting of a nucleotide addition, a nucleotide deletion, or nucleotide substitution. The kit also includes a media packaged with the oligonucleotide. The media contains information for identifying polymorphisms that correlate with mental disorder or a genetic predisposition therefor, the polymorphisms being identifiable using the oligonucleotide as a probe.

The present invention further relates to methods of identifying nGPCR allelic variants that correlates with mental disorders. The methods comprise the steps of providing biological samples that comprise nucleic acid from a human patient diagnosed with a mental disorder, or from the patient's genetic progenitors or progeny, and detecting in the nucleic acid the presence of one or more mutations in an nGPCR that is expressed in the brain. The nGPCR comprises an amino acid sequence selected from the group consisting of SEQ ID Numbers 61, 62, 68, 91, 94, 96, 97, 99, 100, and 111-120, and allelic variants thereof. The nucleic acid includes sequences corresponding to the gene or genes encoding nGPCR. The one or more mutations detected indicate an allelic variant that correlates with a mental disorder.

The present invention further relates to purified polynucleotides, comprising nucleotide sequences encoding alleles of nGPCR-42, 46, 48, 49, 51, 52, 61, 63, or 70 from a human with mental disorder. The polynucleotide hybridizes to the complement of SEQ ID Numbers 1, 2, 8, 31, 34, 36, 37, 39, 40, and 51-60 under the following hybridization conditions: (a) hybridization for 16 hours at 42C in a hybridization solution comprising 50% formamide, 1% SDS, 1 M NaCl, 10% dextran sulfate and (b) washing 2 times for 30 minutes at 60C in a wash solution comprising 0.1x SSC and 1% SDS. The polynucleotide that encodes nGPCR-42, 46, 48, 49, 51, 52, 61, 63, or 70 amino acid sequence of the human differs from SEQ ID Numbers 61, 62, 68, 91, 94, 96, 97, 99, 100, and 111-120 by at least one residue.

The present invention also provides methods for identifying a modulator of biological activity of nGPCR-42, 46, 48, 49, 51, 52, 61, 63, or 70 comprising the steps of contacting a cell that expresses nGPCR-42, 46, 48, 49, 51, 52, 61, 63, or 70 in the presence and in the absence of a putative modulator compound and measuring nGPCR-42, 46, 48, 49, 51, 52, 61, 63, or 70 biological activity in the cell. The decreased or increased nGPCR-42, 46, 48, 49, 51, 52, 61, 63, or 70 biological activity in the presence versus absence of the putative modulator is indicative of a modulator of biological activity.

The present invention further provides methods to identify compounds useful for the treatment of mental disorders. The methods comprise the steps of contacting a composition comprising nGPCR-42, 46, 48, 49, 51, 52, 61, 63, or 70 with a compound suspected of binding nGPCR-42, 46, 48, 49, 51, 52, 61, 63, or 70. The binding between nGPCR-42, 46, 48, 49, 51, 52, 61, 63, or 70 and the compound suspected of binding nGPCR-42,46,48,49,51, 52, 61, 63, or 70 is detected. Compounds identified as binding nGPCR-42, 46, 48, 49, 51, 52, 61, 63, or 70 are candidate compounds useful for the treatment of mental disorder. Compounds identified as binding nGPCR-42, 46, 48, 49, 51, 52, 61, 63, 70, or other nGPCRs can be further tested in other assays including, but not limited to, *in vivo* models, in order to confirm or quantitate their activity.

The present invention further provides methods for identifying a compound useful as a modulator of binding between nGPCR-42, 46, 48, 49, 51, 52, 61, 63, or 70 and a binding partner of nGPCR-42, 46, 48, 49, 51, 52, 61, 63, or 70. The methods comprise the steps of contacting the binding partner and a composition comprising nGPCR-42, 46, 48, 49, 51, 52, 61, 63, or 70 in the presence and in the absence of a putative modulator compound and detecting binding between the binding partner and nGPCR-42,46,48, 49,51,52, 61,63, or 70. Decreased or increased binding between the binding partner and nGPCR-42, 46, 48, 49, 51, 52, 61, 63, or 70 in the presence of the putative modulator, as compared to binding in the absence of the putative modulator is indicative a modulator compound useful for the treatment of a related disease or disorder. Compounds identified as modulating binding between nGPCR-42,46,48, 49, 51, 52, 61, 63, 70, or other nGPCRs and an nGPCR-x binding partner can be further tested in other assays including, but not limited to, *in vivo* models, in order to confirm or quantitate their activity as modulators.

Another aspect of the present invention relates to methods of purifying a G protein from a sample containing a G protein. The methods comprise the steps of contacting the sample with an nGPCR for a time sufficient to allow the G protein to form a complex with the nGPCR, isolating the complex from remaining components of the sample, maintaining the complex under conditions which result in dissociation of the G protein from the nGPCR, and isolating said G protein from the nGPCR.

Another aspect of the present invention relates to methods of identifying a compound that binds to or modulates nGPCR-51. The methods comprise contacting a composition comprising nGPCR-51 and Peptide A with a test compound, or a plurality of test compounds, and determining whether the test compound or compounds compete with Peptide A for binding to nGPCR-51.

### DETAILED DESCRIPTION OF PREFERRED EMBODIMENTS

### Definitions

Various definitions are made throughout this document. Most words have the meaning that would be attributed to those words by one skilled in the art. Words specifically defined either below or elsewhere in this document have the meaning provided in the context of the present invention as a whole and as are typically understood by those skilled in the art.

"Synthesized" as used herein and understood in the art, refers to polynucleotides produced by purely chemical, as opposed to enzymatic, methods. "Wholly" synthesized DNA sequences are therefore produced entirely by chemical means, and "partially" synthesized DNAs embrace those wherein only portions of the resulting DNA were produced by chemical means.

By the term "region" is meant a physically contiguous portion of the primary structure of a biomolecule. In the case of proteins, a region is defined by a contiguous portion of the amino acid sequence of that protein.

The term "domain" is herein defined as referring to a structural part of a biomolecule that contributes to a known or suspected function of the biomolecule. Domains may be co-extensive with regions or portions thereof; domains may also incorporate a portion of a biomolecule that is distinct from a particular region, in addition to all or part of that region. Examples of GPCR protein domains include, but are not limited to, the extracellular (*i.e.,* N-terminal), transmembrane and cytoplasmic (*i.e*., C-terminal) domains, which are co-extensive with like-named regions of GPCRs; each of the seven transmembrane segments of a GPCR; and each of the loop segments (both extracellular and intracellular loops) connecting adjacent transmembrane segments.

As used herein, the term "activity" refers to a variety of measurable indicia suggesting or revealing binding; either direct or indirect; affecting a response, i.e. having a measurable affect in response to some exposure or stimulus, including, for example, the affinity of a compound for directly binding a polypeptide or polynucleotide of the invention, or, for example, measurement of amounts of upstream or downstream proteins or other similar functions after some stimulus or event.

Unless indicated otherwise, as used herein, the abbreviation in lower case (gpcr) refers to a gene, cDNA, RNA or nucleic acid sequence, while the upper case version (GPCR) refers to a protein, polypeptide, peptide, oligopeptide, or amino acid sequence. The term "nGPCR-x" refers to any of the nGPCRs taught herein, while specific reference to a nGPCR (for example nGPCR-63) refers only to that specific nGPCR.

As used herein, the term "antibody" is meant to refer to complete, intact antibodies, and Fab, Fab', F(ab)2, and other fragments thereof. Complete, intact antibodies include monoclonal antibodies such as murine monoclonal antibodies, chimeric antibodies and humanized antibodies.

As used herein, the term "binding" means the physical or chemical interaction between two proteins or compounds or associated proteins or compounds or combinations thereof. Binding includes ionic, non-ionic, Hydrogen bonds, Van der Waals, hydrophobic interactions, etc. The physical interaction, the binding, can be either direct or indirect, indirect being through or due to the effects of another protein or compound. Direct binding refers to interactions that do not take place through or due to the effect of another protein or compound but instead are without other substantial chemical intermediates. Binding may be detected in many different manners. As a non-limiting example, the physical binding interaction between a nGPCR-x of the invention and a compound can be detected using a labeled compound. Alternatively, functional evidence of binding can be detected using, for example, a cell transfected with and expressing a nGPCR-x of the invention. Binding of the transfected cell to a ligand of the nGPCR that was transfected into the cell provides functional evidence of binding. Other methods of detecting binding are well-known to those of skill in the art.

As used herein, the term "compound" means any identifiable chemical or molecule, including, but not limited to, small molecule, peptide, protein, sugar, nucleotide, or nucleic acid, and such compound can be natural or synthetic.

As used herein, the term "complementary" refers to Watson-Crick basepairing between nucleotide units of a nucleic acid molecule.

As used herein, the term "contacting" means bringing together, either directly or indirectly, a compound into physical proximity to a polypeptide or polynucleotide of the invention. The polypeptide or polynucleotide can be in any number of buffers, salts, solutions etc. Contacting includes, for example, placing the compound into a beaker, microtiter plate, cell culture flask, or a microarray, such as a gene chip, or the like, which contains the nucleic acid molecule, or polypeptide encoding the nGPCR or fragment thereof.

As used herein, the phrase "homologous nucleotide sequence," or "homologous amino acid sequence," or variations thereof, refers to sequences characterized by a homology, at the nucleotide level or amino acid level, of at least the specified percentage. Homologous nucleotide sequences include those sequences coding for isoforms of proteins. Such isoforms can be expressed in different tissues of the same organism as a result of, for example, alternative splicing of RNA. Alternatively, isoforms can be encoded by different genes. Homologous nucleotide sequences include nucleotide sequences encoding for a protein of a species other than humans, including, but not limited to, mammals. Homologous nucleotide sequences also include, but are not limited to, naturally occurring allelic variations and mutations of the nucleotide sequences set forth herein. A homologous nucleotide sequence does not, however, include the nucleotide sequence encoding other known GPCRs. Homologous amino acid sequences include those amino acid sequences which contain conservative amino acid substitutions and which polypeptides have the same binding and/or activity. A homologous amino acid sequence does not, however, include the amino acid sequence encoding other known GPCRs. Percent homology can be determined by, for example, the Gap program (Wisconsin Sequence Analysis Package, Version 8 for Unix, Genetics Computer Group, University Research Park, Madison WI), using the default settings, which uses the algorithm of Smith and Waterman (*Adv. Appl. Math.,* 1981, 2, 482-489, which is incorporated herein by reference in its entirety).

As used herein, the term "isolated" nucleic acid molecule refers to a nucleic acid molecule (DNA or RNA) that has been removed from its native environment. Examples of isolated nucleic acid molecules include, but are not limited to, recombinant DNA molecules contained in a vector, recombinant DNA molecules maintained in a heterologous host cell, partially or substantially purified nucleic acid molecules, and synthetic DNA or RNA molecules.

As used herein, the terms "modulates" or "modifies" means an increase or decrease in the amount, quality, or effect of a particular activity or protein.

As used herein, the term "oligonucleotide" refers to a series of linked nucleotide residues which has a sufficient number of bases to be used in a polymerase chain reaction (PCR). This short sequence is based on (or designed from) a genomic or cDNA sequence and is used to amplify, confirm, or reveal the presence of an identical, similar or complementary DNA or RNA in a particular cell or tissue. Oligonucleotides comprise portions of a DNA sequence having at least about 10 nucleotides and as many as about 50 nucleotides, preferably about 15 to 30 nucleotides. They are chemically synthesized and may be used as probes.

As used herein, the term "probe" refers to nucleic acid sequences of variable length, preferably between at least about 10 and as many as about 6,000 nucleotides, depending on use. They are used in the detection of identical, similar, or complementary nucleic acid sequences. Longer length probes are usually obtained from a natural or recombinant source, are highly specific and much slower to hybridize than oligomers. They may be single- or double-stranded and carefully designed to have specificity in PCR, hybridization membrane-based, or ELISA-like technologies.

The term "preventing" refers to decreasing the probability that an organism contracts or develops an abnormal condition.

The term "treating" refers to having a therapeutic effect and at least partially alleviating or abrogating an abnormal condition in the organism.

The term "therapeutic effect" refers to the inhibition or activation factors causing or contributing to the abnormal condition. A therapeutic effect relieves to some extent one or more of the symptoms of the abnormal condition. In reference to the treatment of abnormal conditions, a therapeutic effect can refer to one or more of the following: (a) an increase in the proliferation, growth, and/or differentiation of cells; (b) inhibition (*i.e*., slowing or stopping) of cell death; (c) inhibition of degeneration; (d) relieving to some extent one or more of the symptoms associated with the abnormal condition; and (e) enhancing the function of the affected population of cells. Compounds demonstrating efficacy against abnormal conditions can be identified as described herein.

The term "abnormal condition" refers to a function in the cells or tissues of an organism that deviates from their normal functions in that organism. An abnormal condition can relate to cell proliferation, cell differentiation, cell signaling, or cell survival. An abnormal condition may also include obesity, diabetic complications such as retinal degeneration, and irregularities in glucose uptake and metabolism, and fatty acid uptake and metabolism.

Abnormal cell proliferative conditions include cancers such as fibrotic and mesangial disorders, abnormal angiogenesis and vasculogenesis, wound healing, psoriasis, diabetes mellitus, and inflammation.

Abnormal differentiation conditions include, but are not limited to, neurodegenerative disorders, slow wound healing rates, and slow tissue grafting healing rates. Abnormal cell signaling conditions include, but are not limited to, psychiatric disorders involving excess neurotransmitter activity.

Abnormal cell survival conditions may also relate to conditions in which programmed cell death (apoptosis) pathways are activated or abrogated. A number of protein kinases are associated with the apoptosis pathways. Aberrations in the function of any one of the protein kinases could lead to cell immortality or premature cell death.

The term "administering" relates to a method of incorporating a compound into cells or tissues of an organism. The abnormal condition can be prevented or treated when the cells or tissues of the organism exist within the organism or outside of the organism. Cells existing outside the organism can be maintained or grown in cell culture dishes. For cells harbored within the organism, many techniques exist in the art to administer compounds, including (but not limited to) oral, parenteral, dermal, injection, and aerosol applications. For cells outside of the organism, multiple techniques exist in the art to administer the compounds, including (but not limited to) cell microinjection techniques, transformation techniques and carrier techniques.

The abnormal condition can also be prevented or treated by administering a compound to a group of cells having an aberration in a signal transduction pathway to an organism. The effect of administering a compound on organism function can then be monitored. The organism is preferably a mouse, rat, rabbit, guinea pig or goat, more preferably a monkey or ape, and most preferably a human.

By "amplification" it is meant increased numbers of DNA or RNA in a cell compared with normal cells. "Amplification" as it refers to RNA can be the detectable presence of RNA in cells, since in some normal cells there is no basal expression of RNA. In other normal cells, a basal level of expression exists, therefore in these cases amplification is the detection of at least 1 to 2-fold, and preferably more, compared to the basal level.

As used herein, the phrase "stringent hybridization conditions" or "stringent conditions" refers to conditions under which a probe, primer, or oligonucleotide will hybridize to its target sequence, but to no other sequences. Stringent conditions are sequence-dependent and will be different in different circumstances. Longer sequences hybridize specifically at higher temperatures. Generally, stringent conditions are selected to be about 5C lower than the thermal melting point (Tₘ) for the specific sequence at a defined ionic strength and pH. The Tₘ is the temperature (under defined ionic strength, pH and nucleic acid concentration) at which 50% of the probes complementary to the target sequence hybridize to the target sequence at equilibrium. Since the target sequences are generally present in excess, at Tₘ, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30C for short probes, primers or oligonucleotides (e.g. 10 to 50 nucleotides) and at least about 60C for longer probes, primers or oligonucleotides. Stringent conditions may also be achieved with the addition of destabilizing agents, such as formamide.

The amino acid sequences are presented in the amino to carboxy direction, from left to right. The amino and carboxy groups are not presented in the sequence. The nucleotide sequences are presented by single strand only, in the 5' to 3' direction, from left to right. Nucleotides and amino acids are represented in the manner recommended by the IUPAC-IUB Biochemical Nomenclature Commission or (for amino acids) by three letters code.

### Polynucleotides

The present invention provides purified and isolated polynucleotides (*e.g*., DNA sequences and RNA transcripts, both sense and complementary antisense strands, both single- and double-stranded, including splice variants thereof) that encode unknown G protein-coupled receptors heretofore termed novel GPCRs, or nGPCRs. These genes are described herein and designated herein collectively as nGPCR-x (where x is 42, 44, 45, 46, 47, 48, 49, 50, 51, 52,61, 63, 64, 65, 66, 67, 68, 69, 70, 71, 72, 2001, 2002, 2003, 2004, 2005, 2006, 2007, 2008, 2009, 2010, 2011, 2012, 2013, 2014, 2015, 2016, 2017, 2018, 2019, 2020, 2021, 2022, 2024, 2025, 2026, 2027, 2028, 2029, and 2030). That is, these genes are described herein and designated herein as nGPCR-42, nGPCR-44, etc. Table 1 below identifies the novel gene sequence nGPCR-x designation, the SEQ ID NO: of the gene sequence, the SEQ ID NO: of the polypeptide encoded thereby, and the U.S. Provisional Application in which the gene sequence has been disclosed.

**Table 1**

| **nGPCR** | **Nucleotide Sequence (SEQ ID NO:)** | **Amino acid Sequence (SEQ ID NO:)** | **Originally filed in:** |
|---|---|---|---|
| 0042 | 31 | 91 | D |
| 0042 | 54 | 114 | I |
| 0044 | 32 | 92 | D |
| 0045 | 33 | 93 | D |
| 0046 | 34 | 94 | D |
| 0046 | 55 | 115 | J |
| 0047 | 35 | 95 | D |
| 0048 | 36 | 96 | D |
| 0048 | 56 | 116 | K |
| 0049 | 37 | 97 | D |
| 0049 | 59 | 119 | N |
| 0050 | 38 | 98 | D |
| 0051 | 39 | 99 | D |
| 0051 | 57 | 117 | L |
| 0052 | 40 | 100 | D |
| 0052 | 58 | 118 | M |
| 0061 | 1 | 61 | A |
| 0061 | 60 | 120 | O |
| 0063 | 2 | 62 | A |
| 0063 | 53 | 113 | H |
| 0063 | 51 | 111 | F |
| 0064 | 3 | 63 | A |
| 0065 | 4 | 64 | A |
| 0066 | 5 | 65 | A |
| 0067 | 6 | 66 | A |
| 0068 | 7 | 67 | A |
| 0069 | 10 | 70 | A |
| 0070 | 8 | 68 | A |
| 0070 | 52 | 112 | G |
| 0071 | 9 | 69 | A |
| 0072 | 43 | 103 | E |
| 2001 | 21 | 81 | C |
| 2002 | 22 | 82 | C |
| 2003 | 23 | 83 | C |
| 2004 | 24 | 84 | C |
| 2005 | 25 | 85 | C |
| 2006 | 26 | 86 | C |
| 2007 | 27 | 87 | C |
| 2008 | 28 | 88 | C |
| 2009 | 29 | 89 | C |
| 2010 | 30 | 90 | C |
| 2011 | 11 | 71 | B |
| 2012 | 12 | 72 | B |
| 2013 | 13 | 73 | B |
| 2014 | 14 | 74 | B |
| 2015 | 15 | 75 | B |
| 2016 | 16 | 76 | B |
| 2017 | 17 | 77 | B |
| 2018 | 18 | 78 | B |
| 2019 | 19 | 79 | B |
| 2020 | 20 | 80 | B |
| 2021 | 41 | 101 | E |
| 2022 | 42 | 102 | E |
| 2024 | 44 | 104 | E |
| 2025 | 45 | 105 | E |
| 2026 | 46 | 106 | E |
| 2027 | 47 | 107 | E |
| 2028 | 48 | 108 | E |
| 2029 | 49 | 109 | E |
| 2030 | 50 | 110 | E |

| | | | |
|---|---|---|---|
| **Legend** A= Ser. No. 60/184,305 B= Ser. No. 60/184,304 C= Ser. No. 60/184,303 D= Ser. No. 60/184,397 E= Ser. No. 60/184,247 F= Ser. No. 60/188,880 G= Ser. No. 60/217,369 H=Ser. No. 60/219,492 I= Ser. No. 60/186,810 J= Ser. No. 60/188,064 K= Ser. No. 60/186,457 L= Ser. No. 60/213,861 M= Ser. No. 60/194,344 N= Ser. No. 60/218,337 O= Ser. No. 60/217,370 | | | |

When a specific nGPCR is identified (for example nGPCR-63), it is understood that only that specific nGPCR is being referred to.

As described in Example 5 below, the genes encoding nGPCR-42, 46, 48, 49, 51, 52, 61, 63, or 70 have been detected in brain tissue indicating that these nGPCR proteins are neuroreceptors.

The invention provides purified and isolated polynucleotides *(e.g.,* cDNA, genomic DNA, synthetic DNA, RNA, or combinations thereof, whether single- or double-stranded) that comprise a nucleotide sequence encoding the amino acid sequence of the polypeptides of the invention. Such polynucleotides are useful for recombinantly expressing the receptor and also for detecting expression of the receptor in cells (*e.g*., using Northern hybridization and *in situ* hybridization assays). Such polynucleotides also are useful in the design of antisense and other molecules for the suppression of the expression of nGPCR-x in a cultured cell, a tissue, or an animal; for therapeutic purposes; or to provide a model for diseases or conditions characterized by aberrant nGPCR-x expression. Specifically excluded from the definition of polynucleotides of the invention are entire isolated, non-recombinant native chromosomes of host cells. A preferred polynucleotide has a sequence selected from the group consisting of SEQ ID NO:1 to SEQ ID NO:60, which correspond to naturally occurring nGPCR-x sequences. It will be appreciated that numerous other polynucleotide sequences exist that also encode nGPCR-x having the sequence selected from the group consisting of SEQ ID NO:61 to SEQ ID NO:120, due to the well-known degeneracy of the universal genetic code.

The invention also provides a purified and isolated polynucleotide comprising a nucleotide sequence that encodes a mammalian polypeptide, wherein the polynucleotide hybridizes to a polynucleotide having the sequence set forth in sequences selected from the group consisting of SEQ ID NO:1 to SEQ ID NO:60, or the non-coding strand complementary thereto, under the following hybridization conditions: (a) hybridization for 16 hours at 42C in a hybridization solution comprising 50% formamide, 1% SDS, 1 M NaCl, 10% dextran sulfate; and (b) washing 2 times for 30 minutes each at 60C in a wash solution comprising 0.1% SSC, 1% SDS. Polynucleotides that encode a human allelic variant are highly preferred.

The present invention relates to molecules which comprise the gene sequences that encode the nGPCRs; constructs and recombinant host cells incorporating the gene sequences; the novel GPCR polypeptides encoded by the gene sequences; antibodies to the polypeptides and homologs; kits employing the polynucleotides and polypeptides, and methods of making and using all of the foregoing. In addition, the present invention relates to homologs of the gene sequences and of the polypeptides and methods of making and using the same.

Genomic DNA of the invention comprises the protein-coding region for a polypeptide of the invention and is also intended to include allelic variants thereof. It is widely understood that, for many genes, genomic DNA is transcribed into RNA transcripts that undergo one or more splicing events wherein intron (*i.e.,* non-coding regions) of the transcripts are removed, or "spliced out." RNA transcripts that can be spliced by alternative mechanisms, and therefore be subj ect to removal of different RNA sequences but still encode a nGPCR-x polypeptide, are referred to in the art as splice variants which are embraced by the invention. Splice variants comprehended by the invention therefore are encoded by the same original genomic DNA sequences but arise from distinct mRNA transcripts. Allelic variants are modified forms of a wild-type gene sequence, the modification resulting from recombination during chromosomal segregation or exposure to conditions which give rise to genetic mutation. Allelic variants, like wild type genes, are naturally occurring sequences (as opposed to non-naturally occurring variants that arise from *in vitro* manipulation).

The invention also comprehends cDNA that is obtained through reverse transcription of an RNA polynucleotide encoding nGPCR-x (conventionally followed by second strand synthesis of a complementary strand to provide a double-stranded DNA).

Preferred DNA sequences encoding human nGPCR-x polypeptides are selected from the group consisting of SEQ ID NO:1 to SEQ ID NO:60. A preferred DNA of the invention comprises a double stranded molecule along with the complementary molecule (the "non-coding strand" or "complement") having a sequence unambiguously deducible from the coding strand according to Watson-Crick base-pairing rules for DNA. Also preferred are other polynucleotides encoding the nGPCR-x polypeptide selected from the group consisting of SEQ ID NO:61 to SEQ ID NO:120, which differ in sequence from the polynucleotides selected from the group consisting of SEQ ID NO:1 to SEQ ID NO:60, by virtue of the well-known degeneracy of the universal nuclear genetic code.

The invention further embraces other species, preferably mammalian, homologs of the human nGPCR-x DNA. Species homologs, sometimes referred to as "orthologs," in general, share at least 35%, at least 40%, at least 45%, at least 50%, at least 60%, at least 65%, at least 70%, at least 75%, at least 80%, at least 85%, at least 90%, at least 95%, at least 98%, or at least 99% homology with human DNA of the invention. Generally, percent sequence "homology" with respect to polynucleotides of the invention may be calculated as the percentage of nucleotide bases in the candidate sequence that are identical to nucleotides in the nGPCR-x sequence set forth in sequences selected from the group consisting of SEQ ID NO:1 to SEQ ID NO:60, after aligning the sequences and introducing gaps, if necessary, to achieve the maximum percent sequence identity.

Polynucleotides of the invention permit identification and isolation of polynucleotides encoding related nGPCR-x polypeptides, such as human allelic variants and species homologs, by well-known techniques including Southern and/or Northern hybridization, and polymerase chain reaction (PCR). Examples of related polynucleotides include human and non-human genomic sequences, including allelic variants, as well as polynucleotides encoding polypeptides homologous to nGPCR-x and structurally related polypeptides sharing one or more biological, immunological, and/or physical properties of nGPCR-x. Non-human species genes encoding proteins homologous to nGPCR-x can also be identified by Southern and/or PCR analysis and are useful in animal models for nGPCR-x disorders. Knowledge of the sequence of a human nGPCR-x DNA also makes possible through use of Southern hybridization or polymerase chain reaction (PCR) the identification of genomic DNA sequences encoding nGPCR-x expression control regulatory sequences such as promoters, operators, enhancers, repressors, and the like. Polynucleotides of the invention are also useful in hybridization assays to detect the capacity of cells to express nGPCR-x. Polynucleotides of the invention may also provide a basis for diagnostic methods useful for identifying a genetic alteration(s) in a nGPCR-x locus that underlies a disease state or states, which information is useful both for diagnosis and for selection of therapeutic strategies.

According to the present invention, the nGPCR-x nucleotide sequences disclosed herein may be used to identify homologs of the nGPCR-x, in other animals, including but not limited to humans and other mammals, and invertebrates. Any of the nucleotide sequences disclosed herein, or any portion thereof, can be used, for example, as probes to screen databases or nucleic acid libraries, such as, for example, genomic or cDNA libraries, to identify homologs, using screening procedures well known to those skilled in the art. Accordingly, homologs having at least 50%, more preferably at least 60%, more preferably at least 70%, more preferably at least 80%, more preferably at least 90%, more preferably at least 95%, and most preferably at least 100% homology with nGPCR-x sequences can be identified.

The disclosure herein of full-length polynucleotides encoding nGPCR-x polypeptides makes readily available to the worker of ordinary skill in the art every possible fragment of the full-length polynucleotide.

One preferred embodiment of the present invention provides an isolated nucleic acid molecule comprising a sequence homologous sequences selected from the group consisting of SEQ ID NO:1 to SEQ ID NO:60, and fragments thereof. Another preferred embodiment provides an isolated nucleic acid molecule comprising a sequence selected from the group consisting of SEQ ID NO:1 to SEQ ID NO:60, and fragments thereof.

As used in the present invention, fragments of nGPCR-x-encoding polynucleotides comprise at least 10, and preferably at least 12, 14, 16, 18, 20, 25, 50, or 75 consecutive nucleotides of a polynucleotide encoding nGPCR-x. Preferably, fragment polynucleotides of the invention comprise sequences unique to the nGPCR-x-encoding polynucleotide sequence, and therefore hybridize under highly stringent or moderately stringent conditions only (*i.e*., "specifically") to polynucleotides encoding nGPCR-x (or fragments thereof). Polynucleotide fragments of genomic sequences of the invention comprise not only sequences unique to the coding region, but also include fragments of the full-length sequence derived from introns, regulatory regions, and/or other non-translated sequences. Sequences unique to polynucleotides of the invention are recognizable through sequence comparison to other known polynucleotides, and can be identified through use of alignment programs routinely utilized in the art, *e.g*., those made available in public sequence databases. Such sequences also are recognizable from Southern hybridization analyses to determine the number of fragments of genomic DNA to which a polynucleotide will hybridize. Polynucleotides of the invention can be labeled in a manner that permits their detection, including radioactive, fluorescent, and enzymatic labeling.

Fragment polynucleotides are particularly useful as probes for detection of full-length or fragments of nGPCR-x polynucleotides. One or more polynucleotides can be included in kits that are used to detect the presence of a polynucleotide encoding nGPCR-x, or used to detect variations in a polynucleotide sequence encoding nGPCR-x.

The invention also embraces DNAs encoding nGPCR-x polypeptides that hybridize under moderately stringent or high stringency conditions to the non-coding strand, or complement, of the polynucleotides set forth in sequences selected from the group consisting of SEQ ID NO:1 to SEQ ID NO:60.

Exemplary highly stringent hybridization conditions are as follows: hybridization at 42C in a hybridization solution comprising 50% formamide, 1% SDS, 1 M NaCl, 10% Dextran sulfate, and washing twice for 30 minutes at 60C in a wash solution comprising 0.1 X SSC and 1% SDS. It is understood in the art that conditions of equivalent stringency can be achieved through variation of temperature and buffer, or salt concentration as described Ausubel *et al.* (Eds.), *Protocols in Molecular Biology,* John Wiley & Sons (1994), pp. 6.0.3 to 6.4.10. Modifications in hybridization conditions can be empirically determined or precisely calculated based on the length and the percentage of guanosine/cytosine (GC) base pairing of the probe. The hybridization conditions can be calculated as described in Sambrook, *et* a*l*.*,* (Eds.), *Molecular Cloning: A Laboratory Manual,* Cold Spring Harbor Laboratory Press: Cold Spring Harbor, New York (1989), pp. 9.47 to 9.51.

With the knowledge of the nucleotide sequence information disclosed in the present invention, one skilled in the art can identify and obtain nucleotide sequences which encode nGPCR-x from different sources (*i.e*., different tissues or different organisms) through a variety of means well known to the skilled artisan and as disclosed by, for example, Sambrook et al., "Molecular cloning: a laboratory manual", Second Edition, Cold Spring Harbor Press, Cold Spring Harbor, NY (1989), which is incorporated herein by reference in its entirety.

For example, DNA that encodes nGPCR-x may be obtained by screening of mRNA, cDNA, or genomic DNA with oligonucleotide probes generated from the nGPCR-x gene sequence information provided herein. Probes may be labeled with a detectable group, such as a fluorescent group, a radioactive atom or a chemiluminescent group in accordance with procedures known to the skilled artisan and used in conventional hybridization assays, as described by, for example, Sambrook *et al.*

A nucleic acid molecule comprising any of the nGPCR-x nucleotide sequences described above can alternatively be synthesized by use of the polymerase chain reaction (PCR) procedure, with the PCR oligonucleotide primers produced from the nucleotide sequences provided herein. See U.S. Patent Numbers 4,683,195 to Mullis *et al.* and 4,683,202 to Mullis. The PCR reaction provides a method for selectively increasing the concentration of a particular nucleic acid sequence even when that sequence has not been previously purified and is present only in a single copy in a particular sample. The method can be used to amplify either single- or double-stranded DNA. The essence of the method involves the use of two oligonucleotide probes to serve as primers for the template-dependent, polymerase mediated replication of a desired nucleic acid molecule.

A wide variety of alternative cloning and *in vitro* amplification methodologies are well known to those skilled in the art. Examples of these techniques are found in, for example, Berger *et al., Guide to Molecular Cloning Techniques,* Methods in Enzymology 152, Academic Press, Inc., San Diego, CA (Berger), which is incorporated herein by reference in its entirety.

Automated sequencing methods can be used to obtain or verify the nucleotide sequence of nGPCR-x. The nGPCR-x nucleotide sequences of the present invention are believed to be 100% accurate. However, as is known in the art, nucleotide sequence obtained by automated methods may contain some errors. Nucleotide sequences determined by automation are typically at least about 90%, more typically at least about 95% to at least about 99.9% identical to the actual nucleotide sequence of a given nucleic acid molecule. The actual sequence may be more precisely determined using manual sequencing methods, which are well known in the art. An error in a sequence which results in an insertion or deletion of one or more nucleotides may result in a frame shift in translation such that the predicted amino acid sequence will differ from that which would be predicted from the actual nucleotide sequence of the nucleic acid molecule, starting at the point of the mutation.

The nucleic acid molecules of the present invention, and fragments derived therefrom, are useful for screening for restriction fragment length polymorphism (RFLP) associated with certain disorders, as well as for genetic mapping.

The polynucleotide sequence information provided by the invention makes possible large-scale expression of the encoded polypeptide by techniques well known and routinely practiced in the art.

### Vectors

Another aspect of the present invention is directed to vectors, or recombinant expression vectors, comprising any of the nucleic acid molecules described above. Vectors are used herein either to amplify DNA or RNA encoding nGPCR-x and/or to express DNA which encodes nGPCR-x. Preferred vectors include, but are not limited to, plasmids, phages, cosmids, episomes, viral particles or viruses, and integratable DNA fragments (*i.e*., fragments integratable into the host genome by homologous recombination). Preferred viral particles include, but are not limited to, adenoviruses, baculoviruses, parvoviruses, herpesviruses, poxviruses, adeno-associated viruses, Semliki Forest viruses, vaccinia viruses, and retroviruses. Preferred expression vectors include, but are not limited to, pcDNA3 (Invitrogen) and pSVL (Pharmacia Biotech). Other expression vectors include, but are not limited to, pSPORT™ vectors, pGEM™ vectors (Promega), pPROEXvectors™ (LTI, Bethesda, MD), Bluescript™ vectors (Stratagene), pQE™ vectors (Qiagen), pSE420™ (Invitrogen), and pYES2™(Invitrogen).

Expression constructs preferably comprise GPCR-x-encoding polynucleotides operatively linked to an endogenous or exogenous expression control DNA sequence and a transcription terminator. Expression control DNA sequences include promoters, enhancers, operators, and regulatory element binding sites generally, and are typically selected based on the expression systems in which the expression construct is to be utilized. Preferred promoter and enhancer sequences are generally selected for the ability to increase gene expression, while operator sequences are generally selected for the ability to regulate gene expression. Expression constructs of the invention may also include sequences encoding one or more selectable markers that permit identification of host cells bearing the construct. Expression constructs may also include sequences that facilitate, and preferably promote, homologous recombination in a host cell. Preferred constructs of the invention also include sequences necessary for replication in a host cell.

Expression constructs are preferably utilized for production of an encoded protein, but may also be utilized simply to amplify a nGPCR-x-encoding polynucleotide sequence. In preferred embodiments, the vector is an expression vector wherein the polynucleotide of the invention is operatively linked to a polynucleotide comprising an expression control sequence. Autonomously replicating recombinant expression constructs such as plasmid and viral DNA vectors incorporating polynucleotides of the invention are also provided. Preferred expression vectors are replicable DNA constructs in which a DNA sequence encoding nGPCR-x is operably linked or connected to suitable control sequences capable of effecting the expression of the nGPCR-x in a suitable host. DNA regions are operably linked or connected when they are functionally related to each other. For example, a promoter is operably linked or connected to a coding sequence if it controls the transcription of the sequence. Amplification vectors do not require expression control domains, but rather need only the ability to replicate in a host, usually conferred by an origin of replication, and a selection gene to facilitate recognition of transformants. The need for control sequences in the expression vector will vary depending upon the host selected and the transformation method chosen. Generally, control sequences include a transcriptional promoter, an optional operator sequence to control transcription, a sequence encoding suitable mRNA ribosomal binding and sequences which control the termination of transcription and translation.

Preferred vectors preferably contain a promoter that is recognized by the host organism. The promoter sequences of the present invention may be prokaryotic, eukaryotic or viral. Examples of suitable prokaryotic sequences include the P_{R} and P_{L} promoters ofbacteriophage lambda (The bacteriophage Lambda, Hershey, A. D., Ed., Cold Spring Harbor Press, Cold Spring Harbor, NY (1973), which is incorporated herein by reference in its entirety; Lambda II, Hendrix, R. W., Ed., Cold Spring Harbor Press, Cold Spring Harbor, NY (1980), which is incorporated herein by reference in its entirety); the trp, recA, heat shock, and lacZ promoters of *E. coli* and the SV40 early promoter (Benoist *et al. Nature,* 1981, *290,* 304-310, which is incorporated herein by reference in its entirety). Additional promoters include, but are not limited to, mouse mammary tumor virus, long terminal repeat of human immunodeficiency virus, maloney virus, cytomegalovirus immediate early promoter, Epstein Barr virus, Rous sarcoma virus, human actin, human myosin, human hemoglobin, human muscle creatine, and human metalothionein.

Additional regulatory sequences can also be included in preferred vectors. Preferred examples of suitable regulatory sequences are represented by the Shine-Dalgarno of the replicase gene of the phage MS-2 and of the gene cII of bacteriophage lambda. The Shine-Dalgarno sequence may be directly followed by DNA encoding nGPCR-x and result in the expression of the mature nGPCR-x protein.

Moreover, suitable expression vectors can include an appropriate marker that allows the screening of the transformed host cells. The transformation of the selected host is carried out using any one of the various techniques well known to the expert in the art and described in Sambrook *et al., supra.*

An origin of replication can also be provided either by construction of the vector to include an exogenous origin or may be provided by the host cell chromosomal replication mechanism. If the vector is integrated into the host cell chromosome, the latter may be sufficient. Alternatively, rather than using vectors which contain viral origins of replication, one skilled in the art can transform mammalian cells by the method of co-transformation with a selectable marker and nGPCR-x DNA. An example of a suitable marker is dihydrofolate reductase, (DHFR) or thymidine kinase (*see,* U.S. Patent No. 4,399,216).

Nucleotide sequences encoding GPCR-x may be recombined with vector DNA in accordance with conventional techniques, including blunt-ended or staggered-ended termini for ligation, restriction enzyme digestion to provide appropriate termini, filling in of cohesive ends as appropriate, alkaline phosphatase treatment to avoid undesiderable joining, and ligation with appropriate ligases. Techniques for such manipulation are disclosed by Sambrook et al., *supra* and are well known in the art. Methods for construction of mammalian expression vectors are disclosed in, for example, Okayama *et al., Mol. Cell. Biol.,* **1983*****,** 3*, 280, Cosman *et al., Mol. Immunol*.,**1986**, *23*, 935, Cosman *et al., Nature*,**1984**, *312*, 768, EP-A-0367566, and WO 91/18982, each of which is incorporated herein by reference in its entirety.

### Host cells

According to another aspect of the invention, host cells are provided, including prokaryotic and eukaryotic cells, comprising a polynucleotide of the invention (or vector of the invention) in a manner that permits expression of the encoded nGPCR-x polypeptide. Polynucleotides of the invention may be introduced into the host cell as part of a circular plasmid, or as linear DNA comprising an isolated protein coding region or a viral vector. Methods for introducing DNA into the host cell that are well known and routinely practiced in the art include transformation, transfection, electroporation, nuclear injection, or fusion with carriers such as liposomes, micelles, ghost cells, and protoplasts. Expression systems of the invention include bacterial, yeast, fungal, plant, insect, invertebrate, vertebrate, and mammalian cells systems.

The invention provides host cells that are transformed or transfected (stably or transiently) with polynucleotides of the invention or vectors of the invention. As stated above, such host cells are useful for amplifying the polynucleotides and also for expressing the nGPCR-x polypeptide or fragment thereof encoded by the polynucleotide.

In still another related embodiment, the invention provides a method for producing a nGPCR-x polypeptide (or fragment thereof) comprising the steps of growing a host cell of the invention in a nutrient medium and isolating the polypeptide or variant thereof from the cell or the medium. Because nGPCR-x is a seven transmembrane receptor, it will be appreciated that, for some applications, such as certain activity assays, the preferable isolation may involve isolation of cell membranes containing the polypeptide embedded therein, whereas for other applications a more complete isolation may be preferable.

According to some aspects of the present invention, transformed host cells having an expression vector comprising any of the nucleic acid molecules described above are provided. Expression of the nucleotide sequence occurs when the expression vector is introduced into an appropriate host cell. Suitable host cells for expression of the polypeptides of the invention include, but are not limited to, prokaryotes, yeast, and eukaryotes. If a prokaryotic expression vector is employed, then the appropriate host cell would be any prokaryotic cell capable of expressing the cloned sequences. Suitable prokaryotic cells include, but are not limited to, bacteria of the genera *Escherichia, Bacillus, Salmonella, Pseudomonas, Streptomyces,* and *Staphylococcus.*

If an eukaryotic expression vector is employed, then the appropriate host cell would be any eukaryotic cell capable of expressing the cloned sequence. Preferably, eukaryotic cells are cells of higher eukaryotes. Suitable eukaryotic cells include, but are not limited to, non-human mammalian tissue culture cells and human tissue culture cells. Preferred host cells include, but are not limited to, insect cells, HeLa cells, Chinese hamster ovary cells (CHO cells), African green monkey kidney cells (COS cells), human 293 cells, and murine 3T3 fibroblasts. Propagation of such cells in cell culture has become a routine procedure (*see,* Tissue Culture, Academic Press, Kruse and Patterson, eds. (1973), which is incorporated herein by reference in its entirety).

In addition, a yeast host may be employed as a host cell. Preferred yeast cells include, but are not limited to, the genera *Saccharomyces, Pichia,* and *Kluveromyces.* Preferred yeast hosts are *S. cerevisiae* and *P. pastoris.* Preferred yeast vectors can contain an origin of replication sequence from a 2T yeast plasmid, an autonomously replication sequence (ARS), a promoter region, sequences for polyadenylation, sequences for transcription termination, and a selectable marker gene. Shuttle vectors for replication in both yeast and *E*. *coli* are also included herein.

Alternatively, insect cells may be used as host cells. In a preferred embodiment, the polypeptides of the invention are expressed using a baculovirus expression system (*see,* Luckow *et al., Bio*/*Technology,* **1988**, *6*, 47, Baculovirus Expression Vectors: A Laboratory Manual, O'Rielly *et al.* (Eds.), W.H. Freeman and Company, New York, **1992**, and U.S. Patent No. 4,879,236, each of which is incorporated herein by reference in its entirety). In addition, the MAXBAC™ complete baculovirus expression system (Invitrogen) can, for example, be used for production in insect cells.

Host cells of the invention are a valuable source of immunogen for development of antibodies specifically immunoreactive with nGPCR-x. Host cells of the invention are also useful in methods for the large-scale production of nGPCR-x polypeptides wherein the cells are grown in a suitable culture medium and the desired polypeptide products are isolated from the cells, or from the medium in which the cells are grown, by purification methods known in the art, *e.g*., conventional chromatographic methods including immunoaffinity chromatography, receptor affinity chromatography, hydrophobic interaction chromatography, lectin affinity chromatography, size exclusion filtration, cation or anion exchange chromatography, high pressure liquid chromatography (HPLC), reverse phase HPLC, and the like. Still other methods of purification include those methods wherein the desired protein is expressed and purified as a fusion protein having a specific tag, label, or chelating moiety that is recognized by a specific binding partner or agent. The purified protein can be cleaved to yield the desired protein, or can be left as an intact fusion protein. Cleavage of the fusion component may produce a form of the desired protein having additional amino acid residues as a result of the cleavage process.

Knowledge of nGPCR-x DNA sequences allows for modification of cells to permit, or increase, expression of endogenous nGPCR-x. Cells can be modified (*e.g*., by homologous recombination) to provide increased expression by replacing, in whole or in part, the naturally occurring nGPCR-x promoter with all or part of a heterologous promoter so that the cells express nGPCR-x at higher levels. The heterologous promoter is inserted in such a manner that it is operatively linked to endogenous nGPCR-x encoding sequences. (See, for example, PCT International Publication No. WO 94/12650, PCT International Publication No. WO 92/20808, and PCT International Publication No. WO 91/09955.) It is also contemplated that, in addition to heterologous promoter DNA, amplifiable marker DNA (*e.g.,* ada, dhfr, and the multifunctional CAD gene which encodes carbamoyl phosphate synthase, aspartate transcarbamylase, and dihydroorotase) and/or intron DNA may be inserted along with the heterologous promoter DNA. If linked to the nGPCR-x coding sequence, amplification of the marker DNA by standard selection methods results in co-amplification of the nGPCR-x coding sequences in the cells.

### Knock-outs

The DNA sequence information provided by the present invention also makes possible the development (*e.g*., by homologous recombination or "knock-out" strategies; see Capecchi, *Science 244*:1288-1292 (1989), which is incorporated herein by reference in its entirety) of animals that fail to express functional nGPCR-x or that express a variant of nGPCR-x. Such animals (especially small laboratory animals such as rats, rabbits, and mice) are useful as models for studying the *in vivo* activities of nGPCR-x and modulators of nGPCR-x.

### Antisense

Also made available by the invention are anti-sense polynucleotides that recognize and hybridize to polynucleotides encoding nGPCR-x. Full-length and fragment anti-sense polynucleotides are provided. Fragment antisense molecules of the invention include (i) those that specifically recognize and hybridize to nGPCR-x RNA (as determined by sequence comparison of DNA encoding nGPCR-x to DNA encoding other known molecules). Identification of sequences unique to nGPCR-x encoding polynucleotides can be deduced through use of any publicly available sequence database, and/or through use of commercially available sequence comparison programs. After identification of the desired sequences, isolation through restriction digestion or amplification using any of the various polymerase chain reaction techniques well known in the art can be performed. Anti-sense polynucleotides are particularly relevant to regulating expression of nGPCR-x by those cells expressing nGPCR-x mRNA.

Antisense nucleic acids (preferably 10 to 30 base-pair oligonucleotides) capable of specifically binding to nGPCR-x expression control sequences or nGPCR-x RNA are introduced into cells (*e.g*., by a viral vector or colloidal dispersion system such as a liposome). The antisense nucleic acid binds to the nGPCR-x target nucleotide sequence in the cell and prevents transcription and/or translation of the target sequence. Phosphorothioate and methylphosphonate antisense oligonucleotides are specifically contemplated for therapeutic use by the invention. The antisense oligonucleotides may be further modified by adding poly-L-lysine, transferrin polylysine, or cholesterol moieties at their 5' end. Suppression of nGPCR-x expression at either the transcriptional or translational level is useful to generate cellular or animal models for diseases/conditions characterized by aberrant nGPCR-x expression.

Antisense oligonucleotides, or fragments of sequences selected from the group consisting of SEQ ID NO:1 to SEQ ID NO:60, or sequences complementary or homologous thereto, derived from the nucleotide sequences of the present invention encoding nGPCR-x are useful as diagnostic tools for probing gene expression in various tissues. For example, tissue can be probed *in situ* with oligonucleotide probes carrying detectable groups by conventional autoradiography techniques to investigate native expression of this enzyme or pathological conditions relating thereto. Antisense oligonucleotides are preferably directed to regulatory regions of sequences selected from the group consisting of SEQ ID NO:1 to SEQ ID NO:60, or mRNA corresponding thereto, including, but not limited to, the initiation codon, TATA box, enhancer sequences, and the like.

### Transcription factors

The nGPCR-x sequences taught in the present invention facilitate the design of novel transcription factors for modulating nGPCR-x expression in native cells and animals, and cells transformed or transfected with nGPCR-x polynucleotides. For example, the Cys₂-His₂ zinc finger proteins, which bind DNA via their zinc finger domains, have been shown to be amenable to structural changes that lead to the recognition of different target sequences. These artificial zinc finger proteins recognize specific target sites with high affinity and low dissociation constants, and are able to act as gene switches to modulate gene expression. Knowledge of the particular nGPCR-x target sequence of the present invention facilitates the engineering of zinc finger proteins specific for the target sequence using known methods such as a combination of structure-based modeling and screening of phage display libraries (Segal *et al., Proc. Natl. Acad. Sci.* USA 96:2758-2763 (1999); Liu *et al., Proc. Natl. Acad. Sci.* USA 94:5525-5530 (1997); Greisman *et al., Science* 275:657-661 (1997); Choo *et al., J. Mol. Biol.* 273:525-532 (1997)). Each zinc finger domain usually recognizes three or more base pairs. Since a recognition sequence of 18 base pairs is generally sufficient in length to render it unique in any known genome, a zinc finger protein consisting of 6 tandem repeats of zinc fingers would be expected to ensure specificity for a particular sequence (Segal *et al*.). The artificial zinc finger repeats, designed based on nGPCR-x sequences, are fused to activation or repression domains to promote or suppress nGPCR-x expression (Liu *et al*.). Alternatively, the zinc finger domains can be fused to the TATA box-binding factor (TBP) with varying lengths of linker region between the zinc finger peptide and the TBP to create either transcriptional activators or repressors (Kim *et al., Proc. Natl. Acad. Sci.* USA 94:3616-3620 (1997). Such proteins and polynucleotides that encode them, have utility for modulating nGPCR-x expression *in vivo* in both native cells, animals and humans; and/or cells transfected with nGPCR-x-encoding sequences. The novel transcription factor can be delivered to the target cells by transfecting constructs that express the transcription factor (gene therapy), or by introducing the protein. Engineered zinc finger proteins can also be designed to bind RNA sequences for use in therapeutics as alternatives to antisense or catalytic RNA methods (McColl *et al., Proc. Natl. Acad. Sci.* USA 96:9521-9526 (1997); Wu *et al., Proc. Natl. Acad. Sci.* USA 92:344-348 (1995)). The present invention contemplates methods of designing such transcription factors based on the gene sequence of the invention, as well as customized zinc finger proteins, that are useful to modulate nGPCR-x expression in cells (native or transformed) whose genetic complement includes these sequences.

### Polypeptides

The invention also provides purified and isolated mammalian nGPCR-x polypeptides encoded by a polynucleotide of the invention. Presently preferred is a human nGPCR-x polypeptide comprising the amino acid sequence set out in sequences selected from the group consisting of SEQ ID NO:61 to SEQ ID NO:120, or fragments thereof comprising an epitope specific to the polypeptide. By "epitope specific to" is meant a portion of the nGPCR receptor that is recognizable by an antibody that is specific for the nGPCR, as defined in detail below.

Although the sequences provided are particular human sequences, the invention is intended to include within its scope other human allelic variants; non-human mammalian forms of nGPCR-x, and other vertebrate forms of nGPCR-x.

It will be appreciated that extracellular epitopes are particularly useful for generating ana screening for antibodies and other binding compounds that bind to receptors such as nGPCR-x. Thus, in another preferred embodiment, the invention provides a purified and isolated polypeptide comprising at least one extracellular domain (*e.g.*,the N-terminal extracellular domain or one of the three extracellular loops) of nGPCR-x. Purified and isolated polypeptides comprising the N-terminal extracellular domain of nGPCR-x are highly preferred. Also preferred is a purified and isolated polypeptide comprising a nGPCR-x fragment selected from the group consisting of the N-terminal extracellular domain of nGPCR-x, transmembrane domains of nGPCR-x, an extracellular loop connecting transmembrane domains of nGPCR-x, an intracellular loop connecting transmembrane domains of nGPCR-x, the C-terminal cytoplasmic region of nGPCR-x, and fusions thereof. Such fragments may be continuous portions of the native receptor. However, it will also be appreciated that knowledge of the nGPCR-x gene and protein sequences as provided herein permits recombining of various domains that are not contiguous in the native protein. Using a FORTRAN computer program called "tmtrest.all" (Parodi *et al., Comput. Appl. Biosci.* 5:527-535 (1994)), nGPCR-x was shown to contain transmembrane-spanning domains.

The invention also embraces polypeptides that have at least 99%, at least 95%, at least 90%, at least 85%, at least 80%, at least 75%, at least 70%, at least 65%, at least 60%, at least 55% or at least 50% identity and/or homology to the preferred polypeptide of the invention. Percent amino acid sequence "identity" with respect to the preferred polypeptide of the invention is defined herein as the percentage of amino acid residues in the candidate sequence that are identical with the residues in the nGPCR-x sequence after aligning both sequences and introducing gaps, if necessary, to achieve the maximum percent sequence identity, and not considering any conservative substitutions as part of the sequence identity. Percent sequence "homology" with respect to the preferred polypeptide of the invention is defined herein as the percentage of amino acid residues in the candidate sequence that are identical with the residues in the nGPCR-x sequence after aligning the sequences and introducing gaps, if necessary, to achieve the maximum percent sequence identity, and also considering any conservative substitutions as part of the sequence identity.

In one aspect, percent homology is calculated as the percentage of amino acid residues in the smaller of two sequences which align with identical amino acid residue in the sequence being compared, when four gaps in a length of 100 amino acids may be introduced to maximize alignment (Dayhoff, in *Atlas of Protein Sequence and Structure,* Vol. 5, p. 124, National Biochemical Research Foundation, Washington, D.C. (1972), incorporated herein by reference in its entirety).

Polypeptides of the invention may be isolated from natural cell sources or may be chemically synthesized, but are preferably produced by recombinant procedures involving host cells of the invention. Use of mammalian host cells is expected to provide for such post-translational modifications (*e.g*., glycosylation, truncation, lipidation, and phosphorylation) as may be needed to confer optimal biological activity on recombinant expression products of the invention.Glycosylated and non-glycosylated forms of nGPCR-x polypeptides are embraced by the invention.

The invention also embraces variant (or analog) nGPCR-x polypeptides. In one example, insertion variants are provided wherein one or more amino acid residues supplement a nGPCR-x amino acid sequence. Insertions may be located at either or both termini of the protein, or may be positioned within internal regions of the nGPCR-x amino acid sequence. Insertional variants with additional residues at either or both termini can include, for example, fusion proteins and proteins including amino acid tags or labels.

Insertion variants include nGPCR-x polypeptides wherein one or more amino acid residues are added to a nGPCR-x acid sequence or to a biologically active fragment thereof.

Variant products of the invention also include mature nGPCR-x products, *i.e*., nGPCR-x products wherein leader or signal sequences are removed, with additional amino terminal residues. The additional amino terminal residues may be derived from another protein, or may include one or more residues that are not identifiable as being derived from specific proteins. nGPCR-x products with an additional methionine residue at position -1 (Met⁻¹-nGPCR-x) are contemplated, as are variants with additional methionine and lysine residues at positions -2 and -1 (Met⁻²-Lys⁻¹-nGPCR-x). Variants of nGPCR-x with additional Met, Met-Lys, Lys residues (or one or more basic residues in general) are particularly useful for enhanced recombinant protein production in bacterial host cells.

The invention also embraces nGPCR-x variants having additional amino acid residues that result from use of specific expression systems. For example, use of commercially available vectors that express a desired polypeptide as part of a glutathione-S-transferase (GST) fusion product provides the desired polypeptide having an additional glycine residue at position -1 after cleavage of the GST component from the desired polypeptide. Variants that result from expression in other vector systems are also contemplated.

Insertional variants also include fusion proteins wherein the amino terminus and/or the carboxy terminus of nGPCR-x is/are fused to another polypeptide.

In another aspect, the invention provides deletion variants wherein one or more amino acid residues in a nGPCR-x polypeptide are removed. Deletions can be effected at one or both termini of the nGPCR-x polypeptide, or with removal of one or more non-terminal amino acid residues of nGPCR-x. Deletion variants, therefore, include all fragments of a nGPCR-x polypeptide.

The invention also embraces polypeptide fragments of sequences selected from the group consisting of SEQ ID NO:61 to SEQ ID NO:120, wherein the fragments maintain biological (*e.g*., ligand binding and/or intracellular signaling) immunological properties of a nGPCR-x polypeptide.

In one preferred embodiment of the invention, an isolated nucleic acid molecule comprises a nucleotide sequence that encodes a polypeptide comprising an amino acid sequence homologous to sequences selected from the group consisting of SEQ ID NO:61 to SEQ ID NO:120, and fragments thereof, wherein the nucleic acid molecule encoding at least a portion of nGPCR-x. In a more preferred embodiment, the isolated nucleic acid molecule comprises a sequence that encodes a polypeptide comprising sequences selected from the group consisting of SEQ ID NO:61 to SEQ ID NO:120, and fragments thereof.

As used in the present invention, polypeptide fragments comprise at least 5, 10, 15, 20, 25, 30, 35, or 40 consecutive amino acids of sequences selected from the group consisting of SEQ ID NO:61 to SEQ ID NO:120. Preferred polypeptide fragments display antigenic properties unique to, or specific for, human nGPCR-x and its allelic and species homologs. Fragments of the invention having the desired biological and immunological properties can be prepared by any of the methods well known and routinely practiced in the art.

In still another aspect, the invention provides substitution variants of nGPCR-x polypeptides. Substitution variants include those polypeptides wherein one or more amino acid residues of a nGPCR-x polypeptide are removed and replaced with alternative residues. In one aspect, the substitutions are conservative in nature; however, the invention embraces substitutions that are also non-conservative. Conservative substitutions for this purpose may be defined as set out in Tables 2, 3, or 4 below.

Variant polypeptides include those wherein conservative substitutions have been introduced by modification of polynucleotides encoding polypeptides of the invention. Amino acids can be classified according to physical properties and contribution to secondary and tertiary protein structure. A conservative substitution is recognized in the art as a substitution of one amino acid for another amino acid that has similar properties. Exemplary conservative substitutions are set out in Table 2 (from WO 97/09433, page 10, published March 13, 1997 (PCT/GB96/02197, filed 9/6/96), immediately below.

**Table 2 Conservative Substitutions I**

| **SIDE CHAIN CHARACTERISTIC** | | **AMINO ACID** |
|---|---|---|
| Aliphatic | | |
| | Non-polar | G A P |
| | | I L V |
| | Polar - uncharged | C S T M |
| | | N Q |
| | Polar - charged | D E |
| | | K R |
| Aromatic | | H F W Y |
| Other | | N Q D E |

Alternatively, conservative amino acids can be grouped as described in Lehninger, (*Biochemistry,* Second Edition; Worth Publishers, Inc. NY, NY (1975), pp.71-77) as set out in Table 3, below.

**Table 3 Conservative Substitutions II**

| **SIDE CHAIN CHARACTERISTIC** | **AMINO ACID** |
|---|---|
| Non-polar (hydrophobic) | |
| A. Aliphatic: | A L I V P |
| B. Aromatic: | F W |
| C. Sulfur-containing: | M |
| D. Borderline: | G |

| Uncharged-polar | |
|---|---|
| A. Hydroxyl: | S T Y |
| B. Amides: | N Q |
| C. Sulfhydryl: | C |
| D. Borderline: | G |
| Positively Charged (Basic): | K R H |
| Negatively Charged (Acidic): | D E |

As still another alternative, exemplary conservative substitutions are set out in Table 4, below.

**Table 4 Conservative Substitutions III**

| **Original Residue** | **Exemplary Substitution** |
|---|---|
| Ala (A) | Val, Leu, Ile |
| Arg(R) | Lys, Gln, Asn |
| Asn(N) | Gln, His, Lys, Arg |
| Asp(D) | Glu |
| Cys(C) | Ser |
| Gln(Q) | Asn |
| Glu(E) | Asp |
| His(H) | Asn, Gln, Lys, Arg |
| Ile(I) | Leu, Val, Met, Ala, Phe, |
| Leu(L) | Ile, Val, Met, Ala, Phe |
| Lys(K) | Arg, Gln, Asn |
| Met(M) | Leu, Phe, Ile |
| Phe(F) | Leu, Val, Ile, Ala |
| Pro(P) | Gly |
| Ser(S) | Thr |
| Thr(T) | Ser |
| Trp(W) | Tyr |
| Tyr(Y) | Trp, Phe, Thr, Ser |
| Val(V) | Ile, Leu, Met, Phe, Ala |

It should be understood that the definition of polypeptides of the invention is intended to include polypeptides bearing modifications other than insertion, deletion, or substitution of amino acid residues. By way of example, the modifications may be covalent in nature, and include for example, chemical bonding with polymers, lipids, other organic, and inorganic moieties. Such derivatives may be prepared to increase circulating half-life of a polypeptide, or may be designed to improve the targeting capacity of the polypeptide for desired cells, tissues, or organs. Similarly, the invention further embraces nGPCR-x polypeptides that have been covalently modified to include one or more water-soluble polymer attachments such as polyethylene glycol, polyoxyethylene glycol, or polypropylene glycol. Variants that display ligand binding properties of native nGPCR-x and are expressed at higher levels, as well as variants that provide for constitutively active receptors, are particularly useful in assays of the invention; the variants are also useful in providing cellular, tissue and animal models of diseases/conditions characterized by aberrant nGPCR-x activity.

In a related embodiment, the present invention provides compositions comprising purified polypeptides of the invention. Preferred compositions comprise, in addition to the polypeptide of the invention, a pharmaceutically acceptable (*i.e*., sterile and non-toxic) liquid, semisolid, or solid diluent that serves as a pharmaceutical vehicle, excipient, or medium. Any diluent known in the art may be used. Exemplary diluents include, but are not limited to, water, saline solutions, polyoxyethylene sorbitan monolaurate, magnesium stearate, methyl- and propylhydroxybenzoate, talc, alginates, starches, lactose, sucrose, dextrose, sorbitol, mannitol, glycerol, calcium phosphate, mineral oil, and cocoa butter.

Variants that display ligand binding properties of native nGPCR-x and are expressed at higher levels, as well as, variants that provide for constitutively active receptors, are particularly useful in assays of the invention; the variants are also useful in assays of the invention and in providing cellular, tissue and animal models of diseases/conditions characterized by aberrant nGPCR-x activity.

The G protein-coupled receptor functions through a specific heterotrimeric guanine-nucleotide-binding regulatory protein (G-protein) coupled to the intracellular portion of the G protein-coupled receptor molecule. Accordingly, the G protein-coupled receptor has a specific affinity to G protein. G proteins specifically bind to guanine nucleotides. Isolation of G proteins provides a means to isolate guanine nucleotides. G Proteins may be isolated using commercially available anti-G protein antibodies or isolated G protein-coupled receptors. Similarly, G proteins may be detected in a sample isolated using commercially available detectable anti-G protein antibodies or isolated G protein-coupled receptors.

According to the present invention, the isolated n-GPCR-x proteins of the present invention are useful to isolate and purify G proteins from samples such as cell lysates. Example. 14 below sets forth an example of isolation of G proteins using isolated n-GPCR-x proteins. Such methodolgy may be used in place of the use of commercially available anti-G protein antibodies which are used to isolate G proteins. Moreover, G proteins may be detected using n-GPCR-x proteins in place of commercially available detectable anti-G protein antibodies. Since n-GPCR-x proteins specifically bind to G proteins, they can be employed in any specific use where G protein specific affinity is required such as those uses where commercially available anti-G protein antibodies are employed.

### Antibodies

Also comprehended by the present invention are antibodies (*e.g*., monoclonal and polyclonal antibodies, single chain antibodies, chimeric antibodies, bifunctional/bispecific antibodies, humanized antibodies, human antibodies, and complementary determining region (CDR)-grafted antibodies, including compounds which include CDR sequences which specifically recognize a polypeptide of the invention) specific for nGPCR-x or fragments thereof. Preferred antibodies of the invention are human antibodies that are produced and identified according to methods described in WO93/11236, published June 20, 1993, which is incorporated herein by reference in its entirety. Antibody fragments, including Fab, Fab', F(ab')₂, and Fᵥ, are also provided by the invention. The term "specific for," when used to describe antibodies of the invention, indicates that the variable regions of the antibodies of the invention recognize and bind nGPCR-x polypeptides exclusively (*i.e.,* are able to distinguish nGPCR-x polypeptides from other known GPCR polypeptides by virtue of measurable differences in binding affinity, despite the possible existence of localized sequence identity, homology, or similarity between nGPCR-x and such polypeptides). It will be understood that specific antibodies may also interact with other proteins (for example, *S*. *aureus* protein A or other antibodies in ELISA techniques) through interactions with sequences outside the variable region of the antibodies, and, in particular, in the constant region of the molecule. Screening assays to determine binding specificity of an antibody of the invention are well known and routinely practiced in the art. For a comprehensive discussion of such assays, see Harlow *et al.* (Eds.),*Antibodies A Laboratory Manual*; Cold Spring Harbor Laboratory; Cold Spring Harbor, NY (1988), Chapter 6. Antibodies that recognize and bind fragments of the nGPCR-x polypeptides of the invention are also contemplated, provided that the antibodies are specific for nGPCR-x polypeptides. Antibodies of the invention can be produced using any method well known and routinely practiced in the art.

The invention provides an antibody that is specific for the nGPCR-x of the invention. Antibody specificity is described in greater detail below. However, it should be emphasized that antibodies that can be generated from polypeptides that have previously been described in the literature and that are capable of fortuitously cross-reacting with nGPCR-x (*e.g.,* due to the fortuitous existence of a similar epitope in both polypeptides) are considered "cross-reactive" antibodies. Such cross-reactive antibodies are not antibodies that are "specific" for nGPCR-x. The determination of whether an antibody is specific for nGPCR-x or is cross-reactive with another known receptor is made using any of several assays, such as Western blotting assays, that are well known in the art. For identifying cells that express nGPCR-x and also for modulating nGPCR-x-ligand binding activity, antibodies that specifically bind to an extracellular epitope of the nGPCR-x are preferred.

In one preferred variation, the invention provides monoclonal antibodies. Hybridomas that produce such antibodies also are intended as aspects of the invention. In yet another variation, the invention provides a humanized antibody. Humanized antibodies are useful for *in vivo* therapeutic indications.

In another variation, the invention provides a cell-free composition comprising polyclonal antibodies, wherein at least one of the antibodies is an antibody of the invention specific for nGPCR-x. Antisera isolated from an animal is an exemplary composition, as is a composition comprising an antibody fraction of an antisera that has been resuspended in water or in another diluent, excipient, or carrier.

In still another related embodiment, the invention provides an anti-idiotypic antibody specific for an antibody that is specific for nGPCR-x.

It is well known that antibodies contain relatively small antigen binding domains that can be isolated chemically or by recombinant techniques. Such domains are useful nGPCR-x binding molecules themselves, and also may be reintroduced into human antibodies, or fused to toxins or other polypeptides. Thus, in still another embodiment, the invention provides a polypeptide comprising a fragment of a nGPCR-x-specific antibody, wherein the fragment and the polypeptide bind to the nGPCR-x. By way of non-limiting example, the invention provides polypeptides that are single chain antibodies and CDR-grafted antibodies.

Non-human antibodies may be humanized by any of the methods known in the art. In one method, the non-human CDRs are inserted into a human antibody or consensus antibody framework sequence. Further changes can then be introduced into the antibody framework to modulate affinity or immunogenicity.

Antibodies of the invention are useful for, *e.g.,* therapeutic purposes (by modulating activity of nGPCR-x), diagnostic purposes to detect or quantitate nGPCR-x, and purification of nGPCR-x. Kits comprising an antibody of the invention for any of the purposes described herein are also comprehended. In general, a kit of the invention also includes a control antigen for which the antibody is immunospecific.

### Compositions

Mutations in the nGPCR-x gene that result in loss of normal function of the nGPCR-x gene product underlie nGPCR-x-related human disease states. The invention comprehends gene therapy to restore nGPCR-x activity to treat those disease states. Delivery of a functional nGPCR-x gene to appropriate cells is effected *ex vivo, in situ,* or *in vivo* by use of vectors, and more particularly viral vectors (*e.g*., adenovirus, adeno-associated virus, or a retrovirus), or *ex vivo* by use of physical DNA transfer methods (*e.g.,* liposomes or chemical treatments). See, for example, Anderson, *Nature,* supplement to vol. 392, no. 6679, pp.25-20 (1998). For additional reviews of gene therapy technology see Friedmann, *Science, 244:* 1275-1281 (1989); Verma, *Scientific American:* 68-84 (1990); and Miller, *Nature, 357:* 455-460 (1992). Alternatively, it is contemplated that in other human disease states, preventing the expression of, or inhibiting the activity of, nGPCR-x will be useful in treating disease states. It is contemplated that antisense therapy or gene therapy could be applied to negatively regulate the expression of nGPCR-x.

Another aspect of the present invention is directed to compositions, including pharmaceutical compositions, comprising any of the nucleic acid molecules or recombinant expression vectors described above and an acceptable carrier or diluent. Preferably, the carrier or diluent is pharmaceutically acceptable. Suitable carriers are described in the most recent edition of *Remington's Pharmaceutical Sciences,* A. Osol, a standard reference text in this field, which is incorporated herein by reference in its entirety. Preferred examples of such carriers or diluents include, but are not limited to, water, saline, Ringer's solution, dextrose solution, and 5% human serum albumin. Liposomes and nonaqueous vehicles such as fixed oils may also be used. The formulations are sterilized by commonly used techniques.

Also within the scope of the invention are compositions comprising polypeptides, polynucleotides, or antibodies of the invention that have been formulated with, *e.g*., a pharmaceutically acceptable carrier.

The invention also provides methods of using antibodies of the invention. For example, the invention provides a method for modulating ligand binding of a nGPCR-x comprising the step of contacting the nGPCR-x with an antibody specific for the nGPCR-x, under conditions wherein the antibody binds the receptor.

GPCRs that may be expressed in the brain, such as nGPCR-42,46,48,49, 51, 52, 61, 63, or 70, provide an indication that aberrant nGPCR-42, 46, 48, 49, 51, 52, 61, 63, or 70 signaling activity may correlate with one or more neurological or psychological disorders. The invention also provides a method for treating a neurological or psychiatric disorder comprising the step of administering to a mammal in need of such treatment an amount of an antibody-like polypeptide of the invention that is sufficient to modulate ligand binding to a nGPCR-42, 46, 48, 49, 51, 52, 61, 63, or 70 in neurons of the mammal. nGPCR-42, 46, 48, 49, 51, 52, 61, 63, or 70 may also be expressed in other tissues, including but not limited to, peripheral blood lymphocytes, pancreas, ovary, uterus, testis, salivary gland, thyroid gland, kidney, adrenal gland, liver, bone marrow, prostate, fetal liver, colon, muscle, and fetal brain, and may be found in many other tissues. Within the brain, nGPCR-42, 46, 48, 49, 51, 52, 61, 63, or 70 mRNA transcripts may be found in many tissues, including, but not limited to, frontal lobe, hypothalamus, pons, cerebellum, caudate nucleus, and medulla. Tissues and brain regions where specific nGPCRs of the present invention are expressed are identified in the Examples below. **Kits**

The present invention is also directed to kits, including pharmaceutical kits. The kits can comprise any of the nucleic acid molecules described above, any of the polypeptides described above, or any antibody which binds to a polypeptide of the invention as described above, as well as a negative control. The kit preferably comprises additional components, such as, for example, instructions, solid support, reagents helpful for quantification, and the like.

In another aspect, the invention features methods for detection of a polypeptide in a sample as a diagnostic tool for diseases or disorders, wherein the method comprises the steps of: (a) contacting the sample with a nucleic acid probe which hybridizes under hybridization assay conditions to a nucleic acid target region of a polypeptide having sequences selected from the group consisting of SEQ ID NO:61 to SEQ ID NO:120, said probe comprising the nucleic acid sequence encoding the polypeptide, fragments thereof, and the complements of the sequences and fragments; and (b) detecting the presence or amount of the probe:target region hybrid as an indication of the disease.

In preferred embodiments of the invention, the disease is selected from the group consisting of thyroid disorders (*e.g*. thyreotoxicosis, myxoedema); renal failure; inflammatory conditions (*e.g*., Crohn's disease); diseases related to cell differentiation and homeostasis; rheumatoid arthritis; autoimmune disorders; movement disorders; CNS disorders (*e.g*., pain including migraine; stroke; psychotic and neurological disorders, including anxiety, mental disorder, manic depression, anxiety, generalized anxiety disorder, post-traumatic-stress disorder, depression, bipolar disorder, delirium, dementia, severe mental retardation; dyskinesias, such as Huntington's disease or Tourette's Syndrome; attention disorders including attention deficit disorder (ADD) and attention deficit-hyperactivity disorder (ADHD), and degenerative disorders such as Parkinson's, Alzheimer's; movement disorders, including ataxias, supranuclear palsy, *etc*.); infections, such as viral infections caused by HIV-1 or HIV-2; metabolic and cardiovascular diseases and disorders (*e.g*., type 2 diabetes, impaired glucose tolerance, dyslipidemia, obesity, anorexia, hypotension, hypertension, thrombosis, myocardial infarction, cardiomyopathies, atherosclerosis, *etc.*); proliferative diseases and cancers (*e.g*., different cancers such as breast, colon, lung, *etc*., and hyperproliferative disorders such as psoriasis, prostate hyperplasia, *etc*.); hormonal disorders (*e.g*., male/female hormonal replacement, polycystic ovarian syndrome, alopecia, *etc*.); and sexual dysfunction, among others.

As described above and in Example 5 below, the genes encoding nGPCR-42, 46, 48, 49, 51, 52, 61, 63, and 70 have been detected in brain tissue indicating that these n-GPCR-x proteins are neuroreceptors. Kits may be designed to detect either expression of polynucleotides encoding these proteins or the proteins themselves in order to identify tissue as being neurological. For example, oligonucleotide hybridization kits can be provided which include a container having an oligonucleotide probe specific for the n-GPCR-x-specific DNA and optionally, containers with positive and negative controls and/or instructions. Similarly, PCR kits can be provided which include a container having primers specific for the n-GPCR-x-specific sequences, DNA and optionally, containers with size markers, positive and negative controls and/or instructions.

Hybridization conditions should be such that hybridization occurs only with the genes in the presence of other nucleic acid molecules. Under stringent hybridization conditions only highly complementary nucleic acid sequences hybridize. Preferably, such conditions prevent hybridization of nucleic acids having 1 or 2 mismatches out of 20 contiguous nucleotides. Such conditions are defined supra.

The diseases for which detection of genes in a sample could be diagnostic include diseases in which nucleic acid (DNA and/or RNA) is amplified in comparison to normal cells. By "amplification" is meant increased numbers of DNA or RNA in a cell compared with normal cells.

The diseases that could be diagnosed by detection of nucleic acid in a sample preferably include central nervous system and metabolic diseases. The test samples suitable for nucleic acid probing methods of the present invention include, for example, cells or nucleic acid extracts of cells, or biological fluids. The samples used in the above-described methods will vary based on the assay format, the detection method and the nature of the tissues, cells or extracts to be assayed. Methods for preparing nucleic acid extracts of cells are well known in the art and can be readily adapted in order to obtain a sample that is compatible with the method utilized.

Alternatively, immunoassay kits can be provided which have containers container having antibodies specific for the n-GPCR-x-protein and optionally, containers with positive and negative controls and/or instructions.

Kits may also be provided useful in the identification of GPCR binding partners such as natural ligands or modulators (agonists or antagonists). Substances useful for treatment of disorders or diseases preferably show positive results in one or more *in vitro* assays for an activity corresponding to treatment of the disease or disorder in question. Substances that modulate the activity of the polypeptides preferably include, but are not limited to, antisense oligonucleotides, agonists and antagonists, and inhibitors of protein kinases.

### Methods of inducing immune response

Another aspect of the present invention is directed to methods of inducing an immune response in a mammal against a polypeptide of the invention by administering to the mammal an amount of the polypeptide sufficient to induce an immune response. The amount will be dependent on the animal species, size of the animal, and the like but can be determined by those skilled in the art.

### Methods of identifying ligands

The invention also provides assays to identify compounds that bind nGPCR-x. One such assay comprises the steps of: (a) contacting a composition comprising a nGPCR-x with a compound suspected of binding nGPCR-x; and (b) measuring binding between the compound and nGPCR-x. In one variation, the composition comprises a cell expressing nGPCR-x on its surface. In another variation, isolated nGPCR-x or cell membranes comprising nGPCR-x are employed. The binding may be measured directly, *e.g*., by using a labeled compound, or may be measured indirectly by several techniques, including measuring intracellular signaling of nGPCR-x induced by the compound (or measuring changes in the level of nGPCR-x signaling). Following steps (a) and (b), compounds identified as binding nGPCR-x can be further tested in other assays including, but not limited to, *in vivo* models, in order to confirm or quantitate binding to nGPCR-x.

Specific binding molecules, including natural ligands and synthetic compounds, can be identified or developed using isolated or recombinant nGPCR-x products, nGPCR-x variants, or preferably, cells expressing such products. Binding partners are useful for purifying nGPCR-x products and detection or quantification of nGPCR-x products in fluid and tissue samples using known immunological procedures. Binding molecules are also manifestly useful in modulating (*i.e*., blocking, inhibiting or stimulating) biological activities of nGPCR-x, especially those activities involved in signal transduction.

The DNA and amino acid sequence information provided by the present invention also makes possible identification of binding partner compounds with which a nGPCR-x polypeptide or polynucleotide will interact. Methods to identify binding partner compounds include solution assays, *in vitro* assays wherein nGPCR-x polypeptides are immobilized, and cell-based assays. Identification of binding partner compounds of nGPCR-x polypeptides provides candidates for therapeutic or prophylactic intervention in pathologies associated with nGPCR-x normal and aberrant biological activity.

The invention includes several assay systems for identifying nGPCR-x binding partners. In solution assays, methods of the invention comprise the steps of (a) contacting a nGPCR-x polypeptide with one or more candidate binding partner compounds and (b) identifying the compounds that bind to the nGPCR-x polypeptide. Identification of the compounds that bind the nGPCR-x polypeptide can be achieved by isolating the nGPCR-x polypeptide/binding partner complex, and separating the binding partner compound from the nGPCR-x polypeptide. An additional step of characterizing the physical, biological, and/or biochemical properties of the binding partner compound is also comprehended in another embodiment of the invention, wherein compounds identified as binding nGPCR-x can be further tested in other assays including, but not limited to, *in vivo* models, in order to confirm or quantitate binding to nGPCR-x. In one aspect, the nGPCR-x polypeptide/binding partner complex is isolated using an antibody immunospecific for either the nGPCR-x polypeptide or the candidate binding partner compound.

In still other embodiments, either the nGPCR-x polypeptide or the candidate binding partner compound comprises a label or tag that facilitates its isolation, and methods of the invention to identify binding partner compounds include a step of isolating the nGPCR-x polypeptide/binding partner complex through interaction with the label or tag. An exemplary tag of this type is a poly-histidine sequence, generally around six histidine residues, that permits isolation of a compound so labeled using nickel chelation. Other labels and tags, such as the FLAG® tag (Eastman Kodak, Rochester, NY), well known and routinely used in the art, are embraced by the invention.

In one variation of an *in vitro* assay, the invention provides a method comprising the steps of (a) contacting an immobilized nGPCR-x polypeptide with a candidate binding partner compound and (b) detecting binding of the candidate compound to the nGPCR-x polypeptide. In an alternative embodiment, the candidate binding partner compound is immobilized and binding of nGPCR-x is detected. Immobilization is accomplished using any of the methods well known in the art, including covalent bonding to a support, a bead, or a chromatographic resin, as well as non-covalent, high affinity interactions such as antibody binding, or use of streptavidin/biotin binding wherein the immobilized compound includes a biotin moiety. Detection of binding can be accomplished (i) using a radioactive label on the compound that is not immobilized, (ii) using of a fluorescent label on the non-immobilized compound, (iii) using an antibody immunospecific for the non-immobilized compound, (iv) using a label on the non-immobilized compound that excites a fluorescent support to which the immobilized compound is attached, as well as other techniques well known and routinely practiced in the art.

The invention also provides cell-based assays to identify binding partner compounds of a nGPCR-x polypeptide. In one embodiment, the invention provides a method comprising the steps of contacting a nGPCR-x polypeptide expressed on the surface of a cell with a candidate binding partner compound and detecting binding of the candidate binding partner compound to the nGPCR-x polypeptide. In a preferred embodiment, the detection comprises detecting a calcium flux or other physiological event in the cell caused by the binding of the molecule.

Another aspect of the present invention is directed to methods of identifying compounds that bind to either nGPCR-x or nucleic acid molecules encoding nGPCR-x, comprising contacting nGPCR-x, or a nucleic acid molecule encoding the same, with a compound, and determining whether the compound binds nGPCR-x or a nucleic acid molecule encoding the same. Binding can be determined by binding assays which are well known to the skilled artisan, including, but not limited to, gel-shift assays, Western blots, radiolabeled competition assay, phage-based expression cloning, co-fractionation by chromatography, co-precipitation, cross linking, interaction trap/two-hybrid analysis, southwestern analysis, ELISA, and the like, which are described in, for example, *Current Protocols in Molecular Biology,* **1999**, John Wiley & Sons, NY, which is incorporated herein by reference in its entirety. The compounds to be screened include (which may include compounds which are suspected to bind nGPCR-x, or a nucleic acid molecule encoding the same), but are not limited to, extracellular, intracellular, biologic or chemical origin. The methods of the invention also embrace ligands, especially neuropeptides, that are attached to a label, such as a radiolabel (*e.g*., ¹²⁵I, ³⁵S, ³²P, ³³P, ³H), a fluorescence label, a chemiluminescent label, an enzymic label and an immunogenic label. Modulators falling within the scope of the invention include, but are not limited to, non-peptide molecules such as non-peptide mimetics, non-peptide allosteric effectors, and peptides. The nGPCR-x polypeptide or polynucleotide employed in such a test may either be free in solution, attached to a solid support, borne on a cell surface or located intracellularly or associated with a portion of a cell. One skilled in the art can, for example, measure the formation of complexes between nGPCR-x and the compound being tested. Alternatively, one skilled in the art can examine the diminution in complex formation between nGPCR-x and its substrate caused by the compound being tested.

In another embodiment of the invention, high throughput screening for compounds having suitable binding affinity to nGPCR-x is employed. Briefly, large numbers of different test compounds are synthesized on a solid substrate. The peptide test compounds are contacted with nGPCR-x and washed. Bound nGPCR-x is then detected by methods well known in the art. Purified polypeptides of the invention can also be coated directly onto plates for use in the aforementioned drug screening techniques. In addition, non-neutralizing antibodies can be used to capture the protein and immobilize it on the solid support.

Generally, an expressed nGPCR-x can be used for HTS binding assays in conjunction with its defined ligand, in this case the corresponding neuropeptide that activates it. The identified peptide is labeled with a suitable radioisotope, including, but not limited to, ¹²⁵I, ³H, ³⁵S or ³²P, by methods that are well known to those skilled in the art. Alternatively, the peptides may be labeled by well-known methods with a suitable fluorescent derivative (Baindur *et al., Drug Dev. Res.,* **1994**, *33*, 373-398; Rogers, *Drug Discovery Today,* **1997***, 2*, 156-160). Radioactive ligand specifically bound to the receptor in membrane preparations made from the cell line expressing the recombinant protein can be detected in HTS assays in one of several standard ways, including filtration of the receptor-ligand complex to separate bound ligand from unbound ligand (Williams, *Med. Res. Rev.,* **1991**, *11*, 147-184; Sweetnam *et al., J. Natural Products,* **1993**, *56,* 441-455). Alternative methods include a scintillation proximity assay (SPA) or a FlashPlate format in which such separation is unnecessary (Nakayama, *Cur. Opinion Drug Disc. Dev*.,**1998**, *1*, 85-91 Bossé *et al., J. Biomolecular Screening,* **1998***, 3*, 285-292.). Binding of fluorescent ligands can be detected in various ways, including fluorescence energy transfer (FRET), direct spectrophotofluorometric analysis of bound ligand, or fluorescence polarization (Rogers, *Drug Discovery Today,* **1997**, *2*, 156-160; Hill, *Cur. Opinion Drug Disc. Dev.,* **1998**, *1*, 92-97).

Other assays may be used to identify specific ligands of a nGPCR-x receptor, including assays that identify ligands of the target protein through measuring direct binding of test ligands to the target protein, as well as assays that identify ligands of target proteins through affinity ultrafiltration with ion spray mass spectroscopy/HPLC methods or other physical and analytical methods. Alternatively, such binding interactions are evaluated indirectly using the yeast two-hybrid system described in Fields *et al., Nature,* 340:245-246 (1989), and Fields *et al., Trends in Genetics,* 10:286-292 (1994), both of which are incorporated herein by reference in its entirety. The two-hybrid system is a genetic assay for detecting interactions between two proteins or polypeptides. It can be used to identify proteins that bind to a known protein of interest, or to delineate domains or residues critical for an interaction. Variations on this methodology have been developed to clone genes that encode DNA binding proteins, to identify peptides that bind to a protein, and to screen for drugs. The two-hybrid system exploits the ability of a pair of interacting proteins to bring a transcription activation domain into close proximity with a DNA binding domain that binds to an upstream activation sequence (UAS) of a reporter gene, and is generally performed in yeast. The assay requires the construction of two hybrid genes encoding (1) a DNA-binding domain that is fused to a first protein and (2) an activation domain fused to a second protein. The DNA-binding domain targets the first hybrid protein to the UAS of the reporter gene; however, because most proteins lack an activation domain, this DNA-binding hybrid protein does not activate transcription of the reporter gene. The second hybrid protein, which contains the activation domain, cannot by itself activate expression of the reporter gene because it does not bind the UAS. However, when both hybrid proteins are present, the noncovalent interaction of the first and second proteins tethers the activation domain to the UAS, activating transcription of the reporter gene. For example, when the first protein is a GPCR gene product, or fragment thereof, that is known to interact with another protein or nucleic acid, this assay can be used to detect agents that interfere with the binding interaction. Expression of the reporter gene is monitored as different test agents are added to the system. The presence of an inhibitory agent results in lack of a reporter signal.

The yeast two-hybrid assay can also be used to identify proteins that bind to the gene product. In an assay to identify proteins that bind to a nGPCR-x receptor, or fragment thereof, a fusion polynucleotide encoding both a nGPCR-x receptor (or fragment) and a UAS binding domain (*i.e*., a first protein) may be used. In addition, a large number of hybrid genes each encoding a different second protein fused to an activation domain are produced and screened in the assay. Typically, the second protein is encoded by one or more members of a total cDNA or genomic DNA fusion library, with each second protein-coding region being fused to the activation domain. This system is applicable to a wide variety of proteins, and it is not even necessary to know the identity or function of the second binding protein. The system is highly sensitive and can detect interactions not revealed by other methods; even transient interactions may trigger transcription to produce a stable mRNA that can be repeatedly translated to yield the reporter protein.

Other assays may be used to search for agents that bind to the target protein. One such screening method to identify direct binding of test ligands to a target protein is described in U.S. Patent No. 5,585,277, incorporated herein by reference in its entirety. This method relies on the principle that proteins generally exist as a mixture of folded and unfolded states, and continually alternate between the two states. When a test ligand binds to the folded form of a target protein *(i.e.,* when the test ligand is a ligand of the target protein), the target protein molecule bound by the ligand remains in its folded state. Thus, the folded target protein is present to a greater extent in the presence of a test ligand which binds the target protein, than in the absence of a ligand. Binding of the ligand to the target protein can be determined by any method that distinguishes between the folded and unfolded states of the target protein. The function of the target protein need not be known in order for this assay to be performed. Virtually any agent can be assessed by this method as a test ligand, including, but not limited to, metals, polypeptides, proteins, lipids, polysaccharides, polynucleotides and small organic molecules.

Another method for identifying ligands of a target protein is described in Wieboldt *et al., Anal. Chem.,* 69:1683-1691 (1997), incorporated herein by reference in its entirety. This technique screens combinatorial libraries of 20-30 agents at a time in solution phase for binding to the target protein. Agents that bind to the target protein are separated from other library components by simple membrane washing. The specifically selected molecules that are retained on the filter are subsequently liberated from the target protein and analyzed by HPLC and pneumatically assisted electrospray (ion spray) ionization mass spectroscopy. This procedure selects library components with the greatest affinity for the target protein, and is particularly useful for small molecule libraries.

Determining whether a test compound binds to nGPCR-51 can also be accomplished by measuring the intrinsic fluorescence of nGPCR-51 and determining whether the intrinsic fluorescence is modulated in the presence of the test compound. Preferably, the intrinsic fluorescence of nGPCR-51 is measured as a function of the tryptophan residue(s) of nGPCR-51. Preferably, fluorescence of nGPCR-51 is measured and compared to the fluorescence intensity of nGPCR-51 in the presence of the test compound, wherein a decrease in fluorescence intensity indicates binding of the test compound to nGPCR-51. Preferred methodology is set forth in "Principles of Fluorescence Spectroscopy" by Joseph R. Lakowicz, New York, Plenum Press, **1983** (ISBN 0306412853) and "Spectrophotometry And Spectrofluorometry" by C.L. Bashford and D.A. Harris Oxford, Washington DC, IRL Press, **1987** (ISBN 0947946691), each of which is incorporated herein by reference in its entirety.

Other embodiments of the invention comprise using competitive screening assays in which neutralizing antibodies capable of binding a polypeptide of the invention specifically compete with a test compound for binding to the polypeptide. In this manner, the antibodies can be used to detect the presence of any peptide that shares one or more antigenic determinants with nGPCR-x. Radiolabeled competitive binding studies are described in A.H. Lin *et al. Antimicrobial Agents and Chemotherapy,* **1997**, vol. 41, no. 10. pp. 2127-2131, the disclosure of which is incorporated herein by reference in its entirety.

Another aspect of the present invention relates to methods of identifying a compound that binds to or modulates nGPCR-51. The methods comprise contacting a composition comprising nGPCR-51 and Peptide A with a test compound, or a plurality of test compounds, and detrmining whether the test compound competes with Peptide A for binding to nGPCR-51. A decrease in the amount of the complex between Peptide A, or a protein homologous thereto, and nGPCR-51 in the presence of a test compound or compounds confirms that the compound or compounds binds to nGPCR-51. In some embodiments, the affinity or displacement of Peptide A is measured, wherein a low affinity indicates that the test compound interacts with nGPCR-51. In these methods, the composition that comprises nGPCR-51 and Peptide A can be cells. Compounds identified as binding to nGPCR-51 are also expected to modulate nGPCR-51 activity. Binding of a test compound to nGPCR-51 can be determined by any of the binding assays described above.

As described above and in Example 5 below, the genes encoding nGPCR-42, 46, 48, 49, 51, 52, 61, 63, and 70 have been detected in brain tissue indicating that these n-GPCR-x proteins are neuroreceptors. Accordingly, natural binding partners of these molecules include neurotransmitters.

### Identification of modulating agents

The invention also provides methods for identifying a modulator of binding between a nGPCR-x and a nGPCR-x binding partner, comprising the steps of: (a) contacting a nGPCR-x binding partner and a composition comprising a nGPCR-x in the presence and in the absence of a putative modulator compound; (b) detecting binding between the binding partner and the nGPCR-x; and (c) identifying a putative modulator compound or a modulator compound in view of decreased or increased binding between the binding partner and the nGPCR-x in the presence of the putative modulator, as compared to binding in the absence of the putative modulator. Following steps (a) and (b), compounds identified as modulating binding between nGPCR-x and an nGPCR-x binding partner can be further tested in other assays including, but not limited to, *in vivo* models, in order to confirm or quantitate modulation of binding to nGPCR-x.

nGPCR-x binding partners that stimulate nGPCR-x activity are useful as agonists in disease states or conditions characterized by insufficient nGPCR-x signaling (*e.g*., as a result of insufficient activity of a nGPCR-x ligand). nGPCR-x binding partners that block ligand-mediated nGPCR-x signaling are useful as nGPCR-x antagonists to treat disease states or conditions characterized by excessive nGPCR-x signaling. In addition nGPCR-x modulators in general, as well as nGPCR-x polynucleotides and polypeptides, are useful in diagnostic assays for such diseases or conditions.

In another aspect, the invention provides methods for treating a disease or abnormal condition by administering to a patient in need of such treatment a substance that modulates the activity or expression of a polypeptide having sequences selected from the group consisting of SEQ ID NO:61 to SEQ ID NO:120.

Agents that modulate (*i.e*., increase, decrease, or block) nGPCR-x activity or expression may be identified by incubating a putative modulator with a cell containing a nGPCR-x polypeptide or polynucleotide and determining the effect of the putative modulator on nGPCR-x activity or expression. The selectivity of a compound that modulates the activity of nGPCR-x can be evaluated by comparing its effects on nGPCR-x to its effect on other GPCR compounds. Following identification of compounds that modulate nGPCR-x activity or expression, such compounds can be further tested in other assays including, but not limited to, *in vivo* models, in order to confirm or quantitate their activity. Selective modulators may include, for example, antibodies and other proteins, peptides, or organic molecules that specifically bind to a nGPCR-x polypeptide or a nGPCR-x-encoding nucleic acid. Modulators of nGPCR-x activity will be therapeutically useful in treatment of diseases and physiological conditions in which normal or aberrant nGPCR-x activity is involved. nGPCR-x polynucleotides, polypeptides, and modulators may be used in the treatment of such diseases and conditions as infections, such as viral infections caused by HIV-1 or HIV-2; pain; cancers; metabolic and cardiovascular diseases and disorders (*e.g*., type 2 diabetes, impaired glucose tolerance, dyslipidemia, obesity, anorexia, hypotension, hypertension, thrombosis, myocardial infarction, cardiomyopathies, atherosclerosis, *etc*.); Parkinson's disease; and psychotic and neurological disorders, including anxiety, mental disorder, manic depression, schizophrenia, migraine, major depression, attention disorders including ADD and ADHD, delirium, dementia, severe mental retardation and dyskinesias, such as Huntington's disease or Tourette's Syndrome, among others. nGPCR-x polynucleotides and polypeptides, as well as nGPCR-x modulators, may also be used in diagnostic assays for such diseases or conditions.

Methods of the invention to identify modulators include variations on any of the methods described above to identify binding partner compounds, the variations including techniques wherein a binding partner compound has been identified and the binding assay is carried out in the presence and absence of a candidate modulator. A modulator is identified in those instances where binding between the nGPCR-x polypeptide and the binding partner compound changes in the presence of the candidate modulator compared to binding in the absence of the candidate modulator compound. A modulator that increases binding between the nGPCR-x polypeptide and the binding partner compound is described as an enhancer or activator, and a modulator that decreases binding between the nGPCR-x polypeptide and the binding partner compound is described as an inhibitor. Following identification of modulators, such compounds can be further tested in other assays including, but not limited to, *in vivo* models, in order to confirm or quantitate their activity as modulators.

The invention also comprehends high-throughput screening (HTS) assays to identify compounds that interact with or inhibit biological activity *(i.e.,* affect enzymatic activity, binding activity, *etc*.) of a nGPCR-x polypeptide. HTS assays permit screening of large numbers of compounds in an efficient manner. Cell-based HTS systems are contemplated to investigate nGPCR-x receptor-ligand interaction. HTS assays are designed to identify "hits" or "lead compounds" having the desired property, from which modifications can be designed to improve the desired property. Chemical modification of the "hit" or "lead compound" is often based on an identifiable structure/activity relationship between the "hit" and the nGPCR-x polypeptide.

Another aspect of the present invention is directed to methods of identifying compounds which modulate (*i.e*., increase or decrease) activity of nGPCR-x comprising contacting nGPCR-x with a compound, and determining whether the compound modifies activity of nGPCR-x. The activity in the presence of the test compared is measured to the activity in the absence of the test compound. Where the activity of the sample containing the test compound is higher than the activity in the sample lacking the test compound, the compound will have increased activity. Similarly, where the activity of the sample containing the test compound is lower than the activity in the sample lacking the test compound, the compound will have inhibited activity. Following identification of compounds that modulate an activity of nGPCR-x, such compounds can be further tested in other assays including, but not limited to, *in vivo* models, in order to confirm or quantitate their activity.

The present invention is particularly useful for screening compounds by using nGPCR-x in any of a variety of drug screening techniques. The compounds to be screened include (which may include compounds which are suspected to modulate nGPCR-x activity), but are not limited to, extracellular, intracellular, biologic or chemical origin. The nGPCR-x polypeptide employed in such a test may be in any form, preferably, free in solution, attached to a solid support, borne on a cell surface or located intracellularly. One skilled in the art can, for example, measure the formation of complexes between nGPCR-x and the compound being tested. Alternatively, one skilled in the art can examine the diminution in complex formation between nGPCR-x and its substrate caused by the compound being tested.

The activity of nGPCR-x polypeptides of the invention can be determined by, for example, examining the ability to bind or be activated by chemically synthesized peptide ligands. Alternatively, the activity of nGPCR-x polypeptides can be assayed by examining their ability to bind calcium ions, hormones, chemokines, neuropeptides, neurotransmitters, nucleotides, lipids, odorants, and photons. Alternatively, the activity of the nGPCR-x polypeptides can be determined by examining the activity of effector molecules including, but not limited to, adenylate cyclase, phospholipases and ion channels. Thus, modulators of nGPCR-x polypeptide activity may alter a GPCR receptor function, such as a binding property of a receptor or an activity such as G protein-mediated signal transduction or membrane localization. In various embodiments of the method, the assay may take the form of an ion flux assay, a yeast growth assay, a non-hydrolyzable GTP assay such as a [³⁵S]-GTP γS assay, a cAMP assay, an inositol triphosphate assay, a diacylglycerol assay, an Aequorin assay, a Luciferase assay, a FLIPR assay for intracellular Ca²⁺ concentration, a mitogenesis assay, a MAP Kinase activity assay, an arachidonic acid release assay (*e.g*., using [³H]-arachidonic acid), and an assay for extracellular acidification rates, as well as other binding or function-based assays of nGPCR-x activity that are generally known in the art. In several of these embodiments, the invention comprehends the inclusion of any of the G proteins known in the art, such as G ₁₆, G ₁₅, or chimeric G_{qd5} , G_{qs5}, G_{qo5}, G_{q25}, and the like. nGPCR-x activity can be determined by methodologies that are used to assay for FaRP activity, which is well known to those skilled in the art. Biological activities of nGPCR-x receptors according to the invention include, but are not limited to, the binding of a natural or an unnatural ligand, as well as any one of the functional activities of GPCRs known in the art. Non-limiting examples of GPCR activities include transmembrane signaling of various forms, which may involve G protein association and/or the exertion of an influence over G protein binding of various guanidylate nucleotides; another exemplary activity of GPCRs is the binding of accessory proteins or polypeptides that differ from known G proteins.

The modulators of the invention exhibit a variety of chemical structures, which can be generally grouped into non-peptide mimetics of natural GPCR receptor ligands, peptide and non-peptide allosteric effectors of GPCR receptors, and peptides that may function as activators or inhibitors (competitive, uncompetitive and non-competitive) (*e.g*., antibody products) of GPCR receptors. The invention does not restrict the sources for suitable modulators, which may be obtained from natural sources such as plant, animal or mineral extracts, or non-natural sources such as small molecule libraries, including the products of combinatorial chemical approaches to library construction, and peptide libraries. Examples of peptide modulators of GPCR receptors exhibit the following primary structures: GLGPRPLRFamide, GNSFLRFamide, GGPQGPLRFamide, GPSGPLRFamide, PDVDHVFLRFamide, and pyro-EDVDHVFLRFamide.

Other assays can be used to examine enzymatic activity including, but not limited to, photometric, radiometric, HPLC, electrochemical, and the like, which are described in, for example, *Enzyme Assays: A Practical Approach,* eds. R. Eisenthal and M. J. Danson, **1992**, Oxford University Press, which is incorporated herein by reference in its entirety.

The use of cDNAs encoding GPCRs in drug discovery programs is well-known; assays capable of testing thousands of unknown compounds per day in high-throughput screens (HTSs) are thoroughly documented. The literature is replete with examples of the use of radiolabelled ligands in HTS binding assays for drug discovery (see Williams, *Medicinal Research Reviews,* **1991**, *11*, 147-184.; Sweetnam, *et al., J. Natural Products*, **1993**, *56*, 441-455 for review). Recombinant receptors are preferred for binding assay HTS because they allow for better specificity (higher relative purity), provide the ability to generate large amounts of receptor material, and can be used in a broad variety of formats (see Hodgson, *Bio*/*Technology,* **1992**, *10*, 973-980; each of which is incorporated herein by reference in its entirety).

A variety of heterologous systems is available for functional expression of recombinant receptors that are well known to those skilled in the art. Such systems include bacteria (Strosberg, *et al., Trends in Pharmacological Sciences*, **1992**, *13,* 95-98), yeast (Pausch, *Trends in Biotechnology*, **1997**, *15*, 487-494), several kinds of insect cells (Vanden Broeck, *Int. Rev. Cytology*, **1996**, *164,* 189-268), amphibian cells (Jayawickreme *et al., Current Opinion in Biotechnology,* **1997**, *8,* 629-634) and several mammalian cell lines (CHO, HEK293, COS, etc.; see Gerhardt, *et al., Eur. J. Pharmacology*, **1997**, *334,* 1-23). These examples do not preclude the use of other possible cell expression systems, including cell lines obtained from nematodes (PCT application WO 98/37177).

In preferred embodiments of the invention, methods of screening for compounds that modulate nGPCR-x activity comprise contacting test compounds with nGPCR-x and assaying for the presence of a complex between the compound and nGPCR-x. In such assays, the ligand is typically labeled. After suitable incubation, free ligand is separated from that present in bound form, and the amount of free or uncomplexed label is a measure of the ability of the particular compound to bind to nGPCR-x.

It is well known that activation of heterologous receptors expressed in recombinant systems results in a variety of biological responses, which are mediated by G proteins expressed in the host cells. Occupation of a GPCR by an agonist results in exchange of bound GDP for GTP at a binding site on the G_{α} subunit; one can use a radioactive, non-hydrolyzable derivative of GTP, GTPγ[³⁵S], to measure binding of an agonist to the receptor (Sim *et al.,* Neuroreport, **1996**, *7*, 729-733). One can also use this binding to measure the ability of antagonists to bind to the receptor by decreasing binding of GTPγ[³⁵S] in the presence of a known agonist. One could therefore construct a HTS based on GTPγ[³⁵S] binding, though this is not the preferred method.

The G proteins required for functional expression of heterologous GPCRs can be native constituents of the host cell or can be introduced through well-known recombinant technology. The G proteins can be intact or chimeric. Often, a nearly universally competent G protein (e.g., G_{α16}) is used to couple any given receptor to a detectable response pathway. G protein activation results in the stimulation or inhibition of other native proteins, events that can be linked to a measurable response.

Examples of such biological responses include, but are not limited to, the following: the ability to survive in the absence of a limiting nutrient in specifically engineered yeast cells (Pausch, *Trends in Biotechnology*, **1997**, *15*, 487-494); changes in intracellular Ca²⁺ concentration as measured by fluorescent dyes (Murphy, *et al., Cur. Opinion Drug Disc. Dev.,* **1998,** *1*, 192-199). Fluorescence changes can also be used to monitor ligand-induced changes in membrane potential or intracellular pH; an automated system suitable for HTS has been described for these purposes (Schroeder, *et al., J. Biomolecular Screening*, **1996**, *1*, 75-80). Melanophores prepared from *Xenopus laevis* show a ligand-dependent change in pigment organization in response to heterologous GPCR activation; this response is adaptable to HTS formats (Jayawickreme *et al., Cur. Opinion Biotechnology,* **1997**, *8*, 629-634). Assays are also available for the measurement of common second messengers, including cAMP, phosphoinositides and arachidonic acid, but these are not generally preferred for HTS.

Preferred methods of HTS employing these receptors include permanently transfected CHO cells, in which agonists and antagonists can be identified by the ability to specifically alter the binding of GTPγ[³⁵S] in membranes prepared from these cells. In another embodiment of the invention, permanently transfected CHO cells could be used for the preparation of membranes which contain significant amounts of the recombinant receptor proteins; these membrane preparations would then be used in receptor binding assays, employing the radiolabelled ligand specific for the particular receptor. Alternatively, a functional assay, such as fluorescent monitoring of ligand-induced changes in internal Ca²⁺ concentration or membrane potential in permanently transfected CHO cells containing each of these receptors individually or in combination would be preferred for HTS. Equally preferred would be an alternative type of mammalian cell, such as HEK293 or COS cells, in similar formats. More preferred would be permanently transfected insect cell lines, such as *Drosophila* S2 cells. Even more preferred would be recombinant yeast cells expressing the *Drosophila melanogaster* receptors in HTS formats well known to those skilled in the art (*e.g.,* Pausch, *Trends in Biotechnology,* **1997**, *15*, 487-494).

The invention contemplates a multitude of assays to screen and identify inhibitors of ligand binding to nGPCR-x receptors. In one example, the nGPCR-x receptor is immobilized and interaction with a binding partner is assessed in the presence and absence of a candidate modulator such as an inhibitor compound. In another example, interaction between the nGPCR-x receptor and its binding partner is assessed in a solution assay, both in the presence and absence of a candidate inhibitor compound. In either assay, an inhibitor is identified as a compound that decreases binding between the nGPCR-x receptor and its binding partner. Following identification of compounds that inhibit ligand binding to nGPCR-x receptors, such compounds can be further tested in other assays including, but not limited to, *in vivo* models, in order to confirm or quantitate their activity. Another contemplated assay involves a variation of the dihybrid assay wherein an inhibitor of protein/protein interactions is identified by detection of a positive signal in a transformed or transfected host cell, as described in PCT publication number WO 95/20652, published August 3, 1995.

Candidate modulators contemplated by the invention include compounds selected from libraries of either potential activators or potential inhibitors. There are a number of different libraries used for the identification of small molecule modulators, including: (1) chemical libraries, (2) natural product libraries, and (3) combinatorial libraries comprised of random peptides, oligonucleotides or organic molecules. Chemical libraries consist of random chemical structures, some of which are analogs of known compounds or analogs of compounds that have been identified as "hits" or "leads" in other drug discovery screens, some of which are derived from natural products, and some of which arise from non-directed synthetic organic chemistry. Natural product libraries are collections of microorganisms, animals, plants, or marine organisms which are used to create mixtures for screening by: (1) fermentation and extraction of broths from soil, plant or marine microorganisms or (2) extraction of plants or marine organisms. Natural product libraries include polyketides, non-ribosomal peptides, and variants (non-naturally occurring) thereof. For a review, see *Science* 282:63-68 (1998). Combinatorial libraries are composed of large numbers of peptides, oligonucleotides, or organic compounds as a mixture. These libraries are relatively easy to prepare by traditional automated synthesis methods, PCR, cloning, or proprietary synthetic methods. Of particular interest are non-peptide combinatorial libraries. Still other libraries of interest include peptide, protein, peptidomimetic, multiparallel synthetic collection, recombinatorial, and polypeptide libraries. For a review of combinatorial chemistry and libraries created therefrom, see Myers, *Curr. Opin. Biotechnol.* 8:701-707 (1997). Identification of modulators through use of the various libraries described herein permits modification of the candidate "hit" (or "lead") to optimize the capacity of the "hit" to modulate activity.

Still other candidate inhibitors contemplated by the invention can be designed and include soluble forms of binding partners, as well as such binding partners as chimeric, or fusion, proteins. A "binding partner" as used herein broadly encompasses non-peptide modulators, as well as such peptide modulators as neuropeptides other than natural ligands, antibodies, antibody fragments, and modified compounds comprising antibody domains that are immunospecific for the expression product of the identified nGPCR-x gene.

The polypeptides of the invention are employed as a research tool for identification, characterization and purification of interacting, regulatory proteins. Appropriate labels are incorporated into the polypeptides of the invention by various methods known in the art and the polypeptides are used to capture interacting molecules. For example, molecules are incubated with the labeled polypeptides, washed to remove unbound polypeptides, and the polypeptide complex is quantified. Data obtained using different concentrations of polypeptide are used to calculate values for the number, affinity, and association of polypeptide with the protein complex.

Labeled polypeptides are also useful as reagents for the purification of molecules with which the polypeptide interacts including, but not limited to, inhibitors. In one embodiment of affinity purification, a polypeptide is covalently coupled to a chromatography column. Cells and their membranes are extracted, and various cellular subcomponents are passed over the column. Molecules bind to the column by virtue of their affinity to the polypeptide. The polypeptide-complex is recovered from the column, dissociated and the recovered molecule is subjected to protein sequencing. This amino acid sequence is then used to identify the captured molecule or to design degenerate oligonucleotides for cloning the corresponding gene from an appropriate cDNA library.

Alternatively, compounds may be identified which exhibit similar properties to the ligand for the nGPCR-x of the invention, but which are smaller and exhibit a longer half time than the endogenous ligand in a human or animal body. When an organic compound is designed, a molecule according to the invention is used as a "lead" compound. The design of mimetics to known pharmaceutically active compounds is a well-known approach in the development of pharmaceuticals based on such "lead" compounds. Mimetic design, synthesis and testing are generally used to avoid randomly screening a large number of molecules for a target property. Furthermore, structural data deriving from the analysis of the deduced amino acid sequences encoded by the DNAs of the present invention are useful to design new drugs, more specific and therefore with a higher pharmacological potency.

Comparison of the protein sequence of the present invention with the sequences present in all the available databases showed a significant homology with the transmembrane portion of G protein coupled receptors. Accordingly, computer modeling can be used to develop a putative tertiary structure of the proteins of the invention based on the available information of the transmembrane domain of other proteins. Thus, novel ligands based on the predicted structure of nGPCR-x can be designed.

In a particular embodiment, the novel molecules identified by the screening methods according to the invention are low molecular weight organic molecules, in which case a composition or pharmaceutical composition can be prepared thereof for oral intake, such as in tablets. The compositions, or pharmaceutical compositions, comprising the nucleic acid molecules, vectors, polypeptides, antibodies and compounds identified by the screening methods described herein, can be prepared for any route of administration including, but not limited to, oral, intravenous, cutaneous, subcutaneous, nasal, intramuscular or intraperitoneal. The nature of the carrier or other ingredients will depend on the specific route of administration and particular embodiment of the invention to be administered. Examples of techniques and protocols that are useful in this context are, *inter alia,* found in Remington's Pharmaceutical Sciences, 16^{th} edition, Osol, A (ed.), **1980**, which is incorporated herein by reference in its entirety.

The dosage of these low molecular weight compounds will depend on the disease state or condition to be treated and other clinical factors such as weight and condition of the human or animal and the route of administration of the compound. For treating human or animals, between approximately 0.5 mg/kg of body weight to 500 mg/kg of body weight of the compound can be administered. Therapy is typically administered at lower dosages and is continued until the desired therapeutic outcome is observed.

The present compounds and methods, including nucleic acid molecules, polypeptides, antibodies, compounds identified by the screening methods described herein, have a variety of pharmaceutical applications and may be used, for example, to treat or prevent unregulated cellular growth, such as cancer cell and tumor growth. In a particular embodiment, the present molecules are used in gene therapy. For a review of gene therapy procedures, see e.g. Anderson, *Science,* **1992**, *256,* 808-813, which is incorporated herein by reference in its entirety.

The present invention also encompasses a method of agonizing (stimulating) or antagonizing a nGPCR-x natural binding partner associated activity in a mammal comprising administering to said mammal an agonist or antagonist to one of the above disclosed polypeptides in an amount sufficient to effect said agonism or antagonism. One embodiment of the present invention, then, is a method of treating diseases in a mammal with an agonist or antagonist of the protein of the present invention comprises administering the agonist or antagonist to a mammal in an amount sufficient to agonize or antagonize nGPCR-x-associated functions.

In an effort to discover novel treatments for diseases, biomedical researchers and chemists have designed, synthesized, and tested molecules that modulate the function of G protein coupled receptors. Some small organic molecules form a class of compounds that modulate the function of G protein coupled receptors.

Exemplary diseases and conditions amenable to treatment based on the present invention include, but are not limited to, thyroid disorders (*e.g*. thyreotoxicosis, myxoedema); renal failure; inflammatory conditions (*e.g*., Crohn's disease); diseases related to cell differentiation and homeostasis; rheumatoid arthritis; autoimmune disorders; movement disorders; CNS disorders (*e.g.*, pain including migraine; stroke; psychotic and neurological disorders, including anxiety, mental disorder, manic depression, anxiety, generalized anxiety disorder, post-traumatic-stress disorder, Schizophrenia, depression, bipolar disorder, delirium, dementia, severe mental retardation; dyskinesias, such as Huntington's disease or Tourette's Syndrome; attention disorders including ADD and ADHD, and degenerative disorders such as Parkinson's, Alzheimer's; movement disorders, including ataxias, supranuclear palsy, *etc*.); infections, such as viral infections caused by HIV-1 or HIV-2; metabolic and cardiovascular diseases and disorders (*e.g*., type 2 diabetes, impaired glucose tolerance, dyslipidemia, obesity, anorexia, hypotension, hypertension, thrombosis, myocardial infarction, cardiomyopathies, atherosclerosis, *etc*.); proliferative diseases and cancers (*e.g*., different cancers such as breast, colon, lung, *etc*., and hyperproliferative disorders such as psoriasis, prostate hyperplasia, etc.); hormonal disorders (*e.g*., male/female hormonal replacement, polycystic ovarian syndrome, alopecia, *etc*.); sexual dysfunction, among others.

Methods of determining the dosages of compounds to be administered to a patient and modes of administering compounds to an organism are disclosed in U.S. Application Serial No. *08*/*702,282,* filed August 23, 1996 and International patent publication number WO 96/22976, published August 1 1996, both of which are incorporated herein by reference in their entirety, including any drawings, figures or tables. Those skilled in the art will appreciate that such descriptions are applicable to the present invention and can be easily adapted to it.

The proper dosage depends on various factors such as the type of disease being treated, the particular composition being used and the size and physiological condition of the patient, including such factors as, for example, weight, age, sex, disease state, etc. Therapeutically effective doses for the compounds described herein can be estimated initially from cell culture and animal models. For example, a dose can be formulated in animal models to achieve a circulating concentration range that initially takes into account the IC₅₀ as determined in cell culture assays. The animal model data can be used to more accurately determine useful doses in humans.

Plasma half-life and biodistribution of the drug and metabolites in the plasma, tumors and major organs can also be determined to facilitate the selection of drugs most appropriate to inhibit a disorder. Such measurements can be carried out. For example, HPLC analysis can be performed on the plasma of animals treated with the drug and the location of radiolabeled compounds can be determined using detection methods such as X-ray, CAT scan and MRI. Compounds that show potent inhibitory activity in the screening assays, but have poor pharmacokinetic characteristics, can be optimized by altering the chemical structure and retesting. In this regard, compounds displaying good pharmacokinetic characteristics can be used as a model.

Toxicity studies can also be carried out by measuring the blood cell composition. For example, toxicity studies can be carried out in a suitable animal model as follows: 1) the compound is administered to mice (an untreated control mouse should also be used); 2) blood samples are periodically obtained via the tail vein from one mouse in each treatment group; and 3) the samples are analyzed for red and white blood cell counts, blood cell composition and the percent of lymphocytes versus polymorphonuclear cells. A comparison of results for each dosing regime with the controls indicates if toxicity is present.

At the termination of each toxicity study, further studies can be carried out by sacrificing the animals (preferably, in accordance with the American Veterinary Medical Association guidelines Report of the American Veterinary Medical Assoc. Panel on Euthanasia, Journal of American Veterinary Medical Assoc., 202:229-249,1993). Representative animals from each treatment group can then be examined by gross necropsy for immediate evidence of metastasis, unusual illness or toxicity. Gross abnormalities in tissue are noted and tissues are examined histologically. Compounds causing a reduction in body weight or blood components are less preferred, as are compounds having an adverse effect on major organs. In general, the greater the adverse effect the less preferred the compound.

For the treatment of many diseases, the expected daily dose of a hydrophobic pharmaceutical agent is between 1 to 500 mg/day, preferably 1 to 250 mg/day, and most preferably 1 to 50 mg/day. Drugs can be delivered less frequently provided plasma levels of the active moiety are sufficient to maintain therapeutic effectiveness. Plasma levels should reflect the potency of the drug. Generally, the more potent the compound the lower the plasma levels necessary to achieve efficacy.

nGPCR-42, 46, 48, 49, 51, 52, 61, 63, and 70 mRNA transcripts may found in many tissues, including, but not limited to, brain, peripheral blood lymphocytes, pancreas, ovary, uterus, testis, salivary gland, kidney, adrenal gland, liver, bone marrow, prostate, fetal liver, colon, muscle, and fetal brain, and may be found in many other tissues. Within the brain, nGPCR-42, 46, 48, 49, 51, 52, 61, 63, and 70 mRNA transcripts may be found in many tissues, including, but not limited to, frontal lobe, hypothalamus, pons, cerebellum, caudate nucleus, and medulla. Tissues and brain regions where specific nGPCR mRNA transcripts are expressed are identified in the Examples, below.

Sequences selected from the group consisting of SEQ ID NO:1 to SEQ ID NO:60 will, as detailed above, enable screening the endogenous neurotransmitters/hormones/ligands which activate, agonize, or antagonize nGPCR-x and for compounds with potential utility in treating disorders including, but not limited to, thyroid disorders (*e.g*. thyreotoxicosis, myxoedema); renal failure; inflammatory conditions (*e.g*., Crohn's disease); diseases related to cell differentiation and homeostasis; rheumatoid arthritis; autoimmune disorders; movement disorders; CNS disorders (*e.g.,* pain including migraine; stroke; psychotic and neurological disorders, including anxiety, mental disorder, manic depression, anxiety, generalized anxiety disorder, post-traumatic-stress disorder, Schizophrenia, depression, bipolar disorder, delirium, dementia, severe mental retardation; dyskinesias, such as Huntington's disease or Tourette's Syndrome; attention disorders including ADD and ADHD, and degenerative disorders such as Parkinson's, Alzheimer's; movement disorders, including ataxias, supranuclear palsy, *etc*.); infections, such as viral infections caused by HIV-1 or HIV-2; metabolic and cardiovascular diseases and disorders (*e.g*., type 2 diabetes, impaired glucose tolerance, dyslipidemia, obesity, anorexia, hypotension, hypertension, thrombosis, myocardial infarction, cardiomyopathies, atherosclerosis, etc.); proliferative diseases and cancers (*e.g*., different cancers such as breast, colon, lung, *etc.,* and hyperproliferative disorders such as psoriasis, prostate hyperplasia, etc.); hormonal disorders (*e.g*., male/female hormonal replacement, polycystic ovarian syndrome, alopecia, *etc*.); sexual dysfunction, among others.

For example, nGPCR-x may be useful in the treatment of respiratory ailments such as asthma, where T cells are implicated by the disease. Contraction of airway smooth muscle is stimulated by thrombin. Cicala *et al* (1999) Br J Pharmacol 126:478-484. Additionally, in bronchiolitis obliterans, it has been noted that activation of thrombin receptors may be deleterious. Hauck *et al.* (1999) Am J Physiol 277:L22-L29. Furthermore, mast cells have also been shown to have thrombin receptors. Cirino *et al* (1996) J Exp Med 183:821-827. nGPCR-x may also be useful in remodeling of airway structures in chronic pulmonary inflammation via stimulation of fibroblast procollagen synthesis. See, e.g., Chambers *et al.* (1998) Biochem J 333:121-127; Trejo *et al.* (1996) J Biol Chem 271:21536-21541.

In another example, increased release of sCD40L and expression of CD40L by T cells after activation of thrombin receptors suggests that nGPCR-x may be useful in the treatment of unstable angina due to the role of T cells and inflammation. See Aukrust *et al.* (1999) Circulation 100:614-620.

A further example is the treatment of inflammatory diseases, such as psoriasis, inflammatory bowel disease, multiple sclerosis, rheumatoid arthritis, and thyroiditis. Due to the tissue expression profile of nGPCR-x, inhibition of thrombin receptors may be beneficial for these diseases. See, *e.g., Morris et al.* (1996) Ann Rheum Dis 55:841-843. In addition to T cells, NK cells and monocytes are also critical cell types which contribute to the pathogenesis of these diseases. See, e.g., Naldini & Carney (1996) Cell Immunol 172:35-42; Hoffman & Cooper (1995) Blood Cells Mol Dis 21:156-167; Colotta *et al.* (1994) Am J Pathol 144:975-985.

Expression of nGPCR-x in bone marrow and spleen may suggest that it may play a role in the proliferation of hematopoietic progenitor cells. See DiCuccio *et al.* (1996) Exp Hematol 24:914-918.

As another example, nGPCR-x may be useful in the treatment of acute and/or traumatic brain injury. Astrocytes have been demonstrated to express thrombin receptors. Activation of thrombin receptors may be involved in astrogliosis following brain injury. Therefore, inhibition of receptor activity may be beneficial for limiting neuroinflammation. Scar formation mediated by astrocytes may also be limited by inhibiting thrombin receptors. See, *e.g*, Pindon *et al.* (1998) Eur J Biochem 255:766-774; Ubl & Reiser. (1997) Glia 21:361-369; Grabham & Cunningham (1995) J Neurochem 64:583-591.

nGPCR-x receptor activation may mediate neuronal and astrocyte apoptosis and prevention of neurite outgrowth. Inhibition would be beneficial in both chronic and acute brain injury. See, *e.g*., Donovan *et al.* (1997) J Neurosci 17:5316-5326; Turgeon *et al* (1998) J Neurosci 18:6882-6891; Smith-Swintosky *et al.* (1997) J Neurochem 69:1890-1896; Gill *et al.* (1998) Brain Res 797:321-327; Suidan *et al.* (1996) Semin Thromb Hemost 22:125-133.

The attached Sequence Listing contains the sequences of the polynucleotides and polypeptides of the invention and is incorporated herein by reference in its entirety.

As described above and in Example 5 below, the genes encoding nGPCR-42, 46, 48, 49, 51, 52, 61, 63, and 70 have been detected in brain tissue indicating that these n-GPCR-x proteins are neuroreceptors. The identification of modulators such as agonists and antagonists is therefore useful for the identification of compounds useful to treat neurological diseases and psychiatric disorders. Such neurological diseases and disorders, including but are not limited to, mental disorder, affective disorders, ADHD/ADD, and neural disorders such as Alzheimer's disease, Parkinson's disease, migraine, schizophrenia, and senile dementia as well as depression, anxiety, bipolar disease, epilepsy, neuritis, neurasthenia, neuropathy, neuroses, and the like.

### Methods of Screening Human Subjects

Thus in yet another embodiment, the invention provides genetic screening procedures that entail analyzing a person's genome ― in particular their alleles for GPCRs of the invention - - to determine whether the individual possesses a genetic characteristic found in other individuals that are considered to be afflicted with, or at risk for, developing a mental disorder or disease of the brain that is suspected of having a hereditary component. For example, in one embodiment, the invention provides a method for determining a potential for developing a disorder affecting the brain in a human subject comprising the steps of analyzing the coding sequence of one or more GPCR genes from the human subject; and determining development potential for the disorder in said human subject from the analyzing step.

More particularly, the invention provides a method of screening a human subject to diagnose a disorder affecting the brain or genetic predisposition therefor, comprising the steps of: (a) assaying nucleic acid of a human subject to determine a presence or an absence of a mutation altering the amino acid sequence, expression, or biological activity of at least one seven transmembrane receptor that is expressed in the brain, wherein the seven transmembrane receptor comprises an amino acid sequence selected from the group consisting of SEQ ID Numbers 61, 62, 68, 91, 94, 96, 97, 99, 100, and 111-120, or an allelic variant thereof, and wherein the nucleic acid corresponds to the gene encoding the seven transmembrane receptor; and (b) diagnosing the disorder or predisposition from the presence or absence of said mutation, wherein the presence of a mutation altering the amino acid sequence, expression, or biological activity of allele in the nucleic acid correlates with an increased risk of developing the disorder. In preferred variations, the seven transmembrane receptor is nGPCR-42, 46, 48, 49, 51, 52, 61, 63, or 70 comprising an amino acid sequence set forth in SEQ ID Numbers 61, 62, 68, 91, 94, 96, 97, 99, 100, and 111-120, or an allelic variant thereof, and the disease is mental disorder.

By "human subject" is meant any human being, human embryo, or human fetus. It will be apparent that methods of the present invention will be of particular interest to individuals that have themselves been diagnosed with a disorder affecting the brain or have relatives that have been diagnosed with a disorder affecting the brain.

By "screening for an increased risk" is meant determination of whether a genetic variation exists in the human subject that correlates with a greater likelihood of developing a disorder affecting the brain than exists for the human population as a whole, or for a relevant racial or ethnic human sub-population to which the individual belongs. Both positive and negative determinations (i.e., determinations that a genetic predisposition marker is present or is absent) are intended to fall within the scope of screening methods of the invention. In preferred embodiments, the presence of a mutation altering the sequence or expression of at least one nGPCR-42, 46, 48, 49, 51, 52, 61, 63, or 70 seven transmembrane receptor allele in the nucleic acid is correlated with an increased risk of developing mental disorder, whereas the absence of such a mutation is reported as a negative determination.

The "assaying" step of the invention may involve any techniques available for analyzing nucleic acid to determine its characteristics, including but not limited to well-known techniques such as single-strand conformation polymorphism analysis (SSCP) (Orita *et al.*, *Proc Natl. Acad. Sci. USA, 86:* 2766-2770 (1989)); heteroduplex analysis (White *et al., Genomics, 12:* 301-306 (1992)); denaturing gradient gel electrophoresis analysis (Fischer *et al., Proc. Natl. Acad. Sci. USA, 80:* 1579-1583 (1983); and Riesner *et al.*, *Electrophoresis, 10:* 377-389 (1989)); DNA sequencing; RNase cleavage (Myers *et al., Science, 230:* 1242-1246 (1985)); chemical cleavage of mismatch techniques (Rowley *et al., Genomics, 30:* 574-582 (1995); and Roberts *et al., Nucl. Acids Res., 25*: 3377-3378 (1997)); restriction fragment length polymorphism analysis; single nucleotide primer extension analysis (Shumaker *et al., Hum. Mutat., 7*: 346-354 (1996); and Pastinen *et al., Genome Res., 7*: 606-614 (1997)); 5' nuclease assays (Pease *et al., Proc. Natl. Acad. Sci. USA, 91*:5022-5026 (1994)); DNA Microchip analysis (Ramsay, G., *Nature Biotechnology, 16:* 40-48 (1999); and Chee *et al.,* U.S. Patent No. 5,837,832); and ligase chain reaction (Whiteley *et al.,* U.S. Patent No. 5,521,065). (See generally, Schafer and Hawkins, *Nature Biotechnology, 16:* 33-39 (1998).) All of the foregoing documents are hereby incorporated herein by reference in their entirety.

Thus, in one preferred embodiment involving screening nGPCR-42, 46, 48, 49, 51, 52, 61, 63, or 70 sequences, for example, the assaying step comprises at least one procedure selected from the group consisting of: (a) determining a nucleotide sequence of at least one codon of at least one nGPCR-42, 46, 48, 49, 51, 52, 61, 63, or 70 allele of the human subject; (b) performing a hybridization assay to determine whether nucleic acid from the human subject has a nucleotide sequence identical to or different from one or more reference sequences; (c) performing a polynucleotide migration assay to determine whether nucleic acid from the human subject has a nucleotide sequence identical to or different from one or more reference sequences; and (d) performing a restriction endonuclease digestion to determine whether nucleic acid from the human subject has a nucleotide sequence identical to or different from one or more reference sequences.

In a highly preferred embodiment, the assaying involves sequencing of nucleic acid to determine nucleotide sequence thereof, using any available sequencing technique. (See, *e.g.*, Sanger *et al., Proc. Natl. Acad. Sci. (USA), 74:* 5463-5467 (1977) (dideoxy chain termination method); Mirzabekov, *TIBTECH, 12*: 27-32 (1994) (sequencing by hybridization); Drmanac *et al., Nature Biotechnology, 16:* 54-58 (1998); U.S. Patent No. 5,202,231; and *Science, 260:* 1649-1652 (1993) (sequencing by hybridization); Kieleczawa *et al., Science, 258:* 1787-1791 (1992) (sequencing by primer walking); (Douglas *et al., Biotechniques, 14:* 824-828 (1993) (Direct sequencing of PCR products); and Akane *et al., Biotechniques 16*:238-241 (1994); Maxam and Gilbert, *Meth. Enzymol., 65:* 499-560 (1977) (chemical termination sequencing), all incorporated herein by reference in its entirety). The analysis may entail sequencing of the entire nGPCR gene genomic DNA sequence, or portions thereof; or sequencing of the entire seven transmembrane receptor coding sequence or portions thereof. In some circumstances, the analysis may involve a determination of whether an individual possesses a particular allelic variant, in which case sequencing of only a small portion of nucleic acid -- enough to determine the sequence of a particular codon characterizing the allelic variant -- is sufficient. This approach is appropriate, for example, when assaying to determine whether one family member inherited the same allelic variant that has been previously characterized for another family member, or, more generally, whether a person's genome contains an allelic variant that has been previously characterized and correlated with a mental disorder having a heritable component.

In another highly preferred embodiment, the assaying step comprises performing a hybridization assay to determine whether nucleic acid from the human subject has a nucleotide sequence identical to or different from one or more reference sequences. In a preferred embodiment, the hybridization involves a determination of whether nucleic acid derived from the human subject will hybridize with one or more oligonucleotides, wherein the oligonucleotides have nucleotide sequences that correspond identically to a portion of the GPCR gene sequence taught herein, such as the nGPCR-42, 46, 48, 49, 51, 52, 61, 63, or 70 coding sequence set forth in SEQ ID Numbers 61, 62, 68, 91, 94, 96, 97, 99, 100, and 111-120, or that correspond identically except for one mismatch. The hybridization conditions are selected to differentiate between perfect sequence complementarity and imperfect matches differing by one or more bases. Such hybridization experiments thereby can provide single nucleotide polymorphism sequence information about the nucleic acid from the human subject, by virtue of knowing the sequences of the oligonucleotides used in the experiments.

Several of the techniques outlined above involve an analysis wherein one performs a polynucleotide migration assay, *e.g*., on a polyacrylamide electrophoresis gel (or in a capillary electrophoresis system), under denaturing or non-denaturing conditions. Nucleic acid derived from the human subject is subjected to gel electrophoresis, usually adjacent to (or co-loaded with) one or more reference nucleic acids, such as reference GPCR-encoding sequences having a coding sequence identical to all or a portion of SEQ ID Numbers 61, 62, 68, 91, 94, 96, 97, 99, 100, and 111-120 (or identical except for one known polymorphism). The nucleic acid from the human subject and the reference sequence(s) are subjected to similar chemical or enzymatic treatments and then electrophoresed under conditions whereby the polynucleotides will show a differential migration pattern, unless they contain identical sequences. (See generally Ausubel *et al.* (eds.), *Current Protocols in Molecular Biology,* New York: John Wiley & Sons, Inc. (1987-1999); and *Sambrook et al.,* (eds.), Molecular Cloning, *A Laboratory Manual,* Cold Spring Harbor, New York: Cold Spring Harbor Laboratory Press (1989), both incorporated herein by reference in their entirety).

In the context of assaying, the term "nucleic acid of a human subject" is intended to include nucleic acid obtained directly from the human subject (*e.g*., DNA or RNA obtained from a biological sample such as a blood, tissue, or other cell or fluid sample); and also nucleic acid derived from nucleic acid obtained directly from the human subject. By way of non-limiting examples, well known procedures exist for creating cDNA that is complementary to RNA derived from a biological sample from a human subject, and for amplifying (*e.g*., via polymerase chain reaction (PCR)) DNA or RNA derived from a biological sample obtained from a human subject. Any such derived polynucleotide which retains relevant nucleotide sequence information of the human subject's own DNA/RNA is intended to fall within the definition of "nucleic acid of a human subject" for the purposes of the present invention.

In the context of assaying, the term "mutation" includes addition, deletion, and/or substitution of one or more nucleotides in the GPCR gene sequence (*e.g.*, as compared to the seven transmembrane receptor-encoding sequences set forth of SEQ ID Numbers 61, 62, 68, 91, 94, 96, 97, 99, 100, and 111-120 and other polymorphisms that occur in introns (where introns exist) and that are identifiable via sequencing, restriction fragment length polymorphism, or other techniques. The various activity examples provided herein permit determination of whether a mutation modulates activity of the relevant receptor in the presence or absence of various test substances.

In a related embodiment, the invention provides methods of screening a person's genotype with respect to GPCRs of the invention, and correlating such genotypes with diagnoses for disease or with predisposition for disease (for genetic counseling). For example, the invention provides a method of screening for an nGPCR-63 hereditary mental disorder genotype in a human patient, comprising the steps of: (a) providing a biological sample comprising nucleic acid from the patient, the nucleic acid including sequences corresponding to said patient's nGPCR-42, 46, 48, 49, 51, 52, 61, 63, or 70 alleles; (b) analyzing the nucleic acid for the presence of a mutation or mutations; (c) determining an nGPCR-42, 46, 48, 49, 51, 52, 61,63, or 70 genotype from the analyzing step; and (d) correlating the presence of a mutation in an nGPCR-42, 46, 48, 49, 51, 52, 61, 63, or 70 allele with a hereditary mental disorder genotype. In a preferred embodiment, the biological sample is a cell sample containing human cells that contain genomic DNA of the human subject. The analyzing can be performed analogously to the assaying described in preceding paragraphs. For example, the analyzing comprises sequencing a portion of the nucleic acid (e.g., DNA or RNA), the portion comprising at least one codon of the nGPCR-63 alleles.

Although more time consuming and expensive than methods involving nucleic acid analysis, the invention also may be practiced by assaying protein of a human subject to determine the presence or absence of an amino acid sequence variation in GPCR protein from the human subject. Such protein analyses may be performed, *e.g*., by fragmenting GPCR protein via chemical or enzymatic methods and sequencing the resultant peptides; or by Western analyses using an antibody having specificity for a particular allelic variant of the GPCR.

The invention also provides materials that are useful for performing methods of the invention. For example, the present invention provides oligonucleotides useful as probes in the many analyzing techniques described above. In general, such oligonucleotide probes comprise 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides that have a sequence that is identical, or exactly complementary, to a portion of a human GPCR gene sequence taught herein (or allelic variant thereof), or that is identical or exactly complementary except for one nucleotide substitution. In a preferred embodiment, the oligonucleotides have a sequence that corresponds in the foregoing manner to a human GPCR coding sequence taught herein, and in particular, the coding sequences set forth in SEQ ID Numbers 61, 62, 68, 91, 94, 96, 97, 99, 100, and 111-120. In one variation, an oligonucleotide probe of the invention is purified and isolated. In another variation, the oligonucleotide probe is labeled, e.g., with a radioisotope, chromophore, or fluorophore. In yet another variation, the probe is covalently attached to a solid support. (See generally Ausubel *et al.* and Sambrook *et al., supra*.)

In a related embodiment, the invention provides kits comprising reagents that are useful for practicing methods of the invention. For example, the invention provides a kit for screening a human subject to diagnose a mental disorder or a genetic predisposition therefor, comprising, in association: (a) an oligonucleotide useful as a probe for identifying polymorphisms in a human nGPCR-42, 46, 48, 49, 51, 52, 61, 63, or 70 seven transmembrane receptor gene, the oligonucleotide comprising 6-50 nucleotides that have a sequence that is identical or exactly complementary to a portion of a human nGPCR-42, 46, 48, 49, 51, 52, 61, 63, or 70 gene sequence or nGPCR-42, 46, 48, 49, 51, 52, 61, 63, or 70 coding sequence, except for one sequence difference selected from the group consisting of a nucleotide addition, a nucleotide deletion, or nucleotide substitution; and (b) a media packaged with the oligonucleotide containing information identifying polymorphisms identifiable with the probe that correlate with mental disorder or a genetic predisposition therefor. Exemplary information-containing media include printed paper package inserts or packaging labels; and magnetic and optical storage media that are readable by computers or machines used by practitioners who perform genetic screening and counseling services. The practitioner uses the information provided in the media to correlate the results of the analysis with the oligonucleotide with a diagnosis. In a preferred variation, the oligonucleotide is labeled.

In still another embodiment, the invention provides methods of identifying those allelic variants of GPCRs of the invention that correlate with mental disorders. For example, the invention provides a method of identifying a seven transmembrane allelic variant that correlates with a mental disorder, comprising steps of: (a) providing a biological sample comprising nucleic acid from a human patient diagnosed with a mental disorder, or from the patient's genetic progenitors or progeny; (b) analyzing the nucleic acid for the presence of a mutation or mutations in at least one seven transmembrane receptor that is expressed in the brain, wherein the at least one seven transmembrane receptor comprises an amino acid sequence selected from the group consisting of SEQ ID Numbers 61, 62, 68, 91, 94, 96, 97, 99, 100, and 111-120 or an allelic variant thereof, and wherein the nucleic acid includes sequence corresponding to the gene or genes encoding the at least one seven transmembrane receptor; (c) determining a genotype for the patient for the at least one seven transmembrane receptor from said analyzing step; and (d) identifying an allelic variant that correlates with the mental disorder from the determining step. To expedite this process, it may be desirable to perform linkage studies in the patients (and possibly their families) to correlate chromosomal markers with disease states. The chromosomal localization data provided herein facilitates identifying an involved GPCR with a chromosomal marker.

The foregoing method can be performed to correlate GPCRs of the invention to a number of disorders having hereditary components that are causative or that predispose persons to the disorder. For example, in one preferred variation, the disorder is a mental disorder, and the at least one seven transmembrane receptor comprises nGPCR-42, 46, 48, 49, 51, 52, 61, 63, or 70 having an amino acid sequence set forth in SEQ ID Numbers 61, 62, 68, 91, 94, 96, 97, 99, 100, and 111-120 or an allelic variant thereof.

Also contemplated as part of the invention are polynucleotides that comprise the allelic variant sequences identified by such methods, and polypeptides encoded by the allelic variant sequences, and oligonucleotide and oligopeptide fragments thereof that embody the mutations that have been identified. Such materials are useful in *in vitro* cell-free and cell-based assays for identifying lead compounds and therapeutics for treatment of the disorders. For example, the variants are used in activity assays, binding assays, and assays to screen for activity modulators described herein. In one preferred embodiment, the invention provides a purified and isolated polynucleotide comprising a nucleotide sequence encoding a nGPCR-42, 46, 48, 49, 51, 52, 61, 63, or 70 receptor allelic variant identified according to the methods described above; and an oligonucleotide that comprises the sequences that differentiate the allelic variant from the nGPCR-42, 46, 48, 49, 51, 52, 61, 63, or 70 sequences set forth in SEQ ID Numbers 61, 62, 68, 91, 94, 96, 97, 99, 100, and 111-120. The invention also provides a vector comprising the polynucleotide (preferably an expression vector); and a host cell transformed or transfected with the polynucleotide or vector. The invention also provides an isolated cell line that is expressing the allelic variant GPCR polypeptide; purified cell membranes from such cells; purified polypeptide; and synthetic peptides that embody the allelic variation amino acid sequence. In one particular embodiment, the invention provides a purified polynucleotide comprising a nucleotide sequence encoding a nGPCR-42, 46, 48, 49, 51, 52, 61, 63, or 70 seven transmembrane receptor protein of a human that is affected with a mental disorder; wherein said polynucleotide hybridizes to the complement of SEQ ID Numbers 61, 62, 68, 91, 94, 96, 97, 99, 100, and 111-120 under the following hybridization conditions: (a) hybridization for 16 hours at 42C in a hybridization solution comprising 50% formamide, 1% SDS, 1 M NaCl, 10% dextran sulfate and (b) washing 2 times for 30 minutes at 60C in a wash solution comprising 0.1x SSC and 1% SDS; and wherein the polynucleotide encodes a nGPCR-42,46, 48,49, 51, 52, 61, 63, or 70 amino acid sequence that differs from SEQ ID Numbers 61, 62, 68, 91, 94, 96, 97, 99, 100, and 111-120 by at least one residue.

An exemplary assay for using the allelic variants is a method for identifying a modulator of nGPCR-x biological activity, comprising the steps of: (a) contacting a cell expressing the allelic variant in the presence and in the absence of a putative modulator compound; (b) measuring nGPCR-x biological activity in the cell; and (c) identifying a putative modulator compound in view of decreased or increased nGPCR-x biological activity in the presence versus absence of the putative modulator.

Additional features of the invention will be apparent from the following Examples. Examples 1-6, 12, 14, and 15 are actual, while the remaining Examples are prophetic. Additional features and variations of the invention will be apparent to those skilled in the art from the entirety of this application, including the detailed description, and all such features are intended as aspects of the invention. Likewise, features of the invention described herein can be re-combined into additional embodiments that also are intended as aspects of the invention, irrespective of whether the combination of features is specifically mentioned above as an aspect or embodiment of the invention. Also, only such limitations which are described herein as critical to the invention should be viewed as such; variations of the invention lacking limitations which have not been described herein as critical are intended as aspects of the invention.

### EXAMPLES

### Example 1: Identification of nGPCR-x

### A. Database search

The Celera database was searched using known GPCR receptors as query sequences to find patterns suggestive of novel G protein-coupled receptors. Positive hits were further analyzed with the GCG program BLAST to determine which ones were the most likely candidates to encode G protein-coupled receptors, using the standard (default) alignment produced by BLAST as a guide.

Briefly, the BLAST algorithm, which stands for Basic Local Alignment Search Tool is suitable for determining sequence similarity (Altschul *et al.,* J. Mol. Biol., 1990, 215, 403-410, which is incorporated herein by reference in its entirety). Software for performing BLAST analyses is publicly available through the National Center for Biotechnology Information through the world wide web of the Iriternet (ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighborhood word score threshold (Altschul et al., supra). These initial neighbourhood word hits act as seeds for initiating searches to find HSPs containing them. The word hits are extended in both directions along each sequence for as far as the cumulative alignment score can be increased. Extension for the word hits in each direction are halted when: 1) the cumulative alignment score falls off by the quantity X from its maximum achieved value; 2) the cumulative score goes to zero or below, due to the accumulation of one or more negative-scoring residue alignments; or 3) the end of either sequence is reached. The Blast algorithm parameters W, T and X determine the sensitivity and speed of the alignment. The Blast program uses as defaults a word length (W) of 11, the BLOSUM62 scoring matrix (see Henikoff et al., Proc. Natl. Acad. Sci. USA, 1992, 89, 10915-10919, which is incorporated herein by reference in its entirety) alignments (B) of 50, expectation (E) of 10, M=5, N=4, and a comparison of both strands.

The BLAST algorithm (Karlin *et al.,* Proc. Natl. Acad. Sci. USA, 1993, 90, 5873-5787, which is incorporated herein by reference in its entirety) and Gapped BLAST perform a statistical analysis of the similarity between two sequences. One measure of similarity provided by the BLAST algorithm is the smallest sum probability (P(N)), which provides an indication of the probability by which a match between two nucleotide or amino acid sequences would occur by chance. For example, a nucleic acid is considered similar to a GPCR gene or cDNA if the smallest sum probability in comparison of the test nucleic acid to a GPCR nucleic acid is less than about 1, preferably less than about 0.1, more preferably less than about 0.01, and most preferably less than about 0.001.

Homology searches are performed with the program BLAST version 2.08. A collection of about 200 to about 350 query amino acid sequences derived from GPCRs was used to search the genomic DNA sequence using TBLASTN and alignments with an E-value lower than 0.01 were collected from each BLAST search. The amino acid sequences have been edited to remove regions in the sequence that produce non-significant alignments with proteins that are not related to GPCRs.

Multiple query sequences may have a significant alignment to the same genomic region, although each alignment may not cover exactly the same DNA region. A procedure is used to determine the region of maximum common overlap between the alignments from several query sequences. This region is called the consensus DNA region. The procedure for determining this consensus involves the automatic parsing of the BLAST output files using the program MSPcrunch to produce a tabular report. From this tabular report the start and end of each alignment in the genomic DNA is extracted. This information is used by a PERL script to derive the maximum common overlap. These regions are reported in the form of a unique sequence identifier, a start and the end position in the sequence. The sequences defined by these regions were extracted from the original genomic sequence file using the program fetchdb.

The consensus regions are assembled into a non-redundant set by using the program phrap. After assembly with phrap a set of contigs and singletons were defined as candidate DNA regions coding for nGPCRs. These sequences were then submitted for further sequence analysis.

Further sequence analysis involves the removal of sequences previously isolated and removal of sequences that are related to olfactory GPCRs.

### nGPCR-70

Homology searches were performed with the program BLAST version 2.08. A collection of 286 query amino acid sequences derived from GPCRs was used to search the genomic DNA sequence using TBLASTN and alignments with an E-value lower than 0.1 were collected from each BLAST search. The amino acid sequences were edited to remove regions in the sequence that produce non-significant alignments with proteins that are not related to GPCRs.

The consensus regions were assembled into a non-redundant set by using the program "GelStart": After assembly with GelStart, a set of contigs and singletons were defined as candidate DNA regions coding for nGPCR-70. Further sequence analysis involved the removal of sequences previously isolated and removal of sequences that are related to olfactory GPCRs. The transmembrane regions for the sequences that remained were determined using a FORTRAN computer program called "tmtrest.all" (Parodi *et al.,* Comput. Appl. Biosci. 5:527-535(1994)). Transmembrane regions within the amino acid sequences were visually inspected with an outliner (MaxThink) and the Celera sequences with promising transmembrane regions were kept. In a last step, the blast results were compared against SWPLUS and the transmembrane regions of the sequences and promising Celera sequences for full length cloning were selected. The sequence of nGPCR-70s is shown above in Table 5.

### nGPCR-63

Homology searches were performed with the program BLAST version 2.08. A collection of 286 query amino acid sequences derived from GPCRs was used to search the genomic DNA sequence using TBLASTN and alignments with an E-value lower than 0.1 were collected from each BLAST search. The amino acid sequences were edited to remove regions in the sequence that produce non-significant alignments with proteins that are not related to GPCRs.

The consensus regions were assembled into a non-redundant set by using the program "GelStart". After assembly with "GelStart" a set of contigs and singletons were defined as candidate DNA regions coding for nGPCR-63. Further sequence analysis involved the removal of sequences previously isolated and removal of sequences that are related to olfactory GPCRs. The transmembrane regions for the sequences that remained were determined using a FORTRAN computer program called "tmtrest.all" (Parodi *et al.,* Comput. Appl. Biosci. 5:527-535(1994)). Transmembrane regions within the amino acid sequences were visually inspected with an outliner (MaxThink) and the Celera sequences with promising transmembrane regions were kept. In a last step, the blast results were compared against SWPLUS and the transmembrane regions of the sequences and promising Celera sequences for full length cloning were selected. The sequence of nGPCR-63 is shown above in Table 5.

### nGPCR-42

Homology searches were performed with the program BLAST version 2.08. A collection of 340 query amino acid sequences derived from GPCRs was used to search the genomic DNA sequence using TBLASTN and alignments with an E-value lower than 0.01 were collected from each BLAST search. The amino acid sequences have been edited to remove regions in the sequence that produce non-significant alignments with proteins that are not related to GPCRs.

The consensus regions were assembled into a non-redundant set by using the program phrap. After assembly with phrap a set of contigs and singletons were defined as candidate DNA regions coding for nGPCR-42. These sequences were then submitted for further sequence analysis. Further sequence analysis involves the removal of sequences previously isolated and removal of sequences that are related to olfactory GPCRs. The transmembrane regions for the sequences that remained were determined using a FORTRAN computer program called "tmtrest.all" (Parodi *et al.,* Comput. Appl. Biosci. 5:527-535(1994)). Only sequences that contained transmembrane regions in a pattern found in GPCRs were retained. These nGPCR-42s are shown above in Table 5.

### nGPCR-46

Homology searches are performed with the program BLAST version 2.08. A collection of 340 query amino acid sequences derived from GPCRs was used to search the genomic DNA sequence using TBLASTN and alignments with an E-value lower than 0.01 were collected from each BLAST search. The amino acid sequences have been edited to remove regions in the sequence that produce non-significant alignments with proteins that are not related to GPCRs.

The consensus regions are assembled into a non-redundant set by using the program phrap. After assembly with phrap a set of contigs and singletons were defined as candidate DNA regions coding for nGPCR-46. These sequences were then submitted for further sequence analysis. Further sequence analysis involves the removal of sequences previously isolated and removal of sequences that are related to olfactory GPCRs. The transmembrane regions for the sequences that remained were determined using a FORTRAN computer program called "tmtrest.all" (Parodi *et al.,* Comput. Appl. Biosci. 5:527-535(1994)). Only sequences that contained transmembrane regions in a pattern found in GPCRs were retained. These nGPCR-46s are shown above in Table 5.

### nGPCR-48

Homology searches are performed with the program BLAST version 2.08. A collection of 340 query amino acid sequences derived from GPCRs was used to search the genomic DNA sequence using TBLASTN and alignments with an E-value lower than 0.01 were collected from each BLAST search. The amino acid sequences have been edited to remove regions in the sequence that produce non-significant alignments with proteins that are not related to GPCRs.

The consensus regions are assembled into a non-redundant set by using the program phrap. After assembly with phrap a set of contigs and singletons were defined as candidate DNA regions coding for nGPCR-48. These sequences were then submitted for further sequence analysis. Further sequence analysis involves the removal of sequences previously isolated and removal of sequences that are related to olfactory GPCRs. The transmembrane regions for the sequences that remained were determined using a FORTRAN computer program called "tmtrest.all" (Parodi *et al.,* Comput. Appl. Biosci. 5:527-535(1994)). Only sequences that contained transmembrane regions in a pattern found in GPCRs were retained. These nGPCR-48s are shown above in Table 5.

### nGPCR-49

Homology searches were performed with the program BLAST version 2.08. A collection of 286 query amino acid sequences derived from GPCRs was used to search the genomic DNA sequence using TBLASTN and alignments with an E-value lower than 0.1 were collected from each BLAST search. The amino acid sequences were edited to remove regions in the sequence that produce non-significant alignments with proteins that are not related to GPCRs.

The consensus regions were assembled into a non-redundant set by using the program "GelStart". After assembly with GelStart a set of contigs and singletons were defined as candidate DNA regions coding for nGPCR-49. Further sequence analysis involved the removal of sequences previously isolated and removal of sequences that are related to olfactory GPCRs. The transmembrane regions for the sequences that remained were determined using a FORTRAN computer program called "tmtrest.all" (Parodi *et al.,* Comput. Appl. Biosci. 5:527-535(1994)). Transmembrane regions within the amino acid sequences were visually inspected with an outliner (MaxThink) and the Celera sequences with promising transmembrane regions were kept. In a last step, the blast results were compared against SWPLUS and the transmembrane regions of the sequences and promising Celera sequences for full length cloning were selected. The sequence of nGPCR-49 is shown above in Table 5.

### nGPCR-61

Homology searches were performed with the program BLAST version 2.08. A collection of 286 query amino acid sequences derived from GPCRs was used to search the genomic DNA sequence using TBLASTN and alignments with an E-value lower than 0.1 were collected from each BLAST search. The amino acid sequences were edited to remove regions in the sequence that produce non-significant alignments with proteins that are not related to GPCRs.

The consensus regions were assembled into a non-redundant set by using the program phrap. After assembly with phrap a set of contigs and singletons were defined as candidate DNA regions coding for nGPCR-61. Further sequence analysis involved the removal of sequences previously isolated and removal of sequences that are related to olfactory GPCRs. The transmembrane regions for the sequences that remained were determined using a FORTRAN computer program called "tmtrest.all" (Parodi *et al.,* Comput. Appl. Biosci. 5:527-535(1994)). Transmembrane regions within the amino acid sequences were visually inspected with an outliner (MaxThink) and the Celera sequences with promising transmembrane regions were kept. In a last step, the blast results were compared against SWPLUS and the transmembrane regions of the sequences and promising Celera sequences for full length cloning were selected. The sequence of nGPCR-61 is shown above in Table 5.

### nGPCR-51

Homology searches were performed with the program BLAST version 2.08. A collection of 340 query amino acid sequences derived from GPCRs was used to search the genomic DNA sequence using TBLASTN and alignments with an E-value lower than 0.01 were collected from each BLAST search. The amino acid sequences were edited to remove regions in the sequence that produce non-significant alignments with proteins that are not related to GPCRs.

The consensus regions were assembled into a non-redundant set by using the program phrap. After assembly with phrap a set of contigs and singletons were defined as candidate DNA regions coding for nGPCR-51. These sequences were then submitted for further sequence analysis. Further sequence analysis involved the removal of sequences previously isolated and removal of sequences that are related to olfactory GPCRs. The transmembrane regions for the sequences that remained were determined using a FORTRAN computer program called "tmtrest.all" (Parodi *et al.,* Comput. Appl. Biosci. 5:527-535(1994)). Only sequences that contained transmembrane regions in a pattern found in GPCRs were retained. The sequence of nGPCR-51s is shown above in Table 5.

### nGPCR-52

Homology searches are performed with the program BLAST version 2.08. A collection of 340 query amino acid sequences derived from GPCRs was used to search the genomic DNA sequence using TBLASTN and alignments with an E-value lower than 0.01 were collected from each BLAST search. The amino acid sequences have been edited to remove regions in the sequence that produce non-significant alignments with proteins that are not related to GPCRs.

The consensus regions are assembled into a non-redundant set by using the program phrap. After assembly with phrap a set of contigs and singletons were defined as candidate DNA regions coding for nGPCR-52. These sequences were then submitted for further sequence analysis. Further sequence analysis involves the removal of sequences previously isolated and removal of sequences that are related to olfactory GPCRs. The transmembrane regions for the sequences that remained were determined using a FORTRAN computer program called "tmtrest.all" (Parodi *et al.,* Comput. Appl. Biosci. 5:527-535(1994)). Only sequences that contained transmembrane regions in a pattern found in GPCRs were retained. These nGPCR-52s are shown above in Table 5.

nGPRCR-x cDNAs were sequenced directly using an ABI377 fluorescence-based sequencer (Perkin-Elmer/Applied Biosystems Division, PE/ABD, Foster City, CA) and the ABI PRISM™ Ready Dye-Deoxy Terminator kit with Taq FS™ polymerase. Each ABI cycle sequencing reaction contained about 0.5 µg of plasmid DNA. Cycle-sequencing was performed using an initial denaturation at 98C for 1 minute, followed by 50 cycles using the following parameters: 98C for 30 seconds, annealing at 50C for 30 seconds, and extension at 60C for 4 minutes. Temperature cycles and times were controlled by a Perkin-Elmer 9600 thermocycler. Extension products were purified using Centriflex™ gel filtration cartridges (Advanced Genetic Technologies Corp., Gaithersburg, MD). Each reaction product was loaded by pipette onto the column, which is then centrifuged in a swinging bucket centrifuge (Sorvall model RT6000B tabletop centrifuge) at 1500 x g for 4 minutes at room temperature. Column-purified samples were dried under vacuum for about 40 minutes and then dissolved in 5 µl of a DNA loading solution (83% deionized formamide, 8.3 mM EDTA, and 1.6 mg/ml Blue Dextran). The samples were then heated to 90C for three minutes and loaded into the gel sample wells for sequence analysis using the ABI377 sequencer. Sequence analysis was performed by importing ABI377 files into the Sequencer program (Gene Codes, Ann Arbor, MI). Generally, sequence reads of 700 bp were obtained. Potential sequencing errors were minimized by obtaining sequence information from both DNA strands and by re-sequencing difficult areas using primers annealing at different locations until all sequencing ambiguities were removed.

The following Table 5 contains the sequences of the polynucleotides and polypeptides of the invention. Start and stop codons within the polynucleotide sequence are identified by boldface type. The transmembrane domains within the polypeptide sequence are identified by underlining.

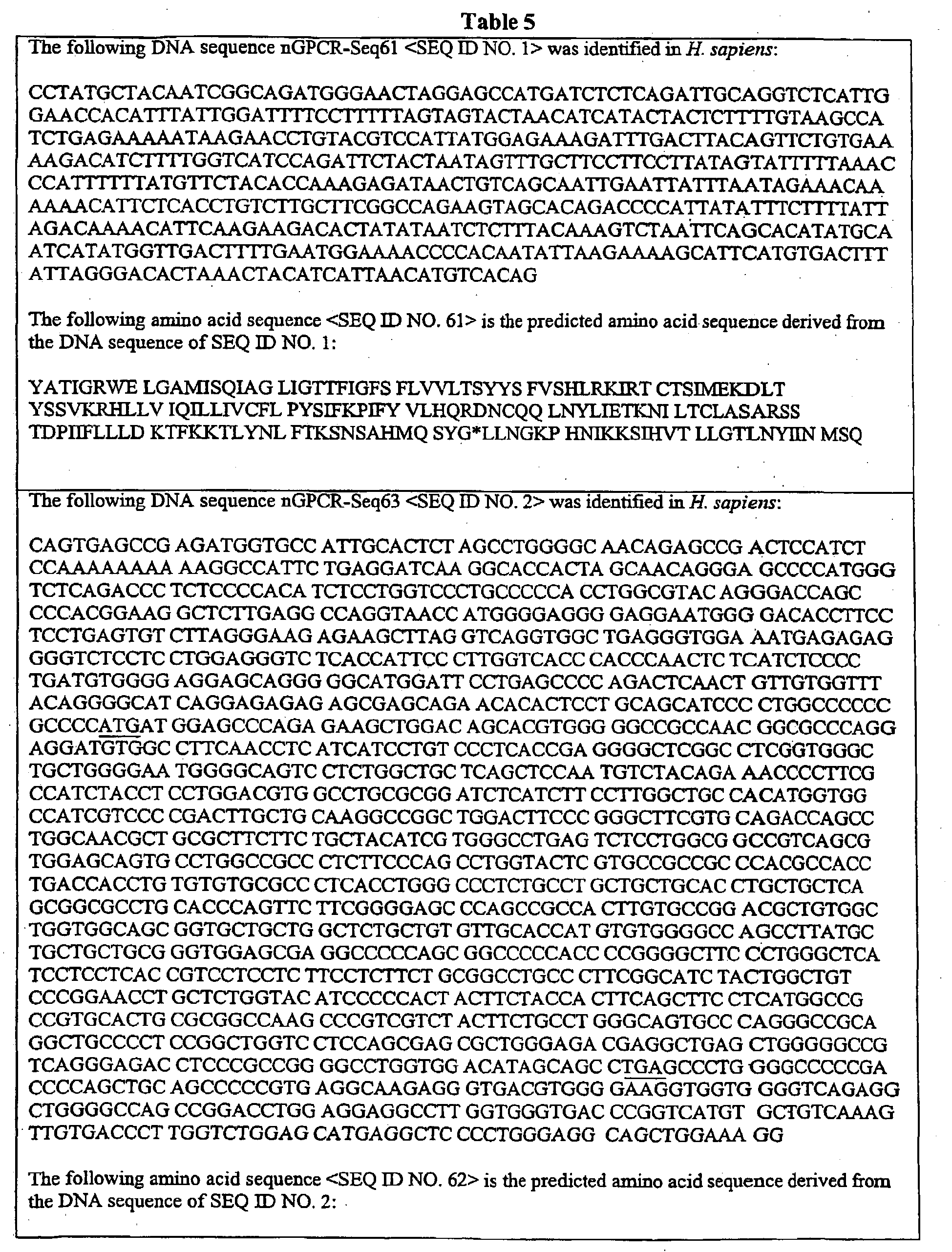

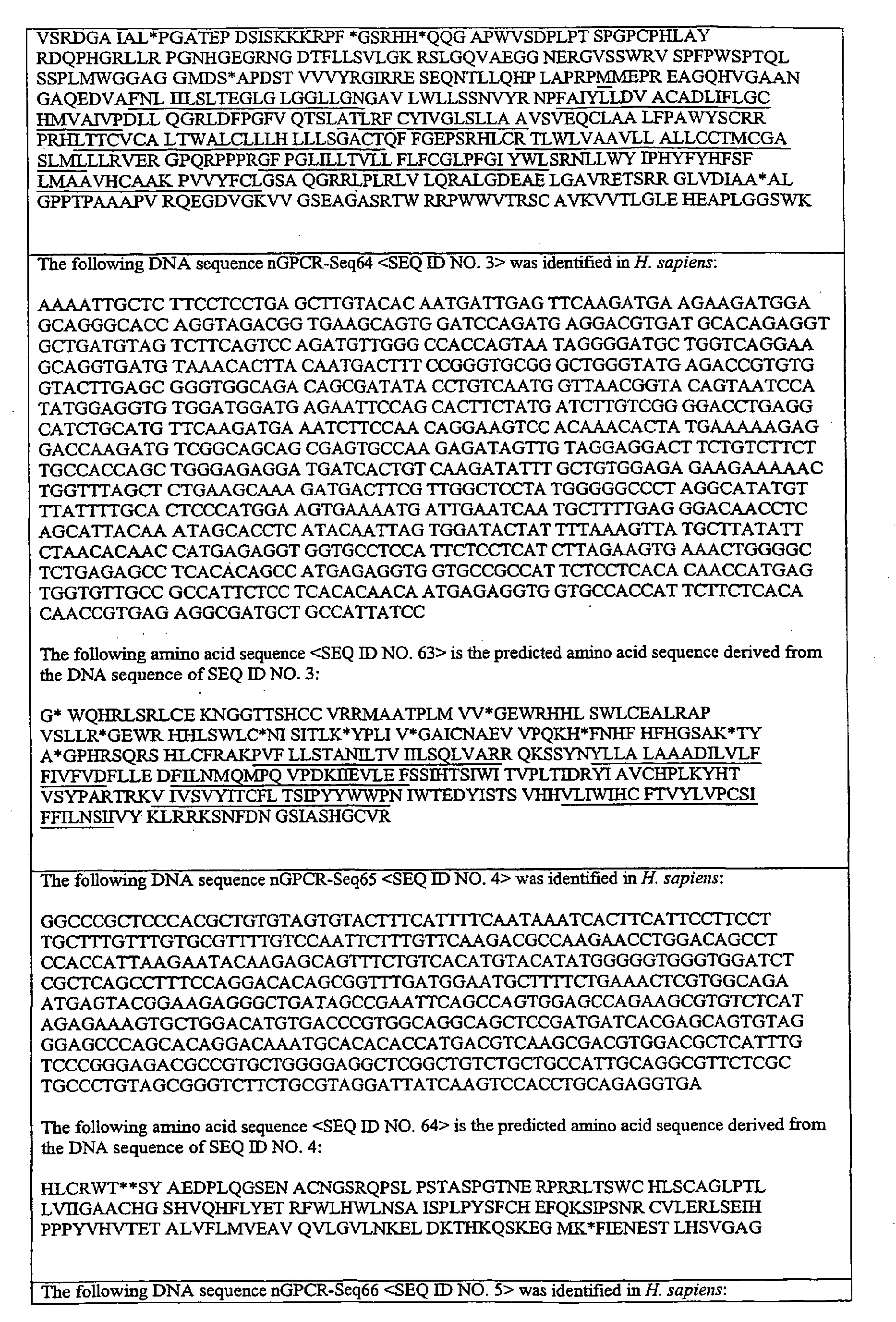

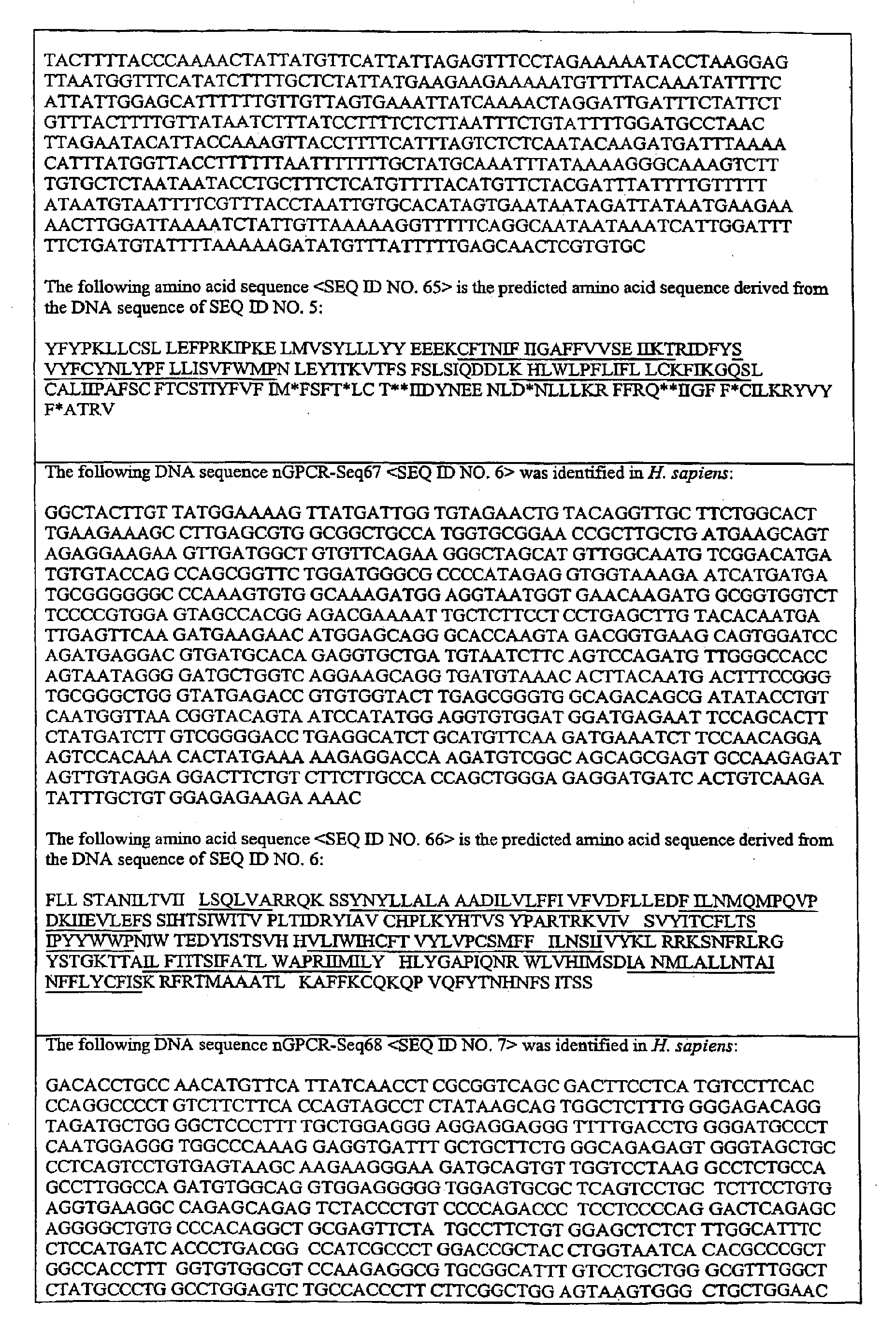

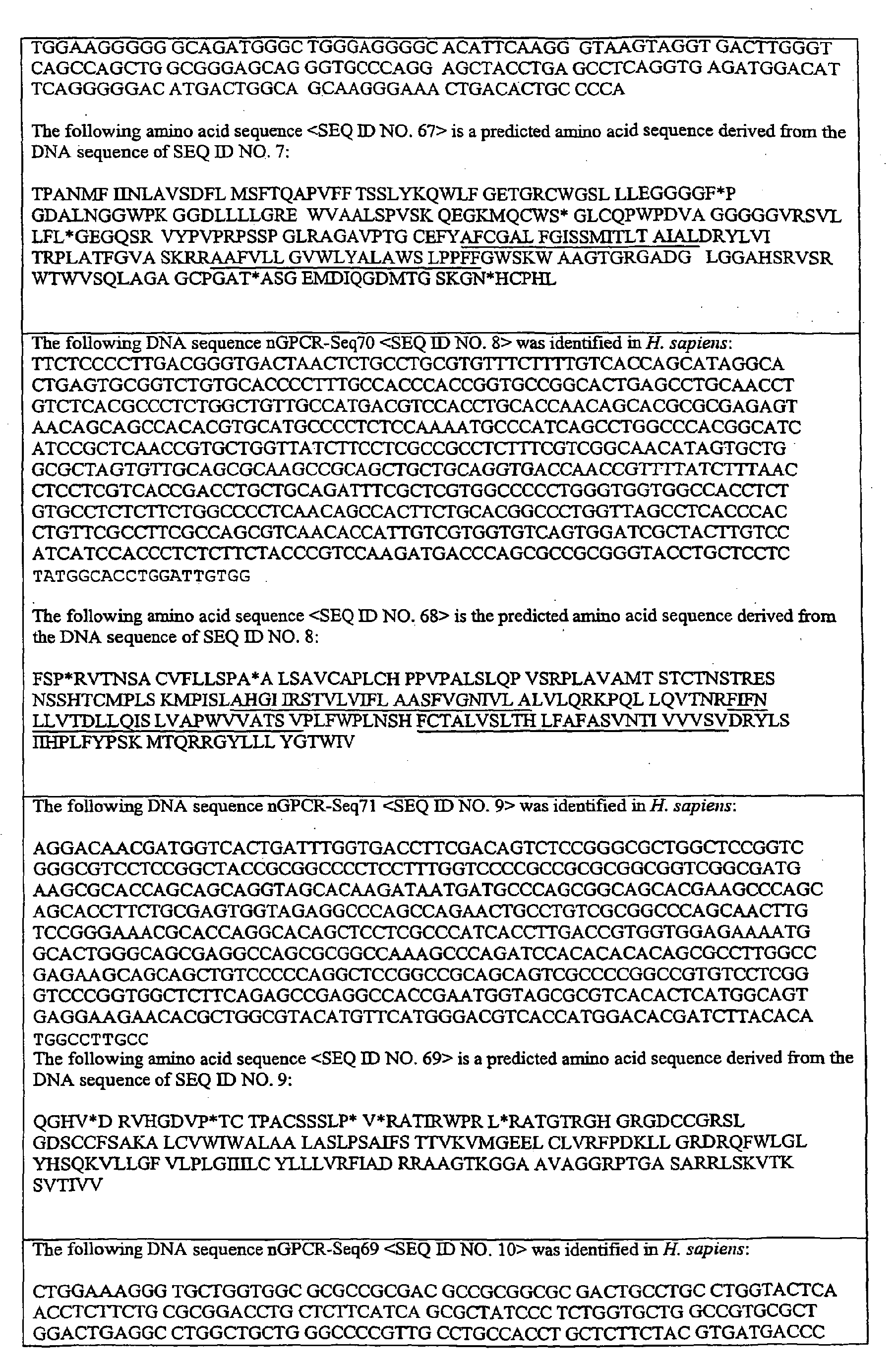

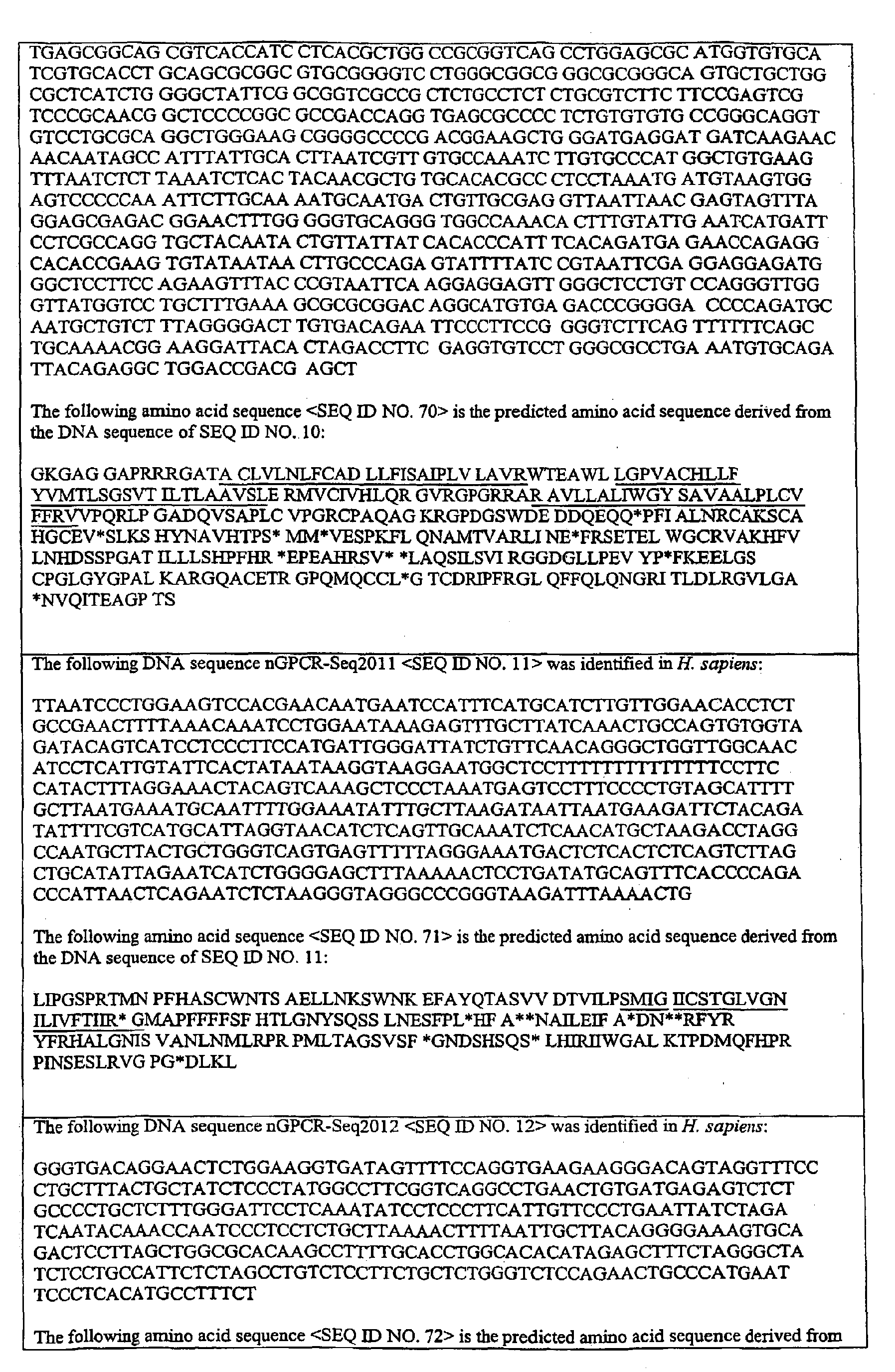

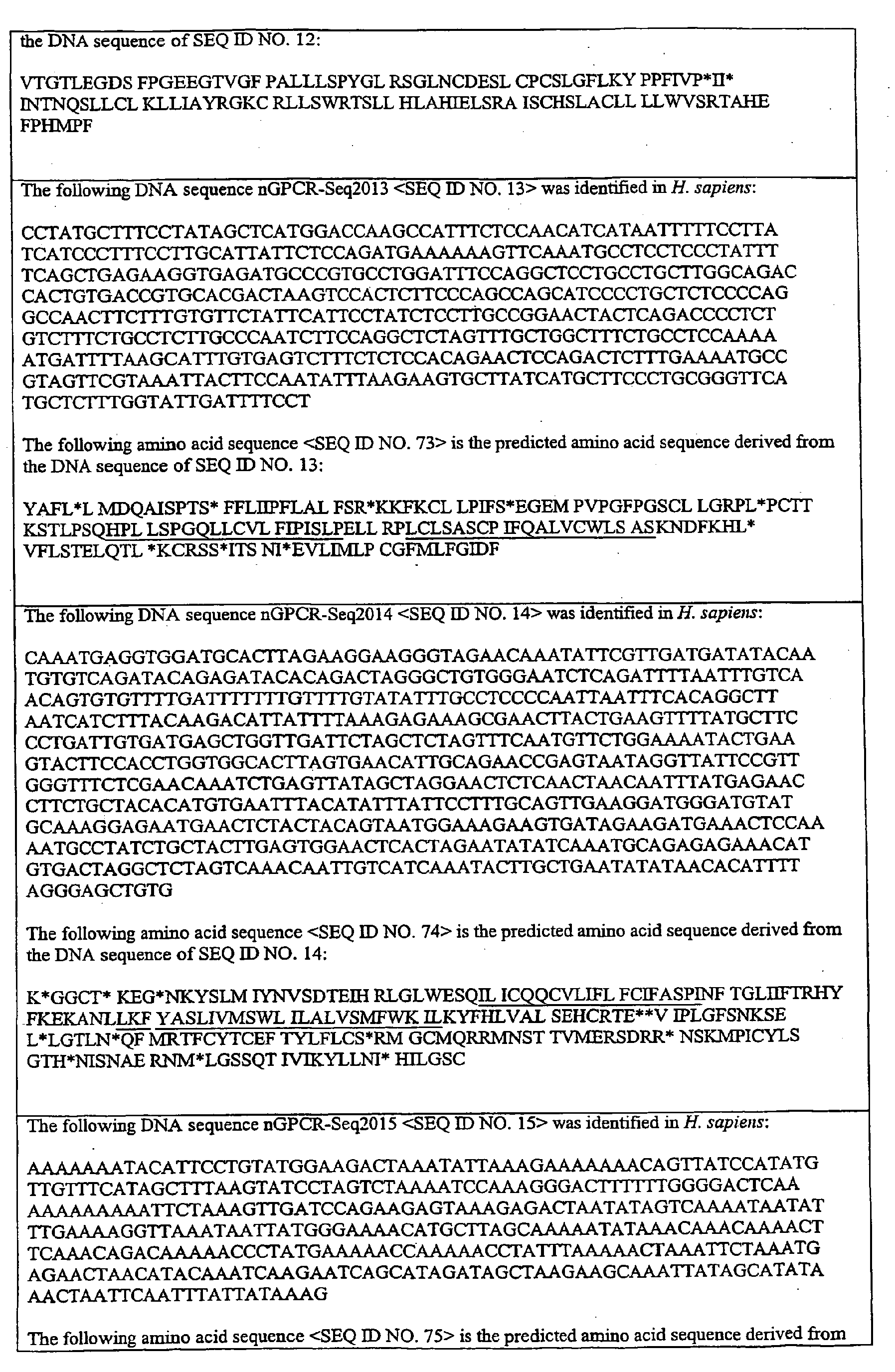

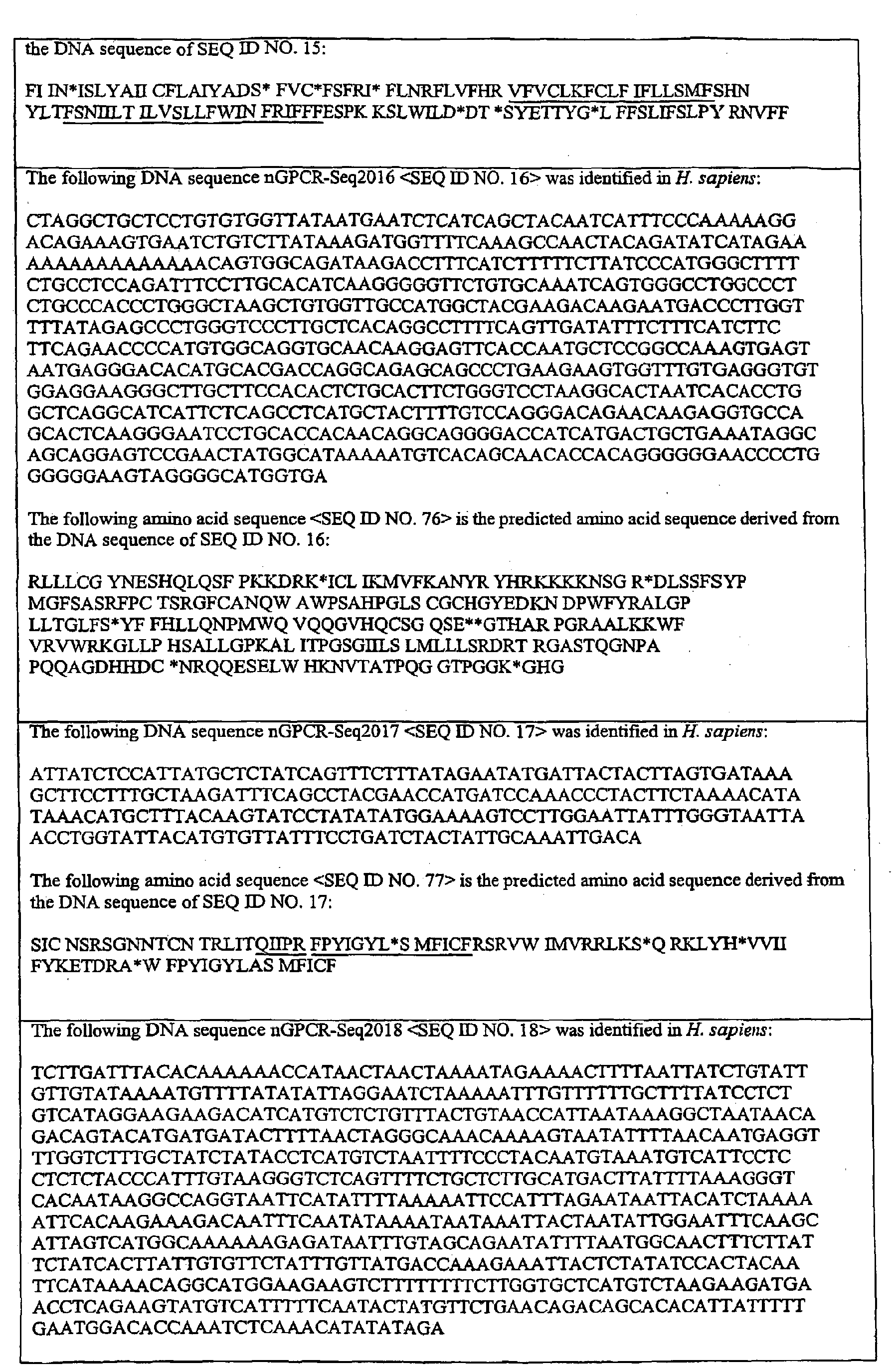

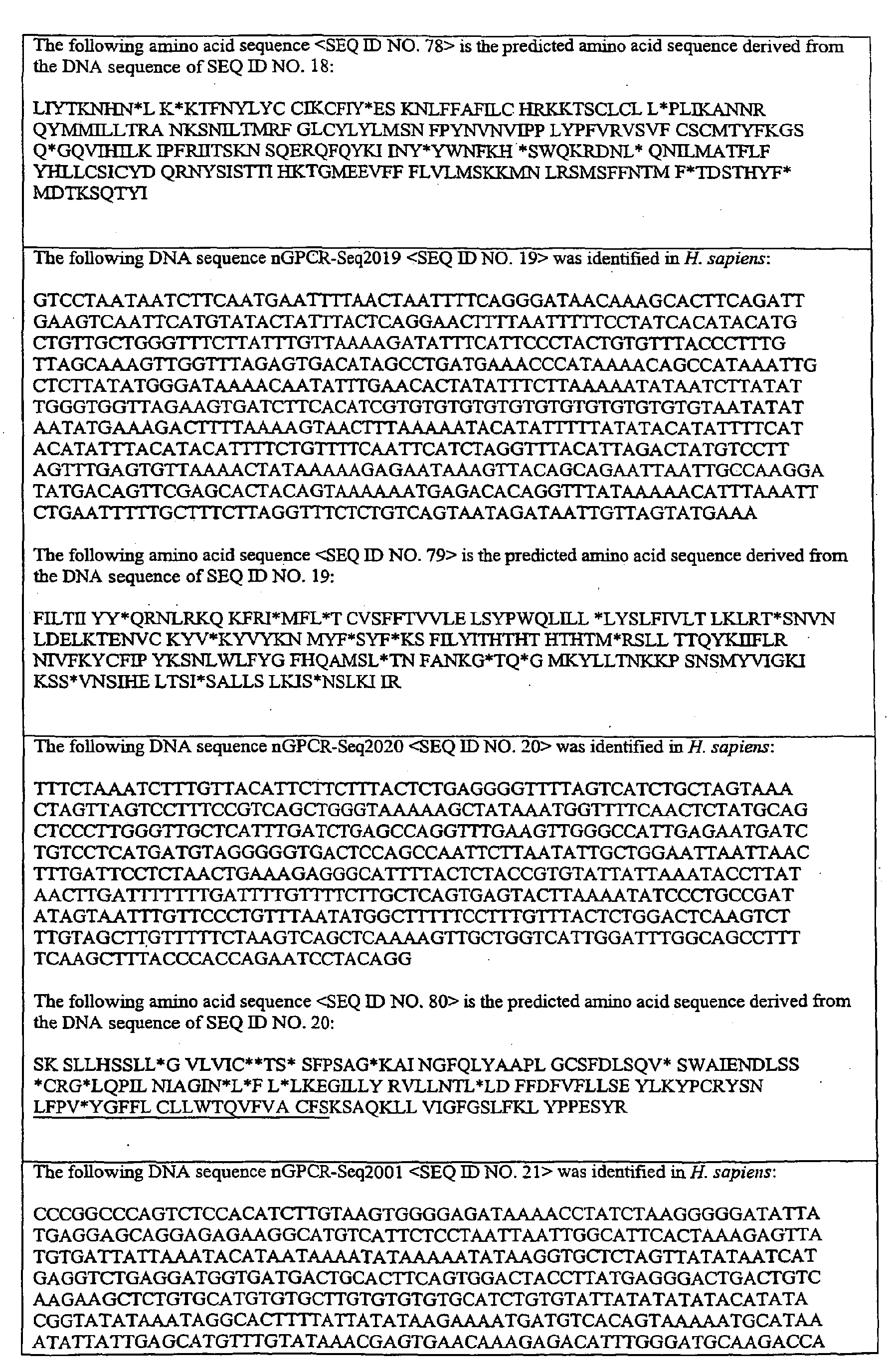

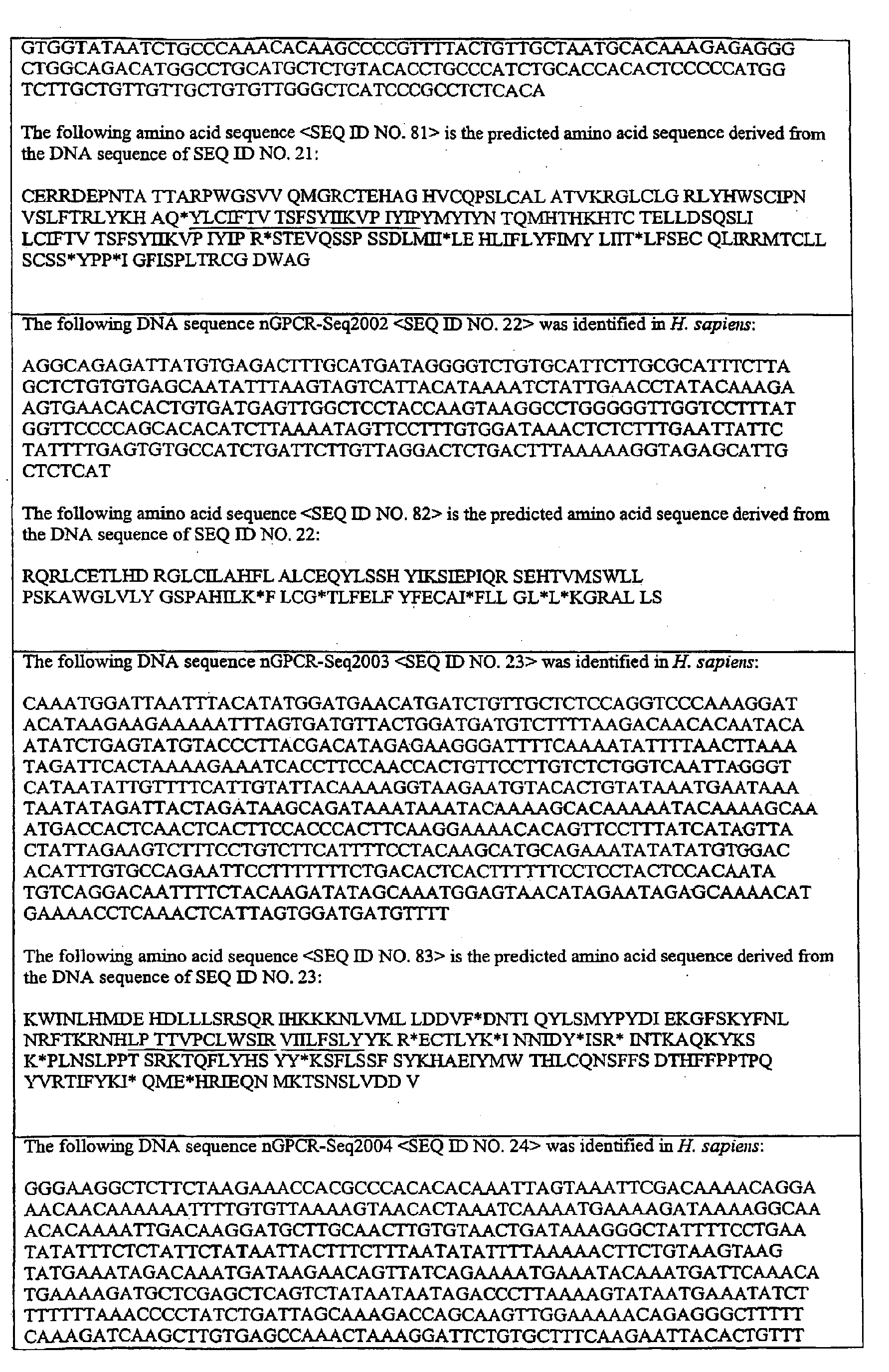

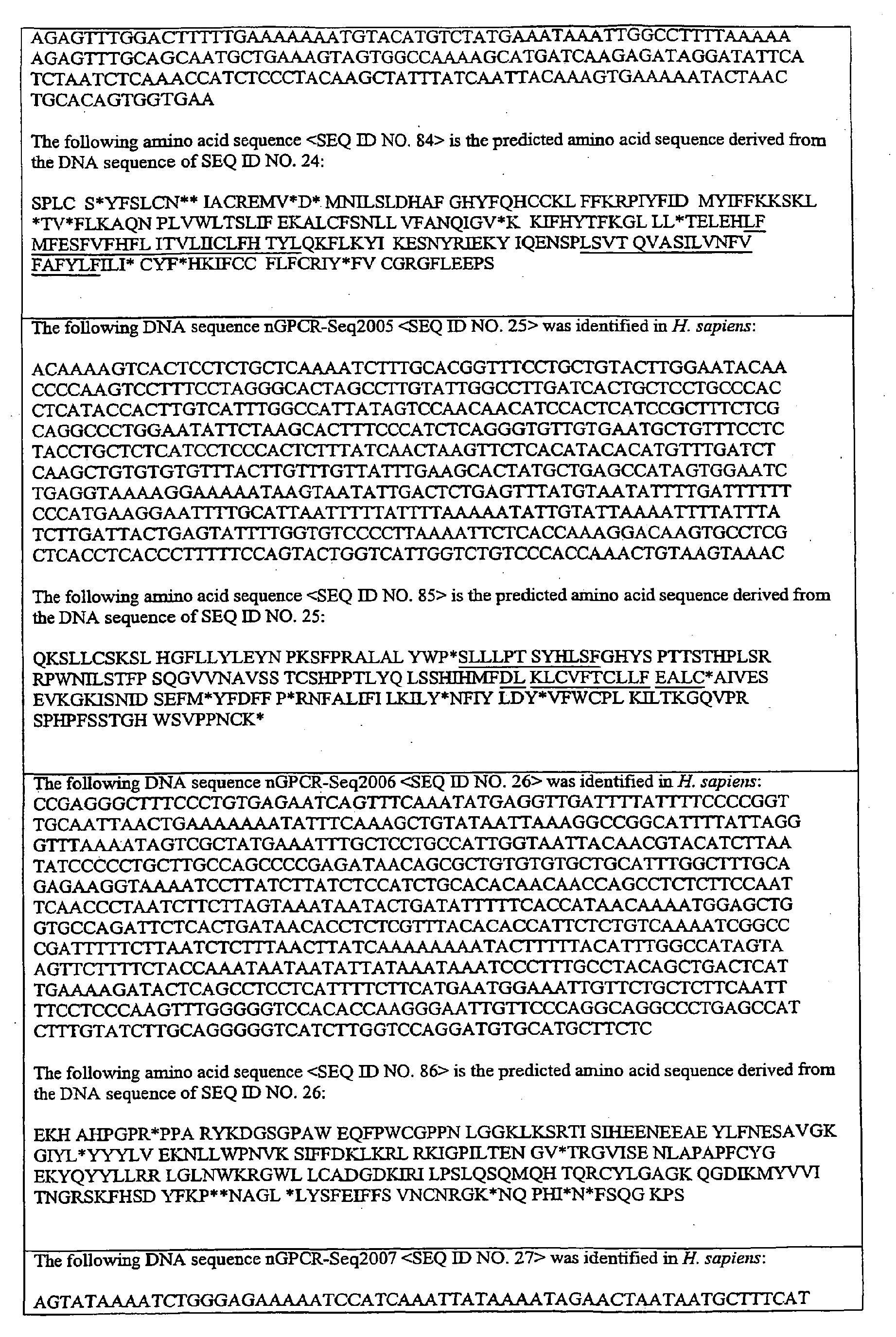

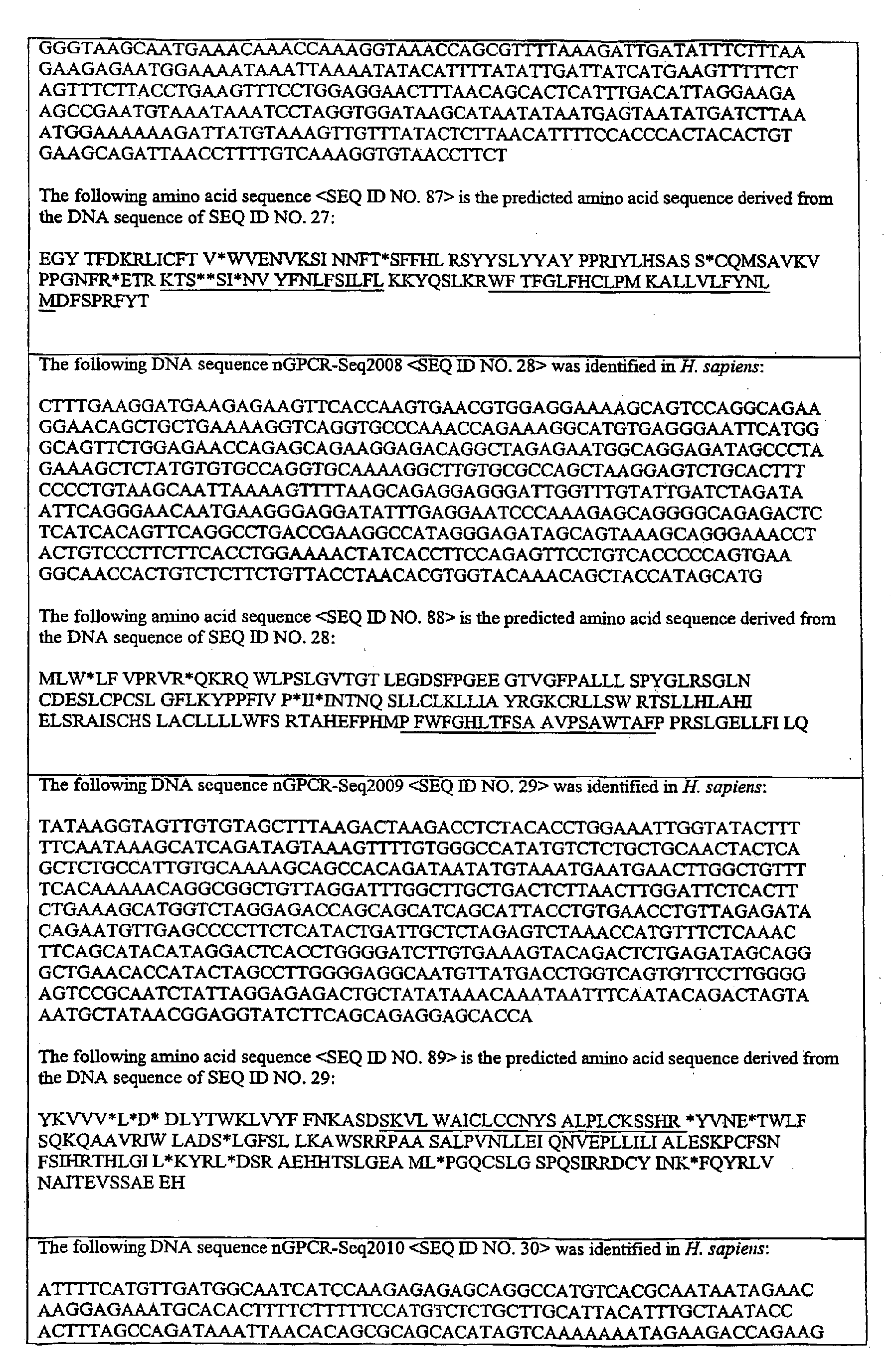

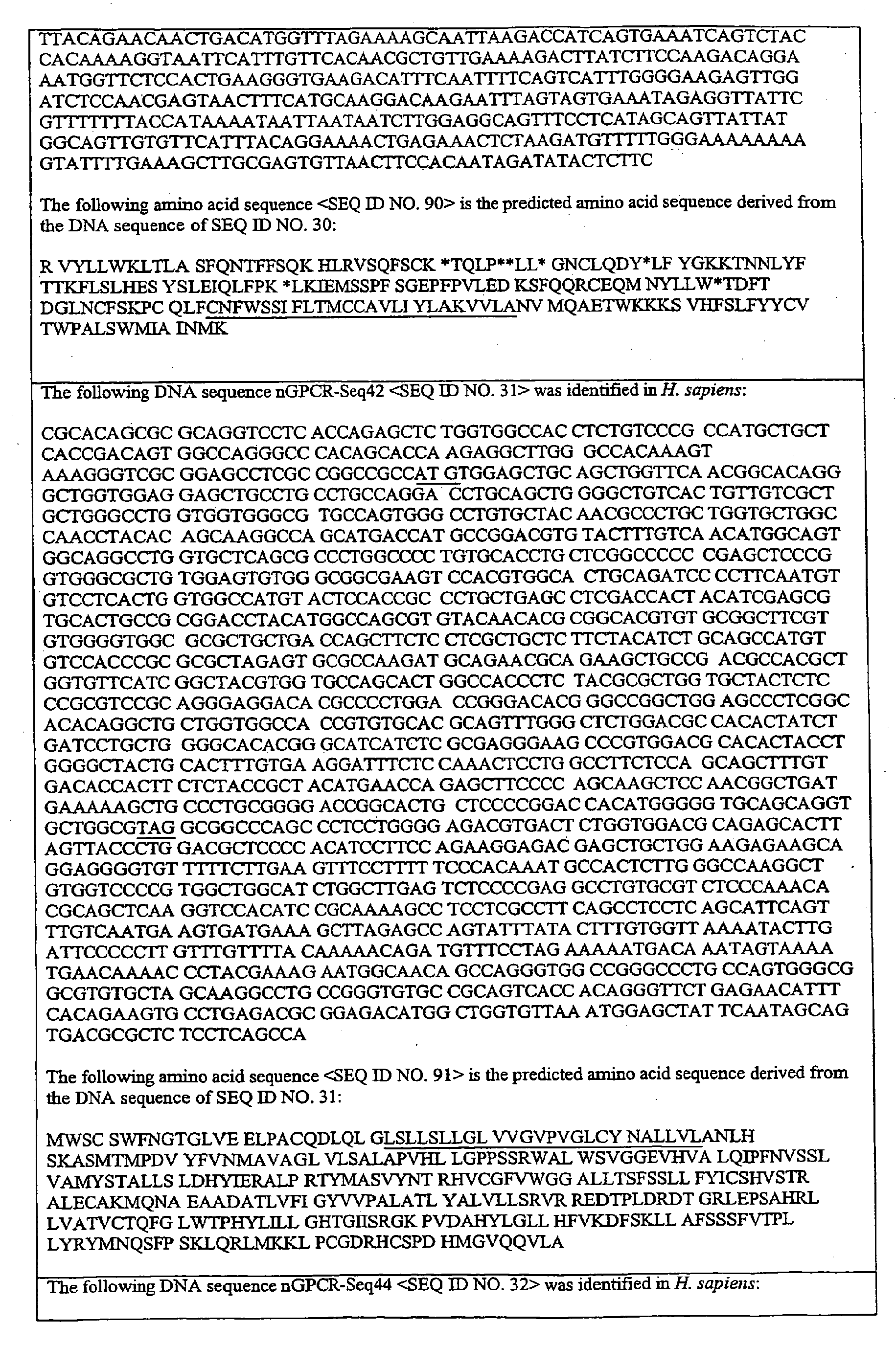

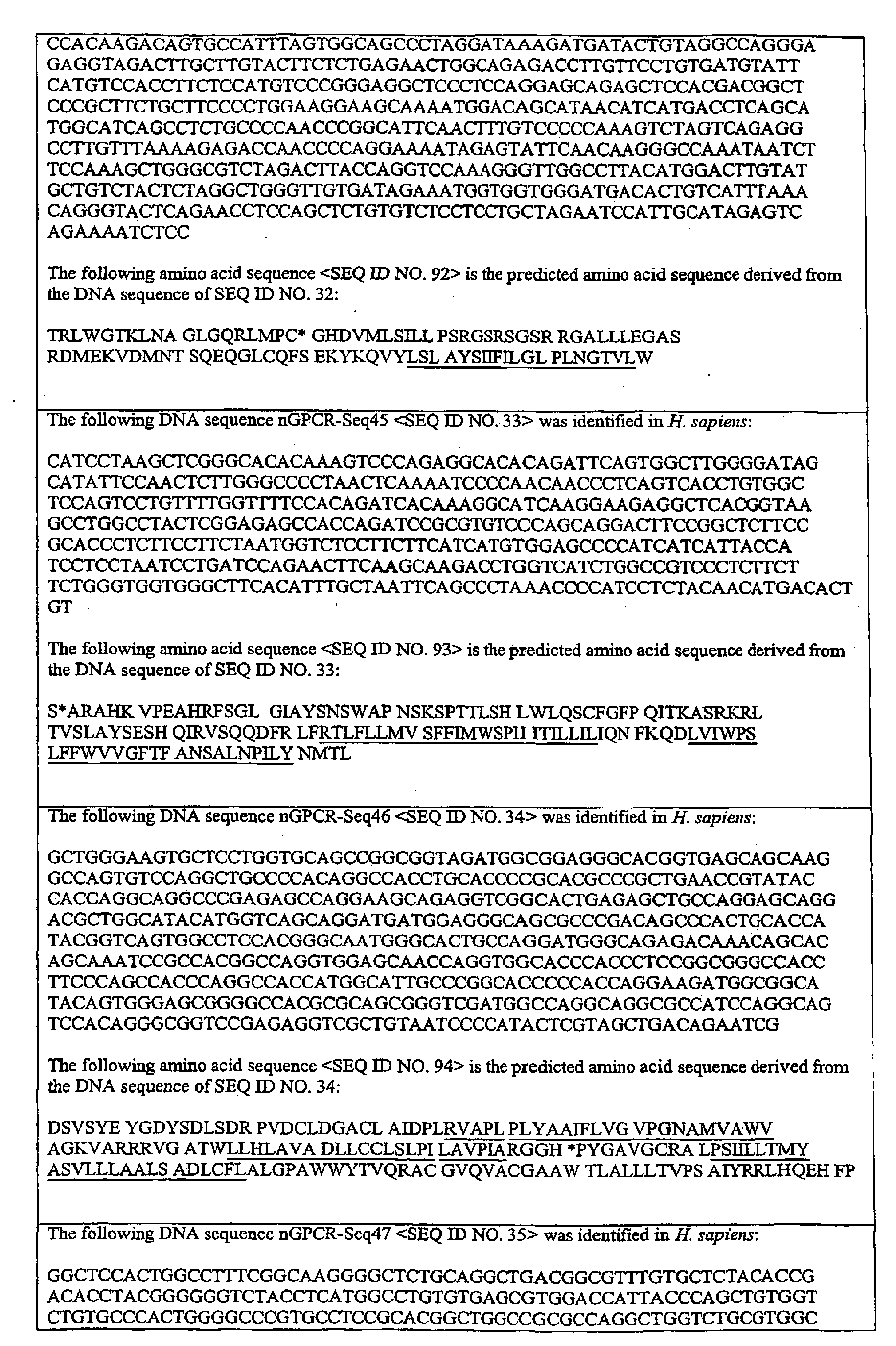

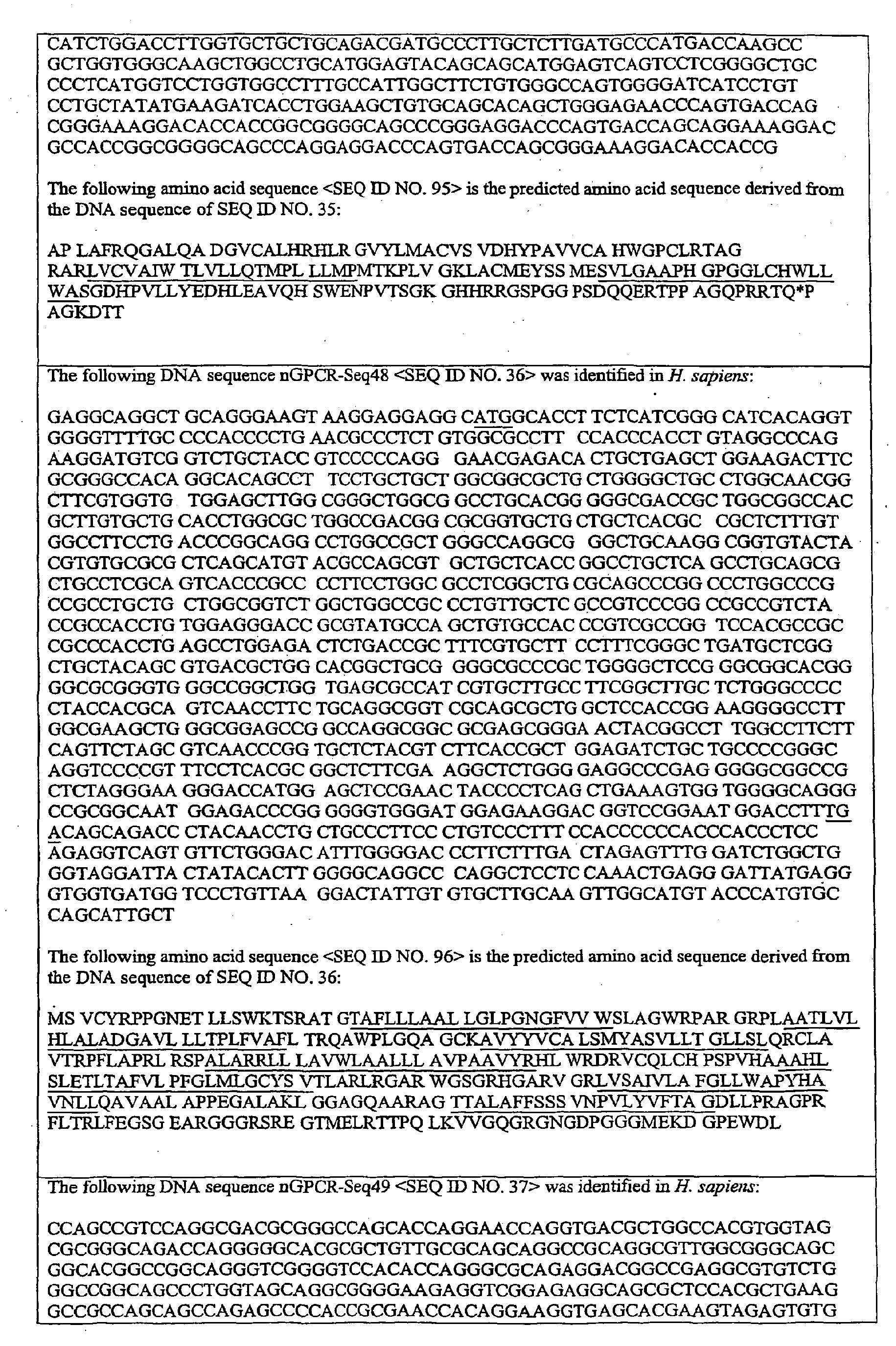

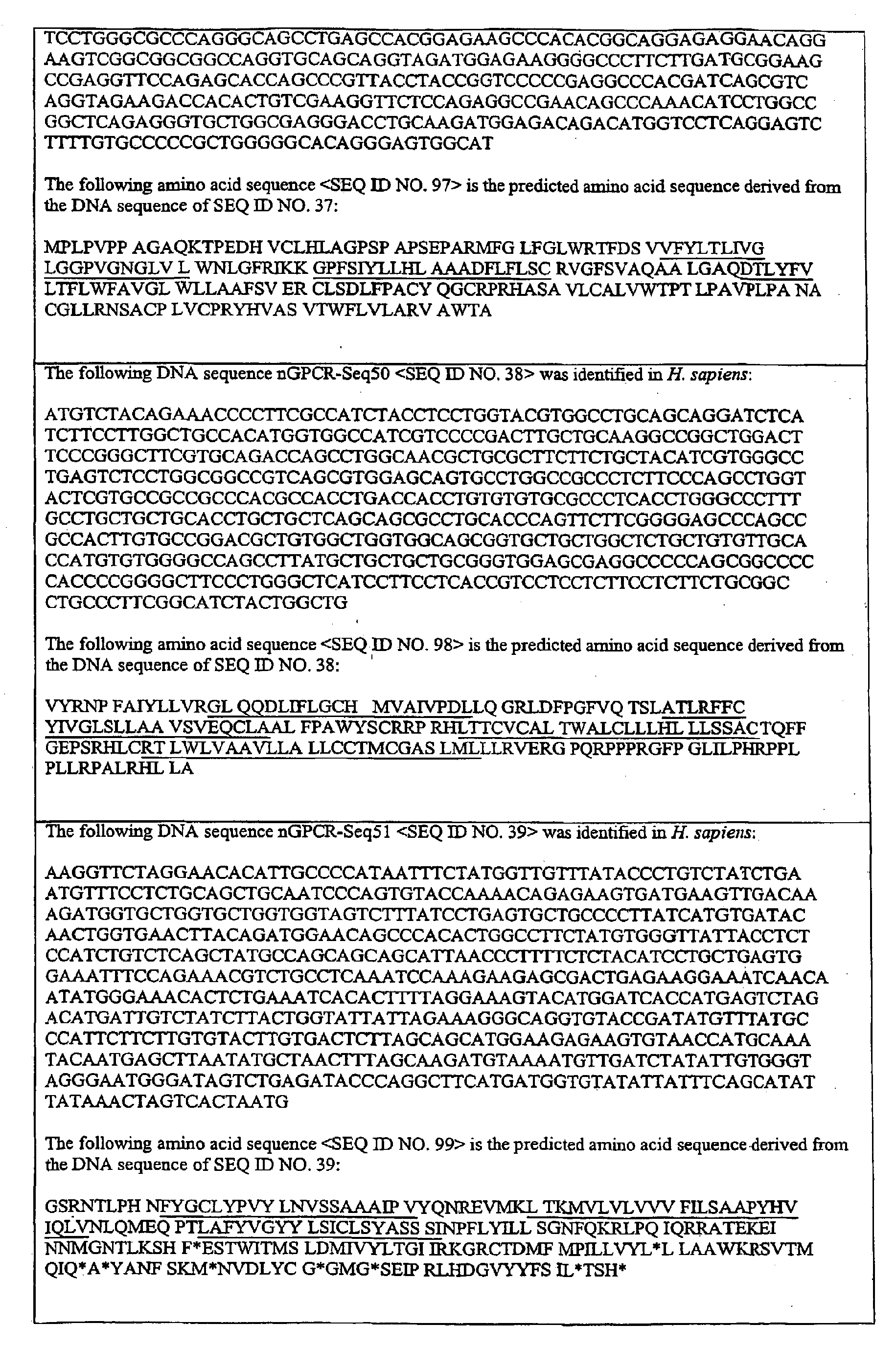

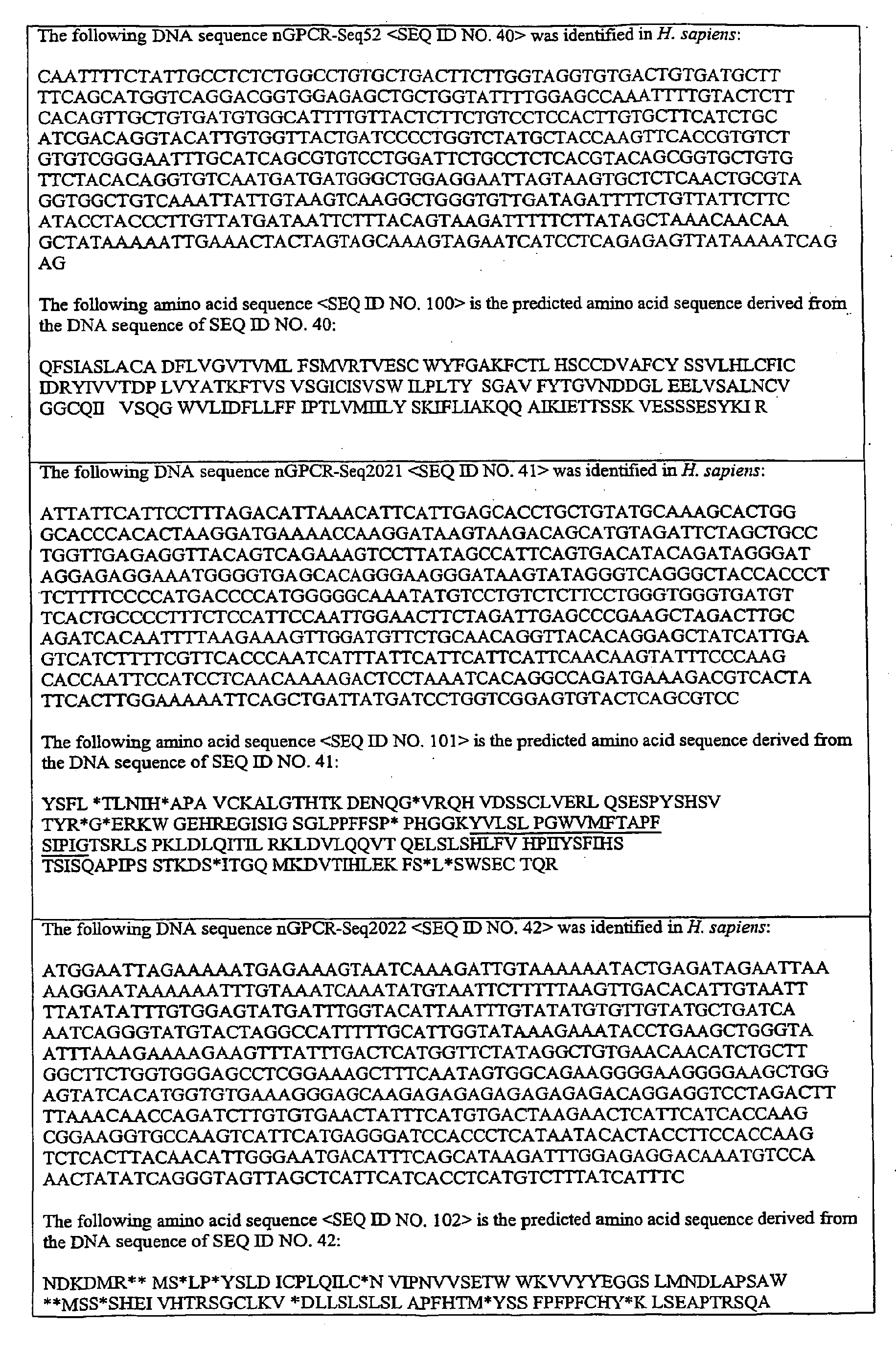

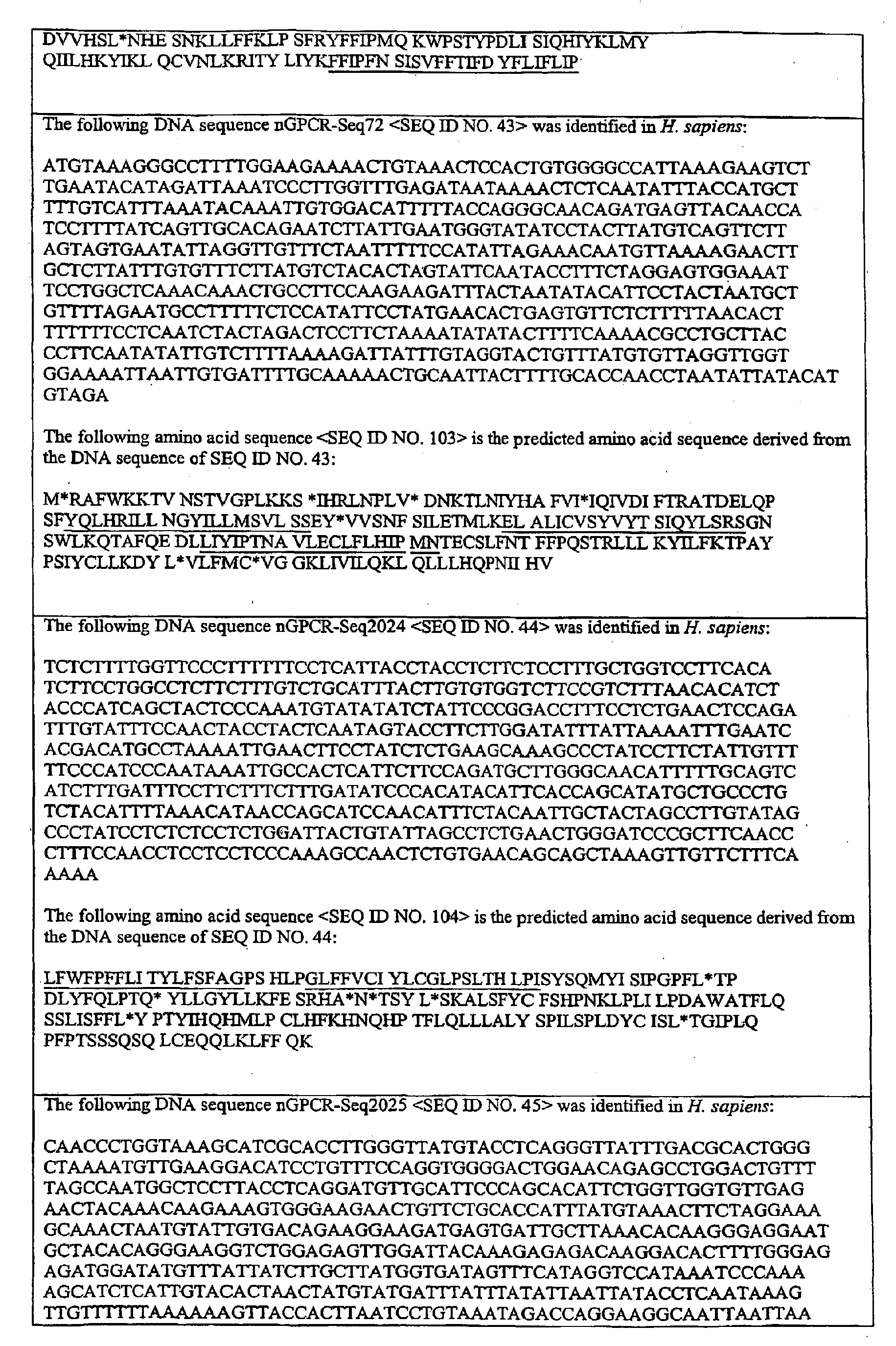

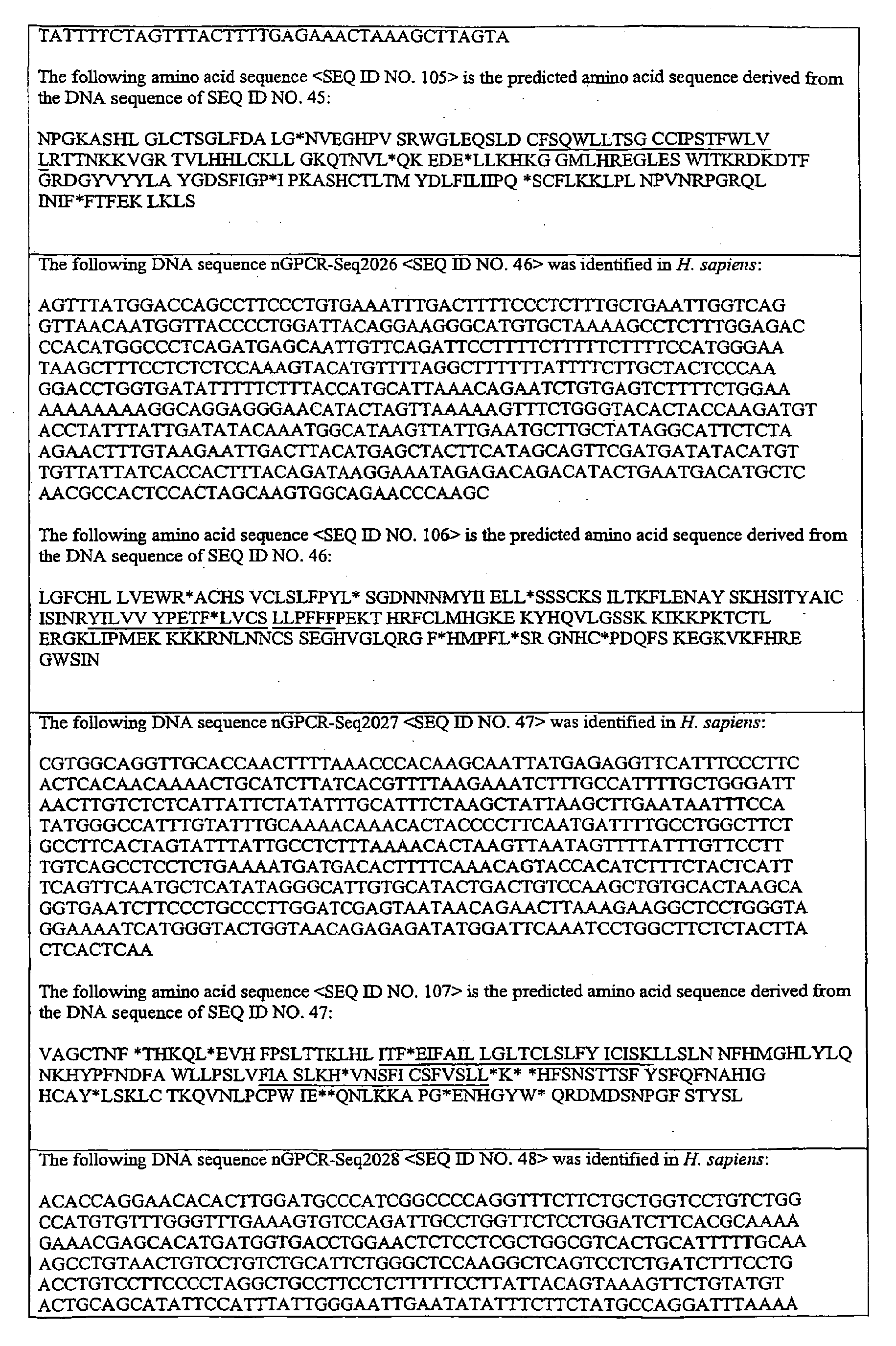

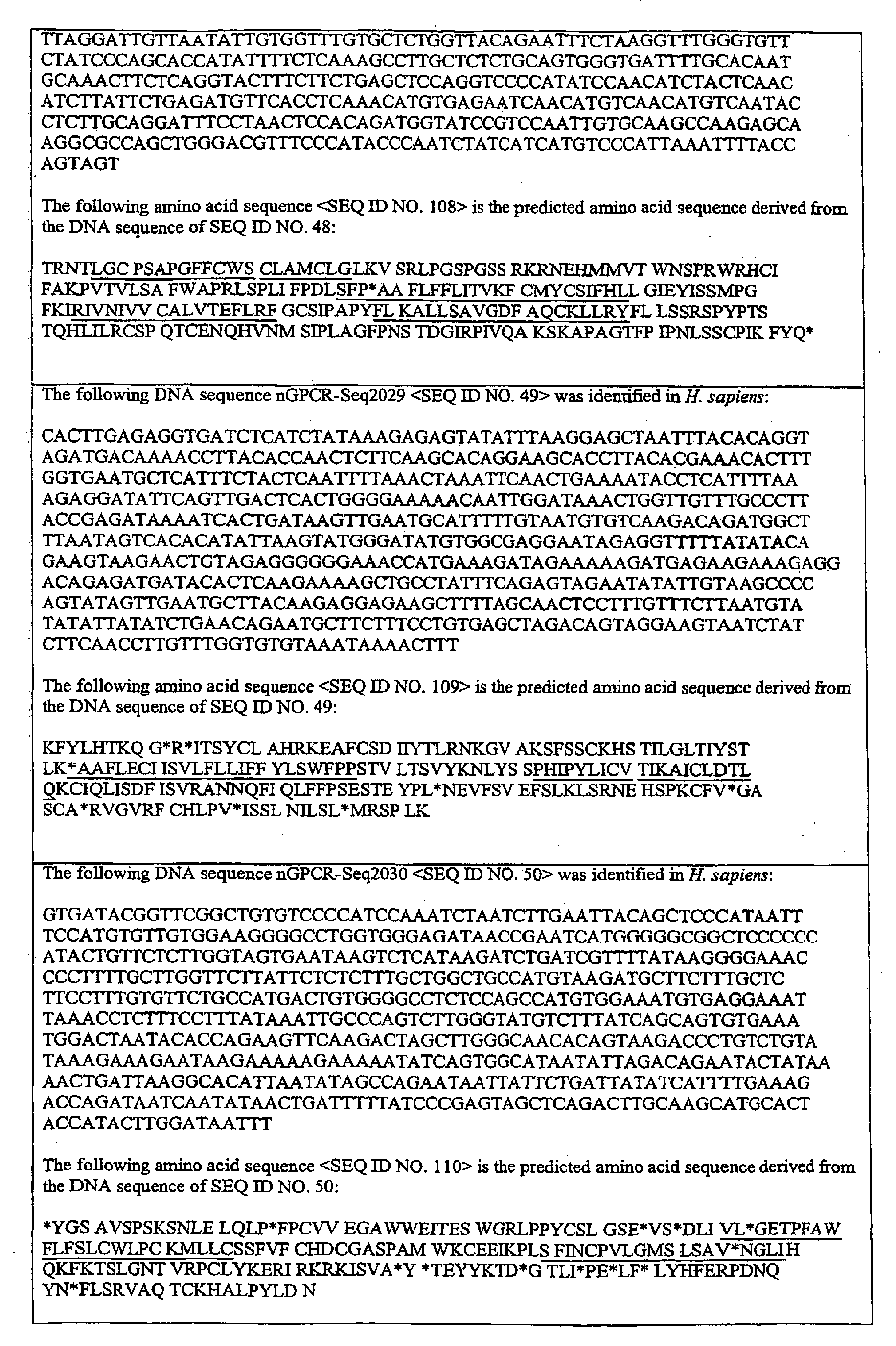

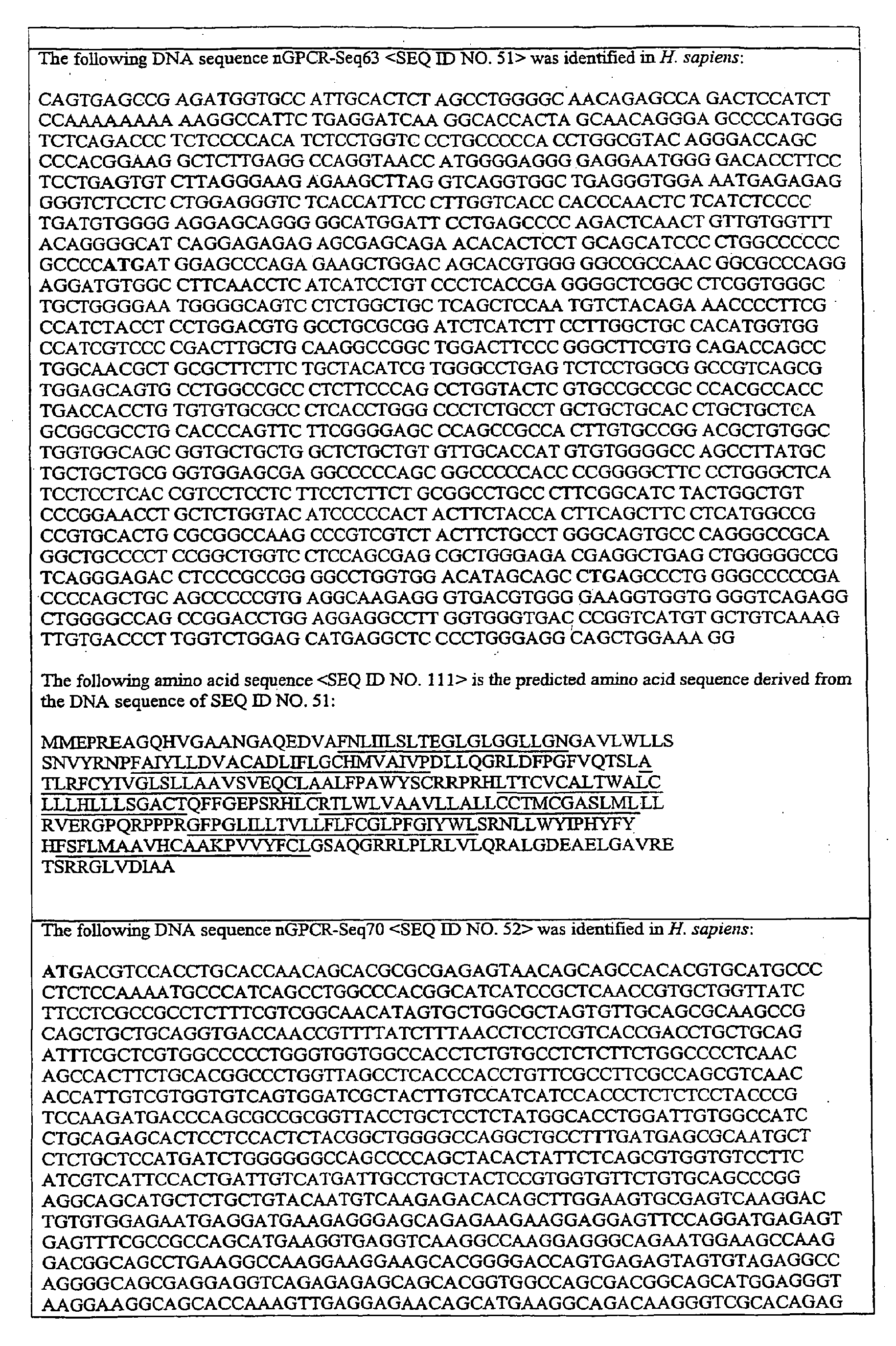

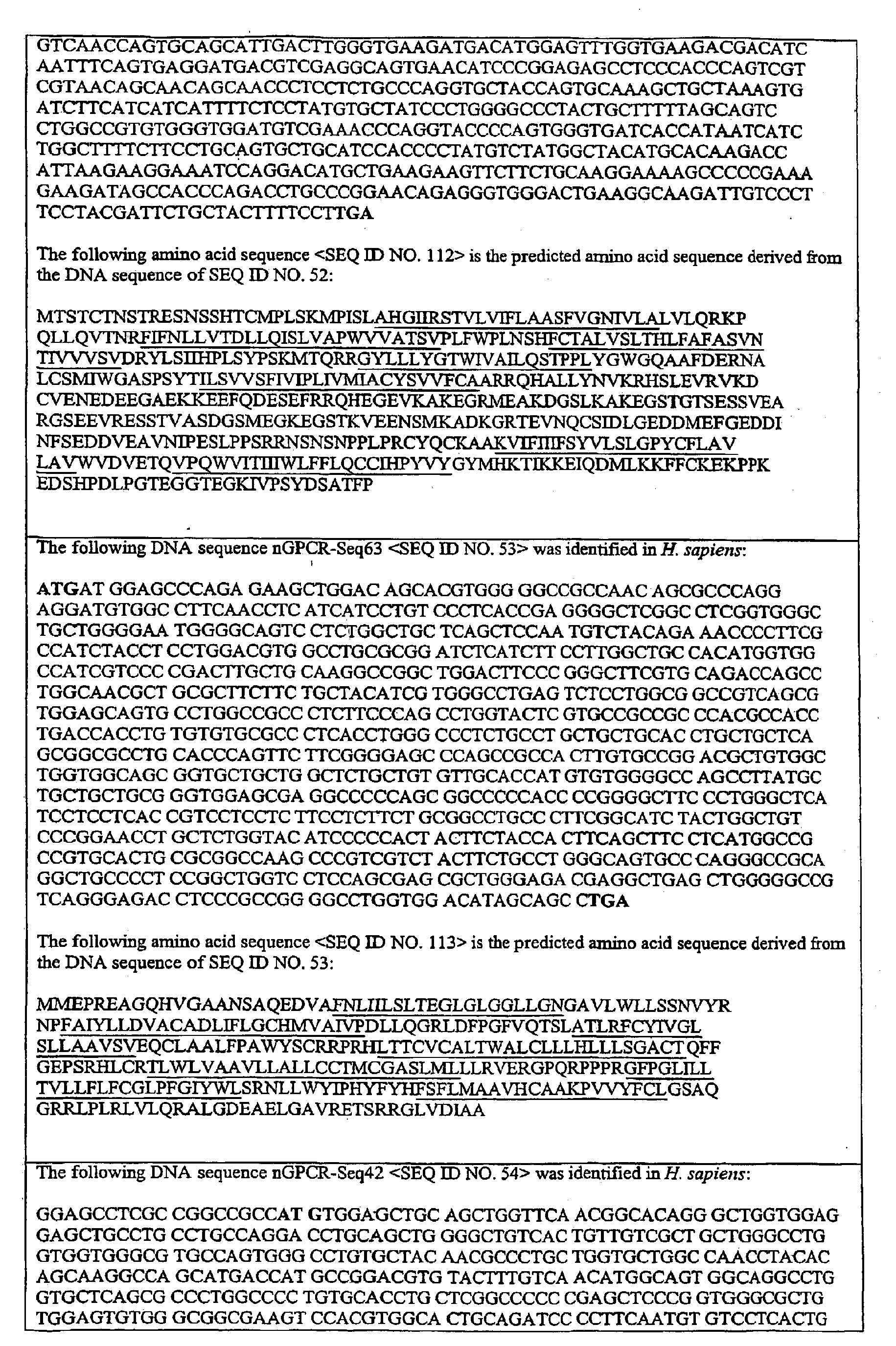

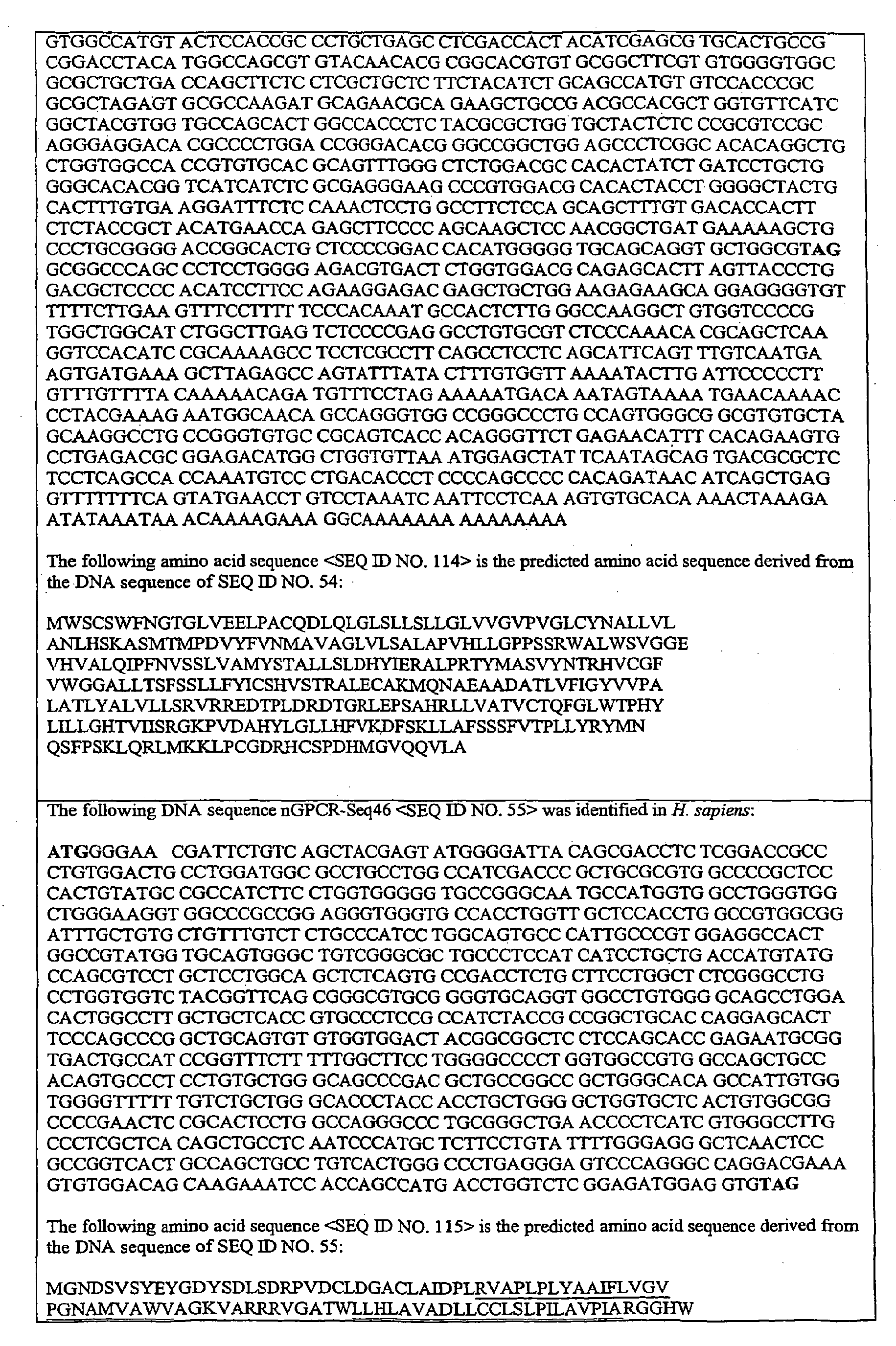

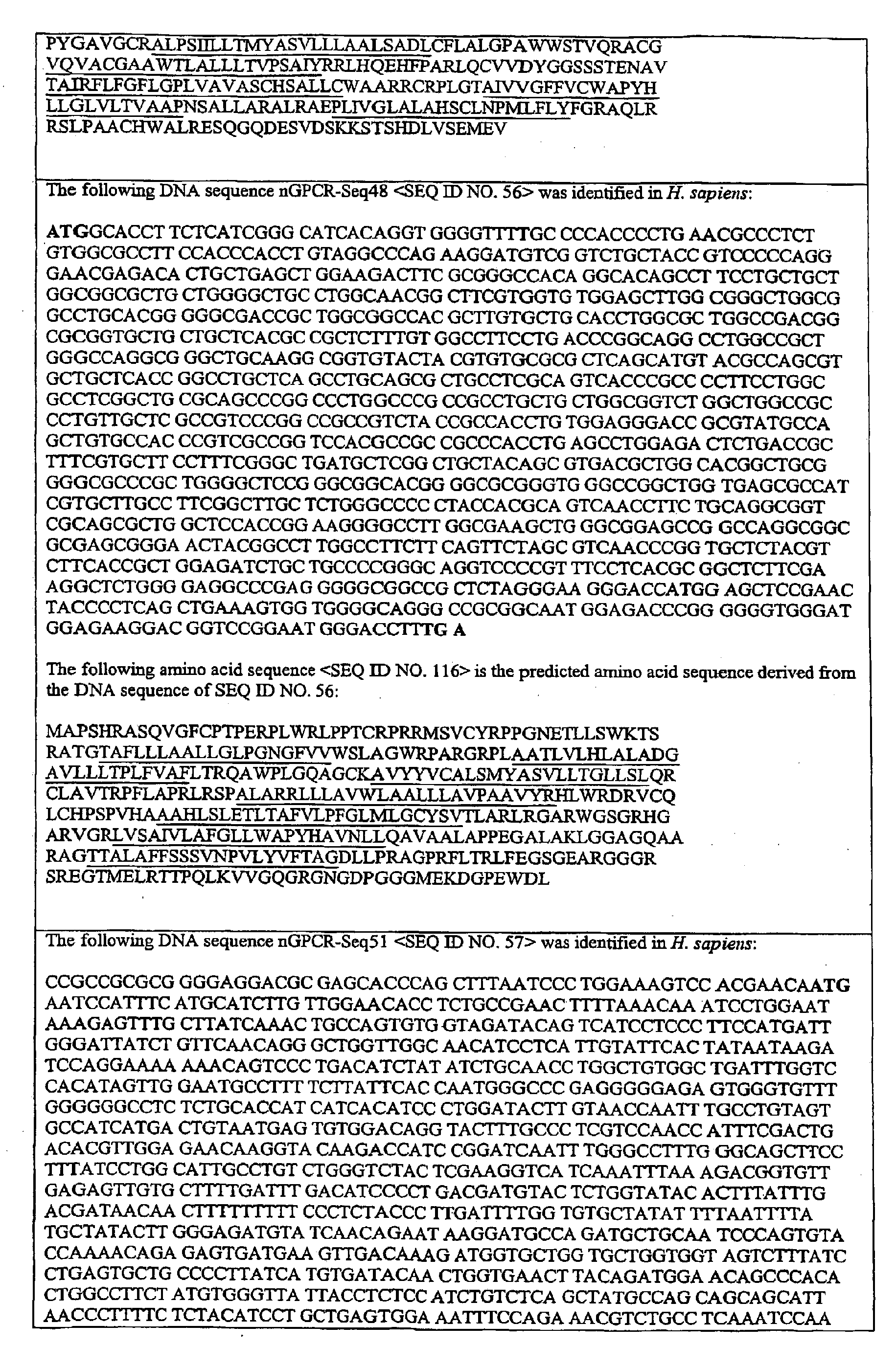

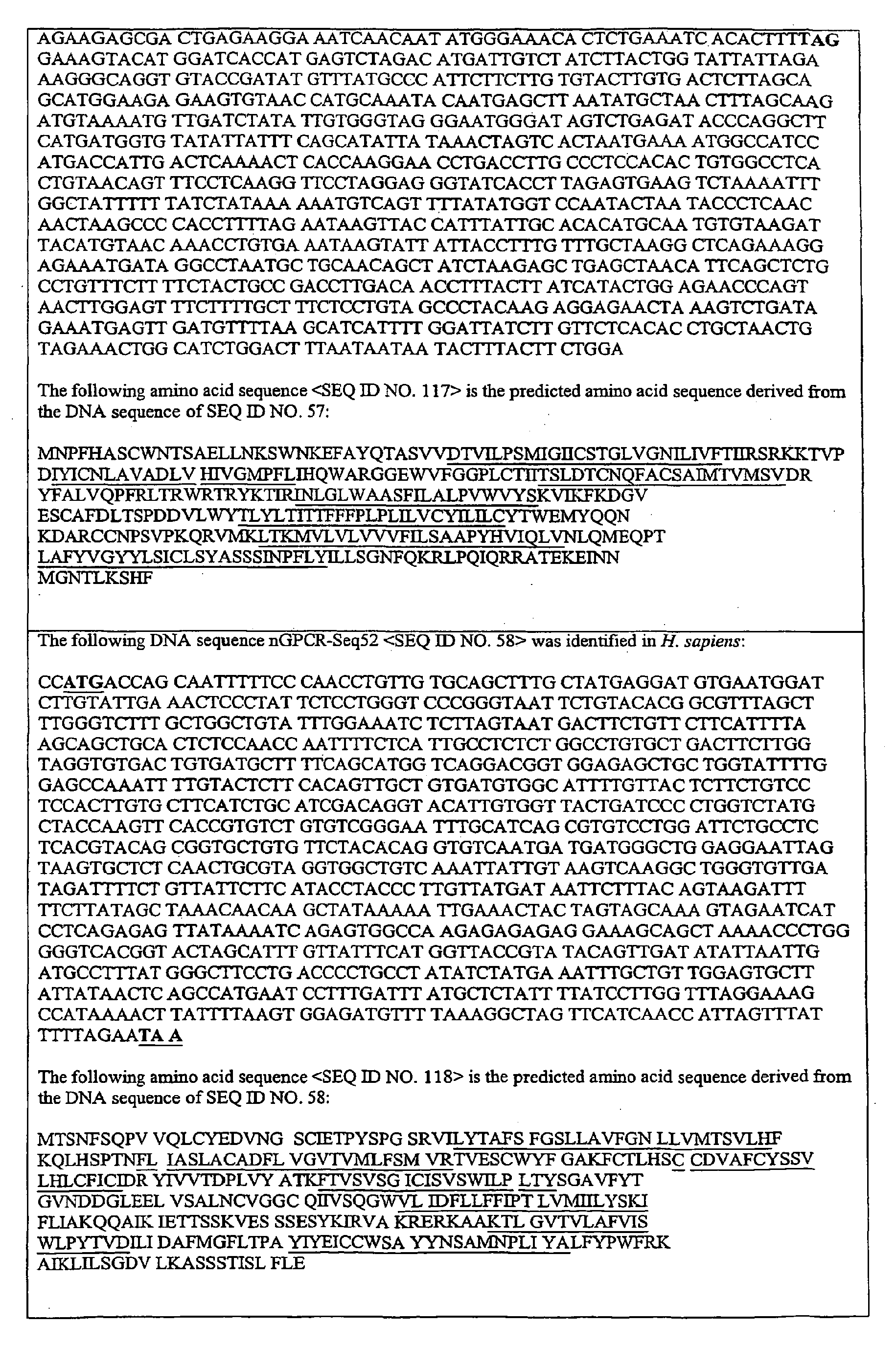

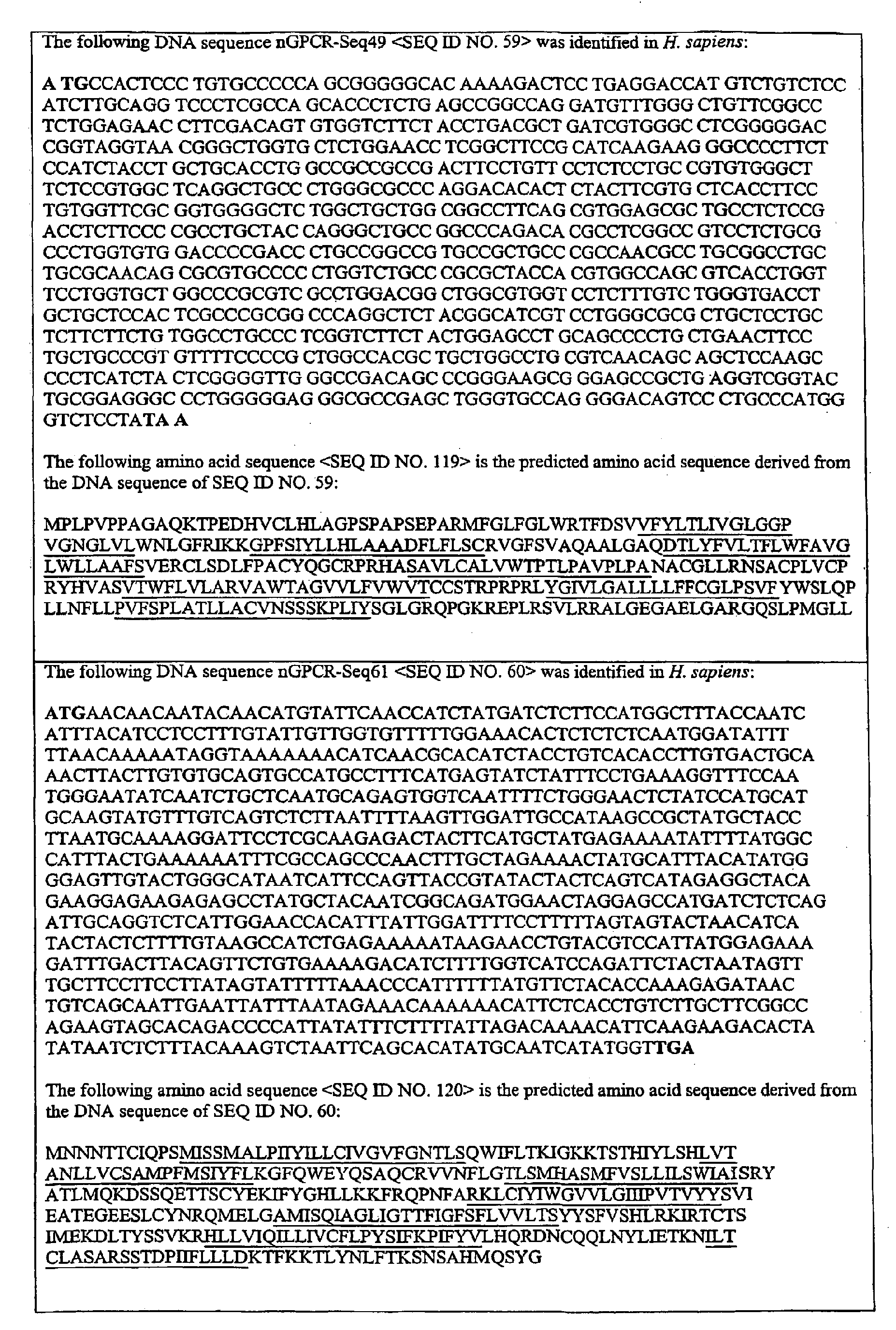

### Example 2: Cloning of nGPCR-x

cDNAs may be sequenced directly using an AB1377 or ABI373A fluorescence-based sequencer (Perkin Elmer/Applied Biosystems Division, PE/ABD, Foster City, CA) and the ABI PRISM Ready Dye-Deoxy Terminator kit with Taq FS polymerase. Each ABI cycle sequencing reaction contains about 0.5 µg of plasmid DNA. Cycle-sequencing is performed using an initial denaturation at 98C for 1 minute, followed by 50 cycles: 98C for 30 seconds, annealing at 50C for 30 seconds, and extension at 60C for 4 minutes. Temperature cycles and times are controlled by a Perkin-Elmer 9600 thermocycler. Extension products are purified using Centriflex gel filtration (Advanced Genetic Technologies Corp., Gaithersburg, MD). Each reaction product is loaded by pipette onto the column, which is then centrifuged in a swinging bucket centrifuge (Sorvall model RT6000B table top centrifuge) at 1500 x g for 4 minutes at room temperature. Column-purified samples are dried under vacuum for about 40 minutes and then dissolved in 5 µl of a DNA loading solution (83% deionized formamide, 8.3 mM EDTA, and 1.6 mg/ml Blue Dextran). The samples are then heated to 90C for three minutes and loaded into the gel sample wells for sequence analysis by the ABI377 sequencer. Sequence analysis is done by importing ABI373A files into the Sequencher program (Gene Codes, Ann Arbor, MI). Generally, sequence reads of 700 bp are obtained. Potential sequencing errors are minimized by obtaining sequence information from both DNA strands and by re-sequencing difficult areas using primers at different locations until all sequencing ambiguities are removed.

To isolate a cDNA clone encoding full length nGPCR, a DNA fragment corresponding to a nucleotide sequence selected from the group consisting of SEQ ID NO:1 to SEQ ID NO:60, or a portion thereof, can be used as a probe for hybridization screening of a phage cDNA library. The DNA fragment is amplified by the polymerase chain reaction (PCR) method. The PCR reaction mixture of 50 ml contains polymerase mixture (0.2 mM dNTPs, 1x PCR Buffer and 0.75 ml Expand High Fidelity Polymerase (Roche Biochemicals)), 1 µg of 3206491 plasmid, and 50 pmoles of forward primer and 50 pmoles of reverse primer. The primers are preferably 10 to 25 nucleotides in length and are determined by procedures well known to those skilled in the art. Amplification is performed in an Applied Biosystems PE2400 thermocycler, using the following program: 95C for 15 seconds, 52C for 30 seconds and 72C for 90 seconds; repeated for 25 cycles. The amplified product is separated from the plasmid by agarose gel electrophoresis, and purified by Qiaquick gel extraction kit (Qiagen).

A lambda phage library containing cDNAs cloned into lambda ZAPII phage-vector is plated with E. coli XL-1 blue host, on 15 cm LB-agar plates at a density of 50,000 pfu per plate, and grown overnight at 37C; (plated as described by Sambrook et al., supra). Phage plaques are transferred to nylon membranes (Amersham Hybond NJ), denatured for 2 minutes in denaturation solution (0.5 M NaOH, 1.5 M NaCl), renatured for 5 minutes in renaturation solution (1 M Tris pH 7.5, 1.5 M NaCl), and washed briefly in 2xSSC (20x SSC: 3 M NaCl, 0.3 M Na-citrate). Filter membranes are dried and incubated at 80C for 120 minutes to cross link the phage DNA to the membranes.

The membranes are hybridized with a DNA probe prepared as described above. A DNA fragment (25 ng) is labeled with α-32P-dCTP (NEN) using Rediprime random priming (Amersham Pharmacia Biotech), according to manufacturers instructions. Labeled DNA is separated from unincorporated nucleotides by S200 spin columns (Amersham Pharmacia Biotech), denatured at 95 C for 5 minutes and kept on ice. The DNA-containing membranes (above) are pre-hybridised in 50 ml ExpressHyb (Clontech) solution at 68C for 90 minutes. Subsequently, the labeled DNA probe is added to the hybridization solution, and the probe is left to hybridise to the membranes at 68C for 70 minutes. The membranes are washed five times in 2x SSC, 0.1% SDS at 42C for 5 minutes each, and finally washed 30 minutes in 0.1x SSC, 0.2% SDS. Filters are exposed to Kodak XAR film (Eastman Kodak Company, Rochester, N.Y., USA) with an intensifying screen at ―80C for 16 hours. One positive colony is isolated from the plates, and replated with about 1000 pfu on a 15 cm LB plate. Plating, plaque lift to filters and hybridization are performed as described above. About four positive phage plaques are isolated form this secondary screening.

cDNA containing plasmids (pBluescript SK-) are rescued from the isolated phages by in vivo excision by culturing XL-1 blue cells co-infected with the isolated phages and with the Excision helper phage, as described by manufacturer (Stratagene). XL-blue cells containing the plasmids are plated on LB plates and grown at 37C for 16 hours. Colonies (18) from each plate are replated on LB plates and grown. One colony from each plate is stricken onto a nylon filter in an ordered array, and the filter is placed on a LB plate to raise the colonies. The filter is then hybridized with a labeled probe as described above. About three positive colonies are selected and grown up in LB medium. Plasmid DNA is isolated from the three clones by Qiagen Midi Kit (Qiagen) according to the manufacturer's instructions. The size of the insert is determined by digesting the plasmid with the restriction enzymes NotI and SalI, which establishes an insert size. The sequence of the entire insert is determined by automated sequencing on both strands of the plasmids.

### nGPCR-70

The PCR reaction was performed in 50 µl containing 31 µl H₂O, 5 µl Buffer II (PE Applied Biosystems AmpliTaq Gold system), 6 µl 25 mM MgCl₂, 2 µl 10 mM dNTP mix, 5 µl Marathon Ready whole human brain cDNA (Clontech #7400-1), 0.3 µl primer VR70C (1 µg/µl)(SEQ ID NO:121), 0.3 µl primer VR70D (1 µg/µl)(SEQ ID NO:122), and 0.4 µl AmpliTaq Gold™ DNA Polymerase. The primer sequence for VR70C is 5'-TTCAAAGCTTATGACGTCCACCTGCACC-3' (SEQ ID NO:121), corresponding to the 5' end of the coding region and containing a HindIII restriction site. The primer sequence for VR70D is 5'- TTCACTCGAGTCAAGGAAAAGTAGCAGAATCGTAG-3' (SEQ ID NO:122), corresponding to the 3' end of the coding region and containing an XhoI restriction site (Genosys).

The PCR reaction was carried out using a GeneAmp PCR 9700 thermocycler (Perkin Elmer Applied Biosystems) and started with 1 cycle of 80C for 20 minutes followed by 95C for 10 minutes, then 12 cycles at 95C for 30 seconds, 72C for 2 minutes decreasing 1C each cycle, 72C for 1 minute, followed by 35 cycles at 95C for 30 seconds, 60C for 30 seconds, 72C for 1 minute. The PCR reaction was loaded on a 0.75% gel. The DNA band was excised from the gel and the DNA was eluted from the agarose using a QIAquick gel extraction kit (Qiagen). The eluted DNA was ethanol-precipitated and resuspended in 4 µl H₂O for ligation. The ligation reaction consisted of 4 µl of fresh ethanol-precipitated PCR product and 1 µl of pCRII-TOPO vector (Invitrogen). The reaction was gently mixed and allowed to incubate for 5 minutes at room temperature followed by the addition of 1 µl of 6x TOPO cloning stop solution and mixing for 10 seconds at room temperature. The sample was then placed on ice and 2 µl was transformed in 50 µl of One Shot cells (Invitrogen) and plated onto ampicillin plates. Seven white colonies were chosen and the presence of an insert was verified by PCR in the following manner. Each colony was resuspended in 2 ml LB broth and incubated at 37C for 2 hours. A 500 µl aliquot was spun down in the microfuge, the supernatant discarded, and the pellet resuspended in 25 µl of H₂O. A 16 µl aliquot was removed and boiled for 5 minutes and the sample was placed on ice. The sample was microfuged briefly to pellet any bacterial debris and PCR was carried out as described above with 15 µl of sample using primers VR70C (SEQ ID NO:121) and VR70D (SEQ ID NO:122).

### nGPCR-63

Isolation of a clone for nGPCR-63 from genomic DNA was performed by PCR in a 50 µl reaction containing Herculase DNA Polymerse blend (Stratagene), with buffer recommendations as supplied by the manufacturer, 200 ng each primers PSK16 and 17 (SEQ ID NO:123 and SEQ ID NO:124, respectively), 150 ng of human genomic DNA (Clontech) and 4% DMSO. The PCR reaction was performed on a on a Robocycler themiocycler (Stratagene) starting with 1 cycle of 94C for 2 minutes followed by 35 cycles of 94C for 30 seconds, 65C for 30 seconds, 72C for 2 minutes. The PCR reaction was purified by the QiaQuick PCR Purification Kit (Qiagen) and eluted in TE. The PCR primer sequences were: PSK16 5'-GATCGAATTC**ATG**ATGGAGCCCAGAGAAGCTGGAC-3' (SEQ ID NO:123) and PSK17 5'-GATCCTCGAG**TCA**GGCTGCTATGTCCACCAGGCC-3' (SEQ ID NO: 124). Translation initiation and termination codons are shown above in bold.

The PCR product was ligated into the pCR-BluntII-TOPO vector (Invitrogen) using the Zero Blunt Topo PCR TA cloning kit as follows. 3µl PCR product DNA, 1 µl pCRII-TOPO vector, and 1 µl TOPOII salt solution (1.2M NaCl, 0.06M MgCl₂) was incubated for 5 minutes at room temperature. To the ligation reaction one microliter of 6X TOPO Cloning Stop Solution was added then the reaction was placed on ice. Two microliters of the ligation reaction was transformed in One-Shot TOP10 cells (Invitrogen), and placed on ice for 30 minutes. The cells were heat-shocked for 30 seconds at 42C, placed on ice for two minutes, 250 µl of SOC was added, then incubated at 37C with shaking for one hour and then plated onto ampicillin plates supplemented with Xgal and IPTG. Single colonies were screened by restriction digestion for the presence of the insert, and a plasmid DNA from colony 63-4-23 was purified using a Qiagen Endo-Free plasmid purification kit.

The clone containing nGPCR-63 was sequenced directly using an ABI377 fluorescence-based sequencer (Perkin Elmer/Applied Biosystems Division, PE/ABD, Foster City, CA) and the ABI BigDyeTM Terminator Cycle Sequencing Ready Reaction kit with Taq FSTM polymerase. Each ABI cycle sequencing reaction contained about 0.5 µg of plasmid DNA. Cycle-sequencing was performed using an initial denaturation at 98C for 1 minute, followed by 50 cycles: 96C for 30 seconds, annealing at 50C for 30 seconds, and extension at 60C for 4 minutes. Temperature cycles and times were controlled by a Perkin-Elmer 9600 thermocycler. Extension products were purified using AGTC (R) gel filtration block (Edge BiosSystems, Gaithersburg, MD). Each reaction product was loaded by pipette onto the column, which was then centrifuged in a swinging bucket centrifuge (Sorvall model RT6000B tabletop centrifuge) at 1500 x g for 4 minutes at room temperature. Column-purified samples were dried under vacuum for about 40 minutes and then dissolved in 3 µl of a DNA loading solution (83% deionized formamide, 8.3 mM EDTA, and 1.6 mg/ml Blue Dextran). The samples were then heated to 90C for 3.5 minutes and loaded into the gel sample wells for sequence analysis by the ABI377 sequencer. Sequence analysis was performed by importing ABI377 files into the Sequencer program (Gene Codes, Ann Arbor, MI).

### nGPCR-42

PCR was performed in a 50 µl reaction containing 36.7 µl H₂O 5 µl 10X TT buffer (140 mM Ammonium Sulfate, 0.1 % gelatin, 0.6 M Tris-tricine pH 8.4), 5 µl 15 mM MgS0₄, 2 µl 10 mM dNTP, 0.3 µl 6541947H1 DNA (1.4 Tg/Tl), 0.3 µl of LW1579 (1 µg/µl), 0.3 µl of LW1580 (1 µg/µl), 0.4 µl High Fidelity Taq polymerase (Boehringer Mannheim). The PCR reaction was performed on a on a Robocycler thermocycler (Stratagene) starting with 1 cycle of 94C for 2 minutes followed by 15 cycles of 94C for 30 seconds, 55C for 30 seconds, 68C for 1.3 minutes. The PCR reaction was loaded onto a 1.2 % agarose gel. The DNA band was excised from the gel, placed in GenElute Agarose spin column (Supelco) and spun for 10 minutes at maximum speed in a microcentrifuge. The eluted DNA was EtOH precipitated and resuspended in 4µl H₂O for ligation. The forward PCR primer was: LW1579 5'-GCATAAGCTTCCATGTGGAGCTGC AGCTGGTTCAACG-3' (SEQ ID NO:125), and the reverse PCR primer was: LW1580 5'-GCATCTCGAGCCTACGCCAGCACCTGCTGCACC-3' (SEQ ID NO:126). The ligation reaction used solutions from the TOPO TA Cloning Kit (Invitrogen) which consisted of 4µl PCR product DNA and 1 µl pCRII-TOPO vector that was incubated for 5 minutes at room temperature. To the ligation reaction one microliter of 6X TOPO Cloning Stop Solution was added then the reaction was placed on ice. Two microliters of the ligation reaction was transformed in One-Shot TOP10 cells (Invitrogen), and placed on ice for 30 minutes. The cells were heat-shocked for 30 seconds at 42C, placed on ice for two minutes, 250 µl of SOC was added, then incubated at 37C with shaking for one hour and then plated onto ampicillin plates. A single colony containing an insert was used to inoculate a 5 ml culture of LB medium. Plasmid DNA was purified using a Concert Rapid Plasmid Miniprep System (GibcoBRL) and then sequenced.

### nGPCR-46

PCR was performed in a 50 µl reaction using components that come with PLATINUM® *Pfx* DNA Polymerase (GibcoBRL) containing 30.5 µl H₂O, 5 µl 10X Pfx Amplification buffer, 5 µl 10X Enhancer solution, 1.5 µl 50mM MgSO₄,2 µl 10 mM dNTP, 5 µl human genomic DNA (0.3µg/µl)(Clontech), 0.3 µl ofLW1626 (1 µg/µl), 0.3 µl ofLW1627 (1µg/µl), 0.4 µl PLATINIUM® *Pfx*- DNA Polymerase (2.5 U/µl). The PCR reaction was performed in a Robocycler Gradient 96 (Stratagene) starting with 1 cycle of 94C for 5 minutes followed by 30 cycles at 94C for 40 seconds, 55C for 2 minutes, 68C for 3 minutes. Following the final cycle, 0.5 µl of AmpliTaq DNA Polymerase (5 U/µl) was added and the tube was incubated at 72C for 5 minutes. The PCR reaction was loaded onto a 1.2 % agarose gel. The DNA band was excised from the gel, placed in GenElute Agarose spin column (Supelco) and spun for 10 minutes at maximum speed in a microcentrifuge. The eluted DNA was EtOH precipitated and resuspended in 12µl H₂O for ligation. The forward PCR primer was: LW1626 5'-GCATAAGCTTCCATGGGGAACGATTCTGTCAGC-3' (SEQ ID NO:127) and the reverse PCR primer was: LW1627 5'-GCATCTCGAGCCTACACCTCCATCTCCGAGACC-3' (SEQ ID NO:128). The ligation reaction used solutions from the TOPO TA Cloning Kit (Invitrogen) which consisted of 4µl PCR product DNA and 1 µl pCRII-TOPO vector that was incubated for 5 minutes at room temperature. To the ligation reaction one microliter of 6X TOPO Cloning Stop Solution was added then the reaction was placed on ice. Two microliters of the ligation reaction was transformed in One-Shot TOP10 cells (Invitrogen), and placed on ice for 30 minutes. The cells were heat-shocked for 30 seconds at 42C, placed on ice for two minutes, 250 µl of SOC was added, then incubated at 37C with shaking for one hour and then plated onto ampicillin plates. A single colony containing an insert was used to inoculate a 5 ml culture of LB medium. Plasmid DNA was purified using a Concert Rapid Plasmid Miniprep System (GibcoBRL) and then sequenced.

### nGPCR-48

PCR was performed in a 50 µl reaction using components that come with PLATINUM® *Pfx* DNA Polymerase (GibcoBRL) containing 30.5 µl H₂O, 5 µl 10X Pfx Amplification buffer, 5 µl 10X Enhancer solution, 1.5 µl 50mM MgS0₄, 2 µl 10 mM dNTP, 5 µl human genomic DNA (0.3µg /µl)(Clontech), 0.3 µl of LW1572 (1 µg/µl), 0.3 µl of LW1573 (1 µg/µl), 0.4 µl PLATINUM® *Pfx* DNA Polymerase (2.5 U/µl). The PCR reaction was performed in a Robocycler Gradient 96 (Stratagene) starting with 1 cycle of 94C for 5 minutes followed by 30 cycles at 94C for 40 seconds, 55C for 2 minutes, 68C for 3 minutes. Following the final cycle, 0.5 µl of AmpliTaq DNA Polymerase (5 U/Tl) was added and the tube was incubated at 72C. for 5 minutes. The PCR reaction was loaded onto a 1.2 % agarose gel. The DNA band was excised from the gel, placed in GenElute Agarose spin column (Supelco) and spun for 10 minutes at maximum speed in a microcentrifuge. The eluted DNA was EtOH precipitated and resuspended in 12 µl H₂O for ligation. The forward primer for PCR was: LW1572 5'-GATCAAGCTTGCA TGGCACCTTCTCATCGGG-3' (SEQ ID NO:129) and the reverse primer was: LW1573 5'-GATCCTCGAGTCAAAGGTCCCATTCCGGACC-3' (SEQ ID NO:130). The ligation reaction used solutions from the TOPO TA Cloning Kit (Invitrogen) which consisted of 4µl PCR product DNA and 1 µl pCRII-TOPO vector that was incubated for 5 minutes at room temperature. To the ligation reaction one microliter of 6X TOPO Cloning Stop Solution was added then the reaction was placed on ice. Two microliters of the ligation reaction was transformed in One-Shot TOP10 cells (Invitrogen), and placed on ice for 30 minutes. The cells were heat-shocked for 30 seconds at 42C, placed on ice for two minutes, 250 µl of SOC was added, then incubated at 37C with shaking for one hour and then plated onto ampicillin plates. A single colony containing an insert was used to inoculate a 5 ml culture of LB medium. Plasmid DNA was purified using a Concert Rapid Plasmid Miniprep System (GibcoBRL) and then sequenced.

### nGPCR-49

The PCR reaction used components that come with PLATINUM® *Pfx* DNA Polymerase (GibcoBRL) containing 30.5 µl H₂O, 5 µl 10X Pfx Amplification buffer, 5 µl 10X Enhancer Solution, 1.5 µl 50mM MgSO₄, 2 µl 10 mM dNTP, 5 µl human genomic DNA (0.3µg/µl) (Clontech), 0.3 µl of LW1726 (1 µg/µl) (SEQ ID NO:131), 0.3 µl of LW1727 (1 µg/µl) (SEQ ID NO:132), 0.4 µl PLATINUM® *Pfx* DNA Polymerase (2.5 U/µl). The PCR reaction was performed in a Robocycler Gradient 96 (Stratagene) starting with 1 cycle of 94C for 2 minutes followed by 35 cycles at 94C for 30 seconds, 55C for 30 seconds, 68C for 2 minutes. Following the final cycle, 0.5 µl of AmpliTaq DNA Polymerase (5 U/µl) was added and the tube was incubated at 72C for 5 minutes. The sequence of LW1726 is: 5'-CATAAGCTTTGGATGCCA CTCCCTGTGCCCCC-3'(SEQ ID NO:131) and for LW1727 is: 5'-GCATCTCGAGTTATAG GAGACCCATGGGCAGGG-3' (SEQ ID NO:132). The underlined portion of the primer matches the 5' and 3' areas, respectively, of the coding region. The ligation reaction used solutions from the TOPO TA Cloning Kit (Invitrogen) which consisted of 4 µl PCR product DNA, 1 µl of salt solution and 1 µl pCRII-TOPO vector that was incubated for 5 minutes at room temperature then the reaction was placed on ice. Two microliters of the ligation reaction was transformed in One-Shot TOP10 cells (Invitrogen), and placed on ice for 30 minutes. The cells were heat-shocked for 30 seconds at 42C, placed on ice for two minutes, 250 µl of SOC was added, then incubated at 37C with shaking for one hour and then plated onto ampicillin plates. A single colony containing an insert was used to inoculate a 5 ml culture of LB medium. Plasmid DNA was purified using a Concert Rapid Plasmid Miniprep System (GibcoBRL) and then sequenced.

The mutation in SEQ-49 in the plasmid above was repaired using the QuikChange Site-Directed Mutagenesis Kit (Stratagene). The PCR reaction contained 40 µ H₂O, 5 µl 10x Reaction buffer, 1 µl mini-prep DNA, 1 µl LW1741 (125 ng/µl) (SEQ ID NO: 133), 1µl LW1742 (125 ng/µl) (SEQ ID NO:134), 1µl 10 mM dNTP, 1 µl Pfu DNA polymerase. The cycle conditions were 95C for 30 seconds then 14 cycles at 95C for 30 seconds, 55C for 1 minute, 68C for 12 minutes. The tube was placed on ice for 2 minutes, then 1 µl ofDpnI was added and the tube incubated at 37C for one hour. One microliter of the DpnI-treated DNA was transformed into Epicurian coli XL1-Blue supercompetent cells and the entire insert was re-sequenced. The primer sequences are for LW1741: 5'-GGTGCTCTGGAACCTCGGCTTCCGCATCAAGAAGGGCCCC-3' (SEQ ID NO:133) and for LW1742 was: 5'-GGGGCCCTTCTTGATGCGGAAGCCGAGGTTCCAGAGCACC-3' (SEQ ID NO:134).

### nGPCR2-61

The PCR reaction was performed in 50 µl containing 34.5 µl H₂O, 5 µl Buffer II (PE Applied Biosystems AmpliTaq Gold system), 6 µl 25 mM MgCl₂, 2 µl 10 mM dNTP mix, 1.5 µl human genomic DNA (Clontech #6550-1, 0.1 µg/µl), 0.3 µl primer VR61C (1 µg/µl) (SEQ ID NO:135), 0.3 µl primer VR61D (1 µg/µl) (SEQ ID NO:136), and 0.4 µl AmpliTaq Gold™ DNA Polymerase. The primer sequence for VR61C was: 5'-TTCAAAGCTTATGAACAAC AATACAACATGTATTCAAC-3' (SEQ ID NO:135), corresponding to the 5' end of the coding region and containing a HindIII restriction site, and the primer sequence for VR61D was: 5'-TTCACTCGAGTCAAACATATGATTGCATATGTG-3' (SEQ ID NO:136), corresponding to the 3' end of the coding region and containing an XhoI restriction site(Genosys). The PCR reaction was carried out using a GeneAmp PCR9700 thermocycler (Perkin Elmer Applied Biosystems) and started with 1 cycle of 95C for 10 minutes, then 14 cycles at 95C for 30 seconds, 72C for 2 minutes decreasing 1C each cycle, 72C for 1 minute, followed by 30 cycles at 95C for 30 seconds, 60C for 30 seconds, 72C for 1 minute. The PCR reaction was loaded on a 0.75% agarose gel. The DNA band was excised from the gel and the DNA was eluted from the agarose using a QIAquick gel extraction kit (Qiagen). The eluted DNA was ethanol-precipitated and resuspended in 4µl H₂O for ligation. The ligation reaction consisted of 4µl of fresh ethanol-precipitated PCR product and 1 µl of pCRII-TOPO vector (Invitrogen). The reaction was gently mixed and allowed to incubate for 5 minutes at room temperature followed by the addition of 1 µl of 6x TOPO cloning stop solution and mixing for 10 seconds at room temperature. The sample was then placed on ice and 2µl was transformed in 50µl of One Shot cells (Invitrogen) and plated onto ampicillin plates. Four white colonies were chosen and the presence of an insert was verified by PCR in the following manner. Each colony was resuspended in 50 µL H₂O. A 16 µl aliquot was removed and boiled for 5 minutes and the sample was placed on ice for 5 minutes. The sample was microfuged briefly to pellet any bacterial debris and PCR was carried out with 15 µl sample using primers VR61C and VR61D, above.

Colonies from the positive clones identified by PCR were used to inoculate a 4 ml culture of LB medium containing 100 µg/ml ampicillin. Plasmid DNA was purified using the Wizard Plus Minipreps DNA purification system (Promega). Since the primers used to PCR SEQ-61 from genomic DNA were engineered to have HindIII and XhoI sites, the cDNA obtained from the minipreps was digested with these restriction enzymes. Clones were verified as having an insert of the correct size by gel electrophoresis. cDNA from one of the clones was then submitted for sequencing. Two mutations were found (bp 939 T→C and bp1004 G→T). The mutation at bp 939 was found to be a silent mutation and was not repaired. The mutation at bp 1004 was repaired as described below.

The mutation at bp 1004 in SEQ-61 was repaired using the QuikChange Site-Directed Mutagenesis Kit (Stratagene). The PCR reaction contained 38.1 µl H₂O, 5 µl 10x reaction buffer, 50 ng mini-prep cDNA from above, 1.25 µl primer VR61G (100 ng/µl) (SEQ ID NO:137), 1.25 µl primer VR61H (100 ng/µl) (SEQ ID NO:138), 2 µl 10 mM dNTP mix, and 1 µl Pfu DNA polymerase. The primer sequence for VR61G was: 5'-GCACATATGCAATCATA T**G**GTTGACTCGAGTGAAAAGGG-3' (SEQ ID NO:137) and the primer sequence for VR61H was: 5'- CCCTTTTCACTCGAGTCAA**C**CATATGATTGCATATGTGC-3' (SEQ ID NO:138), where the base highlighted and underlined is the one being corrected. The PCR cycle conditions were 95C for 30 seconds, then 12 cycles at 95C for 30 seconds, 55C for 1 minute, 68C for 11 minutes. One µl of DpnI was added and the reaction was incubated at 37C for 1 hour. One µl of the DpnI-treated DNA was transformed into 50 µl Epicurian coli XL1-Blue supercompetent cells and plated onto ampicillin plates. Two colonies were chosen and miniprep DNA was purified as described above. The presence of an insert was verified by restriction digest/gel electrophoresis. DNA from both of the samples was submitted for sequencing. Sequencing information showed that the mutation was repaired in both clones. One of the clones was chosen for use in subsequent work.

The clone described above was sequenced directly using an ABI377 fluorescence-based sequencer (Perkin Elmer/Applied Biosystems Division, PE/ABD, Foster City, CA) and the ABI BigDyeTM Terminator Cycle Sequencing Ready Reaction kit with Taq FSTM polymerase. Each ABI cycle sequencing reaction contained 0.5 µg of plasmid DNA. Cycle-sequencing was performed using an initial denaturation at 98C for 1 minute, followed by 50 cycles: 96C for 30 seconds, annealing at 50C for 30 seconds, and extension at 60C for 4 minutes. Temperature cycles and times were controlled by a Perkin-Elmer 9600 thermocycler. Extension products were purified using AGTC (R) gel filtration block (Edge BiosSystems, Gaithersburg, MD). Each reaction product was loaded by pipette onto the column, which was then centrifuged in a swinging bucket centrifuge (Sorvall model RT6000B tabletop centrifuge) at 1500 x g for 4 minutes at room temperature. Column-purified samples were dried under vacuum for about 40 minutes and then dissolved in 3 µl of a DNA loading solution (83% deionized formamide, 8.3 mM EDTA, and 1.6 mg/ml Blue Dextran). The samples were then heated to 90C for 3.5 minutes and loaded into the gel sample wells for sequence analysis by the ABI377 sequencer. Sequence analysis was performed by importing ABI377 files into the Sequencer program (Gene Codes, Ann Arbor, MI).

### nGPCR-51

The cDNA clone for nGPCR-51 was isolated by Life Technologies, Inc. using their GENETRAPPER® cDNA Positive Selection System (Cat. No. 10356-020). Double-stranded plasmid DNA from the SUPERSCRIPT Human Fetal Brain cDNA Library (Cat. No. 10662-013) was used as a substrate. The library was made with fetal brain cDNA directionally cloned into the *NotI-SalI* site of pCMV-SPORT2. An oligonucleotide probe 5'-CTCCATCTGTCTC AGCTATGCCAGCAGCAG-3' (SEQ ID NO:139) was designed from Celera sequence GA_11007426 and then biotinylated for solution hybridization to single-stranded fetal brain DNA (prepared from total cDNA library) and selection with paramagnetic beads.

### Preparation of dsDNA from a Plasmid cDNA Library

One hundred ml of Terrific Broth containing 100 mg/ml ampicillin was inoculated with cells from an amplified library. The cells were grown to saturation at 30C. The sample was centrifuged at 4,800 g for 15 minutes at 4C. The supernatant was decanted, and the cell pellet was resuspended in a total volume of 10 ml of buffer I with RNase (15 mM Tris-HCI (pH 8.0), 10 mM EDTA, RNase A(100 µg/ml), RNase T1 (1,200 units/ml)). Ten ml of buffer II (0.2 M NaOH, 1% SDS) was added to the resuspended cells, and the sample was inverted to mix and allowed to incubate for 5 minutes at room temperature. Ten ml of cold 7.5 M NH₄OAc was added to the cell mixture, and the sample was inverted to mix and allowed to incubate on ice for 10 minutes. The sample was centrifuged at 3,000 g (4,000 rpm in an HS4 rotor) for 15 minutes at 4C. The supernatant was poured through cheesecloth into a fresh 50-ml, centrifuge tube. An equal volume of cold isopropanol (-20C) was added to the tube, mixed well, and the sample was centrifuged at 3,000 g for 15 minutes at 4C. The supernatant was discarded, and the pellet was resuspended in 1 ml of buffer I with RNase and transferred to a microcentrifuge tube. The solution was clarified by centrifugation at 4C for 1 minute at 14,000 g. The supernatant was transferred to a fresh microcentrifuge tube, and incubated at 37C for 30 minutes, after which the tube was incubated at 65C for 5 minutes. The sample was split into two equal parts (~500 µl each) in 1.5-ml microcentrifuge tubes. An equal volume of phenol: chloroform:isoamyl alcohol (25:24:1) was added to each sample and the tube was vortexed. The sample was centrifuged at room temperature for 5 minutes at 14,000 g. Four hundred fifty µl of the upper aqueous phase was transferred to fresh microcentrifuge tubes. The phenol:chloroform extraction procedure above was repeated 3 times. An equal volume of isopropanol (-20C) was added to each tube. The tube was centrifuged at 4C for15 minutes at 14,000 g, and the supernatant was discarded. Five hundred µl of 70% ethanol was carefully added to each tube. The tubes were centrifuged at 4C for 5 minutes at 14,000 g. The supernatant was discarded, and the pellet was dried at room temperature for 10 minutes. The pellets were resuspended in a total of 200µl TE buffer. *Generation of ssDNA*

Five µg ds phagemid cDNA (1 µg/µl), 2 µl 10X Gene II Buffer, and sufficient autoclaved, distilled water to bring the volume to 19 µl was added to a 1.5 ml microcentrifuge tube at room temperature. To each sample, 1 µl of Gene II (phage F1 endonuclease) was added. The sample was vortexed and centrifuged at room temperature for 2 s at 14,000 g to collect the contents to the bottom of the tube. The tubes were incubated in a 30C water bath for 25 minutes. The mixture was heated at 65C for 5 minutes and immediately chilled on ice for 1 minute. Two µl of Exo III were added, and the tube was vortexed and centrifuged at room temperature for 2 s at 14,000 g. The tubes were incubated at 37C for 60 minutes. An equal volume (20 µl) of phenol:chloroform:isoamyl alcohol (25:24:1) was added to each tube, vortexed thoroughly, and centrifuged at room temperature for 5 minutes at 14,000 g to separate the phases. Eighteen µl of the upper aqueous phase was transferred to a fresh 1.5-ml microcentrifuge tube.

### Biotinylation Reaction

The following components were added to a 1.5 ml microcentrifuge tube: 5 µl 5X TdT Buffer; 3 µg oligonucleotide (SEQ ID NO:139),5 µl Biotin-14-dCTP, sufficient autoclaved, distilled water to bring the volume to 23 µl, and 2 µl TdT (Terminal Deoxynucleotidyl Transferase). Each tube is vortexed gently and centrifuged for 2 s at 14,000 g. The tubes were incubated for 1 hour at 30C. After 1 hour, 1 µl of Glycogen (20 µg/µl), 26 µl of 1 M Tris-HCl (pH 7.5), and 120 µl of ethanol were added to the oligonucleotide biotinylation reaction. Each sample was vortexed and stored on dry ice for 10 minutes. Each tube was centrifuged at 4C for 30 minutes at 14,000 g. The supernatant was removed from the microcentrifuge tubes, and 200 µl of 70% ethanol (―20C) was layered over the pellet. Each tube was centrifuged at 4C for 2 minutes at 14,000 g. The ethanol wash was removed from the microcentrifuge tubes, and the ethanol wash was repeated once. The pellets were dried at room temperature for 10 minutes or until completely dry. The biotinylated oligonucleotide was resuspended in 20 µl TE buffer.

### Oligonucleotide Hybridization

The biotinylated oligonucleotide was diluted to 20 ng/µl in TE buffer. The 4X Hybridization Buffer was incubated for 2 minutes at 37C, mixed well, and 6 µl was added to the remaining 17 µl of Gene II/Exo III-treated DNA. The DNA was denatured in a 95C water bath for 1 minutes, and then chilled immediately on ice for 1 minute. One µl of diluted biotinylated oligonucleotide (20 ng) was added to the denatured DNA. The tube was incubated at 37C in a water bath or incubator for 1 hour.

### Streptavidin Paramagnetic Bead Preparation

The beads were gently mixed by pipetting until the beads at the bottom of the tube were completely resuspended. For each reaction, 45 µl of the mixed beads were transferred to the bottom of a microcentrifuge tube. The tubes were placed in the magnet and allowed to sit for 2 minutes. With the tubes still in the magnet, the supernatant was removed by pipetting. One hundred µl of TE buffer was immediately added to the beads. The tubes were removed from the magnet and the beads were resuspended by finger-tapping or vortexing at the lowest setting. The tubes were reinserted into the magnet, and after 2 min, the supernatant was removed. The beads were resuspended in 30 µl of TE buffer.

### cDNA Capture

The hybridization mixture was removed from the 37C water bath and centrifuged at room temperature for 2 s at 14,000 g. The prepared paramagnetic beads were pipetted into the mixture and gently mixed by pipetting. The suspension was incubated for 30 minutes at room temperature. The suspension was gently mixed frequently to resuspend the beads.The tubes were inserted into the magnet, and after 2 min, the supernatant was removed and discarded. One hundred µl of Wash Buffer was then added to the beads. The beads were resuspended and the tubes reinserted into the magnet for 2 minutes. The supernatant was removed and discarded. The washing step was repeated one more time. One hundred µl of Wash Buffer was added to the beads. The beads were gently resuspended by pipetting up and down (not by vortexing) and the solution was transferred to a new tube. The tubes were inserted into the magnet for 5 minutes. The supernatant was removed from the paramagnetic beads and discarded. One hundred µl of Wash Buffer was immediately added and the tubes mixed by finger tapping or gently vortexing. The tubes were placed into the magnet for 5 minutes. For each elution, 14 µl of TE buffer (pH 8.0) was mixed with 7 µl of the 3X Elution Buffer. After the 5 minute incubation, the supernatant was removed and discarded from the paramagnetic beads; 20 µl of 1X elution buffer was added to the beads, and mixed well. The beads were incubated for 5 minutes at room temperature. During the incubation, the beads were mixed for 10 seconds every minute. The tube was inserted into the magnet and allowed to sit for 5 minutes. The supernatant (containing the captured cDNA clone) was collected and transferred to a fresh tube, and the beads were resuspended in 15 µl of TE buffer. The tube was inserted into the magnet and allowed to sit for 5 minutes. The supernatant was transferred from the tube and combined with the previous supernatant. The tube containing the combined supernatants was inserted into the magnet for 10 minutes to remove any remaining paramagnetic beads and the supernatant was transferred to a fresh microcentrifuge tube. To the supernatant (~35 µl), 1 µl of Glycogen, 18 µl of 7.5 M NH ₄OAc, and 135 µl of ethanol (-20C) were added. The tube was mixed well and stored on ice for 10 minutes. The tube was centrifuged at 4C for 30 minutes at 14,000 g. The supernatant was removed from the small pellets. One hundred µl of 70% ethanol was added to each tube. The tubes were centrifuged at room temperature for 2 minutes at 14,000 g. The ethanol was removed and the pellets dried at room temperature until dry. The pellets were resuspended in 5 µl of TE buffer and stored at 4C.

### Repair of Captured cDNA: Repair Reaction

The thermal cycler is programmed for one cycle as described below: 90C denature step for 1 minute; 55C annealing step for 30 seconds; 70C extension step for 15 minutes. A DNA primer/repair mix was prepared for each capture reaction by adding the following to the captured cDNA: captured DNA (5 µl), autoclaved, distilled water (11 µl), 50 ng oligonucleotide (SEQ ID NO:139; not biotinylated)( 1 µl), 10 mM dNTP Mix (0.5 µl), 10X Repair Buffer (2 µl), Repair Enzyme (0.5 µl). The tubes were mixed and centrifuged at room temperature for 2 s at 14,000 g. The DNA primer/repair mix was incubated at 90C for 1 minute. The mix was transferred to 55C and incubated for 30 seconds. The mix was transferred to 70C and incubated for 15 minutes to allow primer extension. The mix was centrifuged at room temperature for 2 s at 14,000 g. The repaired DNA was precipitated by adding 1 µl Glycogen, 11 µl 7.5 M ammonium acetate, and 90 µl of-20C ethanol to each tube. The tubes were vortexed and placed in ice for 10 minutes. The tubes were centrifuged at 4C for 30 minutes at 14,000 g. The ethanol was removed from the small pellet and 100 µl of 70% ethanol (-20C) was layered on the pellet. The tubes were centrifuged at 4C for 2 minutes at 14,000 g. The ethanol was removed and the pellets dried at room temperature until dry. The pellets were dissolved in 10 µl of TE buffer.

### Transformation with ULTRAMAX DH5a a-FT Cells

Competent cells were removed from -70C freezer and thawed on wet ice. The required number of 17 x 100 polypropylene tubes (Falcon 2059) were placed on ice. Immediately after thawing, the cells were gently mixed, then 100 µl of cells were aliquotted into chilled polypropylene tubes. Three µl of the DNA were mixed into each individual tube of cells. The cells were incubated on ice for 30 minutes. Cells were heat-shocked for 45 seconds in a 42C water bath, and then placed on ice for 2 minutes. To each tube, 0.9 ml of room temperature S.O.C medium was added. The tubes were shaken at 225 rpm (37C) for 1 hour. For captured or repaired cDNA samples, 100 µl and 200 µl aliquots were plated onto LB plates containing 100 µg/ml ampicillin. The plates were incubated overnight in a 37C incubator.

### Identification ofDesired cDNA Clones by Colony PCR

A PCR master mix was prepared with the following components: 22.5 µl/clone of 1X PCR SUPERMIX, forward and reverse primers at a final concentration of 200 nM/primer, and autoclaved, distilled water to a final volume of 25 µl. Using a micropipette tip or sterile toothpick, each colony was picked and placed into an individual tube containing master mix. The PCR reaction was as follows: 1 cycle at 94C, 1 minute, then 30 cycles of 94C, 30 seconds; 55C, 30 seconds; 72C, 1 minute. The PCR primer pairs used in colony screening were, forward primer: 5'-CCATCTGTCTCAGCTATGCC-3' (SEQ ID NO:140), and the reverse primer: 5'-TCCTTCTCAGTCGCTCTTC-3' (SEQ ID NO:141).

### nGPCR-52

Two microliters of a human genomic library (~10⁸ PFU/ml) (Clontech) were added to 6 ml of an overnight culture ofK802 cells (Clontech), then distributed as 250 µl aliquots into each of 24 tubes. The tubes were incubated at 37C for 15 minutes. Seven milliliters of 0.8% agarose was added to each tube, mixed, then poured onto LB agar +10 mM MgSO₄ plates and incubated overnight at 37C. To each plate 5 ml of SM (0.1M NaCl, 8.1 TM MgSO₄-7H₂O, 50mM Tris-Cl (pH 7.5), 0.0001% gelatin) phage buffer was added and the top agarose was removed with a microscope slide and placed in a 50 ml centrifuge tube. A drop of chloroform was added and the tube was place in a 37C shaker for 15 minutes, then centrifuged for 20 minutes at 4000 RPM (Sorvall RT6000 table top centrifuge) and the supernatant stored at 4C as a stock solution.

The PCR reaction was performed in 20 µl containing 8.8 µl H₂O, 4 Tl 5X Rapid-Load Buffer (Origene), 2 µl 10xPCR buffer II (Perkin-Elmer), 2 µl 25 mM MgCl₂, 0.8 µl 10 mM dNTP, 0.12 µl LW1632 (SEQ ID NO:142) (1 µg/µl), 0.12 µl LW1633 (SEQ ID NO:143) (1 µg/µl), 0.2 µl AmpliTaq Gold polymerase (Perkin Elmer) and 2 µl of phage from each of the 24 tubes. The PCR reaction involved 1 cycle at 80C for 20 minutes, 95C for 10 minutes, then 22 cycles at 95C for 30 seconds, 72C for 4 minutes decreasing 1C each cycle, 68C for 2 minutes, followed by 30 cycles at 95C for 30 seconds, 55C for 30 seconds, 68C for 60 seconds. The reaction was loaded onto a 2 % agarose gel. From the tube that gave a PCR product of the correct size, 5 µl was used to set up five 1:10 dilutions that were plated onto LB agar + 10 mM MgSO₄ plates and incubated overnight. A BA85 nitrocellulose filter (Schleicher & Schuell) was placed on top of each plate for 1 hour. The filter was removed, placed phage side up in a petri dish, and covered with 4 ml of SM for 15 minutes to elute the phage. One milliliter of SM was removed from each plate and used to set up a PCR reaction as above. The plate of the lowest dilution to give a PCR product was subdivided, filter-lifted and the PCR reaction was repeated. The series of dilutions and subdividing of the plate was continued until a single plaque was isolated that gave a positive PCR band. Once a single plaque was isolated, 10 µl phage supernatant was added to 100 µl SM and 200 µl of K802 cells per plate with a total of 8 plates set up. The plates were incubated overnight at 37C. The top agarose was removed by adding 8 ml of SM, then scrapping off the agarose with a microscope slide and collected in a centrifuge tube. To the tube, 3 drops of chloroform was added, vortexed, incubated at 37C for 15 minutes then centrifuged for 20 minutes at 4000 RPM (Sorvall RT6000 table top centrifuge) to recover the phage, which was used to isolate genomic phage DNA using the Qiagen Lambda Midi Kit. The sequence for primer LW1632 was 5'-CCTCCACTTGTGCTTCATC-3' (SEQ ID NO:142) and for LW1633: 5'-AAAATCTATCAACACCCAGCC-3' (SEQ ID NO:143).

For subcloning the coding region of nGPCR-52, PCR was performed in a 50 µl reaction containing 33 µl H₂O, 5 µl 10X TT buffer (140 mM Ammonium Sulfate, 0.1 % gelatin, 0.6 M Tris-tricine pH 8.4), 5 µl 15 mM MgS0₄, 2 µl 10 mM dNTP, 4 µl genomic phage DNA (0.1 µg/µl), 0.3 µl LW1643 (SEQ ID NO:144) (1µg/µl), 0.3 µl LW1644 (SEQ ID NO:145) (1 µg/µl), 0.4 µl High Fidelity Taq polymerase (Boehringer Mannheim). The PCR reaction was started with 1 cycle of 94C for 2 minutes followed by 15 cycles at 94C for 30 seconds, 55C for 60 seconds, and 68C for 2 minutes. The PCR reaction was loaded onto a 2 % agarose gel. The DNA band was excised from the gel, placed in GenElute Agarose spin column (Supelco) and spun for 10 minutes at maximum speed. The eluted DNA was EtOH precipitated and resuspended in 12µl H₂O for ligation. The PCR primer sequence for LW1643: 5'-GA TCAAGCTTACCATGACCAGCAATTTTTCCC-3' (SEQ ID NO:144) and for LW1644 was: 5'-GATCCTCGAGCTTATTCTAAAAATAAACTAATGG-3' (SEQ ID NO:145). The ligation reaction used solutions from the TOPO TA Cloning Kit (Invitrogen) which consisted of 4µl PCR product DNA and 1 µl pCRII-TOPO vector that was incubated for 5 minutes at room temperature. To the ligation reaction one microliter of 6X TOPO Cloning Stop Solution was added then the reaction was placed on ice. Two microliters of the ligation reaction was transformed in One-Shot TOP10 cells (Invitrogen), and placed on ice for 30 minutes. The cells were heat-shocked for 30 seconds at 42C, placed on ice for two minutes, 250 µl of SOC was added, then incubated at 37C with shaking for one hour and then plated onto ampicillin plates. A single colony containing an insert was used to inoculate a 5 ml culture of LB medium. Plasmid DNA was purified using a Concert Rapid Plasmid Miniprep System (GibcoBRL) and then sequenced.

nGPCR-52 genomic phage DNA was sequenced using the ABI PRISM™ 310 Genetic Analyzer (PE Applied Biosystems) which uses advanced capillary electrophoresis technology and the ABI PRISM™ BigDye™ Terminator Cycle Sequencing Ready Reaction Kit. The cycle-sequencing reaction contained 14 µl of H₂O, 16 µl of BigDye Terminator mix, 7 µl genomic phage DNA (0.1 µg/µl), and 3 µl primer (25 ng/µl). The reaction was performed in a Perkin-Elmer 9600 thermocycler at 95C for 5 minutes, followed by 99 cycles of 95C for 30 seconds, 55C for 20 seconds, and 60C for 4 minutes. The product was purified using a Centriflex™gel filtration cartridges, dried under vacuum, then dissolved in 16 µl of Template Suppression Reagent. The samples were heated at 95C for 5 minutes then placed in the 310 Genetic Analyzer

### Example 3: Subcloning of the Coding Region of nGPCR-x Via PCR

Additional experiments may be conducted to subclone the coding region of nGPCR and place the isolated coding region into a useful vector. Two additional PCR primers are designed based on the coding region of nGPCR, corresponding to either end. To protect against exonucleolytic attack during subsequent exposure to enzymes, e.g., Taq polymerase, primers are routinely synthesized with a protective run of nucleotides at the 5' end that were not necessarily complementary to the desired target.

PCR is performed in a 50 µl reaction containing 34 µl H₂O, 5 µl 10X TT buffer (140 mM ammonium sulfate, 0.1 % gelatin, 0.6 M Tris-tricine, pH 8.4), 5 µl 15 mM MgSO₄, 2 µl dNTP mixture (dGTP, dATP, dTTP, and dCTP, each at 10 mM), 3 µl genomic phage DNA (0.25 µg/µl), 0.3 µl Primer 1 (1 µg/µl), 0.3 µl Primer 2 (1 µg/µl), 0.4 µl High Fidelity Taq polymerase (Boehringer Mannheim). The PCR reaction was started with 1 cycle of 94C for 2 minutes; followed by 25 cycles at 94C for 30 seconds, 55C for 30 seconds, and 72C for 1.3 minutes.

The contents from the PCR reaction are loaded onto a 2% agarose gel and fractionated. The DNA band of expected size is excised from the gel, placed in a GenElute Agarose spin column (Supelco) and spun for 10 minutes at maximum speed in a microfuge. The eluted DNA is precipitated with ethanol and resuspended in 6 µl H₂O for ligation.

The PCR-amplified DNA fragment containing the coding region is cloned into pCR2.1 using a protocol standard in the art. In particular, the ligation reaction consists of 6 µl of GPCR DNA, 1 µl 10X ligation buffer, 2 µl pCR2.1 (25 ng/µl, Invitrogen), and 1 µl T4 DNA ligase (Invitrogen). The reaction mixture is incubated overnight at 14C and the reaction is then stopped by heating at 65C for 10 minutes. Two microliters of the ligation reaction are transformed into One Shot cells (Invitrogen) and plated onto ampicillin plates. A single colony containing a recombinant pCR2.1 bearing an insert is used to inoculate a 5 ml culture of LB medium. Plasmid DNA is purified using the Concert Rapid Plasmid Miniprep System (GibcoBRL) and sequenced. Following confirmation of the sequence, a 50 ml culture of LB medium is inoculated with the transformed One Shot cells, cultured, and processed using a Qiagen Plasmid Midi Kit to yield purified pCR-GPCR.

### nGPCR-70

Colonies from the positive clones identified by PCR were used to inoculate a 4 ml culture of LB medium containing 100 µg/ml ampicillin. Plasmid DNA was purified using the Wizard Plus Minipreps DNA purification system (Promega). Since the primers used to PCR SEQ-70 from human brain cDNA were engineered to have HindIII and XhoI sites, the DNA obtained from the minipreps was digested with these restriction enzymes. Five of the clones were verified by gel electrophoresis to give a DNA band of the correct size. cDNA from each of these clones was then submitted for sequencing. Each of the clones was found to have two or more mutations. The clone containing the fewest mutations (clone #7-2 mutations at bp 561 G→A and bp 1093 G→A) was repaired as described as below.

The mutations in SEQ-70 were repaired sequentially using the QuikChange Site-Directed Mutagenesis Kit (Stratagene). The mutation at bp 561 was repaired first and the primer sequences used were: VR70G 5'-CATGATCTGGGG**G**GCCAGCCCCAGC-3' (SEQ ID NO:146) and VR70H 5'-GCTGGGGCTGGC**C**CCCCAGATCATG-3' (SEQ ID NO:147) where the base highlighted and underlined is the one being corrected. The PCR reaction contained 38.4 µl H₂O, 5 µl 10x reaction buffer, 50 ng mini-prep cDNA from clone #7,2 µl 10 mM dNTP mix, and 1 µl Pfu DNA polymerase. The cycle conditions were 95C for 30 seconds, then 12 cycles at 95C for 30 seconds, 55C for 1 minute, 68C for 11 minutes. One µl of DpnI was added and the reaction was incubated at 37C for 1 hour. One µl of the DpnI-treated DNA was transformed into 50 µl Epicurian coli XL1-Blue supercompetent cells and plated onto ampicillin plates. Four colonies were chosen and miniprep DNA was purified as described above. DNA from one of the preps was used as a template to repair the second mutation at bp 1093. The primer sequences used were: VR701 5'-GACATCAATTTCAGTGAG**G**ATGACGTCGAGGC AG-3' (SEQ ID NO:148) and VR70J 5'-CTGCCTCGACGTCAT**C**CTCACTGAAATTGATG TC-3' (SEQ ID NO:149), where the base highlighted and underlined is the one being corrected. The PCR and transformation reactions were carried out as described above. Mini-prep DNA was prepared from three colonies, and the presence of an insert was verified by restriction digest/gel electrophoresis. DNA from one of the samples was submitted for sequencing. Sequencing information showed that both mutations were repaired but that an additional mutation at bp 560 (G→A) had been introduced. This mutation was adjacent to the original mutation at bp561 and was encompassed in the sequence of the primer that was used. Since it could not be ruled out that there was an error in the synthesis of the primers, the same exact sequence corresponding to primers VR70G (SEQ ID NO:146) and VR70H (SEQ ID NO:147) was reordered and repair of the mutation with the new primers was carried out as above. DNA from 2 clones was submitted for sequencing. One of the clones had no mutations and was used for subsequent work.

The clone described above was sequenced directly using an ABI377 fluorescence-based sequencer (Perkin Elmer/Applied Biosystems Division, PE/ABD, Foster City, CA) and the ABI BigDyeTM Terminator Cycle Sequencing Ready Reaction kit with Taq FSTM polymerase. Each ABI cycle sequencing reaction contained 0.5 µg of plasmid DNA. Cycle-sequencing was performed using an initial denaturation at 98C for 1 minute, followed by 50 cycles: 96C for 30 seconds, annealing at 50C for 30 seconds, and extension at 60C for 4 minutes. Temperature cycles and times were controlled by a Perkin-Ehner 9600 thermocycler. Extension products were purified using AGTC (R) gel filtration block (Edge BiosSystems, Gaithersburg, MD). Each reaction product was loaded by pipette onto the column, which was then centrifuged in a swinging bucket centrifuge (Sorvall model RT6000B tabletop centrifuge) at 1500 x g for 4 minutes at room temperature. Column-purified samples were dried under vacuum for about 40 minutres and then dissolved in 3 µl of a DNA loading solution (83% deionized formamide, 8.3 mM EDTA, and 1.6 mg/ml Blue Dextran). The samples were then heated to 90C for 3.5 minutes and loaded into the gel sample wells for sequence analysis by the ABI377 sequencer. Sequence analysis was performed by importing ABI377 files into the Sequencer program (Gene Codes, Ann Arbor, MI).

### nGPCR-42

The DNA subcloned into pCRII was sequenced using the ABI PRISM™ 310 Genetic Analyzer (PE Applied Biosystems) which uses advanced capillary electrophoresis technology and the ABI PRISM™ BigDye™ Terminator Cycle Sequencing Ready Reaction Kit. Each cycle-sequencing reaction contained 6 µl of H₂O, 8 µl of BigDye Terminator mix, 5 µl mini-prep DNA (0.1 µg/gl), and 1 µl primer (25 ng/µl) and was performed in a Perkin-Elmer 9600 thermocycler with 25 cycles of 96C for 10 seconds, 50C for 10 seconds, and 60C for 4 minutes. The product was purified using a Centriflex™ gel filtration cartridge, dried under vacuum, then dissolved in 16 µl of Template Suppression Reagent (PE Applied Biosystems). The samples were heated at 95C for 5 minutes then placed in the 310 Genetic Analyzer.

### nGPCR-46

The DNA subcloned into pCRII was sequenced using the ABI PRISM™ 310 Genetic Analyzer (PE Applied Biosystems) which uses advanced capillary electrophoresis technology and the ABI PRISM™ BigDye™ Terminator Cycle Sequencing Ready Reaction Kit. Each cycle-sequencing reaction contained 6 µl of H₂O, 8 µl of BigDye Terminator mix, 5 µl mini-prep DNA (0.1 µg/µl), and 1 µl primer (25 ng/µl) and was performed in a Perkin-Elmer 9600 thermocycler with 25 cycles of 96C for 10 seconds, 50C for 10 seconds, and 60C for 4 minutes. The product was purified using a Centriflex™ gel filtration cartridge, dried under vacuum, then dissolved in 16 µl of Template Suppression Reagent (PE Applied Biosystems). The samples were heated at 95C for 5 minutes then placed in the 310 Genetic Analyzer.

The mutation found in the clone was repaired using the QuikChange Site-Directed Mutagenesis Kit (Stratagene). The PCR reaction contained 39 µl H₂O, 5 µl 10xReaction buffer, 1 µl mini-prep DNA (150 ng/µl), 2µl 10 mM dNTP, 1 µl Pfu DNA polymerase, 1 µl LW1638 (SEQ ID NO:150) (125 ng/µl), and 1 µl LW1639 (SEQ ID NO:151) (125 ng/µl). The cycle conditions were 95C for 30 seconds then 12 cycles at 95C for 30 seconds, 55C for 1 minute, 68C for 12 minutes. The tube was placed on ice for 2 minutes, then 1 µl ofDpnI was added and the tube incubated at 37C for one hour. Two microliters of the *DpnI*-treated DNA was transformed into Epicurian coli XL1-Blue supercompetent cells and the entire insert was re-sequenced. The forward PCR primer sequence was: LW1638 5'-CGAACTCCGCACTCCTGG CCAGGGCCCTGCGGGC-3' (SEQ ID NO:150) and the reverse PCR primer sequence was: LW1639 5'-GCCCGCAGGGCCCTGGCCAGGAGTGCGGAGTTC-3' (SEQ ID NO:151).

### nGPCR-48

The DNA subcloned into pCRII was sequenced using the ABI PRISM™ 310 Genetic Analyzer (PE Applied Biosystems) which uses advanced capillary electrophoresis technology and the ABI PRISM™ BigDye™ Terminator Cycle Sequencing Ready Reaction Kit. Each cycle-sequencing reaction contained 6 µl of H₂O, 8 µl of BigDye Terminator mix, 5 µl mini-prep DNA (0.1 µg/µl), and 1 µl primer (25 ng/µl) and was performed in a Perkin-Elmer 9600 thermocycler with 25 cycles of 96C for 10 seconds, 50C for 10 seconds, and 60C for 4 minutes. The product was purified using a Centriflex™ gel filtration cartridge, dried under vacuum, then dissolved in 16 µl of Template Suppression Reagent (PE Applied Biosystems). The samples were heated at 95C for 5 minutes then placed in the 310 Genetic Analyzer.

### nGPCR-49

The DNA subcloned into pCRII was sequenced using the ABI PRISM™ 310 Genetic Analyzer (PE Applied Biosystems) which uses advanced capillary electrophoresis technology and the ABI PRISM™ BigDye™ Terminator Cycle Sequencing Ready Reaction Kit. Each cycle-sequencing reaction contained 6 µl of H₂O, 8 µl of BigDye Terminator mix, 5 µl mini-prep DNA (0.1 µg/µl), and 1 µl primer (25 ng/µl) (SEQ ID NO:131 and SEQ ID NO:132) and was performed in a Perkin-Elmer 9600 thermocycler with 25 cycles of 96C for 10 seconds, 50C for 10 seconds, and 60C for 4 minutes. The product was purified using a Centriflex™ gel filtration cartridge, dried under vacuum, then dissolved in 16 µl of Template Suppression Reagent (PE Applied Biosystems). The samples were heated at 95C for 5 minutes then placed in the 310 Genetic Analyzer.

### nGPCR-52

The DNA subcloned into pCRII was sequenced using the ABI PRISM™ 310 Genetic Analyzer (PE Applied Biosystems) which uses advanced capillary electrophoresis technology and the ABI PRISM™ BigDye™ Terminator Cycle Sequencing Ready Reaction Kit. Each cycle-sequencing reaction contained 6 µl of H₂O, 8 µl of BigDye Terminator mix, 5 µl mini-prep DNA (0.1 µg/µl), and 1 µl primer (25 ng/µl) and was performed in a Perkin-Elmer 9600 thermocycler with 25 cycles of 96C for 10 seconds, 50C for 10 seconds, and 60C for 4 minutes. The product was purified using a Centriflex™ gel filtration cartridge, dried under vacuum, then dissolved in 16 µl of Template Suppression Reagent (PE Applied Biosystems). The samples were heated at 95C for 5 minutes then placed in the 310 Genetic Analyzer.

### Example 4: Hybridization Analysis to Demonstrate nGPCR-X Expression in Brain

The expression of nGPCR-x in mammals, such as the rat, may be investigated by *in situ* hybridization histochemistry. To investigate expression in the brain, for example, coronal and sagittal rat brain cryosections (20 µm thick) are prepared using a Reichert-Jung cryostat. Individual sections are thaw-mounted onto silanized, nuclease-free slides (CEL Associates, Inc., Houston, TX), and stored at -80C. Sections are processed starting with post-fixation in cold 4% paraformaldehyde, rinsed in cold phosphate-buffered saline (PBS), acetylated using acetic anhydride in triethanolamine buffer, and dehydrated through a series of alcohol washes in 70%, 95%, and 100% alcohol at room temperature. Subsequently, sections are delipidated in chloroform, followed by rehydration through successive exposure to 100% and 95% alcohol at room temperature. Microscope slides containing processed cryosections are allowed to air dry prior to hybridization. Other tissues may be assayed in a similar fashion.

A nGPCR-x-specific probe is generated using PCR. Following PCR amplification, the fragment is digested with restriction enzymes and cloned into pBluescript II cleaved with the same enzymes. For production of a probe specific for the sense strand of nGPCR-x, the nGPCR-x clone in pBluescript II is linearized with a suitable restriction enzyme, which provides a substrate for labeled run-off transcripts (*i.e*., cRNA riboprobes) using the vector-borne T7 promoter and commercially available T7 RNA polymerase. A probe specific for the antisense strand of nGPCR-x is also readily prepared using the nGPCR-x clone in pBluescript II by cleaving the recombinant plasmid with a suitable restriction enzyme to generate a linearized substrate for the production of labeled run-off cRNA transcripts using the T3 promoter and cognate polymerase. The riboprobes are labeled with [³⁵S]-UTP to yield a specific activity of about 0.40 x 10⁶ cpm/pmol for antisense riboprobes and about 0.65 x 10⁶ cpm/pmol for sense-strand riboprobes. Each riboprobe is subsequently denatured and added (2 pmol/ml) to hybridization buffer which contained 50% formamide, 10% dextran, 0.3 M NaCl, 10 mM Tris (pH 8.0), 1 mM EDTA, 1X Denhardt's Solution, and 10 mM dithiothreitol. Microscope slides containing sequential brain cryosections are independently exposed to 45 µl of hybridization solution per slide and silanized cover slips are placed over the sections being exposed to hybridization solution. Sections are incubated overnight (15-18 hours) at 52C to allow hybridization to occur. Equivalent series of cryosections are exposed to sense or antisense nGPCR-x-specific cRNA riboprobes.

Following the hybridization period, coverslips are washed off the slides in 1X SSC, followed by RNase A treatment involving the exposure of slides to 20 µg/ml RNase A in a buffer containing 10 mM Tris-HCl (pH 7.4), 0.5 M EDTA, and 0.5 M NaCl for 45 minutes at 37C. The cryosections are then subjected to three high-stringency washes in 0.1 X SSC at 52C for 20 minutes each. Following the series of washes, cryosections are dehydrated by consecutive exposure to 70%, 95%, and 100% ammonium acetate in alcohol, followed by air drying and exposure to Kodak BioMax™ MR-1 film. After 13 days of exposure, the film is developed. Based on these results, slides containing tissue that hybridized, as shown by film autoradiograms, are coated with Kodak NTB-2 nuclear track emulsion and the slides are stored in the dark for 32 days. The slides are then developed and counterstained with hematoxylin. Emulsion-coated sections are analyzed microscopically to determine the specificity of labeling. The signal is determined to be specific if autoradiographic grains (generated by antisense probe hybridization) are clearly associated with cresyl violate-stained cell bodies. Autoradiographic grains found between cell bodies indicates non-specific binding of the probe.

Expression of nGPCR-x in the brain provides an indication that modulators of nGPCR-x activity have utility for treating neurological disorders, including but not limited to, mental disorder, affective disorders, ADHD/ADD (*i.e*., Attention Deficit-Hyperactivity Disorder/Attention Deficit Disorder), and neural disorders such as Alzheimer's disease, Parkinson's disease, migraine, and senile dementia. Some other diseases for which modulators of nGPCR-x may have utility include depression, anxiety, bipolar disease, epilepsy, neuritis, neurasthenia, neuropathy, neuroses, and the like. Use of nGPCR-x modulators, including nGPCR-x ligands and anti-nGPCR-x antibodies, to treat individuals having such disease states is intended as an aspect of the invention.

### In Situ Hybridization of SEQ-51

Coronal and sagittal oriented rat brain sections were cryosectioned (20 um thick) using a Leica CM3050 cryostat. The individual sections were thaw-mounted onto silanated, nuclease-free slides (CEL Associates, Inc., Houston, TX), and stored at -80C. The sections were processed starting with post-fixation in cold 4% paraformaldehyde, rinsed in cold PBS, acetylated using acetic anhydride in triethanolamine buffer and dehydrated through 70%, 95%, and 100% alcohols at room temperature (RT). This was followed with delipidation in chloroform then rehydration in 100% and 95% alcohol at RT. Sections were allowed to air dry prior to hybridization. For nGPCR-51, a 250 bp PCR fragment (spanning nt 83 to 332) was generated that contained T7 polymerase on either the 5' end (sense) with primers LW1743 5'-GCGTAATACGACTCACTATAGGGAGACCTGCCAGTGTGGTAGATACAG-3' (SEQ ID NO:152) and LW1746 5'- GGATGTGATGATGGTGCAG-3' (SEQ ID NO:153) or the 3' end (antisense) with primers LW1744 5'-GCGTAATACGACTCACTATAGGGAGACCGGATG TGATGATGGTGCAG-3' (SEQ ID NO:154) and LW1745 5'-TGCCAGTGTGGTAGATAC AG-3' (SEQ ID NO:155). nGPCR-51 was labeled with ³⁵S-UTP to yield a specific activity of 0.655 x 10⁶ cpm/pmol for antisense and 0.675 x10⁶ cpm/pmol for sense probe. Both riboprobes were denatured and added to hybridization buffer which contained 50% formamide, 10% dextran, 0.3M NaCl, 10 mM Tris, 1 mM EDTA, 1X Denhardts, and 10 mM DTT. Sequential brain cryosections were hybridized with 45 µl/slide of the sense and antisense riboprobe hybridization mixture then coverslipped with silanized glass coverslips. The sections were hybridized overnight (15-18 hours) at 50C in an incubator.

Coverslips were washed off the slides in 1X SSC, followed by RNase A treatment, and high temperature stringency washes (3X, 20 minutes at 49C) in 0.1X SSC. Slides were dehydrated with 70%, 95% and 100% NH₄OAc alcohols, air dried and exposed to Kodak BioMax MR-1 film. After 8 days of exposure, the film was developed. This was followed with coating selected tissue slides with Kodak NTB-2 nuclear track emulsion and storing the slides in the dark for 16 days. The slides were then developed and counterstained with hematoxylin. Emulsion-coated sections were analyzed microscopically to determine the specificity of labeling. Presence of autoradiographic grains (generated by antisense probe hybridization) over cell bodies (versus between cell bodies) was used as an index of specific hybridization.

Sense and antisense ³⁵S-labeled RNA probes were generated using a 250-bp fragment of nGPCR-51 for *in situ* hybridization histochemistry. Specific labeling with the antisense probe showed wide spread distribution of nGPCR-51 mRNA. Localization appears in the piriform ctx, habenula, bed nucleus of stria terminalis, islands of Calleja, olfactory tubercle, hippocampus, hypothalamus, PVN, red nucleus, interpeduncular nucleus, dorsal raphe, substantia nigra pars compacta, and reticular thalamus. Expression of nGPCR-51 in these brain regions provided an indication that modulators of nGPCR-51 activity have utility for treating disorders, including but not limited to, schizophrenia, major depression, bipolar disease, anxiety disorder, Parkinson's disease, endocrine disorders, Alzheimer's disease and the like.

### In Situ Hybridization SEQ-52

Coronal and sagittal oriented rat brain sections were cryosectioned (20 um thick) using a Leica CM3050 cryostat. The individual sections were thaw-mounted onto silanated, nuclease-free slides (CEL Associates, Inc., Houston, TX), and stored at -80C. The sections were processed starting with post-fixation in cold 4% paraformaldehyde, rinsed in cold PBS, acetylated using acetic anhydride in triethanolamine buffer and dehydrated through 70%, 95%, and 100% alcohols at room temperature. This was followed with delipidation in chloroform then rehydration in 100% and 95% alcohol at room temperature. Sections were allowed to air dry prior to hybridization. For nGPCR-52, a 292 bp PCR fragment (spanning nt 2920 to 3211) was generated that contained T7 polymerase on either the 5' end (sense) with primers LW1682 5'-GCGTAATACGACTCACTATAGGGAGACCACCAGCAATTTTTCCCAACC-3' (SEQ ID NO:156) and LW1685 5'-AATACCAGCAGCTCTCCAC-3' (SEQ ID NO:157) or the 3' end (antisense) with primers LW1683 5'-GCGTAATACGACTCACTATAGGGAGACCAATACC AGCAGCTCTCCAC-3' (SEQ ID NO:158) and LW1784 5'-ACCAGCAATTTTTCCCAACC-3' (SEQ ID NO:159). nGPCR-52 was labeled with ³⁵S-UTP to yield a specific activity of 0.686 x 10⁶ cpm/pmol for the antisense probe and 0.601 x 106 cpm/pmol for the sense probe. Both riboprobes were denatured and added to hybridization buffer which contained 50% formamide, 10% dextran, 0.3M NaCl, 10 mM Tris, 1 mM EDTA, 1X Denhardts, and 10 mM DTT. Sequential brain cryosections were hybridized with 45 Tl/slide of the sense and antisense riboprobe hybridization mixture then coverslipped with silanized glass coverslips. The sections were hybridized overnight (15-18 hours) at 49C in an incubator.

Coverslips were washed off the slides in 1X SSC, followed by RNase A treatment, and high temperature stringency washes (3X, 20 minutes at 48C) in 0.1X SSC. Slides were dehydrated with 70%, 95% and 100% NH₄OAc alcohols, air dried and exposed to Kodak BioMax MR-1 film. After 8 days of exposure, the film was developed. This was followed with coating selected tissue slides with Kodak NTB-2 nuclear track emulsion and storing the slides in the dark for 16 days. The slides were then developed and counterstained with hematoxylin. Emulsion-coated sections were analyzed microscopically to determine the specificity of labeling. Presence of autoradiographic grains (generated by antisense probe hybridization) over cell bodies (versus between cell bodies) was used as an index of specific hybridization.

To determine which regions of the brain nGPCR-52 is expressed in, a 292 bp fragment of nGPCR-52 was used to generate sense and antisense ³⁵S-labeled RNA probes for *in situ* hybridization histochemistry. Specific labeling with the antisense probe showed wide spread distribution of nGPCR-52 mRNA. Localization appears in the cortex, piriform ctx, habenula, islands of Calleja, hippocampus, hypothalamus, red nucleus, dorsal raphe, substantia nigra pars compacta, and reticular thalamus. The regions where nGPCR-52 is expressed are within the limbic and neuroendocrine circuitry of the brain.

### Example 5: Tissue Expression Profiling

A PCR-based system (RapidScan™ Gene Expression Panel, OriGene Technologies, Rockville, MD) may be used to generate a comprehensive expression profile of the putative nGPCR-x in human tissue, and in human brain regions. The RapidScan Expression Panel is comprised of first-strand cDNAs from various human tissues and brain regions that are serially diluted over a 4-log range and arrayed into a multi-well PCR plate. Human tissues in the array may include: brain, heart, kidney, spleen, liver, colon, lung, small intestine, muscle, stomach, testis, placenta, salivary gland, thyroid, adrenal gland, pancreas, ovary, uterus, prostate, skin, PBL, bone marrow, fetal brain, and fetal liver.

Expression of nGPCR-X in various tissues is detected using PCR primers designed based on the available sequence of the receptor that will prime the synthesis of a predetermined size fragment in the presence of the appropriate cDNA.

PCR is performed in a 50 µl reaction containing 34 µl H₂O, 5µl 10X TT buffer (140 mM ammonium sulfate, 0.1% gelatin, 0.6 M Tris-tricine, pH 8.4), 5 µl 15 mM MgSO₄, 2 µl dNTP mixture (dGTP, dATP, dTTP, and dCTP, each at 10 mM), 0.3 µl forward primer (1 µg/µl), 0.3 µl reverse primer (1 µg/µl), 0.4 µl High Fidelity Taq polymerase (Boehringer Mannheim). The PCR reaction mixture is added to each well of the PCR plate. The plate is placed in a MJ Research PTC100 thermocycler, and is then exposed to the following cycling parameters: Pre-soak 94C for 3 minutes; denaturation at 94C for 30 seconds; annealing at primer 57C for 45 seconds; extension 72C for 2 minutes; for 35 cycles. PCR productions are then separated and analyzed by electrophoresis on a 1.2% agarose gel stained with ethidium bromide. The 4-log dilution range of cDNA deposited on the plate ensures that the amplification reaction is within the linear range and, hence, facilitates semi-quantitative determination of relative mRNA accumulation in the various tissues or brain regions examined.

### nGPCR-70

Tissue specific expression of the cDNA encoding nGPCR-70 was detected using a PCR-based method. Multiple Choice™ first strand cDNAs (OriGene Technologies, Rockville, MD) from 6 human tissues were serially diluted over a 3-log range and arrayed into a multi-well PCR plate. This array was used to generate a comprehensive expression profile of the putative GPCR in human tissues. Human tissues arrayed included: brain, heart, kidney, peripheral blood leukocytes, lung and testis. PCR primers were designed based on the available sequence of the Celera sequence GA_16417344. The forward primer used was: 5'-GTGACTAACTCTGCCT GCG-3' (SEQ ID NO:160). The reverse primer used was: 5'-TTGCGCTGCAACACTAGCG-3' (SEQ ID NO:161). This primer set primed the synthesis of a 286 base pair fragment in the presence of the appropriate cDNA. For detection of expression within brain regions, the same primer set was used with the Human Brain Rapid Scan™ Panel (OriGene Technologies, Rockville, MD). This panel represented serial dilutions over a 2 log range of first strand cDNA from the following brain regions arrayed in a 96 well format: frontal lobe, temporal lobe, cerebellum, hippocampus, substantia nigra, caudate nucleus, amygdala, thalamus, hypothalamus, pons, medulla and spinal cord. Primers were synthesized by Genosys Corp., The Woodlands, TX. PCR reactions were assembled using the components of the Expand Hi-Fi PCR System™ (Roche Molecular Biochemicals, Indianapolis, IN). Twenty-five microliters of the PCR reaction mixture was added to each well of the RapidScan PCR plate. The plate was placed in a GeneAmp 9700 PCR thermocycler (Perkin Elmer Applied Biosystems). The following cycling program was executed: Pre-soak at (94C for 3 minutes) followed by 35 cycles of 94C for 45 seconds, 53C for 2 minutes and 72C for 45 seconds. PCR reaction products were then separated and analyzed by electrophoresis on a 2.0% agarose gel stained with ethidium bromide.

nGPCR-70 was expressed in the brain, heart and lung. Within the brain, nGPCR-70 was expressed in regions including but not limited to, cerebellum, hippocampus, substantia nigra, thalamus, frontal lobe, caudate nucleus, and spinal cord. Expression of the nGPCR-70 in the brain provides an indication that modulators of nGPCR-70 activity have utility for treating neurological disorders, including but not limited to, movement disorders, affective disorders, metabolic disorders, inflammatory disorders and cancers. Use of nGPCR-70 modulators, including nGPCR-70 ligands and anti-nGPCR-70 antibodies, to treat individuals having such disease states is intended as an aspect of the invention.

### nGPCR-63

Tissue specific expression of the putative nGPCR-63 was detected using a PCR-based RapidScan™ Gene Expression Panel (OriGene Technologies, Rockville, MD). The RapidScan Expression Panel is comprised of first-strand cDNAs from 12 human tissues that are serially diluted over a 3-log range and arrayed into a multi-well PCR plate. This array was used to generate a comprehensive expression profile of the putative GPCR in human tissues. Human tissues arrayed included: brain, heart, kidney, spleen, liver, colon, lung, small intestine, muscle, stomach, testis, placenta, salivary gland, thyroid, adrenal gland, pancreas, ovary, uterus, prostate, skin, PBL, bone marrow, fetal brain, fetal liver. PCR primers were designed based on the available sequence of the Celera sequence HUM_IDS|Contig|11000258115466. The forward primer used was: 5'-ACAGCCCCAAAGCCAAACAC-3' (SEQ ID NO:162). The reverse primer was: 5'-CCGCAGGAGCAATGAAAATCAG-3' (SEQ ID NO:163). This primer set primed the synthesis of a 220 base pair fragment in the presence of the appropriate cDNA. For detection of expression within brain regions, the same primer set was used with the Human Brain RapidScan™ Panel (OriGene Technologies, Rockville, MD). This panel represents serial dilutions over a 2 log range of first strand cDNA from the following brain regions arrayed in a 96 well format: frontal lobe, temporal lobe, cerebellum, hippocampus, substantia nigra, caudate nucleus, amygdala, thalamus, hypothalamus, pons, medulla and spinal cord. Primers were synthesized by Genosys Corp., The Woodlands, TX. PCR reactions were assembled using the components of the Expand Hi-Fi PCR System™ (Roche Molecular Biochemicals, Indianapolis, IN). Twenty-five microliters of the PCR reaction mixture was added to each well of the RapidScan PCR plate. The plate was placed in a GeneAmp 9700 PCR thermocycler (Perkin Elmer Applied Biosystems). The following cycling program was executed: Pre-soak at (94 for 3minutes) followed by 35 cycles of 94C for 45 seconds, 54C for 2 minutes, 72C for 45 seconds. PCR reaction products were then separated and analyzed by electrophoresis on a 2.0% agarose gel, stained with ethidium bromide.

nGPCR-63 was expressed in the brain, heart, kidney, liver, muscle, ovary, prostate, small intestine, spleen, testis, peripheral blood leukocytes, and lung. Within the brain, nGPCR-63 was expressed in regions including but not limited to, cerebellum, amygdala, hypothalamus, medulla, temporal lobe, pons, hippocampus, substantia nigra, thalamus, frontal lobe, caudate nucleus, and spinal cord. Expression of the nGPCR-63 in the brain provides an indication that modulators of nGPCR-63 activity have utility for treating neurological disorders, including but not limited to, movement disorders, affective disorders, metabolic disorders, inflammatory disorders and cancers. Use of nGPCR-63 modulators, including nGPCR-63 ligands and anti-nGPCR-63 antibodies, to treat individuals having such disease states is intended as an aspect of the invention.

### nGPCR-42

Expression of nGPCR-42 in the various tissues was detected by using PCR primers designed based on the sequence of the receptor that prime the synthesis of a 110 bp fragment in the presence of the appropriate cDNA. The forward primer used to detect expression ofnGPCR-42 was: 5'-TCTCCAAACTCCTGGCCTTC-3' (SEQ ID NO:164) and the reverse primer was: 5'-GCAGGGCAGCTTTTTCATCC-3' (SEQ ID NO:165). Primers were synthesized by Genosys Corp., The Woodlands, TX. The primer set was assembled into a PCR reaction using the components of the Expand Hi-Fi PCR System™ (Roche Molecular Biochemicals). Twenty-five microliters of the PCR reaction mixture was added to each well of the RapidScan PCR plate. The plate was placed in a GeneAmp PCR9700 PCR thermocycler (Perkin Elmer Applied Biosystems). The plate was then exposed to the following cycling paramaters: Pre-soak 94C for 3 minutes; denaturation at 94C for 30 seconds; annealing at primer Tₘ for 45 seconds; extension 72C for 2 minutes; for 35 cycles. PCR productions were then separated and analyzed by electrophoresis on a 2.0% agarose gel.

The 4-log dilution range of cDNA deposited on the plate ensured that the amplification reaction was within the linear range and, facilitated semi-quantitative determination of relative mRNA accumulation in the various tissues or brain regions examined.

nGPCR-42 was expressed in the brain, peripheral blood leukocytes, bone marrow, placenta, salivary gland, liver, ovary, uterus, testis, fetal liver, heart, thyroid gland, kidney, adrenal gland, spleen, pancreas, colon, lung, prostate, small intestine, skin, muscle, fetal brain, and stomach. Within the brain, GPCR-42 was expressed in the temporal lobe, cerebellum, substantia nigra, caudate nucleus, amygdala, frontal lobe, thalamus, hippocampus, hypothalamus, pons, medulla, and spinal cord. Expression of the nGPCR-42 in the brain provided an indication that modulators of nGPCR-42 activity have utility for treating disorders, including but not limited to, schizophrenia, affective disorders, metabolic disorders, inflammatory disorders, cancers, ADHD/ADD (i.e., Attention Deficit-Hyperactivity Disorder/Attention Deficit Disorder), and neural disorders such as Alzheimer's disease, Parkinson's disease, migraine, and senile dementia. Some other diseases for which modulators of nGPCR-42 may have utility include depression, anxiety, bipolar disease, epilepsy, neuritis, neurasthenia, neuropathy, neuroses, and the like. Use of nGPCR-42 modulators, including nGPCR-42 ligands and anti-nGPCR-42 antibodies, to treat individuals having such disease states is intended as an aspect of the invention.

### nGPCR-46

Tissue specific expression of the cDNA encoding nGPCR-46 was detected using a PCR-based method. Multiple Choice™ first strand cDNAs (OriGene Technologies, Rockville, MD) from 12 human tissues are serially diluted over a 3-log range and arrayed into a multi-well PCR plate. This array is used to generate a comprehensive expression profile of nGPCR-46 in human tissues. Human tissues arrayed include: brain, heart, kidney, peripheral blood leukocytes, liver, lung, muscle, ovary, prostate, small intestine, spleen and testis. The forward PCR primer was: 5'-ACGCCCGCTGAACCGTATAC-3' (SEQ ID NO: 166) and the reverse primer used was: 5'-GGGTGCCACCTGGTTGCTC-3' (SEQ ID NO:167). This primer set will prime the synthesis of a 242 base pair fragment in the presence of the appropriate cDNA. For detection of expression within brain regions, the same primer set was used with the Human Brain Rapid Scan™ Panel (OriGene Technologies, Rockville, MD). This panel represents serial dilutions over a 2 log range of first strand cDNA from the following brain regions arrayed in a 96 well format: frontal lobe, temporal lobe, cerebellum, hippocampus, substantia nigra, caudate nucleus, amygdala, thalamus, hypothalamus, pons, medulla and spinal cord. Primers were synthesized by Genosys Corp., The Woodlands, TX. PCR reactions were assembled using the components of the Expand Hi-Fi PCR System™ (Roche Molecular Biochemicals, Indianapolis, IN). Twenty-five microliters of the PCR reaction mixture was added to each well of the RapidScan PCR plate. The plate was placed in a GeneAmp 9700 PCR thermocycler (Perkin Elmer Applied Biosystems). The following cycling program was executed: Pre-soak at (94C for 3 minutes) followed by 35 cycles of 94C for 45 seconds, 53C for 2 minutes, 72C for 45 seconds. PCR reaction products were then separated and analyzed by electrophoresis on a 2.0% agarose gel stained with ethidium bromide.

nGPCR-46 was expressed in the brain, peripheral blood lymphocytes, testis, heart, kidney, spleen, prostate, ovary, liver, lung, small intestine, and muscle. Within the brain, nGPCR-46 was expressed in frontal lobe, temporal lobe, cerebellum, hippocampus, substantia nigra, caudate nucleus, amygdala, thalamus, hypothalamus, pons, medulla, and spinal cord. Expression of the nGPCR-46 in the brain indicates that modulators of nGPCR-46 activity have utility for treating neurological disorders, including but not limited to, movement disorders, affective disorders, metabolic disorders, inflammatory disorders and cancers. Use of nGPCR-46 modulators, including nGPCR-46 ligands and anti-nGPCR-46 antibodies, to treat individuals having such disease states is intended as an aspect of the invention.

### nGPCR-48

Tissue specific expression of the cDNA encoding nGPCR-48 was detected using a PCR-based RapidScan™ Gene Expression Panel (OriGene Technologies, Rockville, MD). The RapidScan Expression Panel is comprised of first-strand cDNAs from 24 human tissues that are serially diluted over a 3-log range and arrayed into a multi-well PCR plate. This array is used to generate a comprehensive expression profile of the putative GPCR in human tissues. Human tissues arrayed include: brain, heart, kidney, spleen, liver, colon, lung, small intestine, muscle, stomach, testis, placenta, salivary gland, thyroid, adrenal gland, pancreas, ovary, uterus, prostate, skin, PBL, bone marrow, fetal brain, fetal liver. The forward PCR primer was 5'-ATGGCACCTTCTCATCGG-3' (SEQ ID NO:168) and the reverse primer was 5'- ACGTAGT ACACCGCCTTG-3' (SEQ ID NO:169). This primer set will prime the synthesis of a 392 base pair fragment in the presence of the appropriate cDNA. For detection of expression within brain regions, the same primer set was used with the Human Brain Rapid Scan™ Panel (OriGene Technologies, Rockville, MD). This panel represents serial dilutions over a 2 log range of first strand cDNA from the following brain regions arrayed in a 96 well format: frontal lobe, temporal lobe, cerebellum, hippocampus, substantia nigra, caudate nucleus, amygdala, thalamus, hypothalamus, pons, medulla and spinal cord. Primers were synthesized by Genosys Corp., The Woodlands, TX. PCR reactions were assembled using the components of the Expand Hi-Fi PCR System™ (Roche Molecular Biochemicals, Indianapolis, IN). Twenty-five microliters of the PCR reaction mixture was added to each well of the RapidScan PCR plate. The plate was placed in a GeneAmp 9700 PCR Thermocycler (PE Applied Biosystems). The following cycling program was executed: Pre-soak at (94°Cfor 3min.) followed by 35 cycles of 94C for 45 seconds, 53C for 2 minutes, 72C for 45 seconds. PCR reaction products were then separated and analyzed by electrophoresis on a 2.0% agarose gel, stained with ethidium bromide.

The 4-log dilution range of cDNA deposited on the plate ensured that the amplification reaction was within the linear range and, facilitated semi-quantitative determination of relative mRNA accumulation in the various tissues or brain regions examined.

nGPCR-48 was expressed in the ovary, prostate, skin, stomach bone marrow, placenta, fetal liver, brain, heart, spleen, liver, colon, uterus, lung, small intestine, peripheral blood leukocytes, testis, and fetal brain. Within the brain, nGPCR-48 was expressed in the substantia nigra, frontal lobe, temporal lobe, cerebellum, hippocampus, caudate nucleus, thalamus and spinal cord. Expression of nGPCR-48 in the brain provided an indication that modulators of nGPCR-48 activity have utility for treating disorders, including but not limited to, schizophrenia, affective disorders, ADHD/ADD (i.e., Attention Deficit-Hyperactivity Disorder/Attention Deficit Disorder), neural disorders such as Alzheimer's disease, Parkinson's disease, migraine, senile dementia, depression, anxiety, bipolar disease, epilepsy, neuritis, neurasthenia, neuropathy, neuroses, metabolic disorders, inflammatory disorders, cancers and the like. Use of nGPCR-48 modulators, including nGPCR-48 ligands and anti-nGPCR-48 antibodies, to treat individuals having such disease states is intended as an aspect of the invention.

### nGPCR-49

Tissue specific expression of the cDNA encoding SEQ-49 was detected using a PCR-based method. Multiple Choice™ first strand cDNAs (OriGene Technologies, Rockville, MD) from 6 human tissues were serially diluted over a 3-log range and arrayed into a multi-well PCR plate. This array was used to generate a comprehensive expression profile of the putative GPCRs in human tissues. Human tissues arrayed included: brain, heart, kidney, peripheral blood leukocytes, lung, and testis. PCR primers were designed based on the available sequence of the Celera sequence GA_11585051. The forward primer used was 5'-AGCAGGTAGATGGAGAA GG-3' (SEQ ID NO:170). The reverse primer used was 5'-GACTCCTGAGGACCATGTC- 3' (SEQ ID NO:171). This primer set primed the synthesis of a 320 base pair fragment in the presence of the appropriate cDNA. PCR reactions were performed in a GeneAmp 9700 PCR thermocycler (Perkin Elmer Applied Biosystems). The following cycling program was executed: Pre-soak at 94C for 3minutes followed by 35 cycles of 94C for 45 seconds, 53.5C for 2 minutes, 72C for 45 seconds. PCR reaction products were then separated and analyzed by electrophoresis on a 2.0% agarose gel stained with ethidium bromide. nGPCR-49 was expressed in the brain, heart, testis, peripheral blood leukocytes, kidney, liver, muscle, ovary, prostate, small intestine, spleen, and lung. Within the brain, nGPCR-49 was expressed in regions including but not limited to, cerebellum, hippocampus, substantia nigra, thalamus, hypothalamus, frontal lobe, temporal lobe, amygdala, pons, medulla, caudate nucleus, and spinal cord. Expression of the nGPCR-49 in the brain provides an indication that modulators of nGPCR-49 activity have utility for treating neurological disorders, including but not limited to, movement disorders, affective disorders, metabolic disorders, inflammatory disorders and cancers. Use of nGPCR-49 modulators, including nGPCR-49 ligands and anti-nGPCR-49 antibodies, to treat individuals having such disease states is intended as an aspect of the invention.

### nGPCR-61

Tissue specific expression of the cDNA encoding SEQ-61 was detected using a PCR-based method. Multiple Choice™ first strand cDNAs (OriGene Technologies, Rockville, MD) from 6 human tissues were serially diluted over a 3-log range and arrayed into a multi-well PCR plate. This array was used to generate a comprehensive expression profile of the putative GPCRs in human tissues. Human tissues arrayed included: brain, heart, kidney, peripheral blood leukocytes, lung, and testis. PCR primers were designed based on the available sequence of the Celera sequence GA_13368549. The forward primer used was 5'-CATTGGAACCACATTTAT TGG-3' (SEQ ID NO:172). The reverse primer used was 5'-AAGCAAGACAGGTGAGAATG-3' (SEQ ID NO:173). This primer set primed the synthesis of a 284 base pair fragment in the presence of the appropriate cDNA. PCR reactions were performed in a GeneAmp 9700 PCR thermocycler (Perkin Elmer Applied Biosystems). The following cycling program was executed: Pre-soak at 94C for 3 minutes followed by 35 cycles of 94C for 45 seconds, 53.5C for 2 minutes, 72C for 45 seconds. PCR reaction products were then separated and analyzed by electrophoresis on a 2.0% agarose gel stained with ethidium bromide.

nGPCR-61 was expressed in the brain, heart, testis, peripheral blood leukocytes, and lung. Expression of the nGPCR-61 in the brain provides an indication that modulators of nGPCR-61 activity have utility for treating neurological disorders, including but not limited to, movement disorders, affective disorders, metabolic disorders, inflammatory disorders and cancers. Use of nGPCR-61 modulators, including nGPCR-61 ligands and anti-nGPCR-61 antibodies, to treat individuals having such disease states is intended as an aspect of the invention.

### nGPCR-51

Tissue specific expression of the cDNA encoding SEQ-51 was detected using a PCR-based method. Multiple Choice™ first strand cDNAs (OriGene Technologies, Rockville, MD) from 12 human tissues were serially diluted over a 3-log range and arrayed into a multi-well PCR plate. This array was used to generate a comprehensive expression profile of the putative GPCR in human tissues. Human tissues arrayed include: brain, heart, kidney, peripheral blood leukocytes, liver, lung, muscle, ovary, prostate, small intestine, spleen and testis. PCR primers were designed based on the sequence of nGPCR-51 provided herein as SEQ ID NO:39 and SEQ ID NO:57. The forward primer used was 5'-CCATCTGTCTCAGCTATGCC-3' (SEQ ID NO:174), corresponding base pairs 929 through 948 of SEQ ID NO:57. The reverse primer used was 5'-TCCTTCTCAGTCGCTCTTC-3' (SEQ ID NO:175) corresponding to base pairs 1022 through 1038 of SEQ ID NO:57.This primer set primes the synthesis of a 112 base pair fragment in the presence of the appropriate cDNA. For detection of expression within brain regions, the same primer set was used with the Human Brain Rapid Scan™ Panel (OriGene Technologies, Rockville, MD). This panel represents serial dilutions over a 3 log range of first strand cDNA from the following brain regions arrayed in a 96 well format: frontal lobe, temporal lobe, cerebellum, hippocampus, substantia nigra, caudate nucleus, amygdala, thalamus, hypothalamus, pons, medulla and spinal cord. Primers were synthesized by Genosys Corp., The Woodlands, TX. PCR reactions were assembled using the components of the Expand Hi-Fi PCR System™ (Roche Molecular Biochemicals, Indianapolis, IN). Twenty-five microliters of the PCR reaction mixture was added to each well of the RapidScan PCR plate. The plate was placed in a GeneAmp 9700 PCR thermocycler (Perkin Elmer Applied Biosystems). The following cycling program was executed: Pre-soak at 94C for 3 minutes followed by 35 cycles of 94C for 45 seconds, 52C for 2 minutes, 72C for 45 seconds. PCR reaction products were then separated and analyzed by electrophoresis on a 2.0% agarose gel stained with ethidium bromide.

nGPCR-51 was expressed in the brain, heart, peripheral blood leukocytes, liver, prostate, testis, lung, small intestine, and spleen. Within the brain, nGPCR-51 was expressed in the cerebellum, hippocampus, substantia nigra, thalamus, hypothalamus, pons, frontal lobe, temporal lobe, caudate nucleus, medulla, spinal cord, and amygdala. Expression of the nGPCR-51 in the brain provides an indication that modulators of nGPCR-51 activity have utility for treating neurological disorders, including but not limited to, movement disorders, affective disorders, metabolic disorders, inflammatory disorders, cancers, attention disorders, anxiety, depression, and obesity. Use of nGPCR-51 modulators, including nGPCR-51 ligands and anti-nGPCR-51 antibodies, to treat individuals having such disease states is intended as an aspect of the invention.

### nGPCR-52

Tissue specific expression of the cDNA encoding SEQ-52 was detected using a PCR-based method. Multiple Choice™ first strand cDNAs (OriGene Technologies, Rockville, MD) from 12 human tissues were serially diluted over a 3-log range and arrayed into a multi-well PCR plate. This array was used to generate a comprehensive expression profile of nGPCR-52 in human tissues. Human tissues arrayed include: brain, heart, kidney, peripheral blood leukocytes, liver, lung, muscle, ovary, prostate, small intestine, spleen and testis. The forward primer used was 5'-CAAACAACAAACAGCAGAACC-3' (SEQ ID NO:176) and the reverse primer was 5'-TCACAGTCACACCTACCAAG-3' (SEQ ID NO:177). This primer set primed the synthesis of a 274 base pair fragment in the presence of the appropriate cDNA. For detection of expression within brain regions, the same primer set was used with the Human Brain Rapid Scan™ Panel (OriGene Technologies, Rockville, MD). This panel represents serial dilutions over a 2 log range of first strand cDNA from the following brain regions arrayed in a 96 well format: frontal lobe, temporal lobe, cerebellum, hippocampus, substantia nigra, caudate nucleus, amygdala, thalamus, hypothalamus, pons, medulla and spinal cord. Primers were synthesized by Genosys Corp., The Woodlands, TX. PCR reactions were assembled using the components of the Expand Hi-Fi PCR System™ (Roche Molecular Biochemicals, Indianapolis, IN). Twenty-five microliters of the PCR reaction mixture was added to each well of the RapidScan PCR plate. The plate was placed in a GeneAmp 9700 PCR thermocycler (Perkin Elmer Applied Biosystems). The following cycling program was executed: Pre-soak at 94C for 3 minutes followed by 35 cycles of 94C for 45 seconds, 53C for 2 minutes, 72C for 45 seconds. PCR reaction products were then separated and analyzed by electrophoresis on a 2.0% agarose gel stained with ethidium bromide.

nGPCR-52 was expressed in the brain, the lungs, muscle, small intestine, spleen, testis, heart, peripheral blood leukocytes, and liver. Within the brain, nGPCR-52 was expressed in the frontal lobe, temporal lobe, cerebellum, hippocampus, substantia nigra, caudate nucleus, amygdala, hypothalamus, and medulla. Expression of nGPCR-52 in the brain provides an indication that modulators of nGPCR-52 activity have utility for treating neurological disorders, including but not limited to, movement disorders, affective disorders, metabolic disorders, inflammatory disorders and cancers. Use of nGPCR-52 modulators, including nGPCR-52 ligands and anti-nGPCR-52 antibodies, to treat individuals having such disease states is intended as an aspect of the invention.

### Example 6: Chromosomal Localization

### nGPCR-51

The chromosomal location of the gene encoding SEQ51 was determined using the Stanford G3 Radiation Hybrid Panel (Research Genetics, Inc., Huntsville, AL). This panel contains 83 radiation hybrid clones of the entire human genome created by the Stanford Human Genome Center. PCR reactions were assembled containing 25ng of DNA from each clone and the the components of the Expand Hi-Fi PCR System™ (Roche Molecular Biochemicals, Indianapolis, IN) in a final reaction volume of 15 ul. PCR primers were synthesized by Genosys Corp., The Woodlands, TX. PCR primers were designed based on the available sequence of the Celera sequence GA_11007426. The forward primer used was 5'-CCATCTGTCTCAGCTAT GCC-3' (SEQ ID NO:178) corresponding base pairs 241 through 260 of GA_11007426. The reverse primer used was 5'-TCCTTCTCAGTCGCTCTTC-3' (SEQ ID NO:179) corresponding to base pairs 334 through 352 of GA_11007426. This primer set will prime the synthesis of a 112 base pair fragment in the presence of the appropriate genomic DNA. PCR reactions were incubated in a GeneAmp 9700 PCR thermocycler (Perkin Elmer Applied Biosystems). The following cycling program was executed: Pre-soak at 94C for 3 minutes, and 35 cycles at 94C for 30 seconds, 52C for 60 seconds, 72C for 2 minutes. PCR reaction products were then separated and analyzed by electrophoresis on a 2.0% agarose gel, stained with ethidium bromide. Lanes were scored for the presence or absence of the expected PCR product and the results submitted to the Stanford Human Genome Center via e-mail for analysis (world wide web of the Internet at, for example, shgc.stanford.edu./RH/rhserverformnew.html). G3 Radiation Hybrid Panel Analysis of the SEQ51 places it on chromosome 6, most nearly linked to Stanford marker SHGC-34355 with a LOD score of 12.32. This marker lies at position 6q21. Chromosome 6q21-q22.3 contains a highly significant disease locus for schizophrenia (Cao *et al.,* Genomics 43: 1-8, (1997)). Genes which map to this region of the chromosome are candidate genes for schizophrenia. Any genes localized to chromosomal regions in linkage with schizophrenia are candidate genes for disease suceptibility. Genes in these regions with the potential to play a biochemical/functional role in the disease process (like G protein coupled receptors) have a high probability of being a disease modifying locus. nGPCR-51, because of its chromosomal location, is an attractive target therefor for screening ligands useful in modulating cellular processes involved in schizophrenia.

### nGPCR-52

The chromosomal location of the gene encoding Seq-52 was determined using the Stanford G3 Radiation Hybrid Panel (Research Genetics, Inc., Huntsville, AL). This panel contains 83 radiation hybrid clones of the entire human genome created by the Stanford Human Genome Center. PCR reactions were assembled containing 25ng of DNA from each clone and the components of the Expand Hi-Fi PCR System™ (Roche Molecular Biochemicals, Indianapolis, IN) in a final reaction volume of 15 µl. PCR primers were synthesized by Genosys Corp., The Woodlands, TX. The forward primer used was RRH421: 5'-GTCTATG CTACCAAGTTCACC-3' (SEQ ID NO:180) corresponding to base pairs 3328 through 3348. The reverse primer used was RRH422: 5'-ATTCCTCCAGCCCATCATC-3' (SEQ ID NO:181) corresponding to base pairs 3433 through 3451. This primer set will prime the synthesis of a 124 base pair fragment in the presence of the appropriate genomic DNA. PCR reactions were incubated in a GeneAmp 9700 PCR thermocycler (Perkin Elmer Applied Biosystems). The following cycling program was executed: Pre-soak at 94C for 3 minutes, and 35 cycles of 94C for 30 seconds, 55C for 60 seconds, 72C for 2 minutes. PCR reaction products were then separated and analyzed by electrophoresis on a 2.0% agarose gel, stained with ethidium bromide. Lanes were scored for the presence or absence of the expected PCR product and the results submitted to the Stanford Human Genome Center via e-mail for analysis (world wide web of the Internet at, for example, shgc.stanford.edu./RH/rhserverformnew.html). The chromosomal location of the gene encoding nGPCR-52 was determined using the Stanford G3 Radiation Hybrid Panel (Research Genetics, Inc., Huntsville, AL). This panel contains 83 radiation hybrid clones of the entire human genome created by the Stanford Human Genome Center. Lanes were scored for the presence or absence of the expected PCR product and the results submitted to the Stanford Human Genome Center via e-mail for analysis. The analysis of nGPCR-52 places it on chromosome 6, most nearly linked to Stanford marker SHGC-1836 with a LOD score of 15.65. This marker lies at position 6q21. Chromosome 6q21-q22.3 contains a highly significant disease locus for schizophrenia (Cao *et al.,* Genomics 43: 1-8, (1997)). Genes which map to this region of the chromosome are candidate genes for schizophrenia. Any genes localized to chromosomal regions in linkage with schizophrenia are candidate genes for disease suceptibility. Genes in these regions with the potential to play a biochemical/functional role in the disease process (like G protein coupled receptors) have a high probability of being a disease modifying locus. nGPCR-52 because of its chromosomal location is an attractive target therefor for screening ligands useful in modulating cellular processes involved in schizophrenia.

### Example 7: Northern Blot Analysis

Northern blots are performed to examine the expression of nGPCR-x mRNA. The sense orientation oligonucleotide and the antisense-orientation oligonucleotide, described above, are used as primers to amplify a portion of the GPCR-x cDNA sequence selected from the group consisting of SEQ ID NO:1 to SEQ ID NO:60. Multiple human tissue northern blots from Clontech (Human II # 7767-1) are hybridized with the probe. Pre-hybridization is carried out at 42C for 4 hours in 5xSSC, 1X Denhardt's reagent, 0.1% SDS, 50% formamide, 250 mg/ml salmon sperm DNA. Hybridization is performed overnight at 42C in the same mixture with the addition of about 1.5x10⁶ cpm/ml of labeled probe. The probe is labeled with α-³²P-dCTP by Rediprime™ DNA labeling system (Amersham Pharmacia), purified on Nick Column™ (Amersham Pharmacia) and added to the hybridization solution. The filters are washed several times at 42C in 0.2x SSC, 0.1% SDS. Filters are exposed to Kodak XAR film (Eastman Kodak Company, Rochester, N.Y., USA) with intensifying screen at -80C.

### Example 8: Recombinant Expression of nGPCR-x in Eukaryotic Host Cells

### Expression of nGPCR-x in Mammalian Cells

To produce nGPCR-x protein, a nGPCR-x-encoding polynucleotide is expressed in a suitable host cell using a suitable expression vector and standard genetic engineering techniques. For example, the nGPCR-x-encoding sequence described in Example 1 is subcloned into the commercial expression vector pzeoSV2 (Invitrogen, San Diego, CA) and transfected into Chinese Hamster Ovary (CHO) cells using the transfection reagent FuGENE6™ (Boehringer-Mannheim) and the transfection protocol provided in the product insert. Other eukaryotic cell lines, including human embryonic kidney (HEK293) and COS cells, are suitable as well. Cells stably expressing nGPCR-x are selected by growth in the presence of 100 µg/ml zeocin (Stratagene, LaJolla, CA). Optionally, nGPCR-x may be purified from the cells using standard chromatographic techniques. To facilitate purification, antisera is raised against one or more synthetic peptide sequences that correspond to portions of the nGPCR-x amino acid sequence, and the antisera is used to affinity purify nGPCR-x. The nGPCR-x also may be expressed in-frame with a tag sequence *(e.g.,* polyhistidine, hemagluttinin, FLAG) to facilitate purification. Moreover, it will be appreciated that many of the uses for nGPCR-x polypeptides, such as assays described below, do not require purification of nGPCR-x from the host cell.

### Expression ofnGPCR-x in 293 cells

For expression of nGPCR-x in mammalian cells HEK293 (transformed human, primary embryonic kidney cells), a plasmid bearing the relevant nGPCR-x coding sequence is prepared, using vector pSecTag2A (Invitrogen). Vector pSecTag2A contains the murine IgK chain leader sequence for secretion, the c-myc epitope for detection of the recombinant protein with the anti-myc antibody, a C-terminal polyhistidine for purification with nickel chelate chromatography, and a Zeocin resistant gene for selection of stable transfectants. The forward primer for amplification of this GPCR cDNA is determined by routine procedures and preferably contains a 5' extension of nucleotides to introduce the *HindIII* cloning site and nucleotides matching the GPCR sequence. The reverse primer is also determined by routine procedures and preferably contains a 5' extension of nucleotides to introduce an *XhoI* restriction site for cloning and nucleotides corresponding to the reverse complement of the nGPCR-x sequence. The PCR conditions are 55C as the annealing temperature. The PCR product is gel purified and cloned into the *HindIII-XhoI* sites of the vector.

The DNA is purified using Qiagen chromatography columns and transfected into 293 cells using DOTAP™ transfection media (Boehringer Mannheim, Indianapolis, IN). Transiently transfected cells are tested for expression after 24 hours of transfection, using western blots probed with anti-His and anti-nGPCR-x peptide antibodies. Permanently transfected cells are selected with Zeocin and propagated. Production of the recombinant protein is detected from both cells and media by western blots probed with anti-His, anti-Myc or anti-GPCR peptide antibodies.

### Expression of nGPCR-x in COS cells

For expression of the nGPCR-x in COS7 cells, a polynucleotide molecule having a sequence selected from the group consisting of SEQ ID NO:1 to SEQ ID NO:60 can be cloned into vector p3-CI. This vector is a pUC18-derived plasmid that contains the HCMV (human cytomegalovirus) promoter-intron located upstream from the bGH (bovine growth hormone) polyadenylation sequence and a multiple cloning site. In addition, the plasmid contains the dhrf (dihydrofolate reductase) gene which provides selection in the presence of the drug methotrexane (MTX) for selection of stable transformants.

The forward primer is determined by routine procedures and preferably contains a 5' extension which introduces an *Xbal* restriction site for cloning, followed by nucleotides which correspond to a sequence selected from the group consisting of SEQ ID NO:1 to SEQ ID NO:60. The reverse primer is also determined by routine procedures and preferably contains 5'-extension of nucleotides which introduces a *SalI* cloning site followed by nucleotides which correspond to the reverse complement of a sequence selected from the group consisting of SEQ ID NO:1 to SEQ ID NO:60. The PCR consists of an initial denaturation step of 5 minutes at 95C, 30 cycles of 30 seconds denaturation at 95C, 30 seconds annealing at 58C and 30 seconds extension at 72C, followed by 5 minutes extension at 72C. The PCR product is gel purified and ligated into the *XbaI* and *Sall* sites of vector p3-CI. This construct is transformed into *E. coli* cells for amplification and DNA purification. The DNA is purified with Qiagen chromatography columns and transfected into COS 7 cells using Lipofectamine™ reagent from BRL, following the manufacturer's protocols. Forty-eight and 72 hours after transfection, the media and the cells are tested for recombinant protein expression.

nGPCR-x expressed from a COS cell culture can be purified by concentrating the cell-growth media to about 10 mg of protein/ml, and purifying the protein by, for example, chromatography. Purified nGPCR-x is concentrated to 0.5 mg/ml in an Amicon concentrator fitted with a YM-10 membrane and stored at -80C.

### Expression of nGPCR-x in Insect Cells

For expression of nGPCR-x in a baculovirus system, a polynucleotide molecule having a sequence selected from the group consisting of SEQ ID NO:1 to SEQ ID NO:60 can be amplified by PCR. The forward primer is determined by routine procedures and preferably contains a 5' extension which adds the *NdeI* cloning site, followed by nucleotides which correspond to a sequence selected from the group consisting of SEQ ID NO:1 to SEQ ID NO:60. The reverse primer is also determined by routine procedures and preferably contains a 5' extension which introduces the *KpnI* cloning site, followed by nucleotides which correspond to the reverse complement of a sequence selected from the group consisting of SEQ ID NO:1 to SEQ ID NO:60.

The PCR product is gel purified, digested with *NdeI* and *KpnI,* and cloned into the corresponding sites of vector pACHTL-A (Pharmingen, San Diego, CA). The pAcHTL expression vector contains the strong polyhedrin promoter of the *Autographa californica* nuclear polyhedrosis virus (AcMNPV), and a 6XHis tag upstream from the multiple cloning site. A protein kinase site for phosphorylation and a thrombin site for excision of the recombinant protein precede the multiple cloning site is also present. Of course, many other baculovirus vectors could be used in place of pAcHTL-A, such as pAc373, pVL941 and pAcIM1. Other suitable vectors for the expression of GPCR polypeptides can be used, provided that the vector construct includes appropriately located signals for transcription, translation, and trafficking, such as an in-frame AUG and a signal peptide, as required. Such vectors are described in Luckow *et al.,* Virology 170:31-39, among others. The virus is grown and isolated using standard baculovirus expression methods, such as those described in Summers *et al.* (A Manual of Methods for Baculovirus Vectors and Insect Cell Culture Procedures, Texas Agricultural Experimental Station Bulletin No. 1555 (1987)).

In a preferred embodiment, pAcHLT-A containing nGPCR-x gene is introduced into baculovirus using the "BaculoGold™" transfection kit (Pharmingen, San Diego, CA) using methods established by the manufacturer. Individual virus isolates are analyzed for protein production by radiolabeling infected cells with ³⁵S-methionine at 24 hours post infection. Infected cells are harvested at 48 hours post infection, and the labeled proteins are visualized by SDS-PAGE. Viruses exhibiting high expression levels can be isolated and used for scaled up expression.

For expression of a nGPCR-x polypeptide in a Sf9 cells, a polynucleotide molecule having a sequence selected from the group consisting of SEQ ID NO:1 to SEQ ID.NO:60 can be amplified by PCR using the primers and methods described above for baculovirus expression. The nGPCR-x cDNA is cloned into vector pAcHLT-A (Pharmingen) for expression in Sf9 insect. The insert is cloned into the *NdeI* and *KpnI* sites, after elimination of an internal *NdeI* site (using the same primers described above for expression in baculovirus). DNA is purified with Qiagen chromatography columns and expressed in Sf9 cells. Preliminary Western blot experiments from non-purified plaques are tested for the presence of the recombinant protein of the expected size which reacted with the GPCR-specific antibody. These results are confirmed after further purification and expression optimization in HiG5 cells.

### Example 9: Interaction Trap/Two-Hybrid System

In order to assay for nGPCR-x-interacting proteins, the interaction trap/two-hybrid library screening method can be used. This assay was first described in Fields *et al., Nature,* **1989***, 340*, 245, which is incorporated herein by reference in its entirety. A protocol is published in Current Protocols in Molecular Biology 1999, John Wiley & Sons, NY, and Ausubel, F. M*. et al.* 1992, Short protocols in molecular biology, Fourth edition, Greene and Wiley-interscience, NY, each of which is incorporated herein by reference in its entirety. Kits are available from Clontech, Palo Alto, CA (Matchmaker Two-Hybrid System 3).

A fusion of the nucleotide sequences encoding all or partial nGPCR-x and the yeast transcription factor GAL4 DNA-binding domain (DNA-BD) is constructed in an appropriate plasmid (*i.e.,* pGBKT7) using standard subcloning techniques. Similarly, a GAL4 active domain (AD) fusion library is constructed in a second plasmid (i.e., pGADT7) from cDNA of potential GPCR-binding proteins (for protocols on forming cDNA libraries, see Sambrook *et al.* 1989, Molecular cloning: a laboratory manual, second edition, Cold Spring Harbor Press, Cold Spring Harbor, NY), which is incorporated herein by reference in its entirety. The DNA-BD/nGPCR-x fusion construct is verified by sequencing, and tested for autonomous reporter gene activation and cell toxicity, both of which would prevent a successful two-hybrid analysis. Similar controls are performed with the AD/library fusion construct to ensure expression in host cells and lack of transcriptional activity. Yeast cells are transformed *(ca.* 105 transformants/mg DNA) with both the nGPCR-x and library fusion plasmids according to standard procedures (Ausubel *et al.,* 1992, Short protocols in molecular biology, fourth edition, Greene and Wiley-interscience, NY, which is incorporated herein by reference in its entirety). *In vivo* binding of DNA-BD/nGPCR-x with AD/library proteins results in transcription of specific yeast plasmid reporter genes (*i.e.,* lacZ, HIS3, ADE2, LEU2). Yeast cells are plated on nutrient-deficient media to screen for expression of reporter genes. Colonies are dually assayed for β-galactosidase activity upon growth in Xgal (5-bromo-4-chloro-3-indolyl-β-D-galactoside) supplemented media (filter assay for β-galactosidase activity is described in Breeden *et al.,* Cold Spring Harb. Symp. Quant. Biol., 1985, 50, 643, which is incorporated herein by reference in its entirety). Positive ADlibrary plasmids are rescued from transformants and reintroduced into the original yeast strain as well as other strains containing unrelated DNA-BD fusion proteins to confirm specific nGPCR-x/library protein interactions. Insert DNA is sequenced to verify the presence of an open reading frame fused to GAL4 AD and to determine the identity of the nGPCR-x-binding protein.

### Example 10: Mobility Shift DNA-Binding Assay Using Gel Electrophoresis

A gel electrophoresis mobility shift assay can rapidly detect specific protein-DNA interactions. Protocols are widely available in such manuals as Sambrook *et al.* **1989**, *Molecular cloning: a laboratory* manual, second edition, Cold Spring Harbor Press, Cold Spring Harbor, NY and Ausubel, F. M*. et al.,* 1992, Short Protocols in Molecular Biology, fourth edition, Greene and Wiley-interscience, NY, each of which is incorporated herein by reference in its entirety.

Probe DNA(<300 bp) is obtained from synthetic oligonucleotides, restriction endonuclease fragments, or PCR fragments and end-labeled with ³²P. An aliquot of purified nGPCR-x (*ca.* 15 µg) or crude nGPCR-x extract (*ca.* 15 ng) is incubated at constant temperature (in the range 22-37C) for at least 30 minutes in 10-15 µl of buffer (*i.e*. TAE or TBE, pH 8.0-8.5) containing radiolabeled probe DNA, nonspecific carrier DNA (*ca.* 1 µg), BSA (300 µg/ml), and 10% (v/v) glycerol. The reaction mixture is then loaded onto a polyacrylamide gel and run at 30-35 mA until good separation of free probe DNA from protein-DNA complexes occurs. The gel is then dried and bands corresponding to free DNA and protein-DNA complexes are detected by autoradiography.

### Example 11: Antibodies to nGPCR-x

Standard techniques are employed to generate polyclonal or monoclonal antibodies to the nGPCR-x receptor, and to generate useful antigen-binding fragments thereof or variants thereof, including "humanized" variants. Such protocols can be found, for example, in Sambrook *et al.* (1989) and Harlow *et al.* (Eds.), *Antibodies A Laboratory Manual;* Cold Spring Harbor Laboratory; Cold Spring Harbor, NY (1988). In one embodiment, recombinant nGPCR-x polypeptides (or cells or cell membranes containing such polypeptides) are used as antigen to generate the antibodies. In another embodiment, one or more peptides having amino acid sequences corresponding to an immunogenic portion of nGPCR-x (*e.g*., 6, 7, 8, 9, 10, 11, 12, 13, 14, 15, 16, 17, 18, 19, 20, or more amino acids) are used as antigen. Peptides corresponding to extracellular portions of nGPCR-x, especially hydrophilic extracellular portions, are preferred. The antigen may be mixed with an adjuvant or linked to a hapten to increase antibody production.

### Polyclonal or Monoclonal antibodies

As one exemplary protocol, recombinant nGPCR-x or a synthetic fragment thereof is used to immunize a mouse for generation of monoclonal antibodies (or larger mammal, such as a rabbit, for polyclonal antibodies). To increase antigenicity, peptides are conjugated to Keyhole Lympet Hemocyanin (Pierce), according to the manufacturer's recommendations. For an initial injection, the antigen is emulsified with Freund's Complete Adjuvant and injected subcutaneously. At intervals of two to three weeks, additional aliquots of nGPCR-x antigen are emulsified with Freund's Incomplete Adjuvant and injected subcutaneously. Prior to the final booster injection, a serum sample is taken from the immunized mice and assayed by western blot to confirm the presence of antibodies that immunoreact with nGPCR-x. Serum from the immunized animals may be used as polyclonal antisera or used to isolate polyclonal antibodies that recognize nGPCR-x. Alternatively, the mice are sacrificed and their spleen removed for generation of monoclonal antibodies.

To generate monoclonal antibodies, the spleens are placed in 10 ml serum-free RPMI 1640, and single cell suspensions are formed by grinding the spleens in serum-free RPMI 1640, supplemented with 2 mM L-glutamine, 1 mM sodium pyruvate, 100 units/ml penicillin, and 100 µg/ml streptomycin (RPMI) (Gibco, Canada). The cell suspensions are filtered and washed by centrifugation and resuspended in serum-free RPMI. Thymocytes taken from three naive Balb/c mice are prepared in a similar manner and used as a Feeder Layer. NS-1 myeloma cells, kept in log phase in RPMI with 10% fetal bovine serum (FBS) (Hyclone Laboratories, Inc., Logan, Utah) for three days prior to fusion, are centrifuged and washed as well.

To produce hybridoma fusions, spleen cells from the immunized mice are combined with NS-1 cells and centrifuged, and the supernatant is aspirated. The cell pellet is dislodged by tapping the tube, and 2 ml of 37C PEG 1500 (50% in 75 mM HEPES, pH 8.0) (Boehringer-Mannheim) is stirred into the pellet, followed by the addition of serum-free RPMI. Thereafter, the cells are centrifuged, resuspended in RPMI containing 15% FBS, 100 µM sodium hypoxanthine, 0.4 µM aminopterin, 16 µM thymidine (HAT) (Gibco), 25 units/ml IL-6 (Boehringer-Mannheim) and 1.5 x 10⁶ thymocytes/ml, and plated into 10 Corning flat-bottom 96-well tissue culture plates (Corning, Corning New York).

On days 2, 4, and 6 after the fusion, 100 µl of medium is removed from the wells of the fusion plates and replaced with fresh medium. On day 8, the fusions are screened by ELISA, testing for the presence of mouse IgG that binds to nGPCR-x. Selected fusion wells are further cloned by dilution until monoclonal cultures producing anti-nGPCR-x antibodies are obtained. *Humanization of anti-nGPCR x monoclonal antibodies*

The expression pattern of nGPCR-x as reported herein and the proven track record of GPCRs as targets for therapeutic intervention suggest therapeutic indications for nGPCR-x inhibitors (antagonists). nGPCR-x-neutralizing antibodies comprise one class of therapeutics useful as nGPCR-x antagonists. Following are protocols to improve the utility of anti-nGPCR-x monoclonal antibodies as therapeutics in humans by "humanizing" the monoclonal antibodies to improve their serum half-life and render them less immunogenic in human hosts (*i.e*., to prevent human antibody response to non-human anti-nGPCR-x antibodies).

The principles of humanization have been described in the literature and are facilitated by the modular arrangement of antibody proteins. To minimize the possibility of binding complement, a humanized antibody of the IgG4 isotype is preferred.

For example, a level of humanization is achieved by generating chimeric antibodies comprising the variable domains of non-human antibody proteins of interest with the constant domains of human antibody molecules. (See, *e.g.,* Morrison *et al.,* Adv. Immunol., 44:65-92 (1989)). The variable domains of nGPCR-x-neutralizing anti-nGPCR-x antibodies are cloned from the genomic DNA of a B-cell hybridoma or from cDNA generated from mRNA isolated from the hybridoma of interest. The V region gene fragments are linked to exons encoding human antibody constant domains, and the resultant construct is expressed in suitable mammalian host cells (*e.g*., myeloma or CHO cells).

To achieve an even greater level of humanization, only those portions of the variable region gene fragments that encode antigen-binding complementarity determining regions ("CDR") of the non-human monoclonal antibody genes are cloned into human antibody sequences. (See, *e.g*., Jones *et al.,* Nature 321:522-525 (1986); Riechmann *et al.,* Nature 332:323-327 (1988); Verhoeyen *et al.,* Science 239:1534-36 (1988); and Tempest *et al.,* Bio/Technology 9: 266-71 (1991)). If necessary, the β-sheet framework of the human antibody surrounding the CDR3 regions also is modified to more closely mirror the three dimensional structure of the antigen-binding domain of the original monoclonal antibody. (See Kettleborough *et al.,* Protein Engin., 4:773-783 (1991); and Foote *et al.,* J. Mol. Biol., 224:487-499 (1992)).

In an alternative approach, the surface of a non-human monoclonal antibody of interest is humanized by altering selected surface residues of the non-human antibody, *e.g*., by site-directed mutagenesis, while retaining all of the interior and contacting residues of the non-human antibody. See Padlan, Molecular Immunol., 28(4/5):489-98 (1991).

The foregoing approaches are employed using nGPCR-x-neutralizing anti-nGPCR-x monoclonal antibodies and the hybridomas that produce them to generate humanized nGPCR-x-neutralizing antibodies useful as therapeutics to treat or palliate conditions wherein nGPCR-x expression or ligand-mediated nGPCR-x signaling is detrimental.

### Human nGPCR-x-Neutralizing Antibodies from Phage Display

Human nGPCR-x-neutralizing antibodies are generated by phage display techniques such as those described in Aujame *et al.,* Human Antibodies 8(4):155-168 (1997); Hoogenboom, TIBTECH 15:62-70 (1997); and Rader *et al.,* Curr. Opin. Biotechnol. 8:503-508 (1997), each of which is incorporated herein by reference in is entirety. For example, antibody variable regions in the form of Fab fragments or linked single chain Fv fragments are fused to the amino terminus of filamentous phage minor coat protein pIII. Expression of the fusion protein and incorporation thereof into the mature phage coat results in phage particles that present an antibody on their surface and contain the genetic material encoding the antibody. A phage library comprising such constructs is expressed in bacteria, and the library is screened for nGPCR-x-specific phage-antibodies using labeled or immobilized nGPCR-x as antigen-probe. *Human nGPCR-x-neutralizing antibodies from transgenic mice*

Human nGPCR-x-neutralizing antibodies are generated in transgenic mice essentially as described in Bruggemann *et al.,* Immunol. Today 17(8):391-97 (1996) and Bruggemann *et al.,* Curr. Opin. Biotechnol. 8:455-58 (1997). Transgenic mice carrying human V-gene segments in germline configuration and that express these transgenes in their lymphoid tissue are immunized with a nGPCR-x composition using conventional immunization protocols. Hybridomas are generated using B cells from the immunized mice using conventional protocols and screened to identify hybridomas secreting anti-nGPCR-x human antibodies (*e.g*., as described above).

### Example 12: Assays to Identify Modulators of nGPCR-x Activity

Set forth below are several nonlimiting assays for identifying modulators (agonists and antagonists) of nGPCR-x activity. Among the modulators that can be identified by these assays are natural ligand compounds of the receptor; synthetic analogs and derivatives of natural ligands; antibodies, antibody fragments, and/or antibody-like compounds derived from natural antibodies or from antibody-like combinatorial libraries; and/or synthetic compounds identified by high-throughput screening of libraries; and the like. All modulators that bind nGPCR-x are useful for identifying nGPCR-x in tissue samples (*e.g*., for diagnostic purposes, pathological purposes, and the like). Agonist and antagonist modulators are useful for up-regulating and down-regulating nGPCR-x activity, respectively, to treat disease states characterized by abnormal levels of nGPCR-x activity. The assays may be performed using single putative modulators, and/or may be performed using a known agonist in combination with candidate antagonists (or visa versa).

### A. cAMP Assays

In one type of assay, levels of cyclic adenosine monophosphate (cAMP) are measured in nGPCR-x-transfected cells that have been exposed to candidate modulator compounds. Protocols for cAMP assays have been described in the literature. (See, *e.g.,* Sutherland *et al.,* Circulation 37: 279 (1968); Frandsen *et al.,* Life Sciences 18: 529-541 (1976); Dooley *et al.,* Journal of Pharmacology and Experimental Therapeutics 283 (2): 735-41 (1997); and George *et al.,* Journal of Biomolecular Screening 2 (4): 235-40 (1997)). An exemplary protocol for such an assay, using an Adenylyl Cyclase Activation FlashPlate® Assay from NEN™ Life Science Products, is set forth below.

Briefly, the nGPCR-x coding sequence *(e.g.,* a cDNA or intronless genomic DNA) is subcloned into a commercial expression vector, such as pzeoSV2 (Invitrogen), and transiently transfected into Chinese Hamster Ovary (CHO) cells using known methods, such as the transfection protocol provided by Boehringer-Mannheim when supplying the FuGENE 6 transfection reagent. Transfected CHO cells are seeded into 96-well microplates from the FlashPlate® assay kit, which are coated with solid scintillant to which antisera to cAMP has been bound. For a control, some wells are seeded with wild type (untransfected) CHO cells. Other wells in the plate receive various amounts of a cAMP standard solution for use in creating a standard curve.

One or more test compounds (*i.e*., candidate modulators) are added to the cells in each well, with water and/or compound-free medium/diluent serving as a control or controls. After treatment, cAMP is allowed to accumulate in the cells for exactly 15 minutes at room temperature. The assay is terminated by the addition of lysis buffer containing [¹²⁵I]-labeled cAMP, and the plate is counted using a Packard Topcount™ 96-well microplate scintillation counter. Unlabeled cAMP from the lysed cells (or from standards) and fixed amounts of [¹²⁵I]-cAMP compete for antibody bound to the plate. A standard curve is constructed, and cAMP values for the unknowns are obtained by interpolation. Changes in intracellular cAMP levels of cells in response to exposure to a test compound are indicative of nGPCR-x modulating activity. Modulators that act as agonists of receptors which couple to the Gₛ subtype of G proteins will stimulate production of cAMP, leading to a measurable 3-10 fold increase in cAMP levels. Agonists of receptors which couple to the G_{i/o} subtype of G proteins will inhibit forskolin-stimulated cAMP production, leading to a measurable decrease in cAMP levels of 50-100%. Modulators that act as inverse agonists will reverse these effects at receptors that are either constitutively active or activated by known agonists.

### nGPCR-51

Modulators that act as agonists at receptors which couple to the Gs subtype of G proteins will activate adenyly cyclase leading to a 3-10 fold increase in cyclic adenosine monophosphate (cAMP). Compounds to be tested for the ability to activate nGPCR-51 were assayed for cAMP using an Adenylyl Cyclase Activation FlashPlate® Assay from NEN™ Life Science Products. Briefly, nGPCR-51 cDNA is subcloned into the commercial expression vector pCMVSport (Gibco/Life Technologies) and transiently transfected into CHO or COS 7 cells using the transfection reagent FuGENE 6 (Boehringer-Mannheim) and the transfection protocol provided in the product insert. 24 hours post transfection the cells are harvested by dislodging from the culture flask using Versene (Gibco/BRL). The cells are counted and prepared as a suspension in a buffer included in the assay kit that contains the phophodiesterase inhibitor isobutylmethylxanthine. The assay is conducted in a special 96 well microplate included in the kit which is coated with solid scintillant to which antisera to cAMP has been bound. Dilutions of test compounds to be tested for activation of nGPCR-51 are added to assay wells. Several wells on the plate receive various amounts of cAMP standard solution. After the addition of cells transiently expressing nGPCR-51, cAMP is allowed to accumulate for exactly 15 minutes at room temperature. The assay is terminated by the addition of lysis buffer containing [¹²⁵I] cAMP, and the plate is covered and allowed to incubate at room temperature for 2-24 hours. The plate is then counted using a Packard Topcount™ 96-well microplate scintillation counter. Unlabelled cAMP from cells (or standards) competes with fixed amounts of [¹²⁵I] cAMP for antibody bound to the plate. A standard curve is constructed and cAMP values for the unknowns are obtained by interpolation. Data were analyzed using GraphPad Prism (San Diego, CA). There was no increase in cAMP observed after stimulation with the test compounds at concentrations up to 10 uM.

### B. Aequorin Assays

In another assay, cells (e.g., CHO cells) are transiently co-transfected with both a nGPCR-x expression construct and a construct that encodes the photoprotein apoaquorin. In the presence of the cofactor coelenterazine, apoaquorin will emit a measurable luminescence that is proportional to the amount of intracellular (cytoplasmic) free calcium. (See generally, Cobbold, et al. "Aequorin measurements of cytoplasmic free calcium," In: McCormack J.G. and Cobbold P.H., eds., Cellular Calcium: A Practical Approach. Oxford:IRL Press (1991); Stables et al., Analytical Biochemistry 252: 115-26 (1997); and Haugland, Handbook of Fluorescent Probes and Research Chemicals, Sixth edition. Eugene OR: Molecular Probes (1996).)

In one exemplary assay, nGPCR-x is subcloned into the commercial expression vector pzeoSV2 (Invitrogen) and transiently co-transfected along with a construct that encodes the photoprotein apoaquorin (Molecular Probes, Eugene, OR) into CHO cells using the transfection reagent FuGENE 6 (Boehringer-Mannheim) and the transfection protocol provided in the product insert.

The cells are cultured for 24 hours at 37C in MEM (Gibco/BRL, Gaithersburg, MD) supplemented with 10% fetal bovine serum, 2 mM glutamine, 10 U/ml penicillin and 10 µg/ml streptomycin, at which time the medium is changed to serum-free MEM containing 5 µM coelenterazine (Molecular Probes, Eugene, OR). Culturing is then continued for two additional hours at 37C. Subsequently, cells are detached from the plate using VERSEN (Gibco/BRL), washed, and resuspended at 200,000 cells/ml in serum-free MEM.

Dilutions of candidate nGPCR-x modulator compounds are prepared in serum-free MEM and dispensed into wells of an opaque 96-well assay plate at 50 µl/well. Plates are then loaded onto an MLX microtiter plate luminometer (Dynex Technologies, Inc., Chantilly, VA). The instrument is programmed to dispense 50 µl cell suspensions into each well, one well at a time, and immediately read luminescence for 15 seconds. Dose-response curves for the candidate modulators are constructed using the area under the curve for each light signal peak. Data are analyzed with SlideWrite, using the equation for a one-site ligand, and EC₅₀ values are obtained. Changes in luminescence caused by the compounds are considered indicative of modulatory activity. Modulators that act as agonists at receptors which couple to the Gq subtype of G proteins give an increase in luminescence of up to 100 fold. Modulators that act as inverse agonists will reverse this effect at receptors that are either constitutively active or activated by known agonists.

### nGPCR-51

Agonist activation of receptors that couple to the Gq subtype of G proteins will lead to the release of intracellular calcium. The photoprotein aequorin emits a characteristic luminescence in the presence of calcium and may be expressed in cells along with the receptor of interest in order to report agonist signalling. Peptide A was tested for the ability to activate nGPCR-51 using an assay for aequorin. Peptide A, a cyclic peptide via a disulfide bond between the Cys residues, has the following amio acid sequence: Asp Phe Asp Met Leu Arg Cys Met Leu Gly Arg Val Tyr Arg Pro Cys Trp Gln Val (SEQ ID NO: 183). Preferably, Peptide A is used in a solution containing 0.17M acetic acid, or the like, and a stabilizer such as a 0.2% BSA:acetonitrile 90:10. Briefly, nGPCR-51 cDNA is subcloned into the commercial expression vector pCMVSport (Gibco/Life Technologies) and transiently transfected along with the expression construct mtAequorin (Molecular Probes, Eugene, OR) into COS 7 cells using the transfection reagent FuGENE 6 (Boehringer-Mannheim) and the transfection protocol provided in the product insert. 24 hours post transfection the cells are harvested by dislodging from the culture flask using Versene (Gibco/BRL) and prepared as a suspension in assay buffer (Dulbecco's Modified Eagle's Medium with high glucose, pyridoxine HCl, L-glutamine, sodium pyruvate, and 0.1% fetal bovine serum (Gibco/BRL)) and containing the cofactor coelenterazine (Molecular Probes). The cell suspension is incubated for 4 hours at room temperature with gentle stirring. After the coelenterazine loading incubation, the cells are counted and diluted to 1 x 10⁶ cells/ml in assay buffer. Dilutions of test compound are prepared in assay buffer and pipetted into wells of an opaque 96-well assay plate, 50 µl/well. Plates are loaded onto an MLX microtiter plate luminometer (Dynex Technologies, Inc., Chantilly, VA). The instrument is programmed to dispense 50 µl cell suspension into each well, one well at a time, and immediately read luminescence for 20 seconds. Dose response curves are constructed using the area under the curve for each light signal peak. Data are analyzed with SlideWrite, using the equation y = a⁰ * a¹/(x + a¹) + a² where a⁰ is the maximum response minus the baseline; a¹ is the EC₅₀; and a² is the maximum response.

Peptide A stimulation of nGPCR-51 expressing COS 7 cells resulted in a dose dependent increase in aequorin luminescence with an EC₅₀ of approximately 40 nM and a maximum response of 52-fold over baseline.

### C. Luciferase Reporter Gene Assay

The photoprotein luciferase provides another useful tool for assaying for modulators of nGPCR-x activity. Cells (e.g., CHO cells or COS 7 cells) are transiently co-transfected with both a nGPCR-x expression construct (e.g., nGPCR-x in pzeoSV2) and a reporter construct which includes a gene for the luciferase protein downstream from a transcription factor binding site, such as the cAMP-response element (CRE), AP-1, or NF-kappa B. Agonist binding to receptors coupled to the Gₛ subtype of G proteins leads to increases in cAMP, thereby activating the CRE transcription factor and resulting in expression of the luciferase gene. Agonist binding to receptors coupled to the Gq subtype of G protein leads to production of diacylglycerol that activates protein kinase C, which activates the AP-1 or NF-kappa B transcription factors, in turn resulting in expression of the luciferase gene. Expression levels of luciferase reflect the activation status of the signaling events. (See generally, George *et al., Journal of Biomolecular Screening,* 2(4): 235-240 (1997); and Stratowa *et al.,* Current Opinion in Biotechnology 6: 574-581 (1995)). Luciferase activity may be quantitatively measured using, e.g., luciferase assay reagents that are commercially available from Promega (Madison, WI).

In one exemplary assay, CHO cells are plated in 24-well culture dishes at a density of 100,000 cells/well one day prior to transfection and cultured at 37C in MEM (Gibco/BRL) supplemented with 10% fetal bovine serum, 2 mM glutamine, 10 U/ml penicillin and 10 µg/ml streptomycin. Cells are transiently co-transfected with both a nGPCR-x expression construct and a reporter construct containing the luciferase gene. The reporter plasmids CRE-luciferase, AP-1-luciferase and NF-kappaB-luciferase may be purchased from Stratagene (LaJolla, CA). Transfections are performed using the FuGENE 6 transfection reagent (Boehringer-Mannheim) according to the supplier's instructions. Cells transfected with the reporter construct alone are used as a control. Twenty-four hours after transfection, cells are washed once with PBS pre-warmed to 37C. Serum-free MEM is then added to the cells either alone (control) or with one or more candidate modulators and the cells are incubated at 37C for five hours. Thereafter, cells are washed once with ice-cold PBS and lysed by the addition of 100 µl of lysis buffer per well from the luciferase assay kit supplied by Promega. After incubation for 15 minutes at room temperature, 15 µl of the lysate is mixed with 50 µl of substrate solution (Promega) in an opaque-white, 96-well plate, and the luminescence is read immediately on a Wallace model 1450 MicroBeta scintillation and luminescence counter (Wallace Instruments, Gaithersburg, MD).

Differences in luminescence in the presence versus the absence of a candidate modulator compound are indicative of modulatory activity. Receptors that are either constitutively active or activated by agonists typically give a 3 to 20-fold stimulation of luminescence compared to cells transfected with the reporter gene alone. Modulators that act as inverse agonists will reverse this effect.

### nGPCR-51

nGPCR-51 was transfected in N7 cells, stable HOS (human osteosarcoma) cells expressing NFkB-Luciferase reporter gene Briefly, LipofectAMINE, PLUS reagent, and 4 µg DNA were added to cells in Opti-MEM. After 4 hours, serum-containing medium was added to each plate. Twenty-four hour after transfection the cells were trypsinized and seeded in COSTAR white 96-well plate at a cell density of about 20,000 cells per well. Another 24 hours later the culture medium was replaced with fresh growth medium and Peptide A was added. After 5-hr incubation with Peptide A, the medium was aspirated and 100 µL diluted Steady Glo was added. The plates were gently shake at room temperature for 30 minutes and luminescence determined by TopCount at the SPC mode.

The dose-response effect of Peptide A on luciferase expression in Seq51-transfected N7 cells was tested in 2 separate experiments. The EC₅₀ value of Peptide A for the 2 experiments was 1.6 nM and 1.7 nM, respectively. Salmon Peptide B (Asp Thr Met Arg Cys Met Val Gly Arg Val Tyr Arg Pro Cys Trp Glu Val; SEQ ID NO:184), a variant of Peptide A, and another variant of Peptide A were also tested in the second experiment. The EC₅₀ value for salmon Peptide B was approximately 25 nM while variant of Peptide A had no effect on these Seq51-transfected cells at concentration up to 1 µM.

Peptide A, a cyclic heptadecapeptide, is a prominent hypothalamic neuropeptide expressed in cells which are part of an extensive network of neurons projecting to multiple parts of the brain. Despite the large numbers of hypothalamic Peptide A-expressing cells, the Peptide A cell groups are not part of the major hypothalamic nuclei (paraventricular, supraoptic, dorsomedial, ventromedial, arcuate, lateral tuberal or mammillary) (Knigge *et al., J. Peptides,* **1996,** *17*, 1063-1073), but they have terminal projections to most of these hypothalamic nulei. Additionally, Peptide A projections from the hypothalamus to the rest of the brain are extensive, including to the cerebellar cortex, subcortical nuclei, limbic areas, thalamus, brain stem and spinal cord.

Peptide A projections are included in the circuitry involved in processing visual and auditory information, suggesting a general role in arousal and sensorimotor integration (Herview *et al., Eur. J. Neurosci.,* **2000**, *12*, 1194-1216). Intracerebroventricular injection of Peptide A causes a decrease in the conditioning amplitude of the second of a pair of auditory evoked potentials recorded from the hippocampus (hippocampal auditory gating paradigm) and reverses the increases in the conditioning amplitude induced by alpha MSH (Miller *et al., Peptides,* **1993**, *14,* 431-440). These data would suggest that Peptide A inhibits the normal auditory gating process. Schizophrenics also present with a loss of auditory gating which may contribute to an inability to filter inappropriate information. Dysregulation of Peptide A may contribute to this phenotype in humans and antagonists active at the Peptide A receptors may effectively treat these deficits in schizophrenia.

Major portions of the Peptide A cell group are in areas considered to be part of the extrapyramidal motor circuits. It has been suggested that this location would afford the Peptide A neuronal system the capability of coordinating hypothalamic visceral activity with appropriate motor activity (Knigge *et al.,* **1996**).

Peptide A localization in the hippocampal formation, the amygdaloid regions, the bed nucleus of the stria terminalis all suggest a role in cognition and learning and in support of this Peptide A has been shown to alter passive avoidance performance (McBride *et al., Peptides,* **1994**, *15*, 757-759).

Central injections of Peptide A in rats leads to a reduction in peak circadian ACTH levels and inhibit stress-induced ACTH secretion (Bluet Pajot *et al., J. Neuroendocrinol.,* **1995**, 7, 297-303; and Ludwig *et al., Am. J. Physiology,* **1998,** *274,* E627-E633). However, injections of Peptide A into the medial preoptic area of the hypothalamus of rats result in decreases in time spent in open arms in the elevated plus maze assay indicative of increased anxiety. Injections of the peptide also stimulated female sexual receptivity (Gonzalez *et al., Peptides,* **1996***, 17*, 171 - 177).

The lateral hypothalamus has a prominent role in feeding-related behaviors and in fact Peptide A has been shown to be involved in energy balance and food intake. Ob/ob mice express more Peptide A mRNA compared with lean littermates (Qu *et al., Nature,* **1996***, 380,* 243-247). Additionally, fasting increases hypothalamic Peptide A mRNA levels (Qu *et al.,* **1996**). Intracerebroventricular injection of Peptide A leads to an increase in food intake in rats (Qu *et al., Endocrinology,* **1997**,*138*, 351-355).

Targeted deletion of the gene which encodes the preprohormone for Peptide A resulted in mice which have reduced body weight and are lean due to reduced feeding. Additionally these mice have an increased metabolic rate in the presence of decreased leptin levels and POMC levels (Shimada *et al., Nature,* **1998***, 396,* 670-673). These mice also have a loss of expression of the NEI peptide as well as Peptide A. Nevertheless, the prominent phenotypic feature of these mice is their reduced size. Antagonists of Peptide A receptors may be effective in the treatment of obesity.

The SLC-1 gene product was recently identified as a G-protein coupled receptor which is potently activated by Peptide A *(*Chambers *et al., Nature*,**1999**, *400,* 261-265; and Saito *et al., Nature,* **1999**, *400,* 265-269). nGPCR-51 has been identified as a gene encoding a second Peptide A receptor which is also potently activated by Peptide A. Like that of the SLC-1 gene, expression of nGPCR-51 is extensive throughout the brain. The multiple actions of Peptide A could be differentially mediated by each of the two Peptide A receptors. Selective agonism or antagonism at nGPCR-51 could result in a therapeutic effective in treating schizophrenia, attention disorders, anxiety, depression, obesity, and the like.

### D. Intracellular calcium measurement using FLIPR

Changes in intracellular calcium levels are another recognized indicator of G protein-coupled receptor activity, and such assays can be employed to screen for modulators of nGPCR-x activity. For example, CHO cells stably transfected with a nGPCR-x expression vector are plated at a density of 4 x 10⁴ cells/well in Packard black-walled, 96-well plates specially designed to discriminate fluorescence signals emanating from the various wells on the plate. The cells are incubated for 60 minutes at 37C in modified Dulbecco's PBS (D-PBS) containing 36 mg/L pyruvate and 1 g/L glucose with the addition of 1% fetal bovine serum and one of four calcium indicator dyes (Fluo-3™ AM, Fluo-4™ AM, Calcium Green™-1 AM, or Oregon Green™ 488 BAPTA-1 AM), each at a concentration of 4 µM. Plates are washed once with modified D-PBS without 1% fetal bovine serum and incubated for 10 minutes at 37C to remove residual dye from the cellular membrane. In addition, a series of washes with modified D-PBS without 1% fetal bovine serum is performed immediately prior to activation of the calcium response.

A calcium response is initiated by the addition of one or more candidate receptor agonist compounds, calcium ionophore A23187 (10 µM; positive control), or ATP (4 µM; positive control). Fluorescence is measured by Molecular Device's FLIPR with an argon laser (excitation at 488 nm). (See, e.g., Kuntzweiler et al., Drug Development Research, 44(1):14-20 (1998)). The F-stop for the detector camera was set at 2.5 and the length of exposure was 0.4 milliseconds. Basal fluorescence of cells was measured for 20 seconds prior to addition of candidate agonist, ATP, or A23187, and the basal fluorescence level was subtracted from the response signal. The calcium signal is measured for approximately 200 seconds, taking readings every two seconds. Calcium ionophore A23187 and ATP increase the calcium signal 200% above baseline levels. In general, activated GPCRs increase the calcium signal approximately 10-15% above baseline signal.

### nGPCR-51

HEK293 cells were transiently transfected with an expression vector for nGPCR-51 and empty vector using Lipofectamine plus (Gibco) according to the manufacturer's instructions. The next day, the cells were seeded into 96-well plates at 25,000 cells per well. The following day, cells were loaded with 1 uM Fluo-4-acetoxymethyl fluorescent indicator dye (Molecular Probes) in MEM (minimal essential media) containing 0.1% bovine serum albumin, 0.04% pluronic acid and 2.5 mM probenecid for 30 minutes at 37C. The cells were washed with pre-warmed (37C) assay buffer (Hanks buffer containing 15 mM HEPES, 2.5 mM probenecid and 0.1% bovine serum albumin). Assay buffer (100 ul) was added to each well and plates were incubated at 37C for 15 minutes. Various concentrations (0.03 pM-10 nM) of human Peptide A or salmon Peptide B were added and fluorescence produced by fluo-4 (a calcium sensitive dye) was measured every second for 150 seconds on a fluorometric imaging plate reader (FLIPR1; Molecular Devices). Human Peptide A and salmon Peptide B-induced calcium mobilization in nGPCR-51-transfected cells with EC₅₀ values of 3 nM and 6 nM, respectively. Human Peptide A and salmon Peptide B had no effect on vector-transfected cells.

### E. Mitogenesis Assay

In a mitogenesis assay, the ability of candidate modulators to induce or inhibit nGPCR-x-mediated cell division is determined. (See, e.g., Lajiness et al., Journal of Pharmacology and Experimental Therapeutics 267(3): 1573-1581 (1993)). For example, CHO cells stably expressing nGPCR-x are seeded into 96-well plates at a density of 5000 cells/well and grown at 37□C in MEM with 10% fetal calf serum for 48 hours, at which time the cells are rinsed twice with serum-free MEM. After rinsing, 80 µl of fresh MEM, or MEM containing a known mitogen, is added along with 20 µl MEM containing varying concentrations of one or more candidate modulators or test compounds diluted in serum-free medium. As controls, some wells on each plate receive serum-free medium alone, and some receive medium containing 10% fetal bovine serum. Untransfected cells or cells transfected with vector alone also may serve as controls.

After culture for 16-18 hours, 1 µCi of [³H]-thymidine (2 Ci/mmol) is added to the wells and cells are incubated for an additional 2 hours at 37C. The cells are trypsinized and collected on filter mats with a cell harvester (Tomtec); the filters are then counted in a Betaplate counter. The incorporation of [³H]-thymidine in serum-free test wells is compared to the results achieved in cells stimulated with serum (positive control). Use of multiple concentrations of test compounds permits creation and analysis of dose-response curves using the non-linear, least squares fit equation: A = B x [C/ (D + C)] + G where A is the percent of serum stimulation; B is the maximal effect minus baseline; C is the EC₅₀; D is the concentration of the compound; and G is the maximal effect. Parameters B, C and G are determined by Simplex optimization.

Agonists that bind to the receptor are expected to increase [³H]-thymidine incorporation into cells, showing up to 80% of the response to serum. Antagonists that bind to the receptor will inhibit the stimulation seen with a known agonist by up to 100%.

### F. [³⁵S]GTPγS Binding Assay

Because G protein-coupled receptors signal through intracellular G proteins whose activity involves GTP binding and hydrolysis to yield bound GDP, measurement of binding of the non-hydrolyzable GTP analog [³⁵S]GTPγS in the presence and absence of candidate modulators provides another assay for modulator activity. (See, e.g., Kowal et al., Neuropharmacology 37:179-187 (1998).)

In one exemplary assay, cells stably transfected with a nGPCR-x expression vector are grown in 10 cm tissue culture dishes to subconfluence, rinsed once with 5 ml of ice-cold Ca²⁺/Mg²⁺-free phosphate-buffered saline, and scraped into 5 ml of the same buffer. Cells are pelleted by centrifugation (500 x g, 5 minutes), resuspended in TEE buffer (25 mM Tris, pH 7.5, 5 mM EDTA, 5 mM EGTA), and frozen in liquid nitrogen. After thawing, the cells are homogenized using a Dounce homogenizer (one ml TEE per plate of cells), and centrifuged at 1,000 x g for 5 minutes to remove nuclei and unbroken cells.

The homogenate supernatant is centrifuged at 20,000 x g for 20 minutes to isolate the membrane fraction, and the membrane pellet is washed once with TEE and resuspended in binding buffer (20 mM HEPES, pH 7.5, 150 mM NaCl, 10mM MgCl₂, 1 mM EDTA). The resuspended membranes can be frozen in liquid nitrogen and stored at -70C until use.

Aliquots of cell membranes prepared as described above and stored at -70C are thawed, homogenized, and diluted into buffer containing 20 mM HEPES, 10 mM MgCl₂, 1 mM EDTA, 120 mM NaCl, 10 µM GDP, and 0.2 mM ascorbate, at a concentration of 10-50 µg/ml. In a final volume of 90 µl, homogenates are incubated with varying concentrations of candidate modulator compounds or 100 µM GTP for 30 minutes at 30C and then placed on ice. To each sample, 10 µl guanosine 5'-O-(3[³⁵S]thio) triphosphate (NEN, 1200 Ci/mmol; [³⁵S]-GTPγS), was added to a final concentration of 100-200 pM. Samples are incubated at 30C for an additional 30 minutes, 1 ml of 10mM HEPES, pH 7.4, 10 mM MgCl₂, at 4C is added and the reaction is stopped by filtration.

Samples are filtered over Whatman GF/B filters and the filters are washed with 20 ml ice-cold 10 mM HEPES, pH 7.4,10 mM MgCl₂. Filters are counted by liquid scintillation spectroscopy. Nonspecific binding of [³⁵S]-GTPγS is measured in the presence of 100 µM GTP and subtracted from the total. Compounds are selected that modulate the amount of [³⁵S]-GTPγS binding in the cells, compared to untransfected control cells. Activation of receptors by agonists gives up to a five-fold increase in [³⁵S]GTPγS binding. This response is blocked by antagonists.

### G. MAP Kinase Activity Assay

Evaluation of MAP kinase activity in cells expressing a GPCR provides another assay to identify modulators of GPCR activity. (See, e.g., Lajiness et al., Journal of Pharmacology and Experimental Therapeutics 267(3):1573-1581 (1993) and Boulton et al., Cell 65:663-675 (1991).)

In one embodiment, CHO cells stably transfected with nGPCR-x are seeded into 6-well plates at a density of 70,000 cells/well 48 hours prior to the assay. During this 48-hour period, the cells are cultured at 37C in MEM medium supplemented with 10% fetal bovine serum, 2mM glutamine, 10 U/ml penicillin and 10µg/ml streptomycin. The cells are serum-starved for 1-2 hours prior to the addition of stimulants.

For the assay, the cells are treated with medium alone or medium containing either a candidate agonist or 200 nM Phorbol ester- myristoyl acetate (i.e., PMA, a positive control), and the cells are incubated at 37C for varying times. To stop the reaction, the plates are placed on ice, the medium is aspirated, and the cells are rinsed with 1 ml of ice-cold PBS containing 1mM EDTA. Thereafter, 200µl of cell lysis buffer (12.5 mM MOPS, pH 7.3, 12.5 mM glycerophosphate, 7.5mM MgCl₂, 0.5mM EGTA, 0.5 mM sodium vanadate, 1mM benzamidine, 1mM dithiothreitol, 10 µg/ml leupeptin, 10 µg/ml aprotinin, 2µg/ml pepstatin A, and 1µM okadaic acid) is added to the cells. The cells are scraped from the plates and homogenized by 10 passages through a 23 3/4 G needle, and the cytosol fraction is prepared by centrifugation at 20,000 x g for 15 minutes.

Aliquots (5-10 µl containing 1-5 µg protein) of cytosol are mixed with 1 mM MAPK Substrate Peptide (APRTPGGRR; SEQ ID NO:182), Upstate Biotechnology, Inc., N.Y.) and 50µM [γ-³²P]ATP (NEN, 3000 Ci/mmol), diluted to a final specific activity of ~2000 cpm/pmol, in a total volume of 25 µl. The samples are incubated for 5 minutes at 30C, and reactions are stopped by spotting 20 µl on 2 cm² squares of Whatman P81 phosphocellulose paper. The filter squares are washed in 4 changes of 1% H₃PO₄, and the squares are subj ected to liquid scintillation spectroscopy to quantitate bound label. Equivalent cytosolic extracts are incubated without MAPK substrate peptide, and the bound label from these samples are subtracted from the matched samples with the substrate peptide. The cytosolic extract from each well is used as a separate point. Protein concentrations are determined by a dye binding protein assay (Bio-Rad Laboratories). Agonist activation of the receptor is expected to result in up to a five-fold increase in MAPK enzyme activity. This increase is blocked by antagonists.

### H. [³H]Arachidonic Acid Release

The activation of GPCRs also has been observed to potentiate arachidonic acid release in cells, providing yet another useful assay for modulators of GPCR activity. (See, e.g., Kanterman et al., Molecular Pharmacology 39:364-369 (1991).).For example, CHO cells that are stably transfected with a nGPCR-x expression vector are plated in 24-well plates at a density of 15,000 cells/well and grown in MEM medium supplemented with 10% fetal bovine serum, 2 mM glutamine, 10 U/ml penicillin and 10 µg/ml streptomycin for 48 hours at 37C before use. Cells of each well are labeled by incubation with [³H]-arachidonic acid (Amersham Corp., 210 Ci/mmol) at 0.5 µCi/ml in 1 ml MEM supplemented with 10mM HEPES, pH 7.5, and 0.5% fatty-acid-free bovine serum albumin for 2 hours at 37C. The cells are then washed twice with 1 ml of the same buffer.

Candidate modulator compounds are added in 1 ml of the same buffer, either alone or with 10µM ATP and the cells are incubated at 37C for 30 minutes. Buffer alone and mock-transfected cells are used as controls. Samples (0.5 ml) from each well are counted by liquid scintillation spectroscopy. Agonists which activate the receptor will lead to potentiation of the ATP-stimulated release of [³H]-arachidonic acid. This potentiation is blocked by antagonists.

### I. Extracellular Acidification Rate

In yet another assay, the effects of candidate modulators of nGPCR-x activity are assayed by monitoring extracellular changes in pH induced by the test compounds. (See, e.g., Dunlop et al., Journal of Pharmacological and Toxicological Methods 40(1):47-55 (1995).) In one embodiment, CHO cells transfected with a nGPCR-x expression vector are seeded into 12 mm capsule cups (Molecular Devices Corp.) at 4 x10⁵ cells/cup in MEM supplemented with 10% fetal bovine serum, 2 mM L-glutamine, 10 U/ml penicillin, and 10 µg/ml streptomycin. The cells are incubated in this medium at 37C in 5% CO₂ for 24 hours.

Extracellular acidification rates are measured using a Cytosensor microphysiometer (Molecular Devices Corp.). The capsule cups are loaded into the sensor chambers of the microphysiometer and the chambers are perfused with running buffer (bicarbonate-free MEM supplemented with 4 mM L-glutamine, 10 units/ml penicillin, 10 µg/ml streptomycin, 26 mM NaCl) at a flow rate of 100 µl/minute. Candidate agonists or other agents are diluted into the running buffer and perfused through a second fluid path. During each 60-second pump cycle, the pump is run for 38 seconds and is off for the remaining 22 seconds. The pH of the running buffer in the sensor chamber is recorded during the cycle from 43-58 seconds, and the pump is re-started at 60 seconds to start the next cycle. The rate of acidification of the running buffer during the recording time is calculated by the Cytosoft program. Changes in the rate of acidification are calculated by subtracting the baseline value (the average of 4 rate measurements immediately before addition of a modulator candidate) from the highest rate measurement obtained after addition of a modulator candidate. The selected instrument detects 61 mV/pH unit. Modulators that act as agonists of the receptor result in an increase in the rate of extracellular acidification compared to the rate in the absence of agonist. This response is blocked by modulators which act as antagonists of the receptor.

### J. Radioligand Binding Assay

HEK 293 or COS7 cells transiently expressing nGPCR-51, or CHO K-1 cells stable expressing nGPCR-51, were grown to sub-confluence (2 days post-transfection for transients), harvested from flasks in Dulbecco's PBS and pelleted by low speed centrifugation (2500 rpm) for 10 minutes. Cell pellets were homogenized in 10 ml tissue buffer (50 mM Hepes, 10 mM MgCl₂, 2 mM EGTA, 1 µg/ml aprotinin, 1 µg/ml leupeptin hemisulfate, 1 µg/ml pepstatin A, pH 7.0) using a dounce, 10 strokes. Homogenate was centrifuged at 47,000 x g for 15 minutes. Membrane pellet was resuspended in 1 ml tissue buffer using the dounce, 10 strokes. An aliquot of the membrane preparation was used to determine protein concentration. For measurement of saturation binding, 10 µg of cell membranes were incubated with various concentrations of [¹²⁵I] Peptide A (iodinated by routine procedures via the Tyr residue) in 300 µl binding assay buffer (tissue buffer plus 0.15 mM Bacitracin and 0.1 % ovalbumin) for 90 minutes at room temperature in 96-well plates. Non-specific binding was defined by the inclusion of 1 µM Peptide A. After the binding incubation, plates were harvested onto GF/C filters presoaked in 0.3% non-fat dry milk. Filters were dried, and 30 µl scintillant was added to each well. Filter plates were then counted in a Packard Topcount™ 96-well microplate scintillation counter. Data were analyzed using GraphPad Prism (San Diego, CA) and Kd and Bmax values were calculated.

In a saturation binding experiment using COS 7 cells transiently expressing nGPCR-51 the Kd for [¹²⁵I] Peptide A was determined to be 0.254 nM, with a Bmax of 75 fmol/mg. HEK 293 cells transiently expressing nGPCR-51 were shown to have an estimated Bmax of 108 fmol/mg. Untransfected cells have no specific radioligand binding for [¹²⁵I] Peptide A.

### Example 13: Using nGPCR-x Proteins to Isolate Neurotransmitters

The isolated nGPCR-x proteins, particularly nGPCR-42, 46, 48, 49, 51, 52, 61, 63, or 70 (SEQ ID Numbers 61, 62, 68, 91, 94, 96, 97, 99, 100, and 111-120) can be used to isolate novel or known neurotransmitters (Saito et al., Nature, 400: 265-269, 1999). The cDNAs that encode the isolated nGPCR-x can be cloned into mammalian expression vectors and used to stably or transiently transfect mammalian cells including CHO, Cos or HEK293 cells. Receptor expression can be determined by Northern blot analysis of transfected cells and identification of an appropriately sized mRNA band (predicted size from the cDNA). Brain regions shown by mRNA analysis to express each of the nGPCR-x proteins could be processed for peptide extraction using any of several protocols ((Reinsheidk R.K. et al., Science 270: 243-247, 1996; Sakurai, T., et al., Cell 92; 573-585, 1998; Hinuma, S., et al., Nature 393: 272-276, 1998). Chromotographic fractions of brain extracts could be tested for ability to activate nGPCR-x proteins by measuring second messenger production such as changes in cAMP production in the presence or absence of forskolin, changes in inositol 3-phosphate levels, changes in intracellular calcium levels or by indirect measures of receptor activation including receptor stimulated mitogenesis, receptor mediated changes in extracellular acidification or receptor mediated changes in reporter gene activation in response to cAMP or calcium (these methods should all be referenced in other sections of the patent). Receptor activation could also be monitored by co-transfecting cells with a chimeric GI_{q/i3} to force receptor coupling to a calcium stimulating pathway (Conklin et al., Nature 363; 274-276, 1993). Neurotransmitter mediated activation of receptors could also be monitored by measuring changes in [³⁵ S]-GTPKS binding in membrane fractions prepared from transfected mammalian cells. This assay could also be performed using baculoviruses containing nGPCR-x proteins infected into SF9 insect cells.

The neurotransmitter which activates nGPCR-x proteins can be purified to homogeneity through successive rounds of purification using nGPCR-x proteins activation as a measurement of neurotransmitter activity. The composition of the neurotransmitter can be determined by mass spectrometry and Edman degradation if peptidergic. Neurotransmitters isolated in this manner will be bioactive materials which will alter neurotransmission in the central nervous system and will produce behavioral and biochemical changes.

### Example 14: Using nGPCR-x Proteins to Isolate and Purify G Proteins

cDNAs encoding nGPCR-x proteins are epitope-tagged at the amino terminuus end of the cDNA with the cleavable influenza-hemagglutinin signal sequence followed by the FLAG epitope (IBI, New Haven, CT). Additionally, these sequences are tagged at the carboxyl terminus with DNA encoding six histidine residues. (Amino and Carboxyl Terminal Modifications to Facilitate the Production and Purification of a G Protein-Coupled Receptor, B.K. Kobilka , Analytical Biochemistry, Vol. 231, No. 1, Oct 1995, pp. 269-271). The resulting sequences are cloned into a baculovirus expression vector such as pVL1392 (Invitrogen). The baculovirus expression vectors are used to infect SF-9 insect cells as described (Guan *et al.,* (1992) J. Biol. Chem. 267, 21995-21998). Infected SF-9 cells could be grown in 1000-ml cultures in SF900 II medium (Life Technologies, Inc.) containing 5% fetal calf serum (Gemini, Calabasas, CA) and 0.1 mg/ml gentamicin (Life Technologies, Inc.) for 48 hours at which time the cells could be harvested. Cell membrane preparations could be separated from soluble proteins following cell lysis. nGPCR-x protein purification is carried out as described for purification of the ϑ2 receptor (Kobilka, Anal. Biochem., 231 (1): 269-271, 1995) including solubilization of the membranes in 0.8-1.0 % n-dodecyl-D-maltoside (DM) (CalBiochem, La Jolla, CA) in buffer containing protease inhibitors followed by Ni-column chromatography using chelating Sepharose™ (Pharmacia, Uppsala, Sweden). The eluate from the Ni-column is further purified on an M1 anti-FLAG antibody column (IBI). Receptor containing fractions are monitored by using receptor specific antibodies following western blot analysis or by SDS-PAGE analysis to look for an appropriate sized protein band (appropriate size would be the predicted molecular weight of the protein). This method of purifying G protein is particularly useful to isolate G proteins that bind to the nGPCR-x proteins in the absence of an activating ligand.

Some of the preferred embodiments of the invention described above are outlined below and include, but are not limited to, the following embodiments. As those skilled in the art will appreciate, numerous changes and modifications may be made to the preferred embodiments of the invention without departing from the spirit of the invention. It is intended that all such variations fall within the scope of the invention. The entire disclosure of each publication cited herein is incorporated herein by reference in its entirety.

### Example 15: Clone Deposit Information

In accordance with the Budapest Treaty, clones of the present invention have been deposited at the Agricultural Research Culture Collection (NRRL) International Depository Authority, 1815 N. University Street, Peoria, Illinois 61604, U.S.A. Accession numbers and deposit dates are provided below in Table 6.

**Table 6: Deposit Information**

| Clone | SEQ ID NO: | NRRL Accession Number | Budapest Treaty Deposit Date |
|---|---|---|---|
| nGPCR-42 | 31,54 | B-30246 | 2000 January 18 |
| nGPCR-48 | 36,56 | B30263 | 2000 February 22 |
| nGPCR-46 | 34, 55 | B-30269 | 2000 March 10 |
| nGPCR-52 | 40,58 | B-30270 | 2000 March 10 |
| nGPCR-70 | 8, 52 | B-30300 | 2000 June 2 |
| nGPCR-61 | 1,60 | B-30301 | 2000 June 2 |
| nGPCR-49 | 37,59 | B-30313 | 2000 July 6 |
| nGPCR-51 | 39,57 | B-30314 | 2000 July 6 |
| nGPCR-63 | 3,51,53 | B-30315 | 2000 July 6 |

## Claims

1. An isolated polypeptide selected from:
a) a fragment of a polypeptide comprising the amino acid sequence of SEQ ID NO:117 wherein the fragment comprises at least 16 consecutive amino acids;
b) a naturally occurring allelic variant of a polypeptide comprising the amino acid sequence of SEQ ID NO:117, wherein the polypeptide is encoded by a nucleic acid molecule which hybridizes to a nucleic acid molecule comprising SEQ ID NO:57 under stringent conditions;
c) a polypeptide which is encoded by a nucleic acid molecule comprising a nucleotide sequence which is at least 95% homologous to a nucleic acid comprising the nucleotide sequence of SEQ ID NO:57; and
d) a polypeptide comprising an amino acid sequence which is at least 95% homologous to the amino acid sequence of SEQ ID NO:117.

2. The isolated polypeptide of claim 1, wherein the polypeptide encodes a peptide A receptor.

3. The isolated polypeptide of claim 1, comprising SEQ ID NO: 117.

4. An isolated antibody which specifically binds to an epitope on a polypeptide of claim 1.

5. The antibody of claim 4, which is a monoclonal antibody.

6. An isolated nucleic acid molecule selected from:
a) a nucleic acid molecule comprising a nucleotide sequence which is at least 95% homologous to a nucleotide sequence of SEQ ID NO:57, or a complement thereof;
b) a nucleic acid molecule which encodes a polypeptide comprising an amino acid sequence at least about 95% homologous to the amino acid sequence of SEQ ID NO:117;
c) a nucleic acid molecule which encodes a fragment of a polypeptide comprising the amino acid sequence of SEQ ID NO:117, said polypeptide fragment comprising at least 16 contiguous amino acids; and
d) a nucleic acid molecule which encodes a naturally occurring allelic variant of a polypeptide comprising the amino acid sequence of SEQ ID NO:117 wherein the nucleic acid molecule hybridizes to a nucleic acid molecule comprising SEQ ID NO:57 under stringent conditions.

7. The isolated nucleic acid molecule of claim 6, wherein said nucleotide sequence encodes a peptide A receptor.

8. The isolated nucleic acid molecules of claim 6, comprising SEQ ID NO:57.

9. An expression vector comprising the nucleic acid molecule of claim 6.

10. A host cell transformed with the expression vector of claim 9.
